# EUROPEAN PATENT APPLICATION

(11) **EP 1 316 909 A1**
(43) Date of publication of application: **04.06.2003**
(21) Application number: 01128633.3
(22) Date of filing: 30.11.2001
(51) Int. Cl.: G06F 19/00, G01N 33/68, C12Q 1/26

(54) **In silico screening method for inhibitors of glutamyl-tRNA reductase**

(71) Applicant: Gesellschaft für Biotechnologische Forschung mbH (GBF), D-38124 Braunschweig (DE)
(72) Inventor: Heinz, Dirk, Dr., 38124 Braunschweig (DE); Schubert, Wolf-Dieter, Dr., 38124 Braunschweig (DE); Jahn, Dieter, Prof. Dr., c/o TU Braunschweig, 38106 Braunschweig (DE); Moser, Jürgen, Dr., c/o TU Braunschweig, 38106 Braunschweig (DE)
(74) Representative: Boeters, Hans Dietrich, Dr.

(57) **Abstract**

The invention relates to methods for the development of new antibiotics and herbicides and herbicide resistent plants and the improvement of vitamin B12 production based on the three-dimensional crystal structure of glutamyl-tRNA reductase.

## Description

The invention relates to methods for the development of new antibiotics and herbicides and herbicide resistent plants and the improvement of vitamin B12 production based on the three-dimensional crystal structure of glutamyl-tRNA reductase.

### Field of the Invention

The invention allows rational structure-based design of novel herbicides and antibiotics as well as protein engineering approaches for the development of herbicide resistent plants and the improvement of vitamin B₁₂ production based on the newly determined crystal structure of *Methanopyrus kandleri* glutamyl-tRNA reductase.

A list of the cited authors with full bibliographic data can be found at the end of this specification.

### Background of the Invention

The common precursor of all tetrapyrroles, including hemes, chlorophylls and vitamin B₁₂ is 5-aminolevulinic acid (Jahn *et al.*, 1992). Nature employs two distinct routes for 5-aminolevulinic acid formation. Humans, animals and fungi are using the one-step condensation of succinyl-CoA and glycine catalysed by aminolevulinic acid synthase (Kikuchi *et al.*, 1958; Ferreira & Gong, 1995). In plants, archaea and the majority of bacteria 5-aminolevulinic acid is synthesized via a two-step reaction from glutamyl-tRNA (Jahn *et al.*, 1992). The initial enzyme of the pathway, glutamyl-tRNA reductase (GluTR) performs the NADPH-dependent reduction of tRNA-bound glutamate to glutamate-1-semialdehyde. The formed aldehyde gets subsequently transaminated by glutamate-1-semialdehyde-2,1-aminomutase (GSAM) to form 5-aminolevulinic acid. A cysteine residue of glutamyl-tRNA reductase performs a nucleophilic attack of the α-carbon of tRNA^{Glu}-bound glutamate with the formation of an enzyme localized thioester-intermediate and the release of tRNA^{Glu}. Subsequent hydride transfer from enzyme-bound NADPH leads to glutamate-1-semialdehyde formation (Moser *et al*., 1999). GluTRs are subject to heme inhibition (Vothknecht *et al.*, 1998) and regulated proteolysis (Wang *et al*., 1999). Various structurally not related inhibitors of the enzyme have been described (Loida et al., 1999, Moser et al., 1999).

As GluTRs are enzyme unique to plants, archaea and most bacteria, the present high resolution crystal structure of GluTR will open up new avenues to discovering and developing reagents by structure-based rational drug design. These new reagents could be designed to either interfere with the active site pocket of the enzyme or the large interface between the enzyme and the substrate.

An object of the invention is to provide a method for the rational development of new antibiotics and herbicides based on the three-dimensional crystal structure of glutamyl-tRNA reductase.

Accordingly, the invention relates to a method for the preparation of antibiotics and herbicides comprising the steps of
(a) generating a space-filling model of glutamyl-tRNA reductase, or of functional domains thereof, and of candidate compounds,
(b) docking said candidate compounds to at least one of the substrate and cofactor binding sites, the active site pocket as well as the extended interaction surfaces with substrate (tRNA) and the following enzyme (GSAM) of said space-filling model of said glutamyl-tRNA reductase, or of functional domains thereof (furthermore also called surface), and estimating the fitting accuracy on the basis of the three-dimensional structure and functional surface amino acid residues information mediated by said space-filling model of said glutamyl-tRNA reductase,
(c) selecting and chemically synthesizing candidate compounds which fit to said surface of said space-filling model of said glutamyl-tRNA reductase,
(d) qualitatively and quantitatively detecting the potential inhibiting activity of said candidate compounds for glutamyl-tRNA reductase obtained in step (c) in a biological assay, e.g. the assay described by Moser et al., 1999.

In the vitamin B12 biosynthesis of plants, archea and the majority of bacteria, glutamyl-tRNA-Reductase is the target for feedback inhibition by heme and/or other tetrapyrroles. The binding regions for said inhibitors have been determined experimentally. Binding of said inhibitors leads to enzyme inhibition and to proteolytic degradation. At an increased vitamin B12 production, said tetrapyrroles accumulate thus inhibiting their own biosynthesis. Due to the knowledge of the three-dimensional structure of glutamyl-tRNA-reductase according to the present invention, a site-directed modification of the corresponding binding regions for preventing an inhibition or a degration, respectively, can be carried out for the first time.

Based on the inhibitor glutamycin and the inhibitor citrate, as well as the interaction domains for substrate (tRNA) and cofactor (NADPH) known by structure determination of the present invention, new inhibitors can be designed, developed and tested by directed rational drug design.

Based on the new inhibitors and the 3-dimensional structure of glutamyl-tRNA-reductase, a new glutamyl-tRNA-reductase enzyme can be designed and produced which is resistent to the inhibitors. First, a herbicide is developed, followed by making the glutamyl-tRNA-reductase enzyme from a useful plant resistent against this agent.

Large scale screening cannot be used since the enzyme glutamyl-tRNA-reductase is essential to bacteria, so that any not-site-directed mutation can be lethal to the organism, rendering tests with living cells unpracticable. High throughput in vitro tests failed because of a) an unstable, highly complex substrate (tRNA), b) an even less stable product (GSA), the detection whereof is practically impossible, and c) it leads to a necessarily complex test system with e.g. three enzymes and HPLC-analysis.

By the knowledge of the 3-dimensional structure of the enzyme, the inventors are in the unique situation to carry out amino acid exchanges, site-directed or randomized, at relevant positions of the binding regions (surfaces) with a calculable effect. The two small molecule inhibitors glutamycin and citrate serve as modell compounds to design, calculate and, optionally, synthesize new and highly potent inhibitors.

In a further aspect the invention relates to compounds having glutamyl-tRNA reductase inhibiting activity obtainable by conducting a method as defined above, or a salt, solvate, hydrate or formulation thereof which is acceptable for antibiotics or herbicides.

In another aspect the invention relates to pro-drugs, which are composed of a compound as defined above and at least one protective group which is acceptable for antibiotics or herbicides and will be cleaved off under physiological conditions.

In yet another aspect the invention relates to compositions containing a least one compound as defined above as the active agent or at least one pro-drug as defined above and optionally carriers and/or diluents and/or adjuvants.

In yet another aspect the invention relates to the use of a compound, a pro-drug or a composition as defined above for the manufacture of antibiotics or herbicides.

Further advantageous and/or preferred embodiments of the invention are subject-matter of the subclaims.

In one embodiment of the inventive method as defined above is in step (b) as said functional domain of said glutamyl-tRNA reductase one ore more domain selected form the group consisting of the catalytic or active site domain, the glutamyl-tRNA substrate binding domain, the NADPH binding domain, the glutamate-1-semialdehyde-2,1-aminomutase docking domain and the glutamyl-tRNA reductase dimerization domain used.

In the following the invention is described in more detail by reference to examples and figures. The examples or specific embodiments are, however, not to be construed as any limitation of the scope of the attached claims.

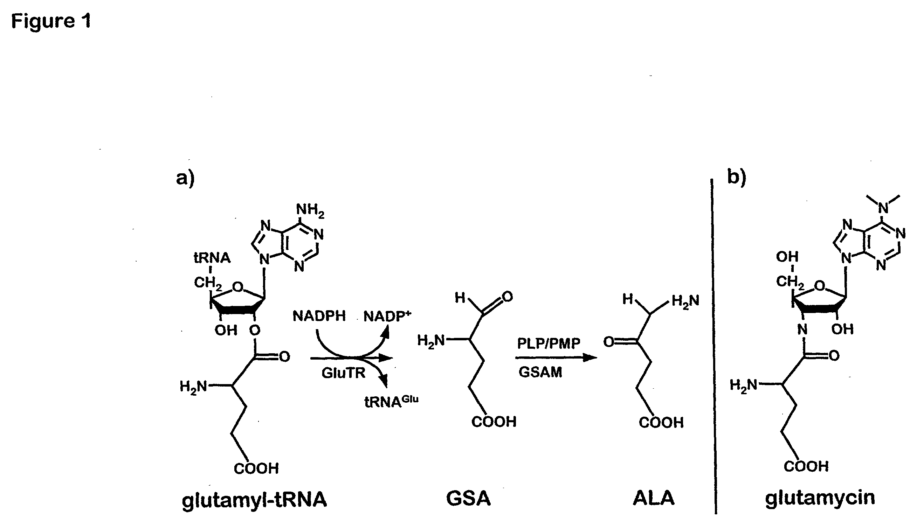

The function of transfer RNA (tRNA) is primarily associated with ribosomal protein biosynthesis (Al-Karadaghi et *al.,* 2000). Central to this process is the tRNA-mediated activation of amino acids catalyzed by aminoacyl-tRNA synthetases. In protein biosynthesis this activation is the energetic prerequisite for peptide bond formation at the ribosome. Intriguingly, tRNA-activated glutamate is also utilized to initiate a second major metabolic pathway, the tetrapyrrole biosynthesis in plants, archaea and the majority of bacteria (Jahn *et al*., 1992). Here the common precursor of all tetrapyrroles, 5-aminolevulinic acid (ALA), is generated from glutamyl-tRNA. In a first step, the activated glutamate is reduced to glutamate-1-semialdehyde (GSA) by the NADPH-dependent enzyme glutamyl-tRNA reductase (GluTR). In this reaction, a cysteine residue nucleophilically attacks the aminoacyl bond of glutamyl-tRNA leading to a highly reactive thioester intermediate (Moser *et al.,* 1999). Subsequently the thioester is reduced via hydride transfer from NADPH leading to the formation of GSA. In a second step, GSA is transaminated by glutamate-1-semialdehyde aminomutase (GSAM) leading to the formation of ALA (Smith *et al.*, 1992) (Figure 1).

Here we present the first crystal structure of a GluTR, in complex with the competitive substrate-like inhibitor glutamycin. The structure was solved by multiple isomorphous replacement (MIR) (Table I) and refined at a resolution of 1.95 Å (Table II).

The inventors solved the first crystal structure of a GluTR (from the archaeon *Methanopyrus kandleri*) in complex with the inhibitor glutamycin. The dimeric enzyme shows a unique V-shaped structure where a catalytic domain, a NADPH-binding domain and a dimerization domain are linked together via a curved 110 Å α-helix. Bindung of the puromycin-derived inhibitor glutamycin to the active site led to the identification of catalytically important amino acids and the confirmation of the postulated catalytic mechanism (Moser *et al.,* 1999). Docking of the glutamyl-tRNA substrate (Sekine *et al*., 2001) on the enzyme structure revealed an extensive surface complementarity between both macromolecules. The structure also suggests that the void formed between both monomers in the GluTR structure could be occupied *in vivo* by GSAM (Hennig *et al.*, 1997), the following enzyme in the pathway, allowing efficient metabolic channeling of the aldehyde product of GluTR into the active site of GSAM.

The coordinates of the GluTR structure presented here is attached in Brookhaven Protein Data Base (PDB) ASCII format (see diskette) and can be easily visualized and manipulated using freely available programs like RasMol (Sayle & Milner-White, 1995) and Swiss PDBViewer (Guex & Peitsch, 1997) and a state-of-the-art personal computer (PC).

### Tools for protein structure-based drug design

A detailed understanding of interactions between small molecules and proteins forms the basis for rational drug-design strategies (reviewed by Verlinde & Hol, 1994; Whittle & Blundell, 1994; Lybrand, 1995). Freely and commercially available programs for docking small molecular ligands to the surface of proteins are abundant and have been successfully used in the past (e.g. Lorber & Shoichet, 1998; Stigler et al., 1999; Otyepka et al., 2000; Taylor & Burnett, 2000). Other programs like GenStar (Rotstein & Murcko, 1993a) and GroupBuild (Rotstein & Murcko, 1993b) can be utilized to generate chemically reasonable structures that fit to the protein surface and that might subsequently serve the chemist as lead structures for drug development.

### Description of the structure

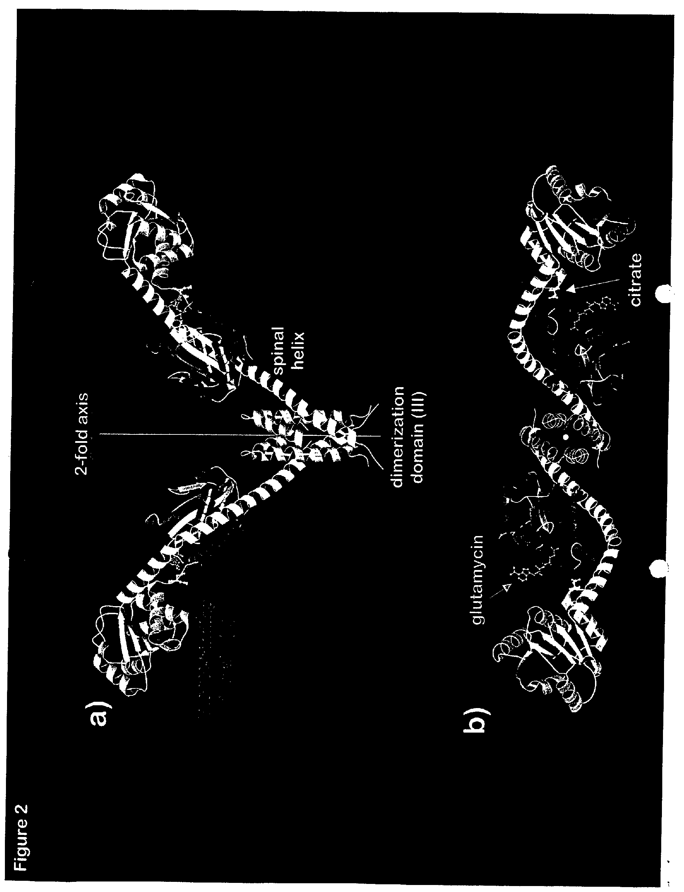

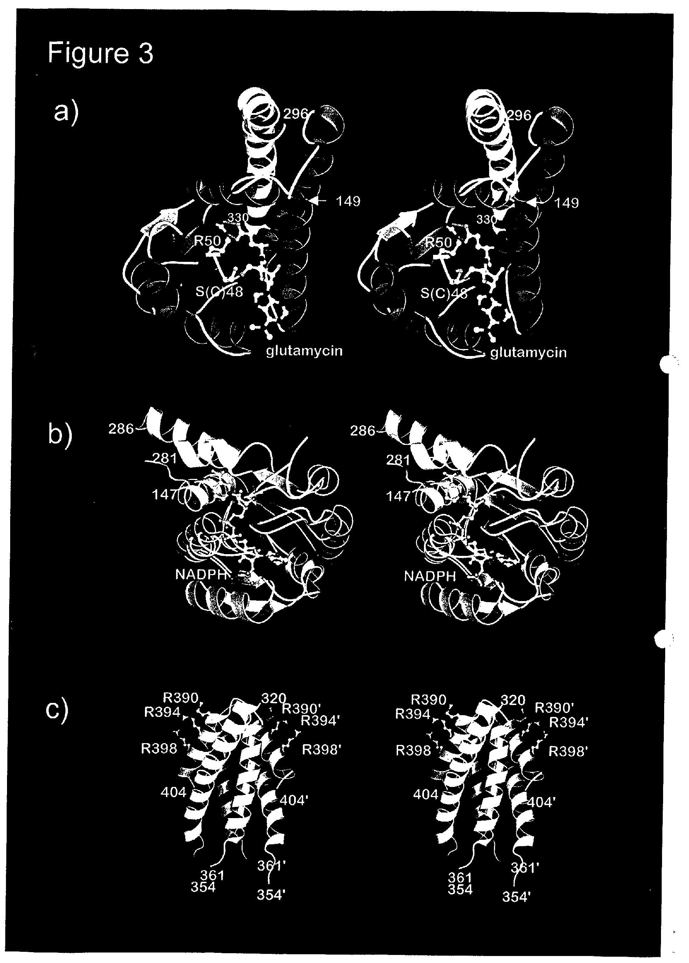

GluTR is a dimeric protein with an unusual V-shaped fold (Figure 2). The monomers, each constituting one leg of the V, are about 125 Å in length, 45 x 45 Å² in cross-section and together span a maximum width of ∼165 Å. Each monomer consists of three distinct domains linearly arranged along a common axis and connected by a curved "spinal" α-helix of 110 Å in length. Residues 1-145 constitute the N-terminal domain (domain I) consisting of two subdomains (Figure 3a). Residues 1-77 form a central antiparallel four-stranded β-sheet decorated on one side by 3 α-helices. The topology, though modified to βαββααβ, most closely resembles the common βαββαβ-motif of, amongst others, RNA-binding proteins (Lindahl *et al.*, 1994; Schluenzen *et al*., 2000). Residues 78-141 form three antiparallel α-helices packed against the central β-sheet. Together with the central part of the spinal α-helix they constitute a distorted antiparallel 4-helix bundle. A short linker (residues 142-148) connects domain I to domain II (residues 149-285). Domain II has a classical nucleotidebinding fold (Carugo and Argos, 1997) composed of a central 6-stranded parallel β-sheet, characteristic βαβ-motifs and a conserved glycine-rich loop (Figure 3b). Domain II is directly followed by the spinal α-helix (residues 286-352) comprising 18 α-helical turns with a visible kink induced by Pro305. C-terminally the spinal α-helix becomes part of a 3-helix dimerization domain (domain III), which forms an unusual 6-helix bundle with the neighboring GluTR monomer around a crystallographic 2-fold axis (Figure 3c). Residues 355-360, connecting the spinal helix to the second helix of domain III are not visible in the electron density map. As a result, it is unclear whether α-helices from one subunit are arranged alongside each other or whether α-helices from alternate subunits interdigitate.

### Active site and catalytic mechanism

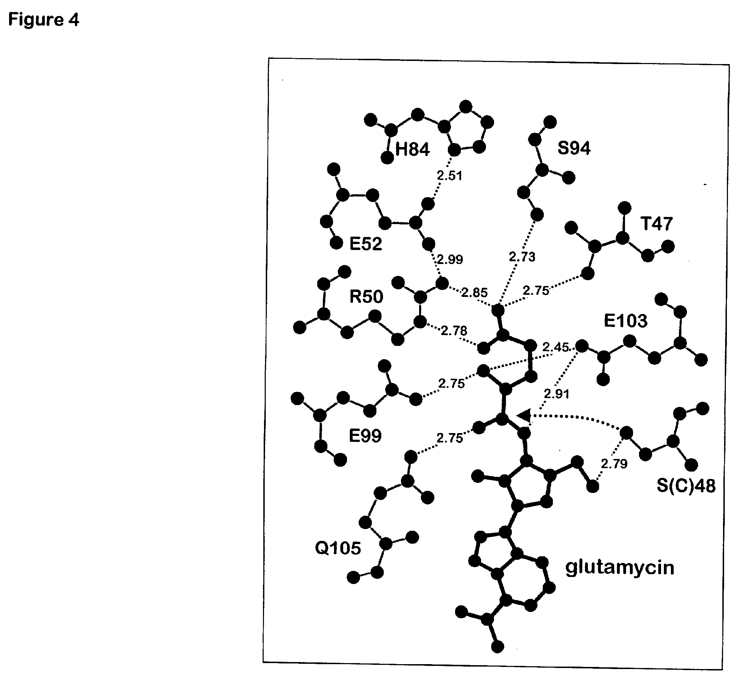

The present crystal structure of GluTR is a complex with the inhibitor glutamycin (Moser *et al.,* 1999), a synthetic glutamate analogue of the aminonucleoside antibiotic compound puromycin (Figure 1).Glutamycin is bound within a deep pocket in domain I of GluTR specifically recognized by an array of strictly conserved residues (Figures 3a and 4). The principal interaction at the bottom of the pocket is the bidentate salt-bridge between the carboxylate group of glutamycin and Arg50 - allowing for the differentiation of glutamate as opposed to glutamine. Other hydrogen bonds to the glutamycin carboxylate involve the side-chains of Ser94 and Thr47. The α-amino-group of glutamycin is recognized by Glu99, Glu101 and Gln105 and the amide group and ribose 03-atom again by Glu101. Arg50, which is largely buried, is held in position by an intricate hydrogen-bonding network of likewise highly conserved residues including His84, which has been shown to be essential for catalytic activity (Moser *et al.*, 1999). Analogously, in aspartyl-tRNA synthetase (Eiler *et al.*, 1999) and presumably glutamyl-tRNA synthetase (Sekine *et al.*, 2001) the substrate carboxylate group is also specifically recognized via a conserved arginine at the bottom of the amino acid recognition pocket.

The proposed catalytic mechanism for the enzyme (Moser *et al*., 1999) is in good agreement with the crystal structure (Figure 4). The activated α-carboxylate of glutamyl-tRNA is nucleophilically attacked by a conserved cysteine (Cys48) leading to a covalent thioacyl intermediate and release of tRNA^{Glu}. In the present structure Cys48 (as well as other cysteines) had to be substituted by serine to prevent nonspecific intermolecular disulfide bond formation during crystallization. The mutation C48S, apart from preventing oligomerization, leads to the complete inactivation the enzyme (Moser *et al*., 1999). The side-chain of Ser48, exposed at the rim of the binding pocket, is in close proximity (3.9 Å) to the α-carbonyl carbon atom of glutamycin. In a second step the highly reactive thioacyl intermediate is then reduced to GSA by an S_{N}2-like hydride transfer from NADPH. The precise position of the nicotinamide moiety in the active complex cannot be derived from the present structure as the NADPH-binding site in domain II is too remote from the glutamate recognition pocket (see below).

Domain II closely resembles the NADPH-binding domain of human tetrahydrofolate dehydrogenase/cyclohydrolase (Allaire et *al*., 1998; Holm and Sander, 1996), the r.m.s. deviation for main chain atoms being 2.3 Å for 104 common residues with structurally well conserved binding pockets for the adenine moiety (Figure 3b). In GluTR, however, the NADPH-binding site is empty despite attempts to co-crystallize GluTR with NADPH or smaller analogues. It appears that the second α-helix (αB, residues 176-190) and the glycine-rich loop (connecting β1-αB, residues 174-176) are laterally shifted in GluTR leading to a slight compression of the diphosphate recognition pocket (between the glycine-rich loop and loop β4-αE, residues 234-236) (Carugo and Argos, 1997), thus preventing NADPH binding. Based on superpositions with homologous structures, we have nevertheless derived a theoretical position for NADPH (Figure 3b). The distance between the nicotinamide moiety and the glutamate-binding pocket is, however, about 21 Å. The NADPH-binding pocket obliquely faces the large active site crevice between domains I and II, suggesting that domain II is required to tilt substantially relative to the remaining structure to position NADPH for substrate reduction. Possibly this tipping of domain II induces the opening of the NADPH binding pocket and occurs in concert with glutamyl-tRNA- binding. The present open structure of GluTR may therefore be described as a »pre-active« state.

The large crevice separating domains I and II is delimited at its deep end by the N-terminal third of the spinal α-helix and the loop connecting domains I and II. Within this crevice, we have identified a well-defined citrate anion specifically contacting residues of both domains I (Gln100) and II (Ser152), as well as the connecting loop (Val148) and the spinal α-helix (Arg300) (Figure 2). The citrate proved to be mandatory for the successful crystallization of the enzyme presumably by stabilizing the present 'pre-active' GluTR conformation. Interestingly, Arg300, which binds the citrate anion with a total of three different hydrogen bonds, is conserved in all plant and Archaea GluTR-sequences, not however in all bacteria (see http://srs.ebi.ac.uk/srs6bin/cgibin/wgetz?-id+2R07NlHLqM4+[pfama-AccNumber:PF00745]+-e). Its side chain extends through a curve in the interdomain loop, presenting its positive charge at the bottom of the crevice. It is plausible that the citrate anion may partly mimic the acceptor stem of the glutamyl-tRNA^{Glu} substrate, either as a counter ion for a tRNA backbone phosphate or for the specific base recognition.

### Comparison of GluTR and its theoretical model

Using multiple sequence alignments, secondary structure prediction, homology modelling and biochemical evidence, Brody *et al*. (1999) have proposed a theoretical model of GluTR. Subunit B of succinyl CoA synthase served as an overall template. In principle, the model correctly identifies the domains of GluTR, including the glutamyl-thioester region (part of domain I), the NADPH binding region (domain II), and the α-helical, glutamyl-tRNA binding region (domain III).

Of the three domains, only the NADPH-binding domain, however, resembles its true three-dimensional structure. Clearly, the large collection of related domains available from known protein structures favors a structural comparison. Intricate details of glutamate recognition and the overall glutamyl-tRNA surface are, of course, beyond the scope of such a model. However, aspects including the dimerization domain were similarly not identified by the theoretical model.

### Glutamyl-tRNA recognition

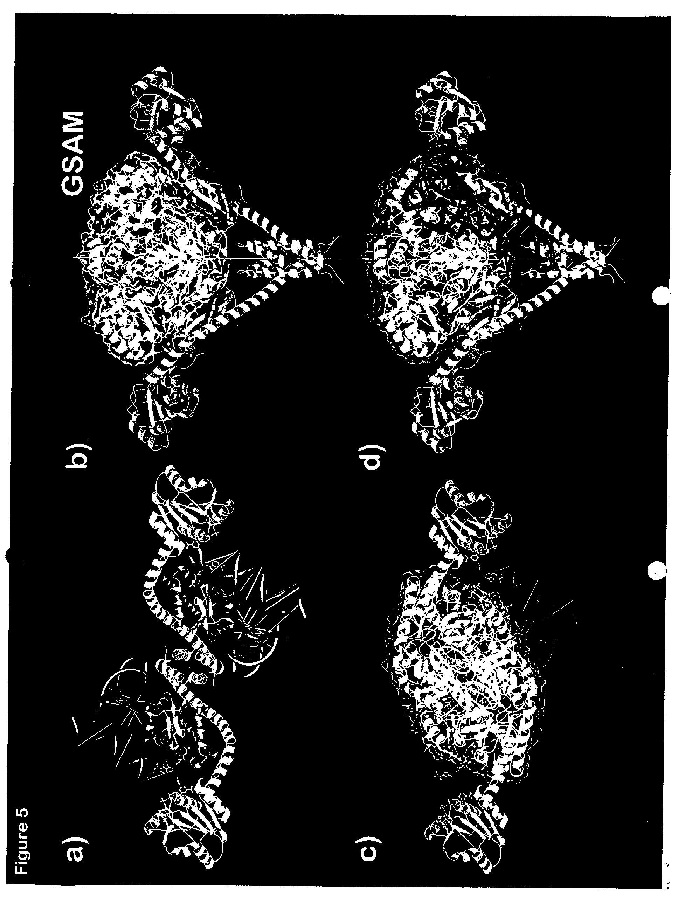

Does the present GluTR structure allow for the prediction of binding of the natural substrate glutamyl-tRNA? Based on the well-defined location of glutamycin in the present structure, we manually positioned the acceptor-stem bearing both the 5'- and 3'-ends of *Thermus thermophllus* tRNA^{Glu} (Sekine *et al*., 2001) into the active site of GluTR and rotated the tRNA around the RNA double helical axis of the acceptor stem. This procedure favors a single orientation for the tRNA^{Glu} in which substantial surface complementarity between the tRNA^{Glu} and the enzyme is achieved without incurring steric clashes (Figure 5a). In this orientation, the 3'-strand of the amino acid acceptor stem (ribonucleotides 68-70) interacts with domain I (helix αA), while nucleotides 11-13 and 24-25 of the dihydrouridine hairpin fit into two neighboring shallow depressions on the surface of domain I formed by α-helices B and C. Also the anticodon bases 34-36 of the anticodon hairpin are in close proximity to α-helical domain III, thus allowing in principle for the specific recognition of the anticodon. This mode of tRNA-recognition is reminiscent of the discriminating GluRS, where the anticodon of glutamyl-tRNA is recognized by an all α-helical domain (Sekine *et al*., 2001). Organisms lacking glutaminyl-tRNA synthetase (GlnRS) (Schön *et al.*, 1988) mischarge glutaminyl-tRNA with glutamate and convert this to glutamine using an amidotransferase. In these organisms, GluTR needs to discriminate between glutamyltRNA^{Gln} and glutamyl-tRNA^{Glu}. Anticodon recognition critically supports such discrimination. Our proposed model is corroborated by studies of barley GluTR where tRNA^{Glu}-recognition has been shown to involve nucleotides in the anticodon stem of the tRNA^{Glu} (Willows *et al*., 1995). In addition, the mutual extended dimensions of GluTR and the tRNA substrate, and the proposed anticodon recognition suggest that the fidelity of tRNA^{Glu}-recognition is ensured by a multitude of individual interactions.

In our present model of the enzyme-substrate complex, tRNA^{Glu} and domain II of GluTR interact only marginally. In fact, glutamyl-tRNA and domain I together create a large shallow surface that could accommodate domain II. To achieve this, the latter would need to swing around the N-terminal tip of the spinal α-helix. Simulating this movement in the model (arrow in Figure 5d), places NADPH in close proximity to the glutamate binding pocket. Similarly, a slight relaxation of the elbow angle of tRNA^{Glu} would significantly improve the fit around domain I. Such deviations from free tRNA-structures have been observed before (Nissen *et al.,* 1995; Nissen *et al*., 1999; Schmitt *et al*., 1998). Especially the flexible, single-stranded 3'-end of tRNA^{Glu} as taken from the complex with *Thermus thermophilus* glutamyl-tRNA synthetase (Sekine et *al.*, 2001) is required to adopt a tight turn conformation reminiscent of glutaminyl-tRNA bound to glutaminyl-tRNA synthetase (Rath *et al.,* 1998). This conformational change has been incorporated into the model as seen in Figure 5a.

### Model of a ternary complex between GluTR, glutamyl-tRNA and GSAM

In animals, fungi and some bacteria, ALA is synthesized in a one-step condensation of succinyl-CoA and glycine by ALA-synthase (Kikuchi *et al*., 1958, May *et al*., 1995). The tRNA-dependent ALA-formation in plants and most bacteria, by contrast, requires the concerted action of the two enzymes GluTR and GSAM (Figure 1). As yet no biochemical evidence has indicated a direct interaction between both enzymes despite being metabolically linked by the reactive and therefore transient aldehyde GSA. A close spatial proximity between GluTR and GSAM, therefore would ensure the efficient formation of ALA by preventing leakage of the aldehyde from this synthetic pathway. The V-shaped structure of the GluTR suggests an attractive solution to this metabolic problem. Placing GluTR alongside the similarly dimeric structure of GSAM from *Synechococcus sp.* (Hennig *et al.,* 1997) immediately suggests that the open space delimited by the GluTR monomers is strikingly similar to the volume occupied by GSAM. Docking both enzymes along their two-fold axes leads to a model complex (Figure 5b) with a striking degree of surface complementarity. In the case of GluTR, domain I and the spinal α-helix of each monomer are involved in inter-protein contacts. To further improve the mutual fit requires a small tilting of GSAM, which translates into a slight widening of the GluTR-grip by moving the catalytic domains outward relative to the dimerization domain. Concomitantly, this movement of domain I relative to domain III, brings the tRNA anticodon loop of tRNA^{Glu} into closer contact with domain III of GluTR, which, as proposed, may have a role in anticodon recognition (see above). In addition, widening the GluTR-embrace allows GSAM to approach the flexible loop connecting the two C-terminal α-helices in domain III.

As described, both tRNA^{Glu} and GSAM may separately be docked onto GluTR, each in a single plausible position. Though separately docked, the model of the ternary complex of GluTR, tRNA^{Glu} and GSAM does not lead to steric clashes between GSAM and tRNA^{Glu} (Figures 5c and d). Instead GSAM and tRNA^{Glu} appear to share a substantial binding surface, indicating that the proposed ternary complex is stabilized by mutual recognition between each pair of the three components. The common binding interface for tRNA^{Glu} therefore appears to be prerequisite for tRNA^{Glu} recognition and binding.

Residues on the surface of both GluTR and GSAM are not particularly strongly conserved. Similarly, both tRNA sequences and nucleotide modifications vary from one species to another. Therefore local complementarity via salt bridges or hydrophobic interactions is most likely species dependent.

### Metabolic channeling between GluTR and GSAM

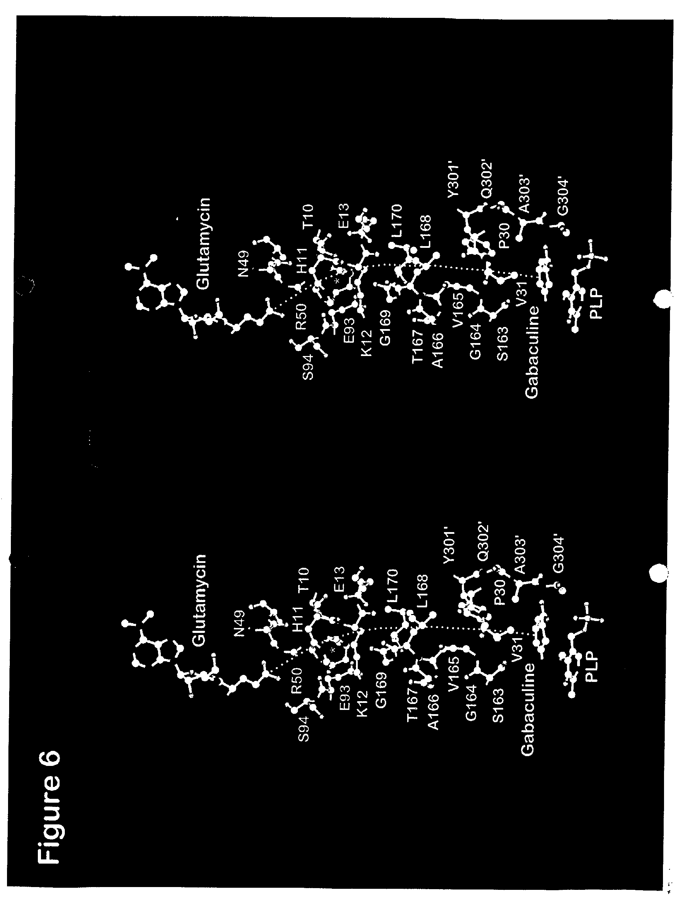

The most striking result of the GluTR/tRNA^{Glu}/GSAM model complex is, however, that the putative active site entrance of each GSAM monomer (Hennig *et al.,* 1997) is positioned opposite a partly opened depression in domain I of GluTR. This depression, defined largely by Thr10, His11, Glu13, His84, and Glu93, is separated from the glutamate recognition pocket by only Arg50. The proposed complex thus implies that the GluTR-product GSA could leave the enzyme via this »back door« of the glutamate recognition pocket and directly channel to the active site of GSAM, a distance of about 26 Å, without coming into contact with the aqueous environment (red dotted line in Figure 5c, Figure 6). The proposed channeling would consequently ensure the catalytic efficiency of this pathway. Despite extended surface complementarity between GluTR and GSAM, residues contributing to this interface are not conserved. Instead species-specific interactions may have evolved. As the two protein structures used in the present modelling originate from distantly related organisms, predictions of individual pairs of interacting residues is not possible.

GSAM is known to be an allosteric dimeric enzyme (Hennig *et* al., 1997; Contestabile *et al.*, 2000) potentially dictating the overall reaction rate of the complex. As indicated by the disordered lid covering the active site of one monomer (Hennig *et al.,* 1997), GSAM oscillates between two states where at any particular time one monomer is in a contracted, active state, while the other is in a relaxed state, allowing the product ALA to leave the complex. In oscillating between the two states, GSAM would, in our model, induce corresponding changes in the GluTR monomers, possibly optimizing the tRNA-^{Glu}-recognition surface. Once glutamyl-tRNA had bound, GSA would be produced and channeled to GSAM. The complex could then switch to the second state, inducing the conversion of GSA to ALA.

### Conclusions and outlook

The unusual V-shaped structure of GluTR suggests that the enzyme is capable of forming multiple and extended interactions not only with its natural substrate glutamyl-tRNA but also simultaneously with the subsequent enzyme of tetrapyrrole biosynthesis, GSAM. The proposed ternary complex provides the basis for the analysis of the complex protein-protein as well as protein-RNA-interactions required for the initiation of tetrapyrrole biosynthesis in plants and most bacteria. In addition it may represent a unique model system to study metabolic channeling between two essential enzymes both at the functional and structural level. As this pathway is ubiquitous throughout to the biosphere, merely excluding animals and some prokaryotes, the proposed complex may furthermore allow the rational design of novel herbicides and antibiotics.

### Materials and methods

### Protein crystallization and data collection

Recombinant production and purification of GluTR have been published elsewhere (Moser *et al.,* 1999). GluTR was crystallized by hanging-drop vapor-diffusion at 4°C with a protein concentration of 9.6 mg/ml in 0.2 M NaCl, 2 mM MgCl₂, 100 mM HEPES pH 7.5, 0.2 M sodium citrate and 30 % (v/v) MPD as precipitant. Lens-shaped crystals grew to a size of 0.3 x 0.1 x 0.1 mm³ within two weeks.

### Phasing, model building and refinement

Crystals contain one molecule per asymmetric unit and belong to space group *P*2₁2₁2 with a = 78.3 Å, b = 98.7 Å and c = 68.6 Å. All data were collected at 100 K using an MSC-Rigaku R-AXIS IV⁺⁺ imaging plate detector on a Rigaku RU-H3R X-ray generator equipped with Osmic Confocal MaxFlux™ Optics. Data were processed with *DENZO*/*SCALEPACK* (Otwinowski and Minor, 1997) and *TRUNCATE* (CCP4, 1994). »Native« data were obtained from crystals of a mutant, where cysteines at positions 6, 42, 48, 90 and 393 were replaced by serine. Heavy atom derivative soaks were performed using the triple mutant C42S/C48S/C90S. Phasing was initiated with difference Patterson search using *SOLVE* (Terwilliger and Berendzen, 1996) and performed with *SHARP* (de la Fortelle and Bricogne, 1997), making use of multiple isomorphous and anomalous scattering (MIRAS) phase probabilities. The subsequent phase-modified map (using *SOLOMON* (Abrahams and Leslie, 1996), was readily traced with *O* (Jones *et al.,* 1991). Refinement was performed using *CNS* (Brunger *et al*., 1998) followed by *REFMAC* (Murshudov *et al.*, 1997). *PROCHECK* (Laskowski *et al.*, 1993) was used for structure validation. The current model of GluTR contains residues 1 to 404 but excludes residues 355-360 which are disordered. 85% of the residues were in the most favored regions of the Ramachandran plot. Outliers cumulate in two poorly defined loop regions located in domain III (residues A349, R352, L353, Q363, L384, P385, D386). Other outliers of possible functional relevance include D18, R23, R59, R390 and A391. Coordinates and structure factors of GluTR have been deposited at the Protein Data Base (entry code ***noch einfügen***).

### Legends to Figures

**Fig. 1.** Pathway of tRNA-dependent formation of 5-aminolevulinic acid (ALA) and structure of the substrate-like inhibitor glutamycin. a) Glutamyl-tRNA reductase (GluTR) reduces tRNA-bound glutamate to glutamate-1-semialdehyde (GSA). Glutamate-1-semialdehyde aminomutase (GSAM) transaminates GSA to ALA. b) The inhibitor glutamycin is an analogue of the 3'- terminal nucleotide of acylated tRNA^{Glu}.

**Fig. 2.** Structure of the GluTR dimer viewed **a)** perpendicular to and **b)** along the 2-fold-axis. Monomers consist of three structural domains: I) an N-terminal catalytic domain (blue), II) an NAPDH-binding domain (green) and III) a C-terminal dimerization domain (orange) - connected by an extended 18-turn 'spinal' a-helix (dark-yellow). Glutamycin (red) binds at the catalytic domain. At the deep end of the large crevice between domains I and II a citrate anion (yellow) is bound. Figures 2,3 and 5 were generated using *MOLSCRIPT* (Kraulis, 1991) rendered with *RASTER3D* (Merrit and Murphy, 1994).

**Fig. 3.** Stereo images of the three domains of GlutTR. Colouring of the domains and the spinal helix as in Fig. 2. a) Catalytic domain with bould glutamycin (orange) viewed towards the glutamate recognition pocket. The salt-bridge like interactions between the glutamate moiety of glutamycin and Arg50 (pink) are indicated by green lines. Also the catalytic side chain of Ser/Cys48 is shown. **b)** NADPH-binding domain of GluTR superimposed on the NADPH-binding domain of tetrahydrofolate dehydrogenase/cyclohydrolase (light grey). NADPH bound to this domain is shown in pink. **c)** Dimerization domain. Residues of the second monomer are primed. Also shown are the side-chains of Arg390, Arg394 and Arg398 that we postulate may interact with the anticodon region of glutamyl-tRNA.

**Fig. 4.** Glutamycin (red) bound within the glutamate recognition pocket of GluTR (orange bonds). Primarily the glutamate moiety of glutamycin is recognized through a network of specific hydrogen bonds, the ribose moiety to a lesser extent. The initial step of GluTR catalysis, the nucleophilic attack of the carbonyl carbon by Cys48 (here Ser48) is indicated (dotted arrow). Adapted from *LIGPLOT* (Wallace et *al.,* 1995).

**Fig. 5.** Model complexes. **a)** Model of GluTR - 2tRNA^{Glu} viewed along the 2-fold axis. A GluTR-dimer can independently bind two tRNAs (blue and violet) . **b)** The open V-shape of GluTR provides sufficient space to allow the following enzyme of the tetrapyrrole biosynthesis pathway GSAM (white and pink) to bind. The translucent surface of GSAM (gray) indicates significant surface complementarity between both protein dimers, which share a two-fold symmetry axis. **c)** and **d)** Combining complexes **a)** and **b)** gives rise to a proposed ternary complex GluTR / GSAM / 2tRNA^{Glu} without steric clashes between individual constituents. The NADPH-binding domain would need to tip and rotate around the end of the spinal helix (green arrow in **d)**), to close the active center and initiate the reaction. The proposed substrate path of GSA from the active site of GluTR to that of GSAM is indicated as a dotted red line in **c)**. The active sites of the GSAM monomers are emphasized by two red rings marking the PLP cofactors.

**Fig. 6.** In the proposed complex GluTR/GSAM (blue/pink residues) the respective active sites are separated ∼26Å. Glutamate-1-semialdehyde (GSA), the product of GluTR and the substrate of GSAM, would channel from GluTR to GSAM (green, dotted line). In GluTR, there is an opening immediately behind the central active-site residue Arg50 surrounded by the loop residues Thr10 to Glu13, Glu93 to Ser94 and His84 (position indicated by a blue star). In the putative complex, this comes to lie opposite a partly disordered loop 159-172 (Ser163 to Leu170 shown) proposed to be the active site entrance for GSAM (Hennig *et al*., 1997) and shown to be essential for catalysis (Contestabile *et al.*, 2000). Gabaculine, an inhibitor of GSAM, indicates the position GSA would presumably occupy to initiate its conversion to ALA.

**Table I.**

| MIR phasing statistics | | | | |
|---|---|---|---|---|
| Data sets: | Native | TlCl | K₂IrCl₆ | K₂HgI₄ |
| Soak (mM) Soaking time | - | 0.6 | 3 | 2 |
| (d) | - | 2 | 2 | 3 |
| Resolution (Å) Completeness | 1.9 | 2.5 | 2.6 | 3.0 |
| (%) | 91.1 | 100.0 | 99.7 | 99.6 |
| (last shell) | 69.1 | 99.7 | 99.6 | 99.3 |
| Redundancy | 2.5 | 5.4 | 5.2 | 5.7 |
| Average *I*/*σ* | 12.0 | 15.9 | 9.8 | 9.2 |
| R_{sym} (%)* | 7.3 | 8.3 | 7.4 | 8.5 |
| (last shell) | 32.4 | 30.5 | 24.4 | 21.0 |
| Sites | - | 1 | 1 | 1 |
| R_{cullis} (%)^{‡} | - | 0.80 | 0.67 | 0.73 |
| Phasing power Figure of | - | 1.53 | 1.60 | 1.19 |
| merit | | 0.79 | | |

| | | | | |
|---|---|---|---|---|
| *R_{sym} = Σ*j* \| *Ij-*<*I*>\| / *Σj Ij* with *j* = (*hkl, i*), where *Ij* is the intensity for the reflection *j,* and <*I*> is the mean intensity for multiply measured reflections. | | | | |
| ^{‡}R_{cullis} = Σ*j* \| \| *F*_{PH} - *F*_{P} \| - *F*_{H} \| / *Σj F*_{H} with *j* = (centric *hkl*), where F_{H} is the calculated heavy atom structure factor. | | | | |

**Table II.**

| Refinement statistics | |
|---|---|
| Resolution (Å) | 1.95 |
| Number of atoms: protein, inihibitor, water | 3143, 29, 374 |
| R-factor (%)† | 19.7 |
| Rfree (%)‡ | 26.0 |
| Rmsd bond lengths (Å) | 0.03 |
| Rmsd bond angles (degrees) | 3.0 |

| | |
|---|---|
| †R-factor = Σ \| Fₒ - F_{c} \| / Σ Fₒ where Fₒ and F_{c} are the observed and calculated structure factors, respectively. ‡R_{free} is the cross-validated R-factor computed for a test set of a 5 % of the reflections, which were omitted during refinement (Brünger, 1992). | |

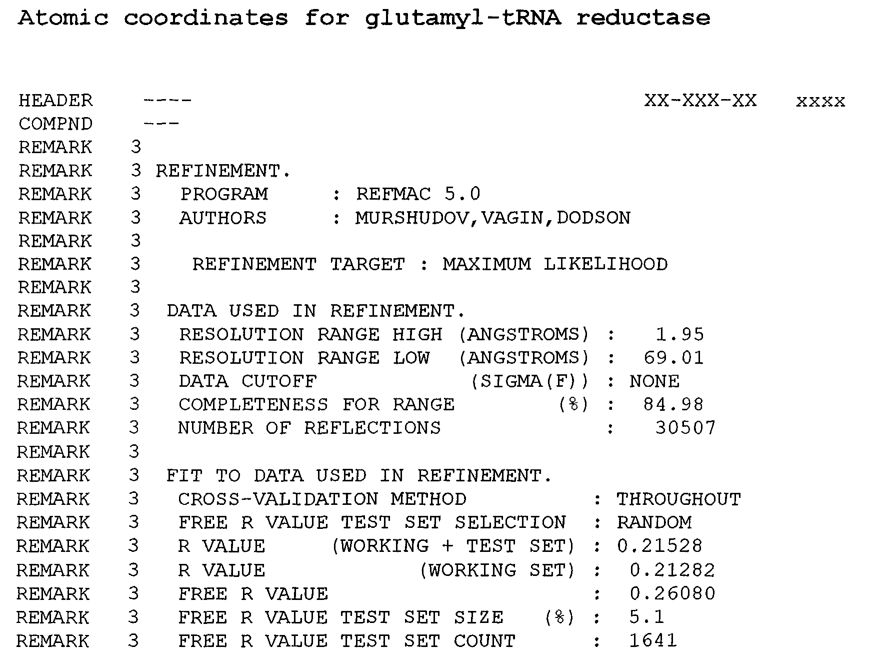

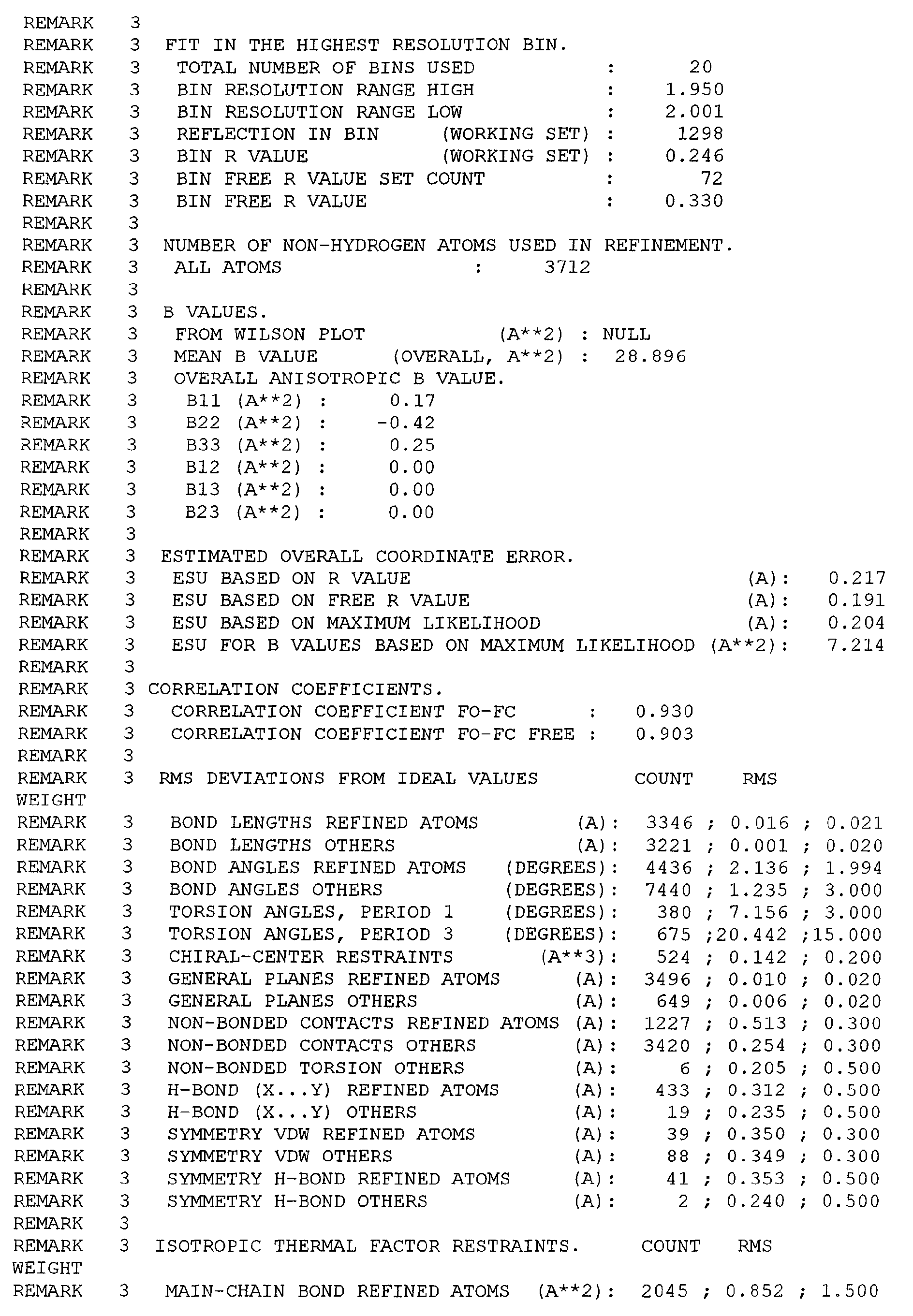

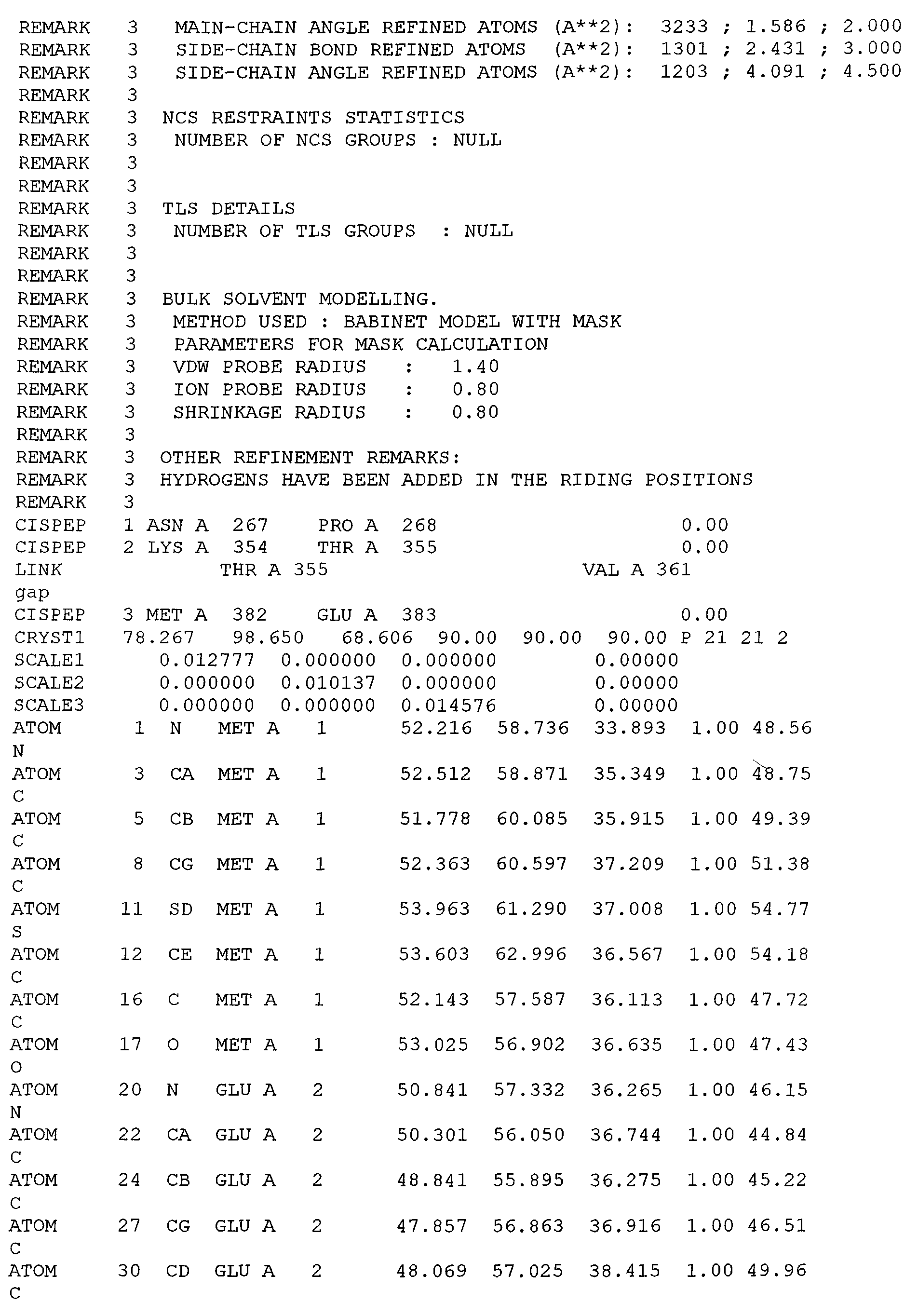

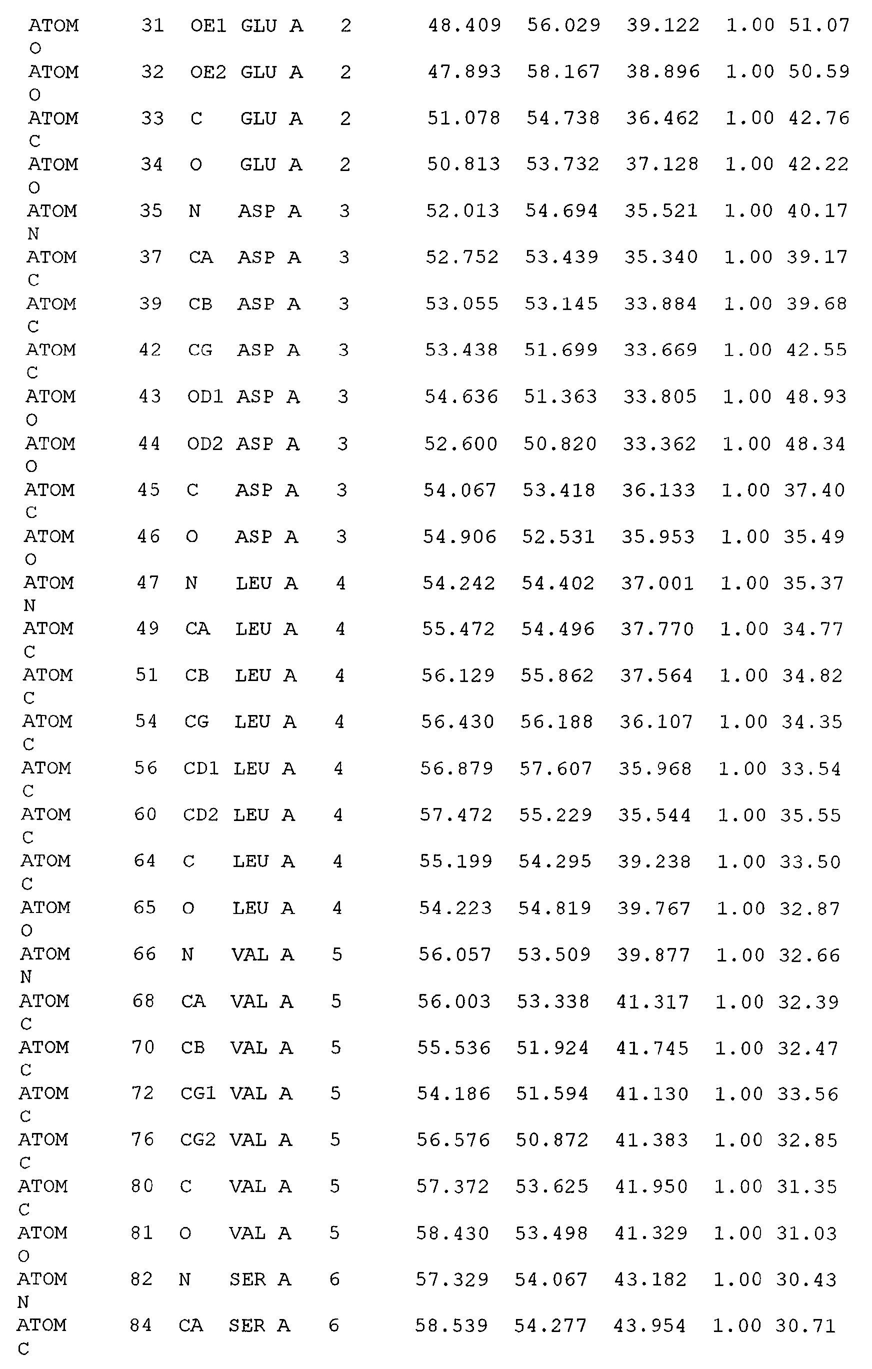

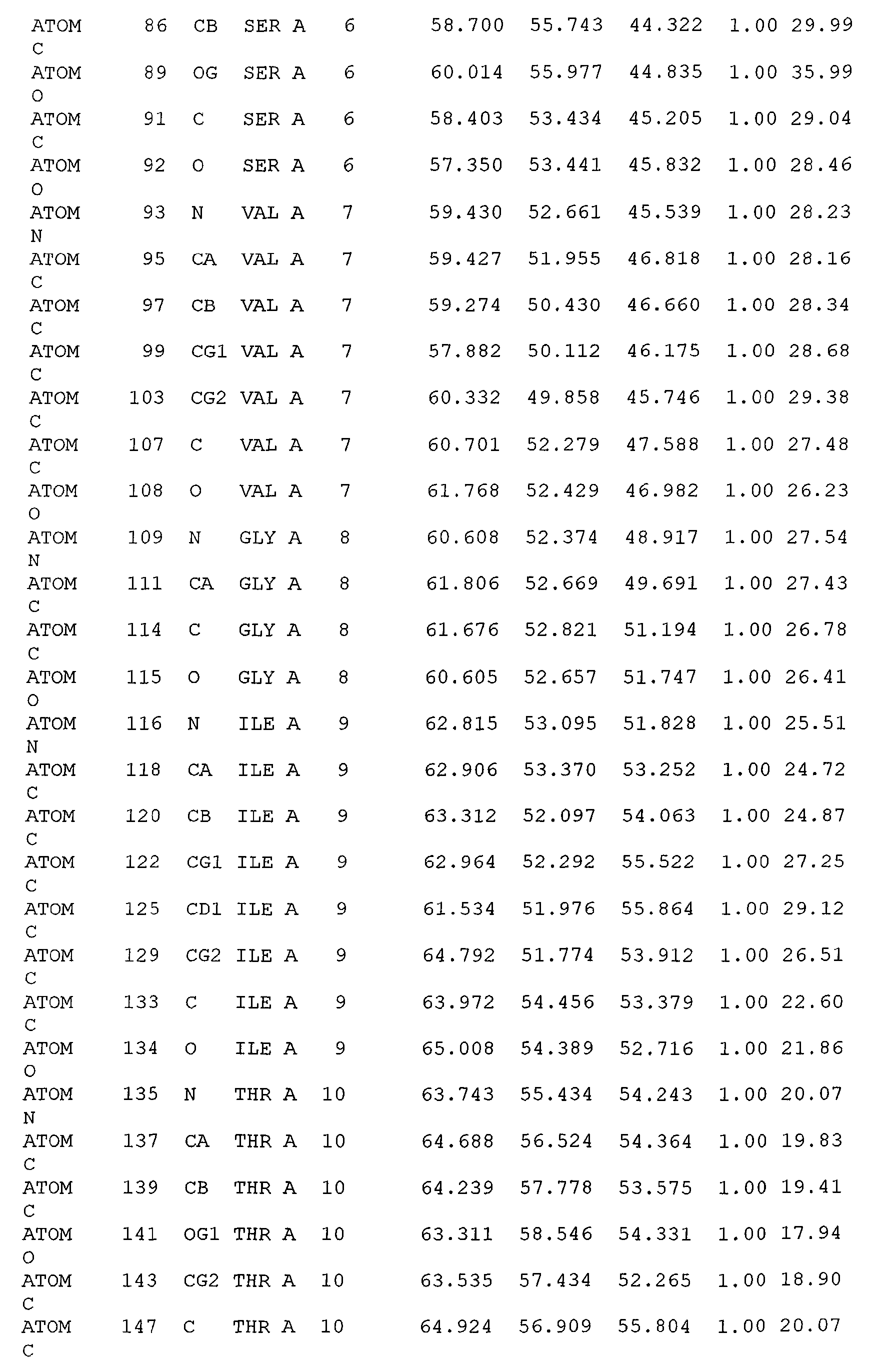

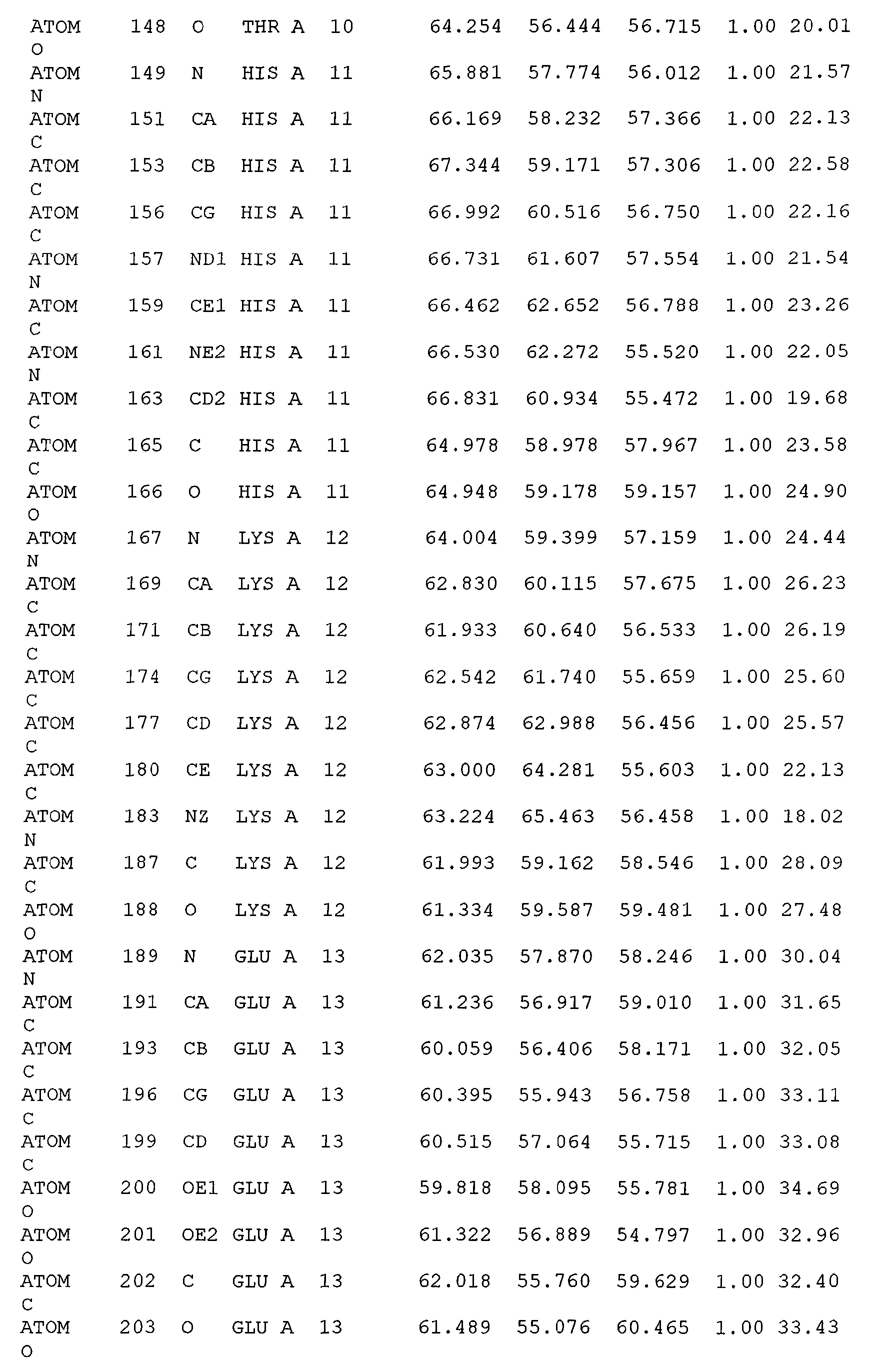

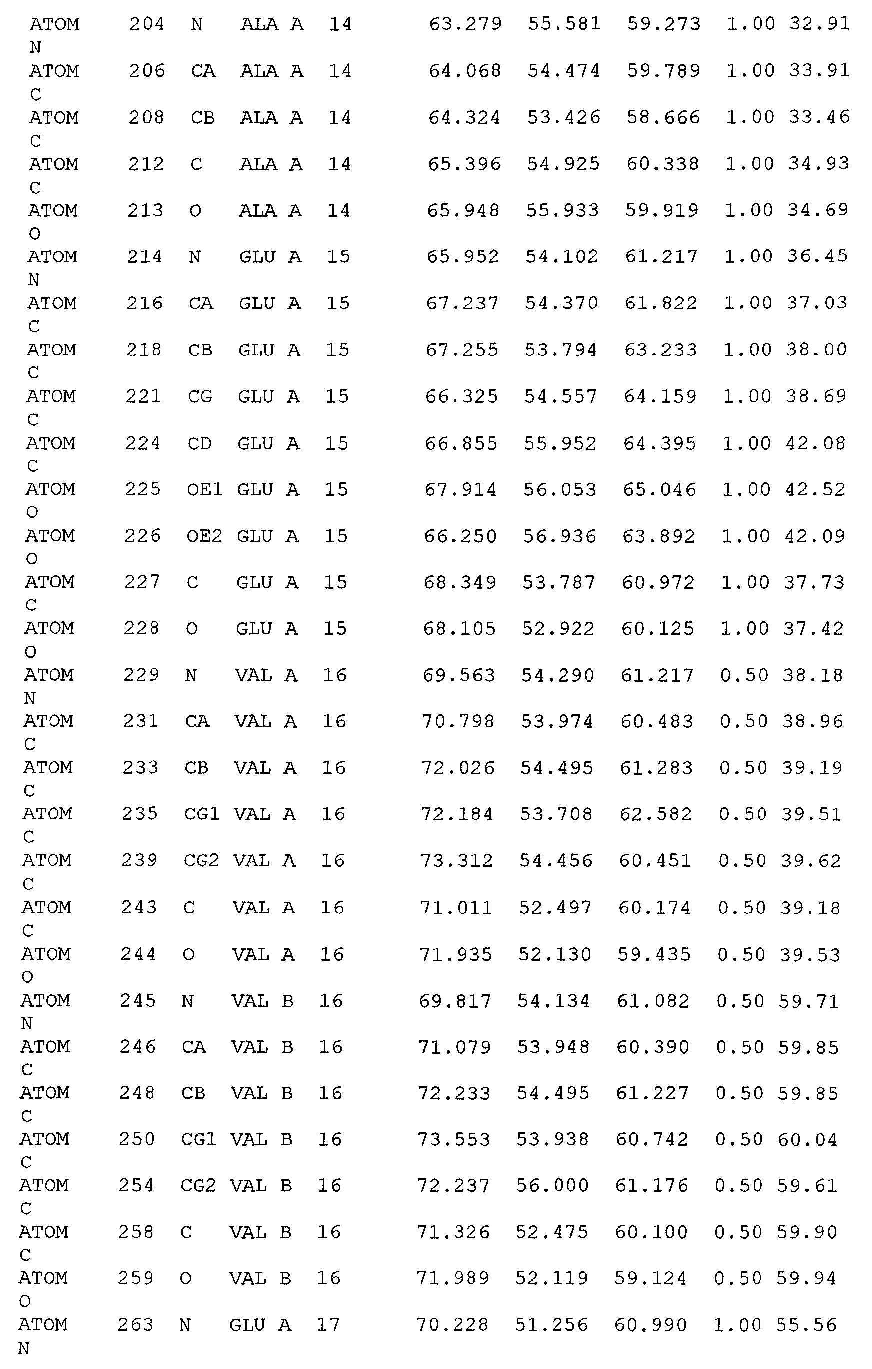

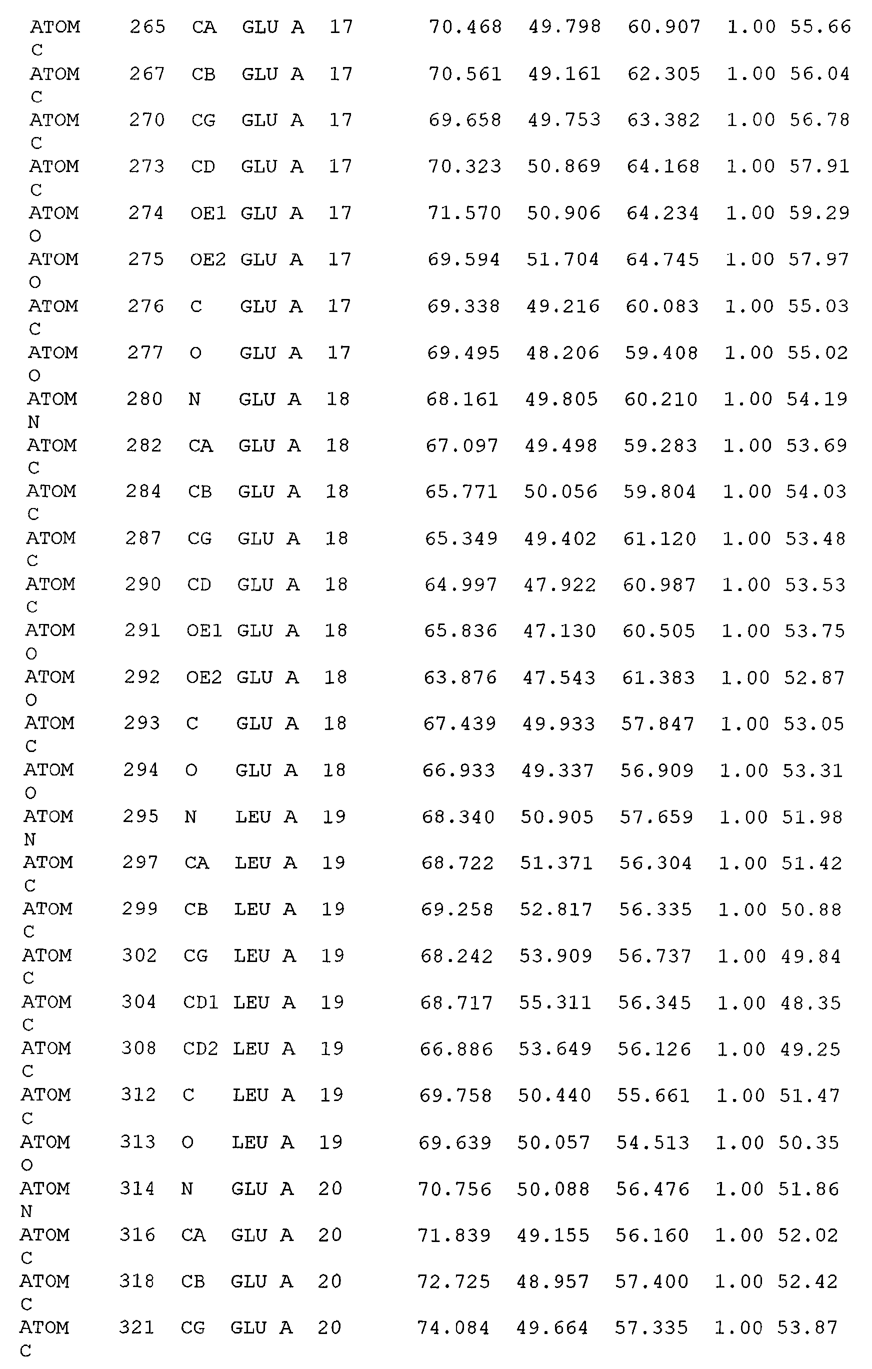

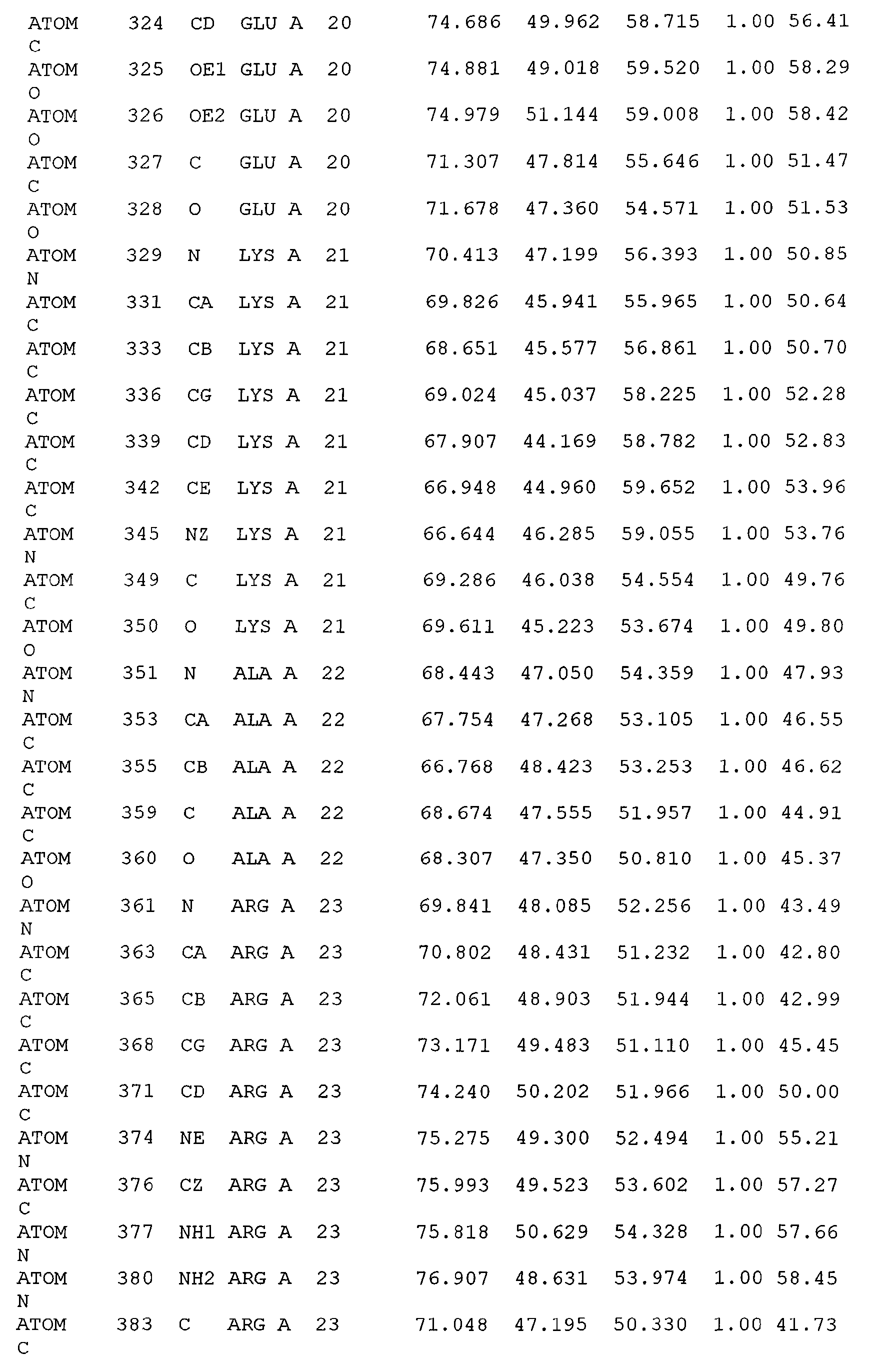

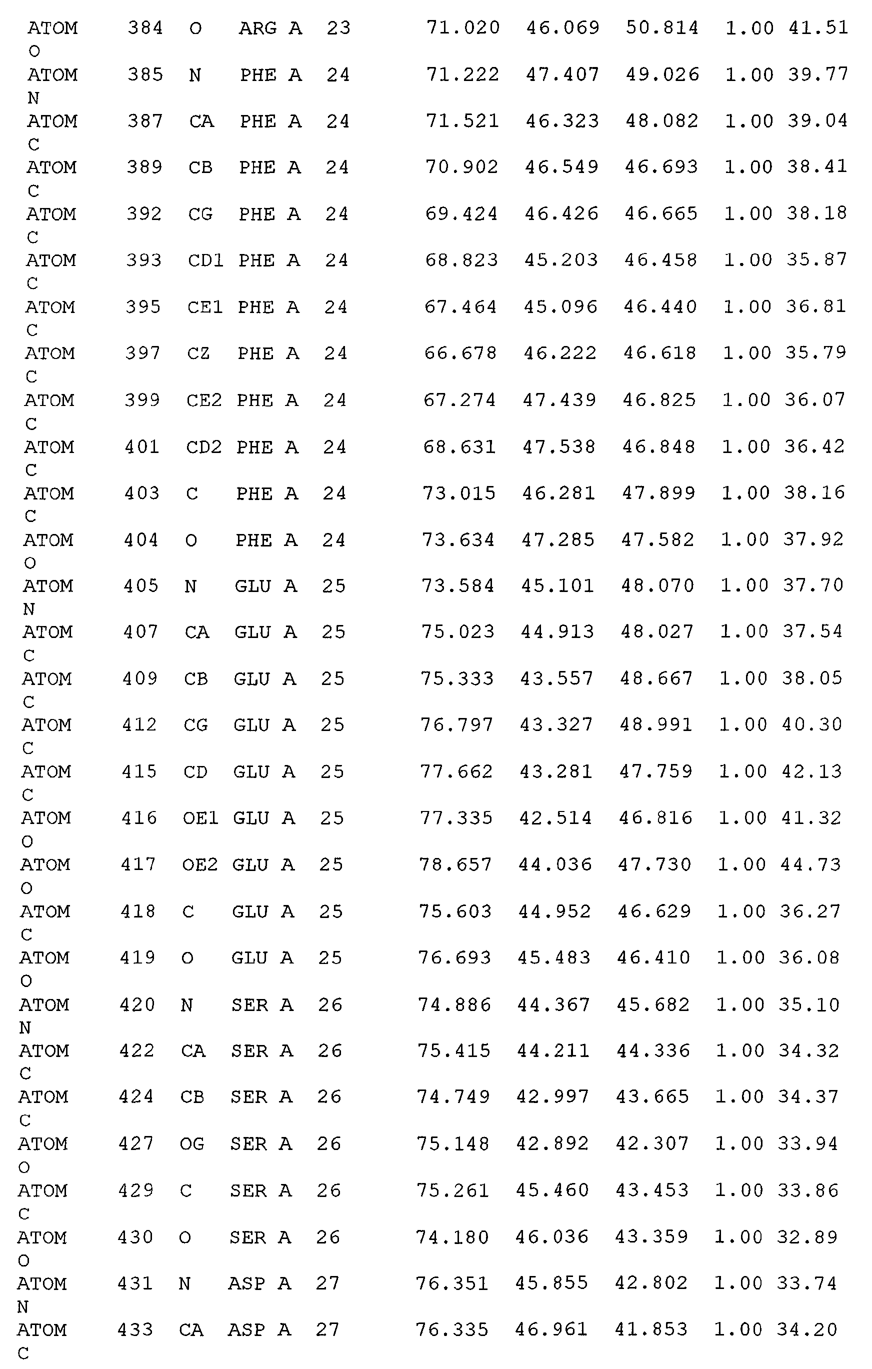

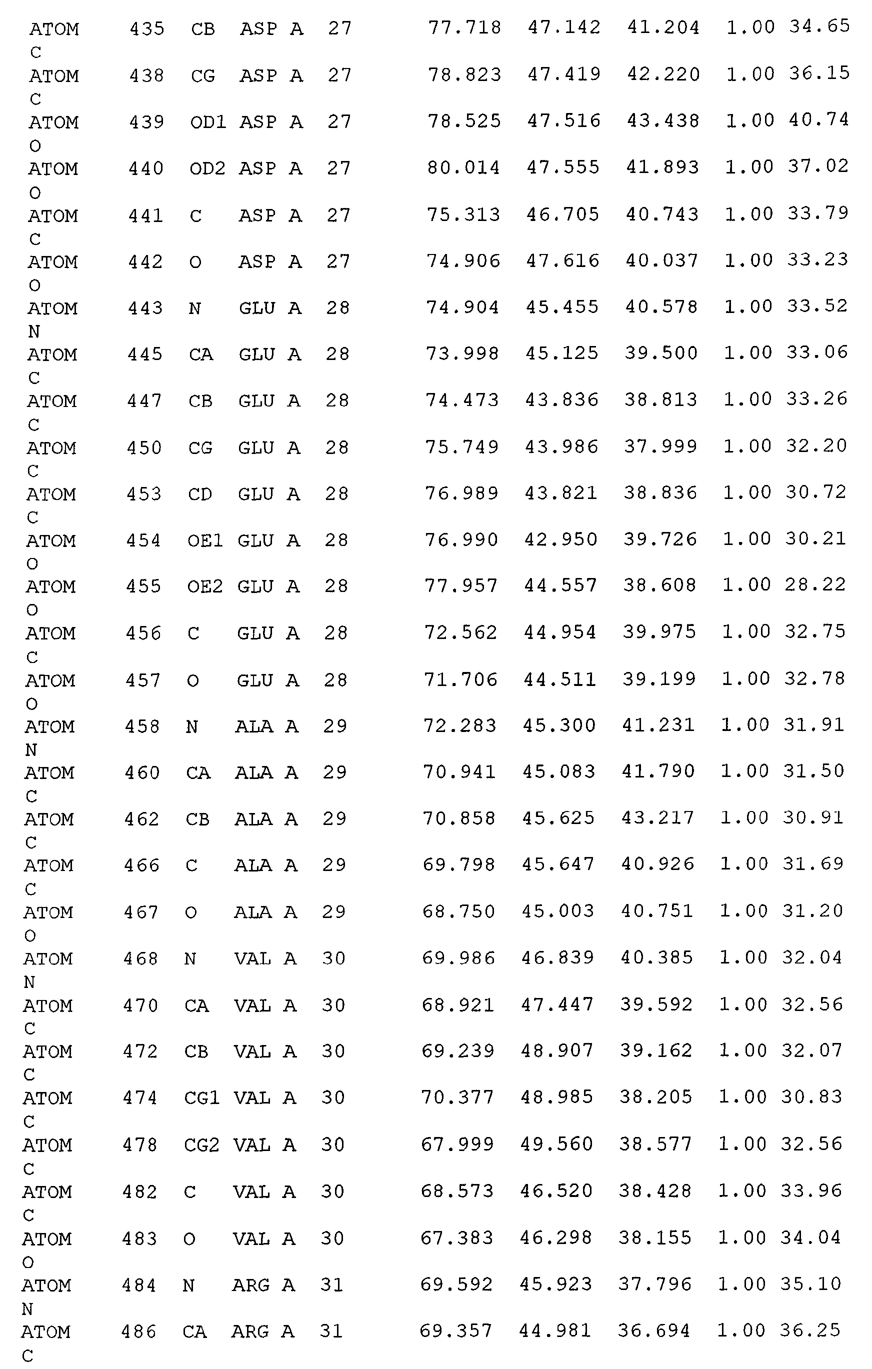

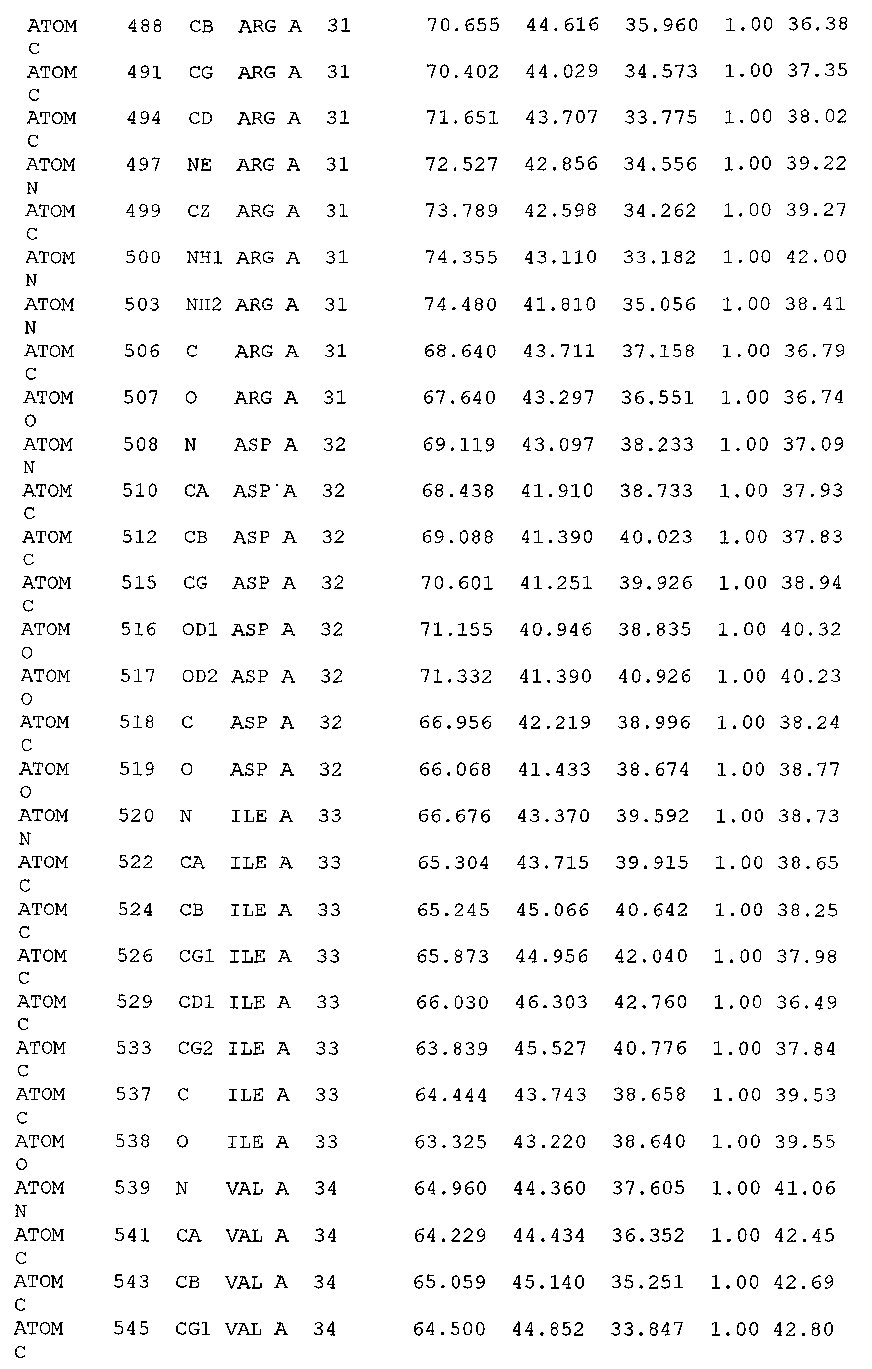

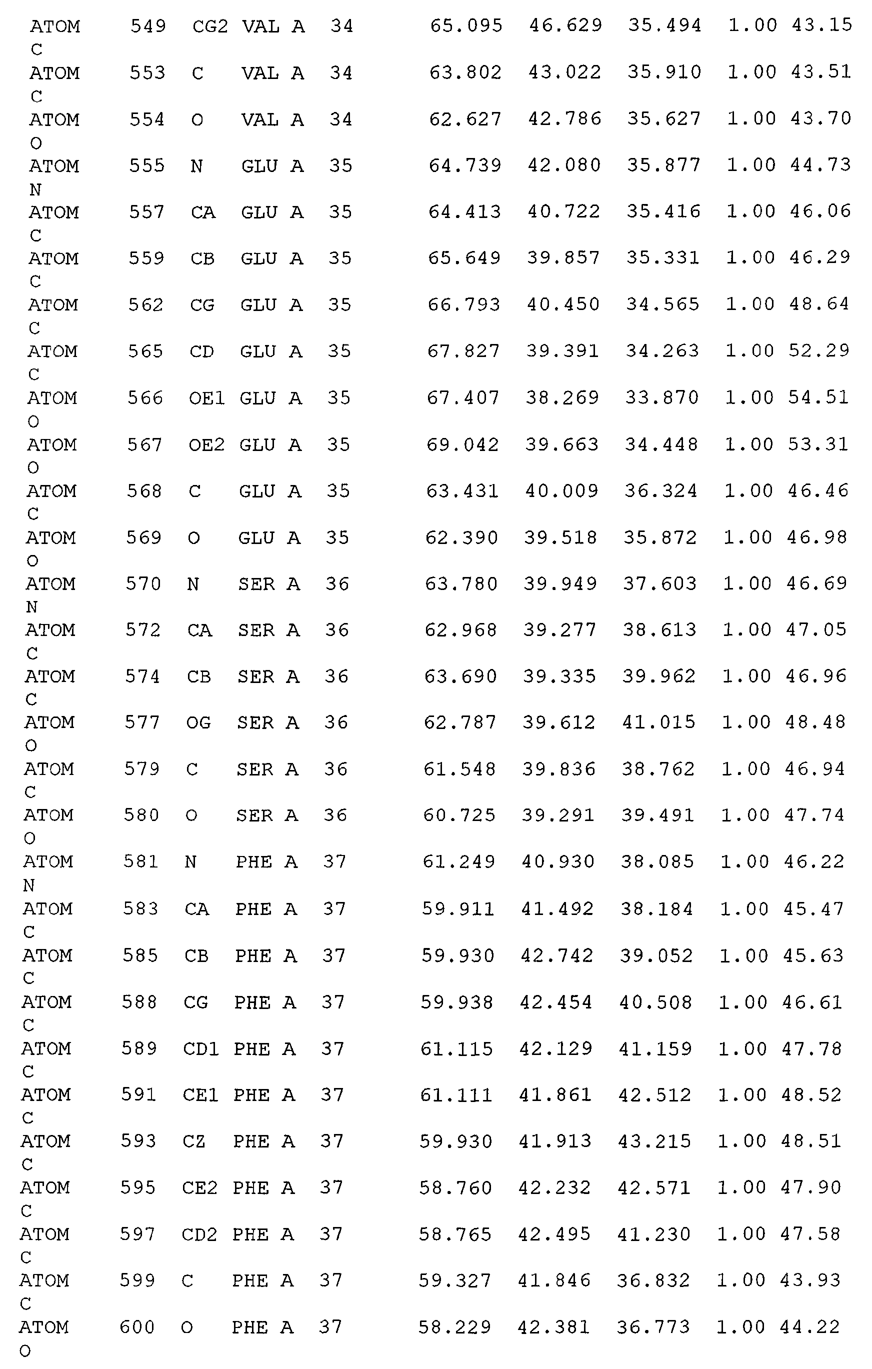

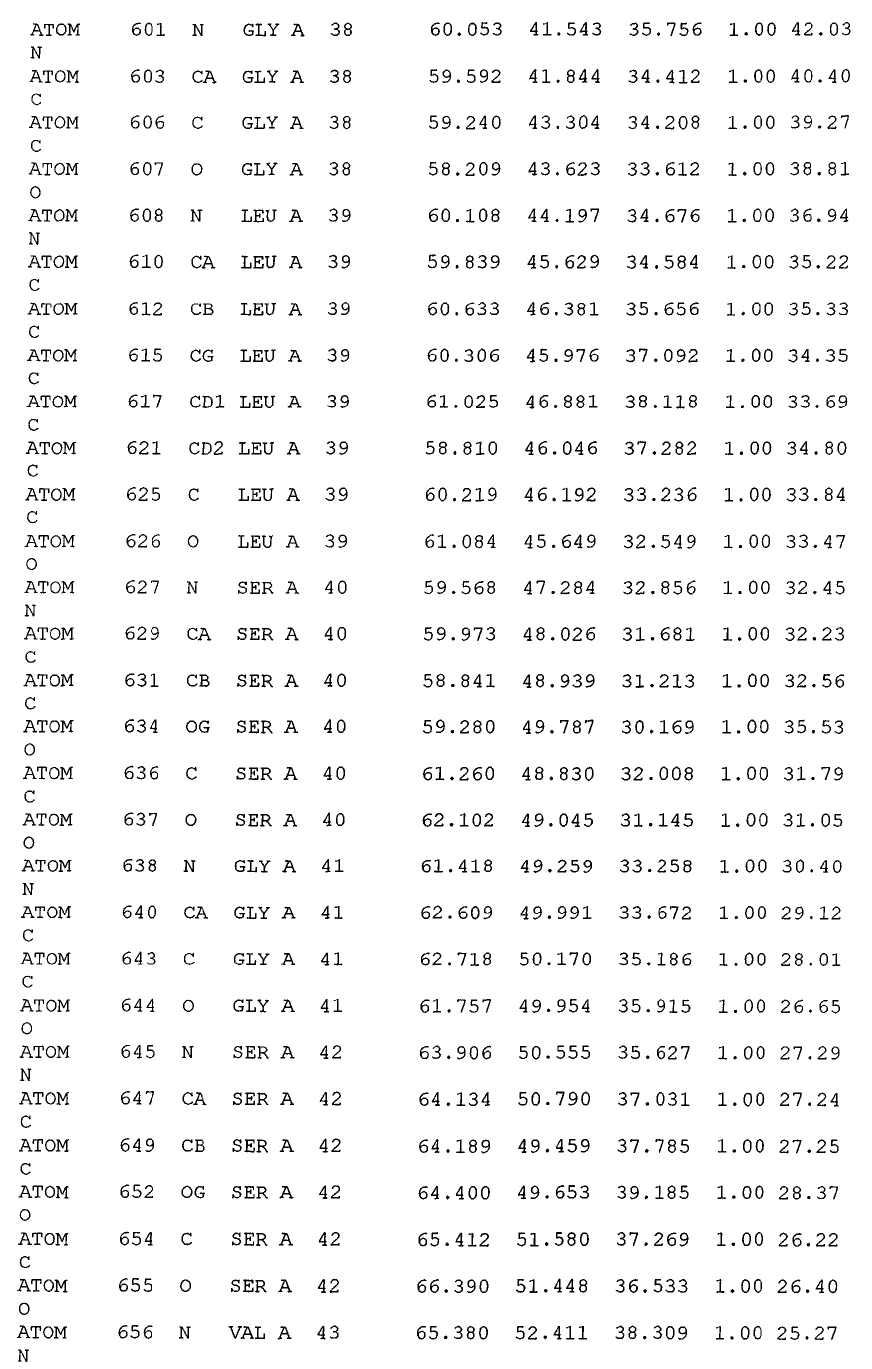

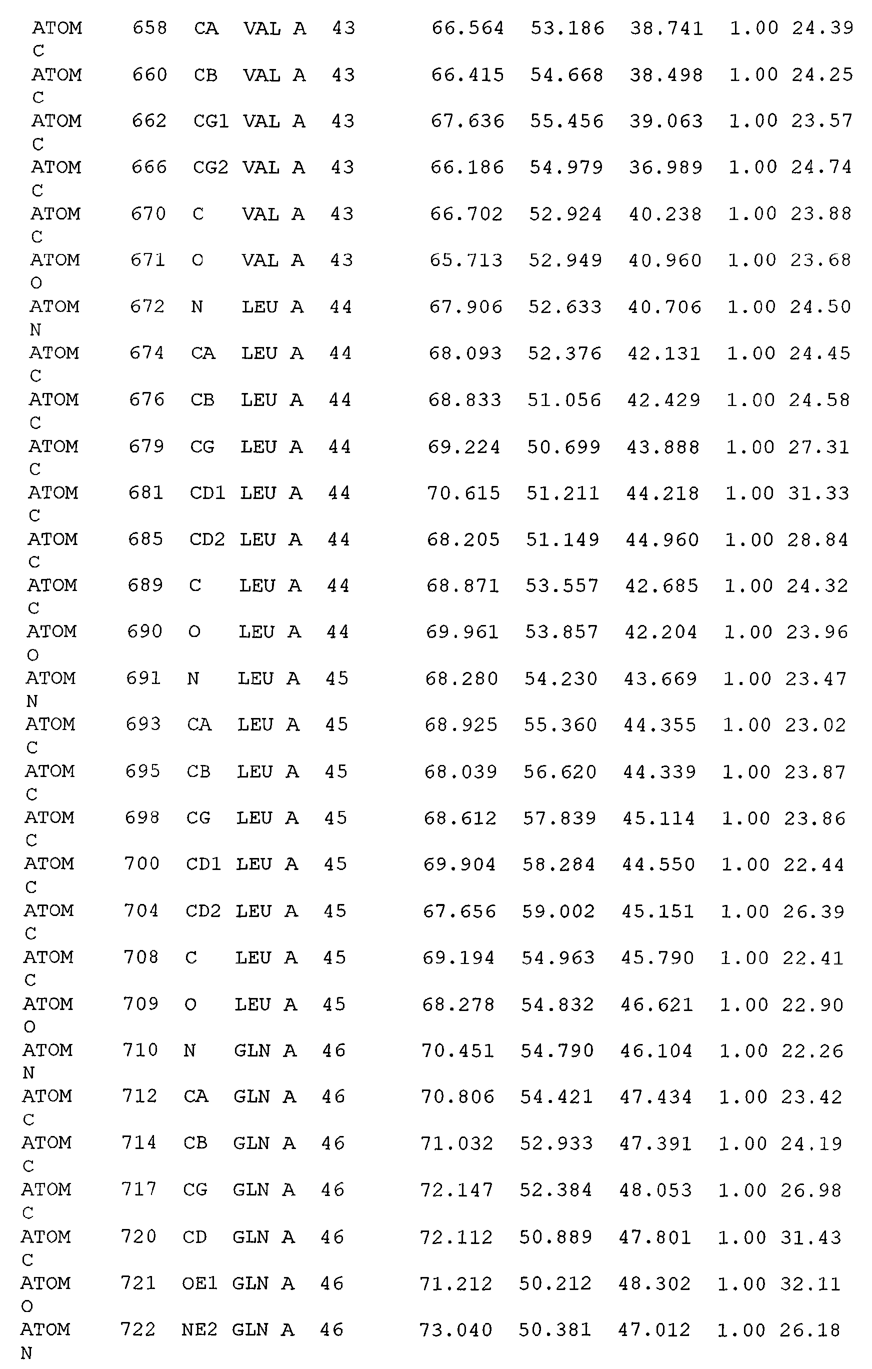

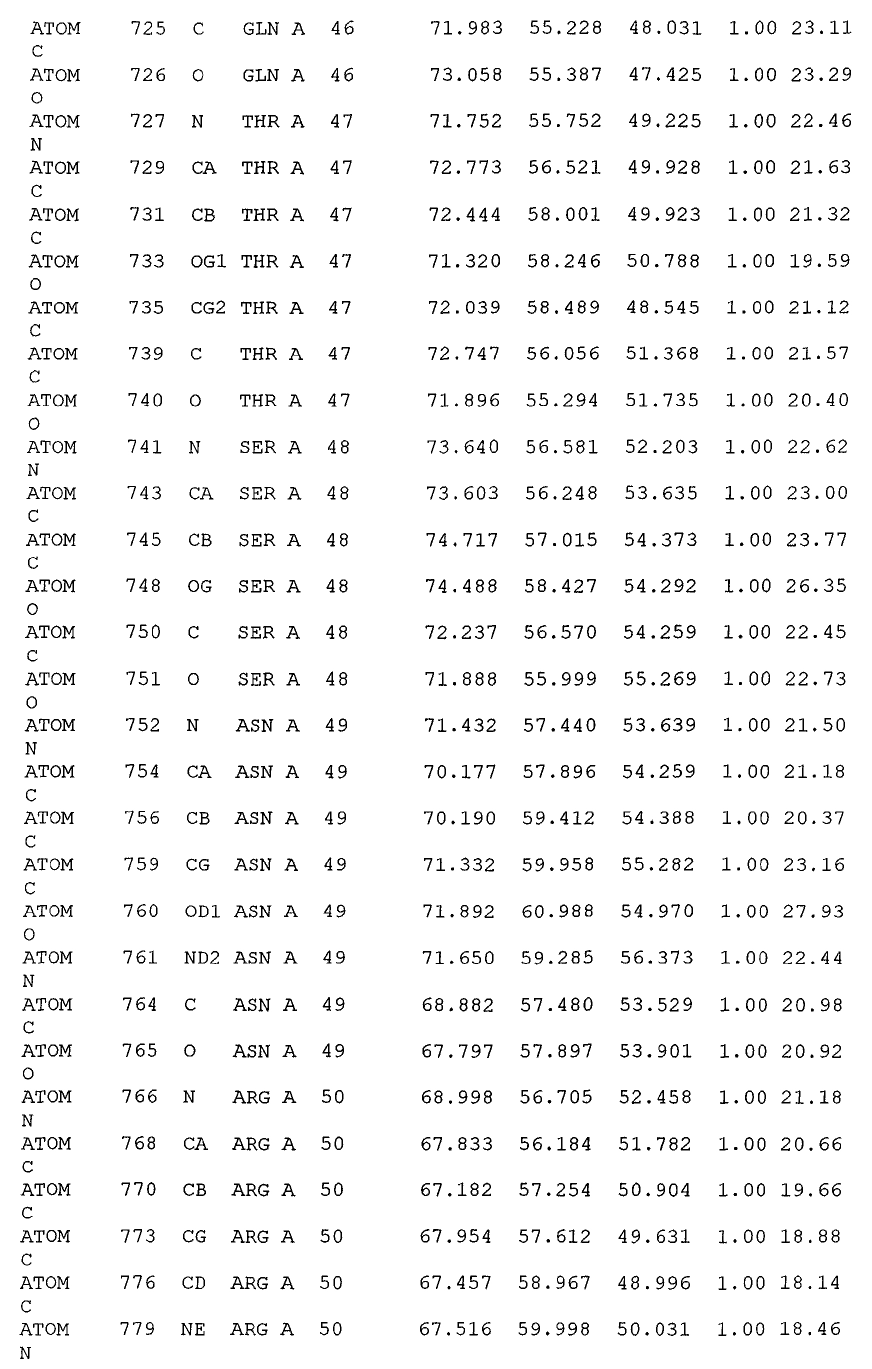

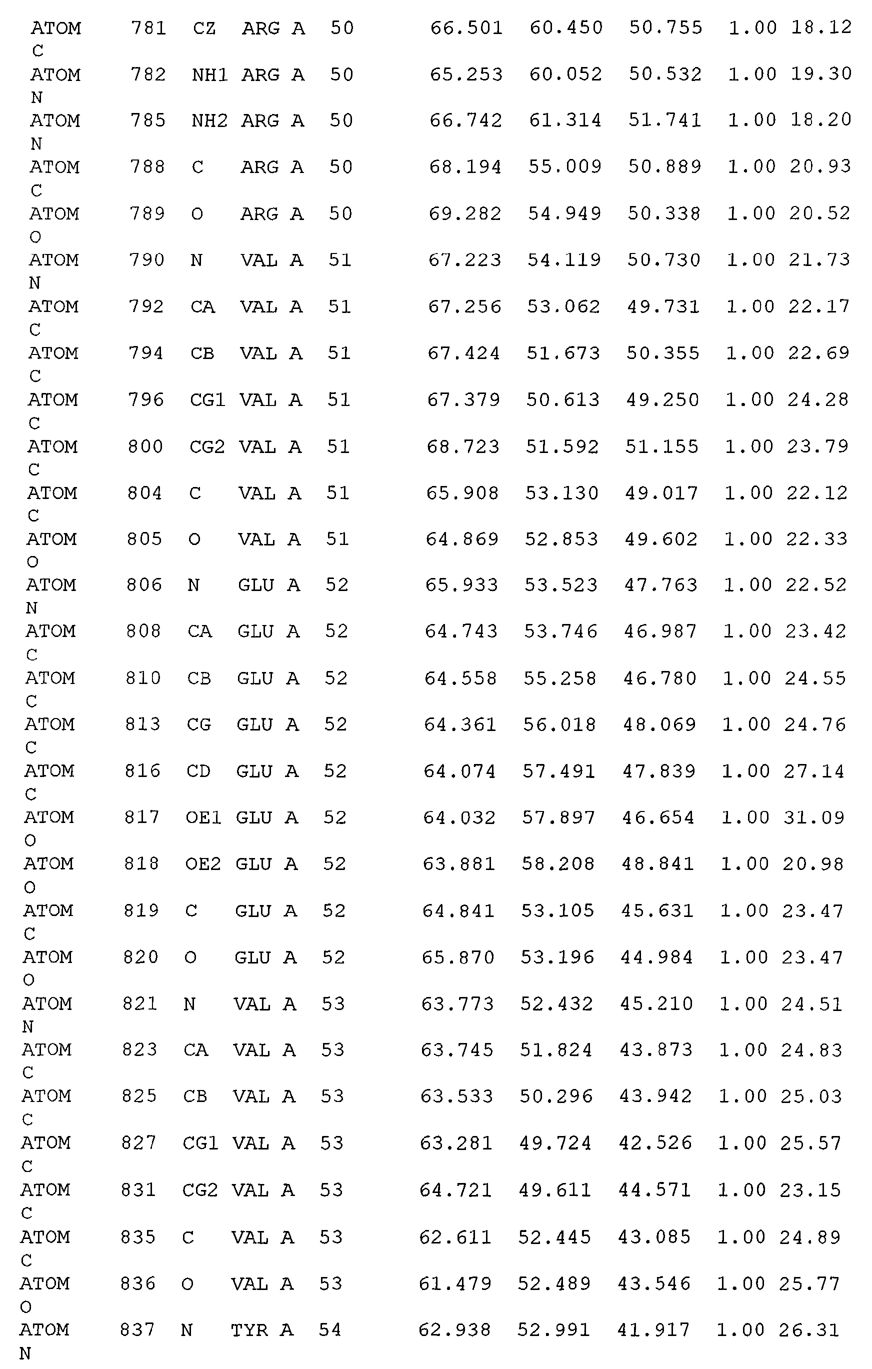

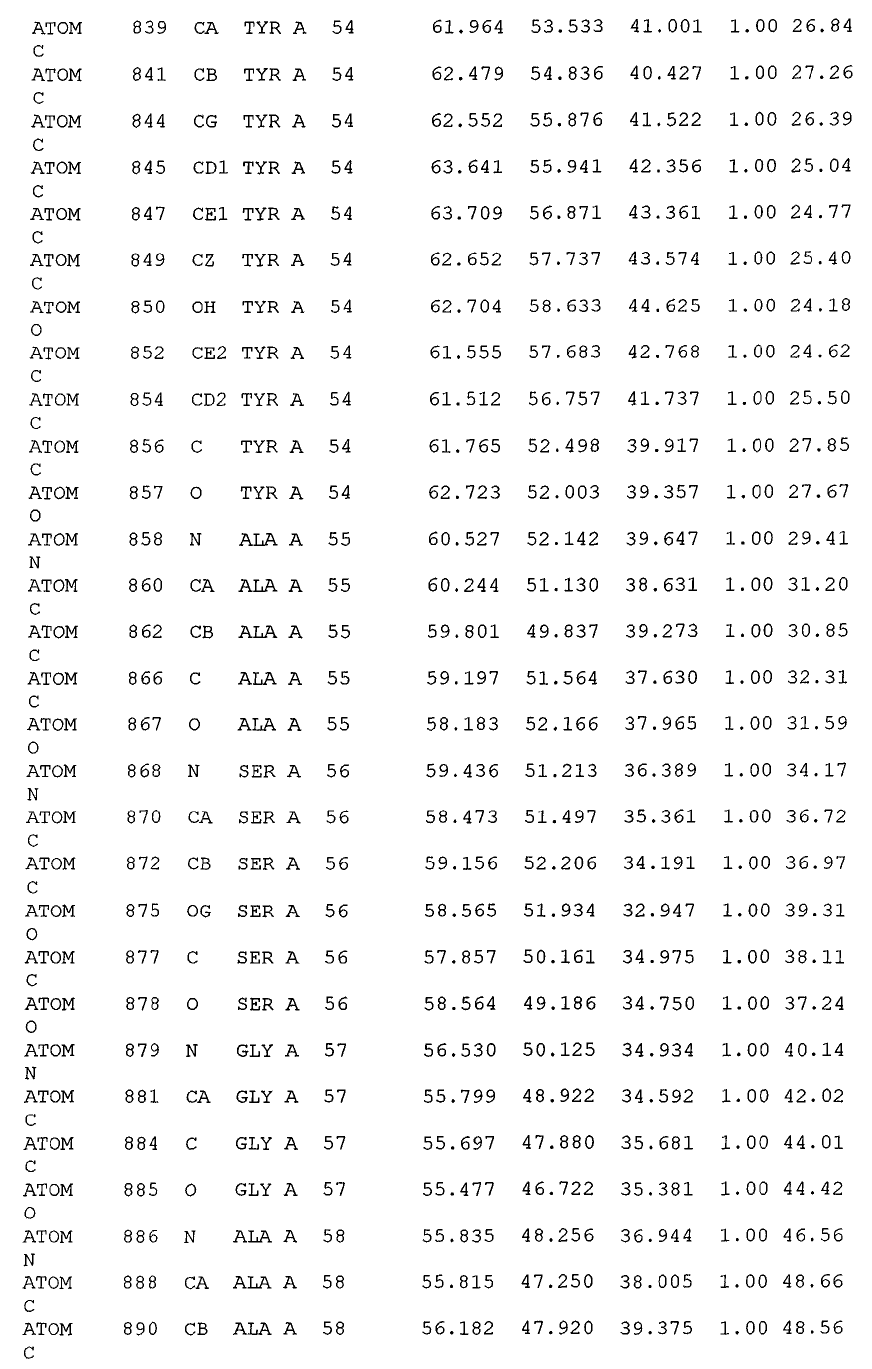

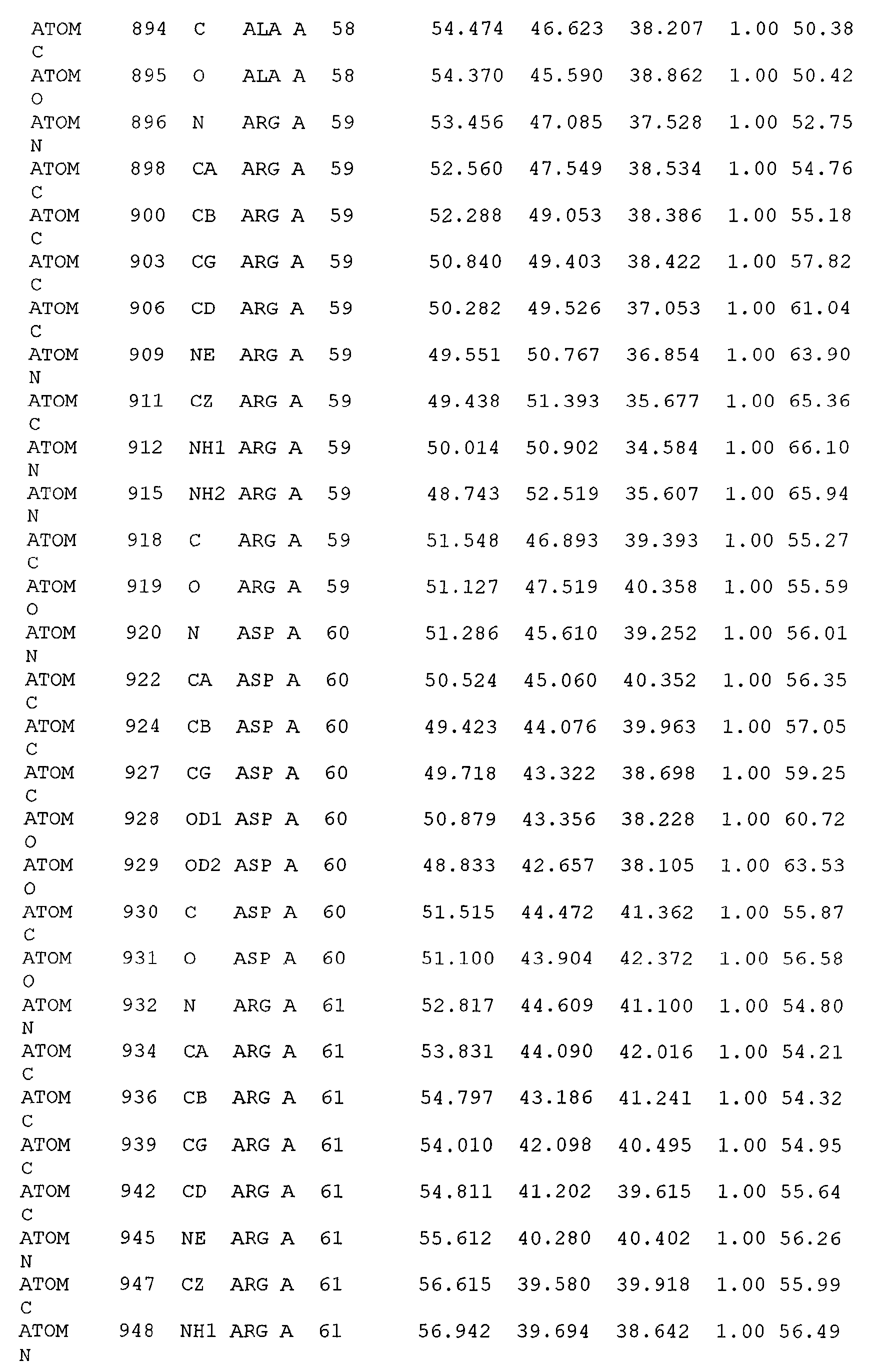

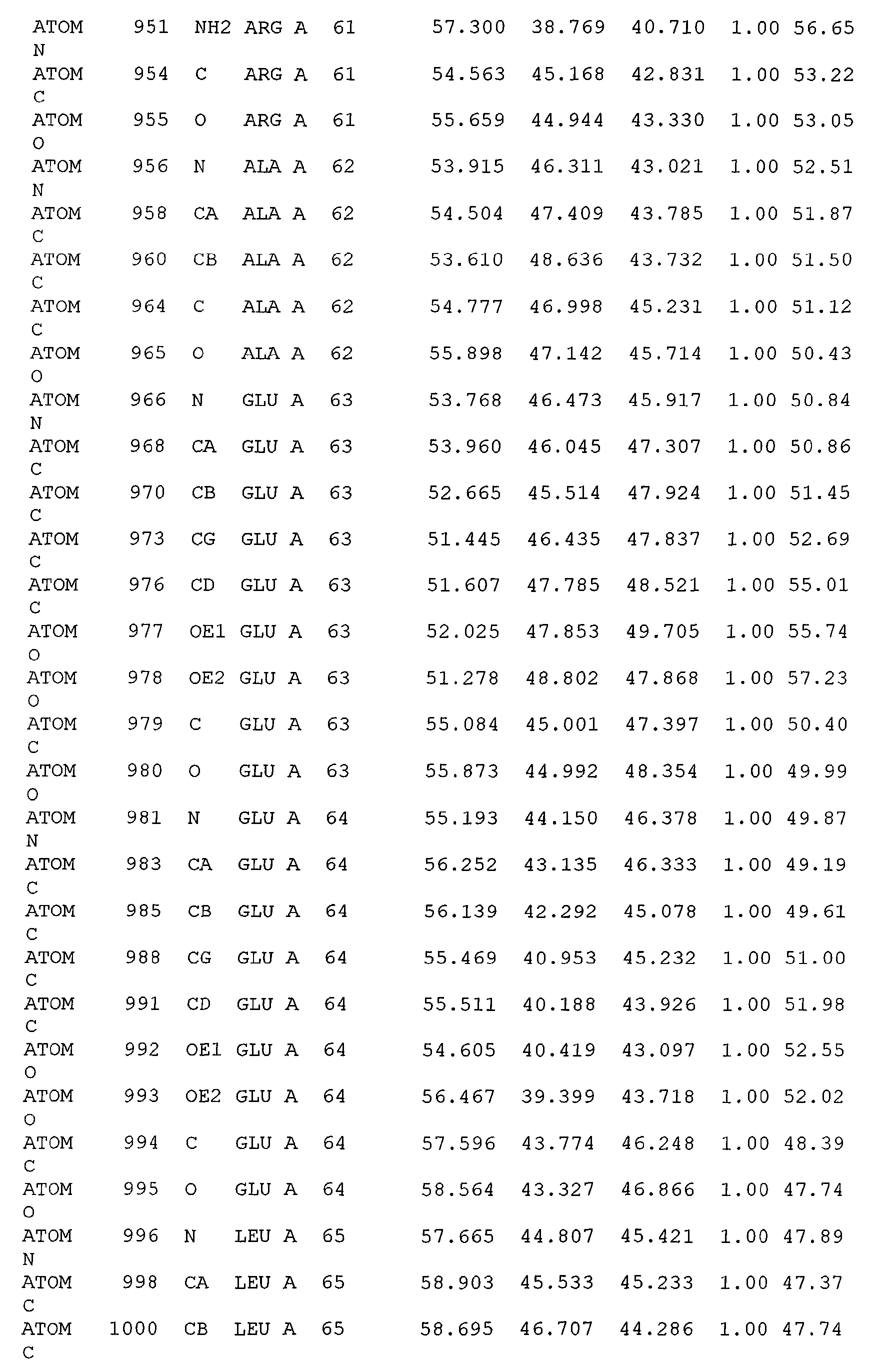

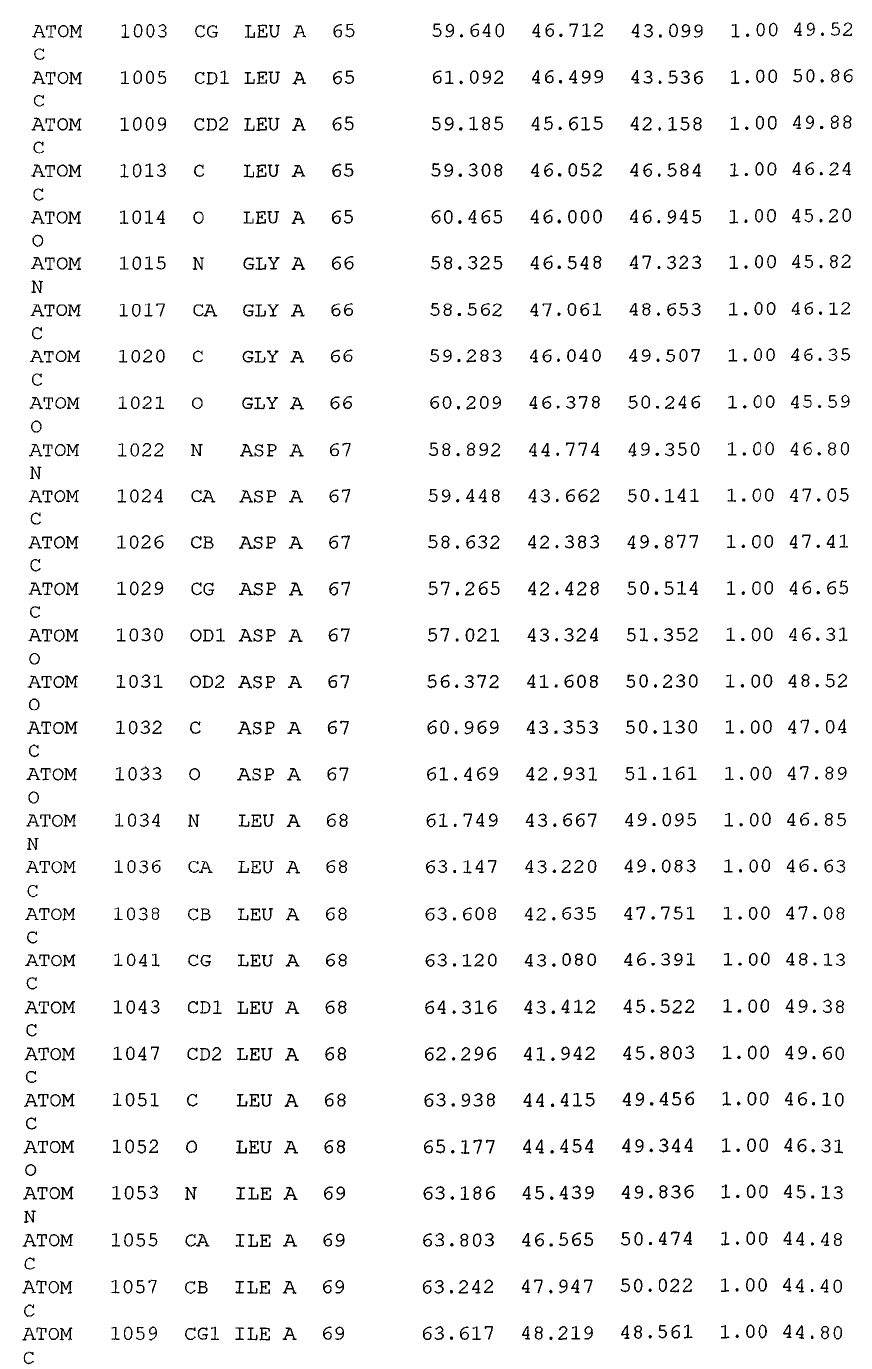

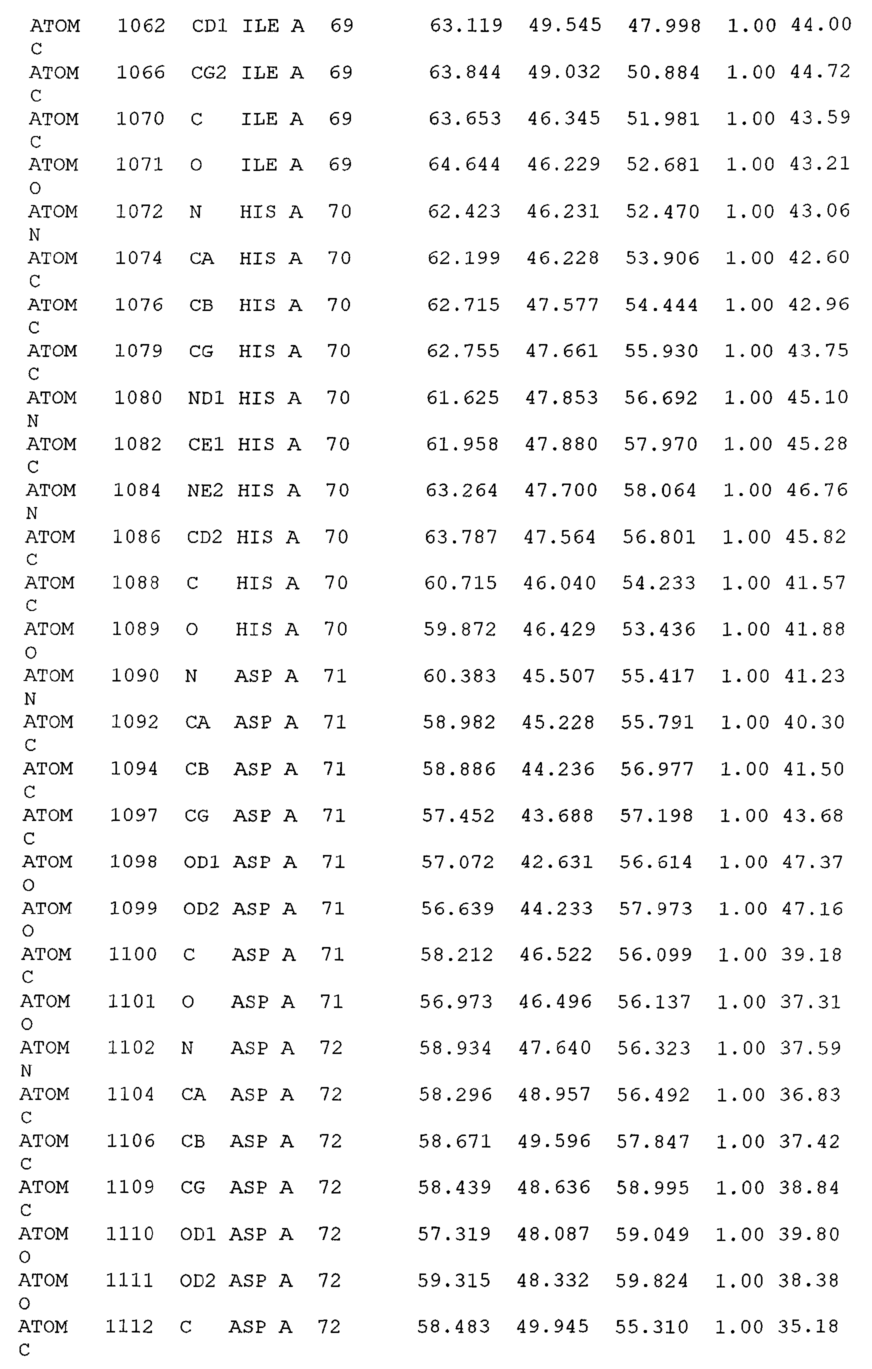

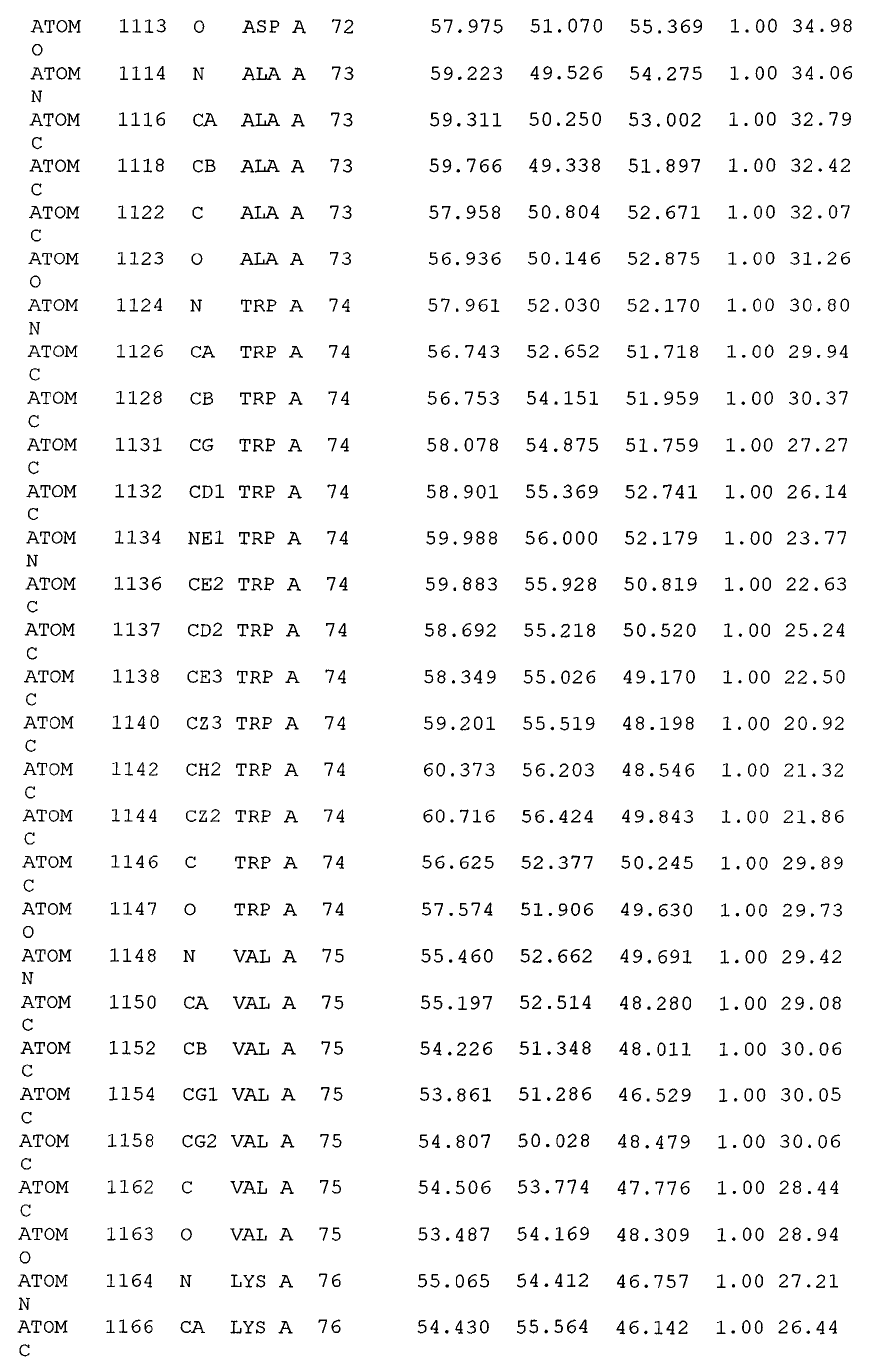

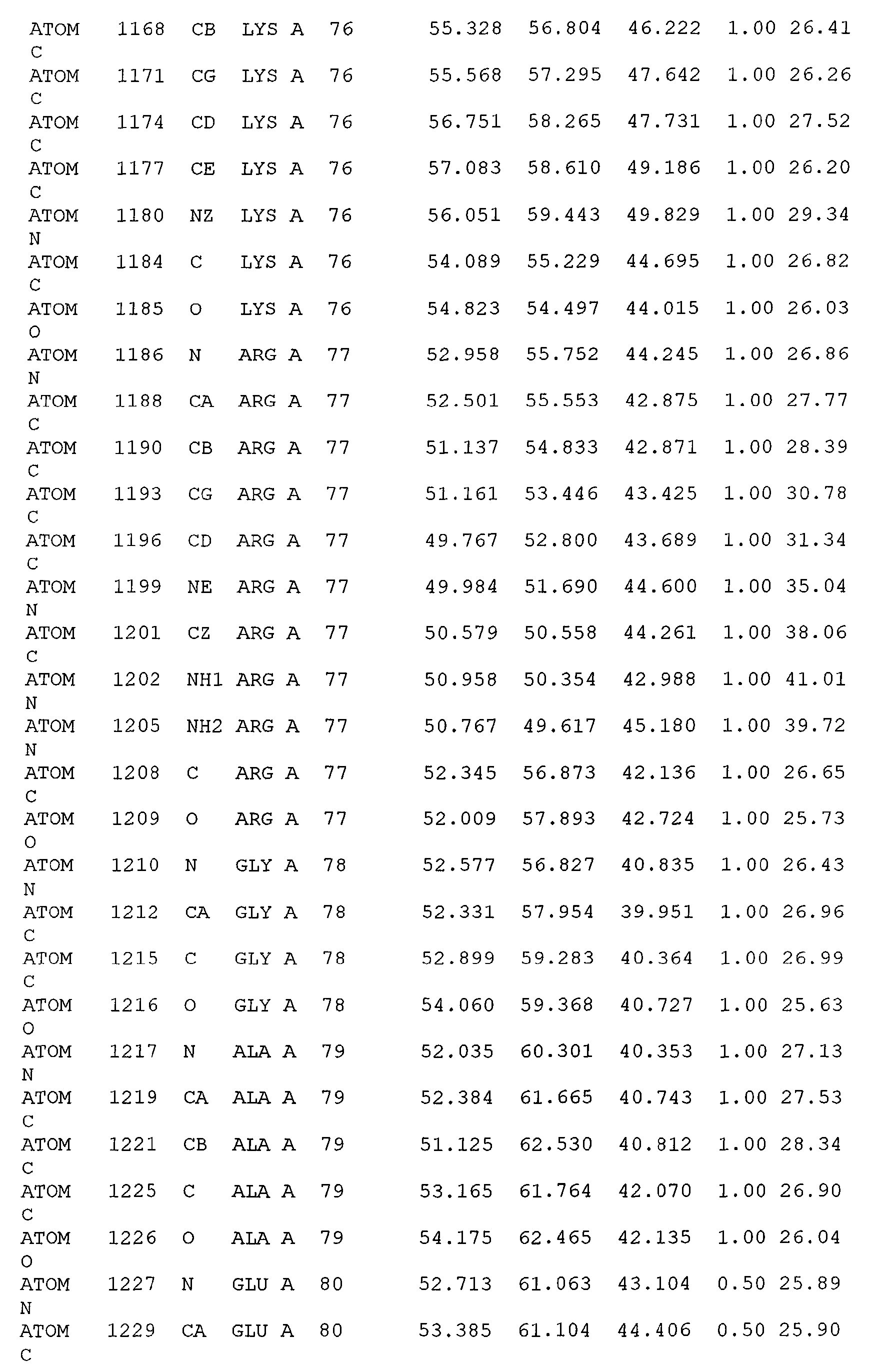

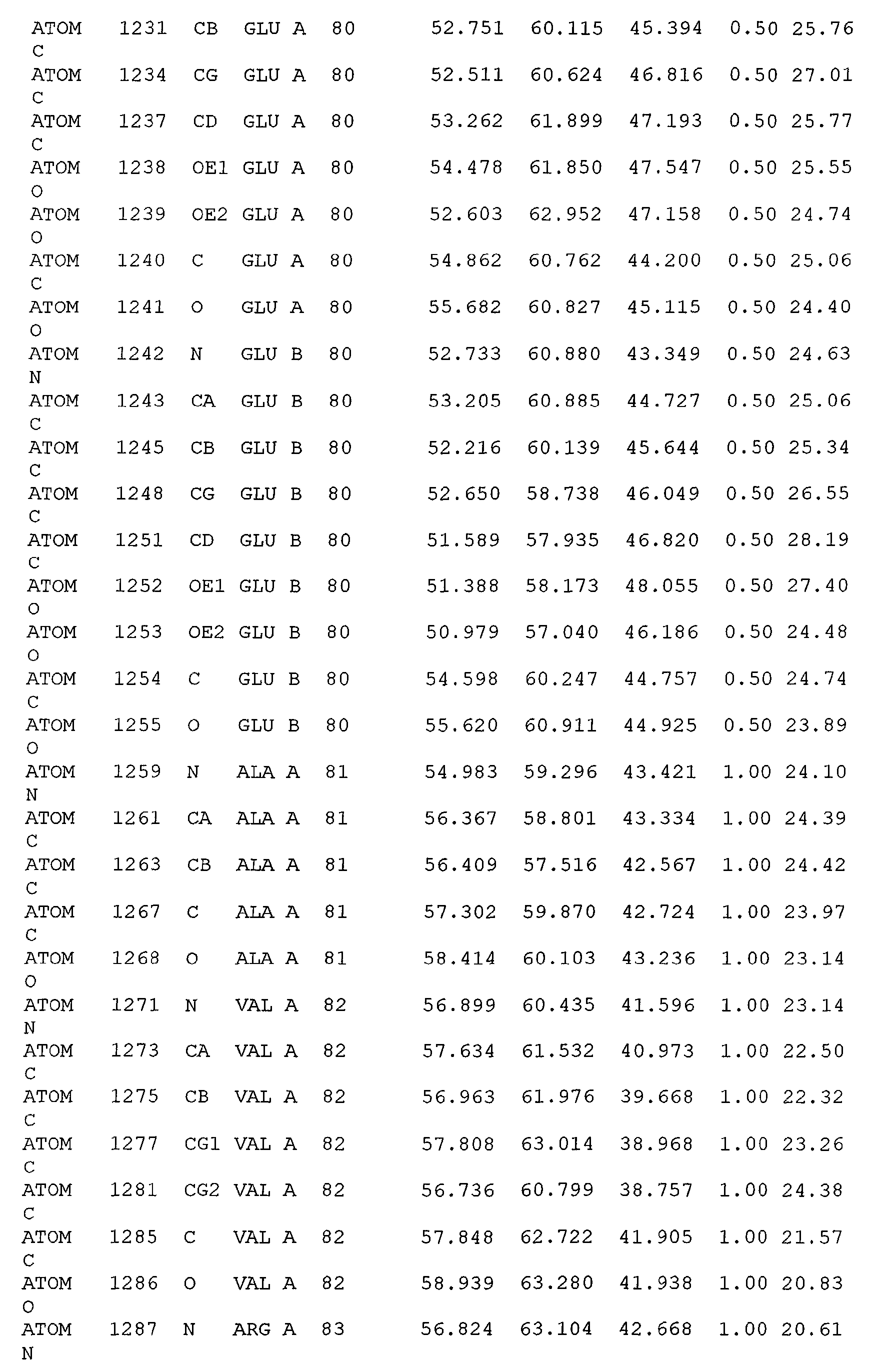

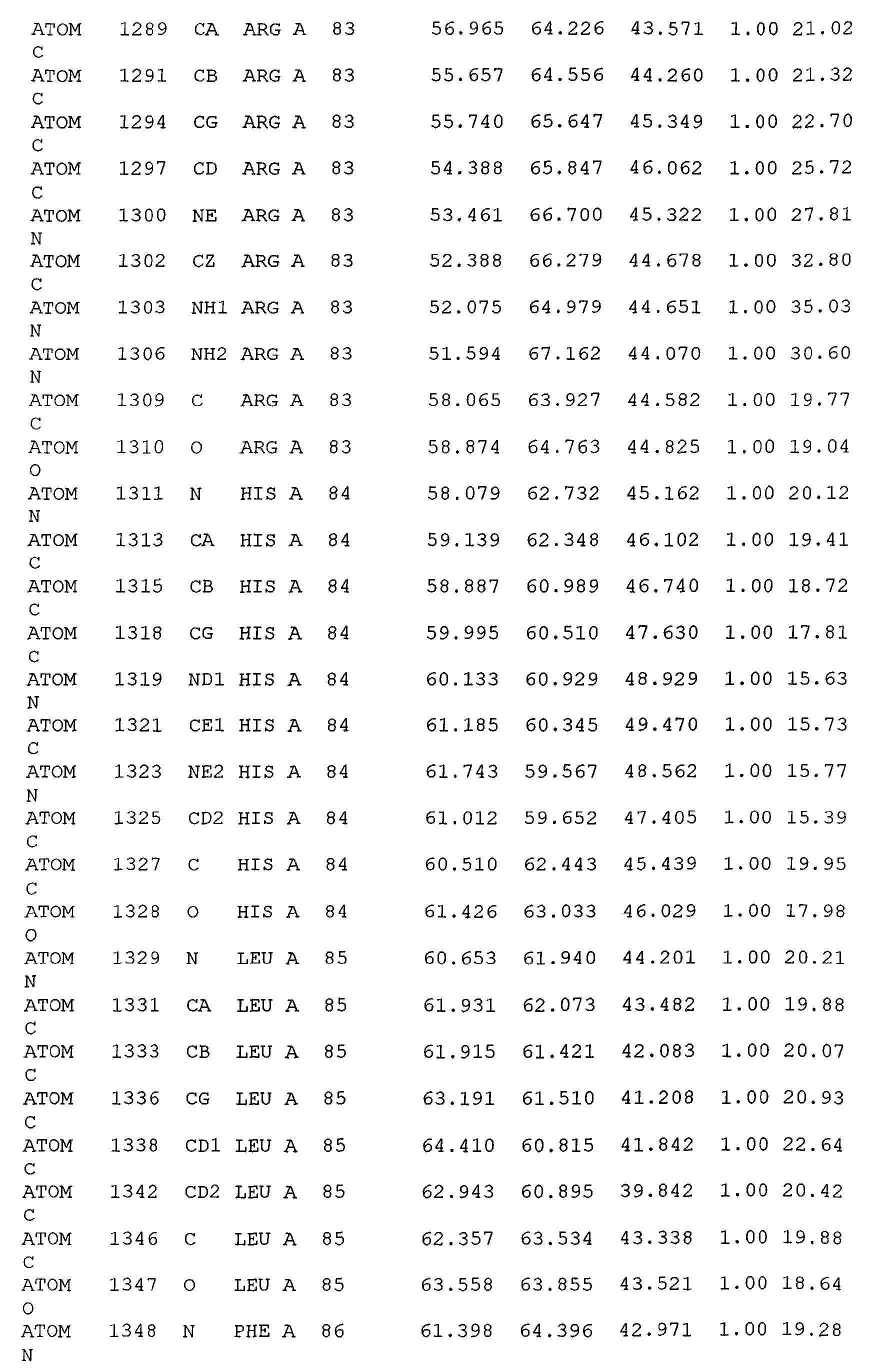

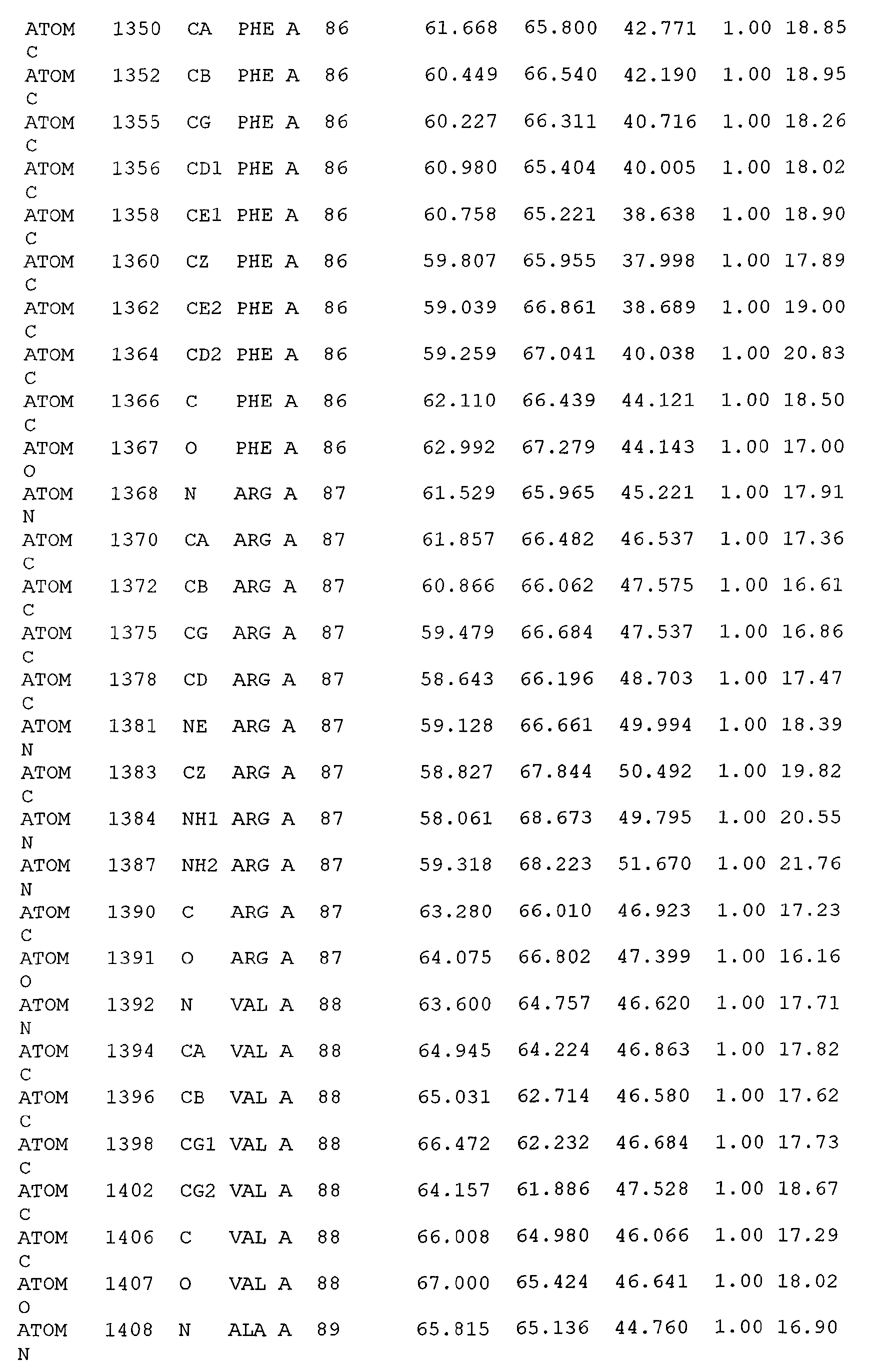

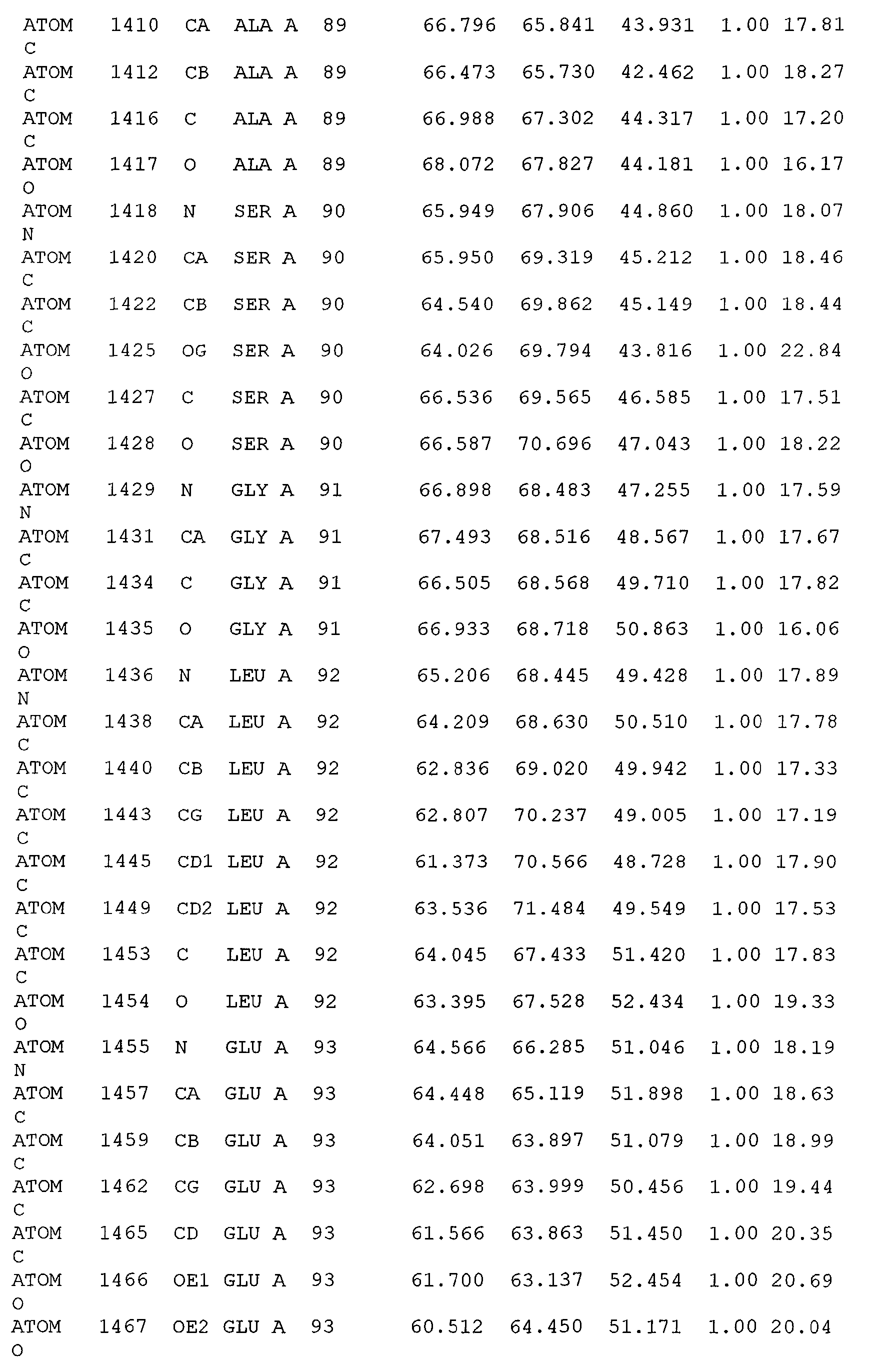

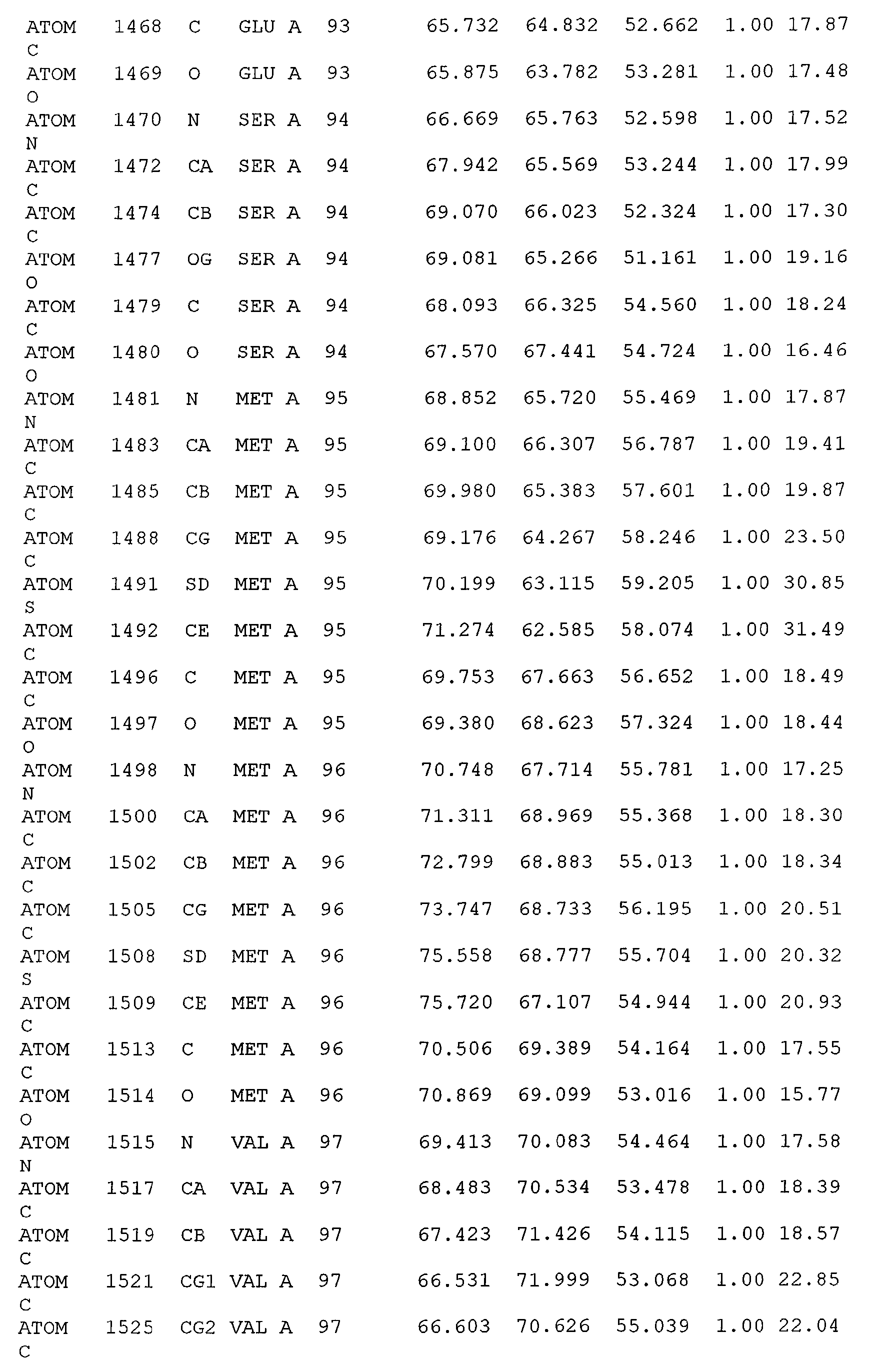

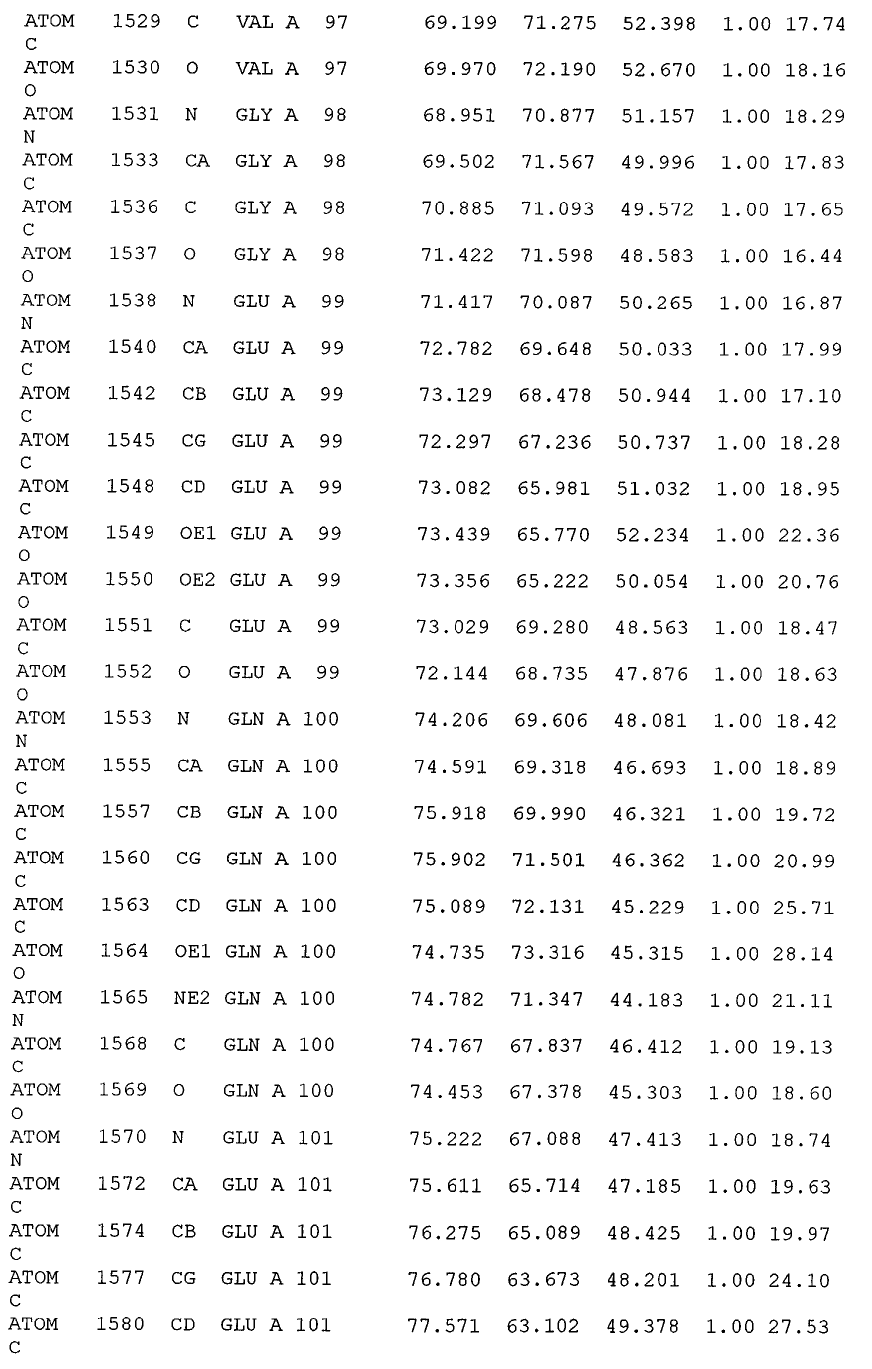

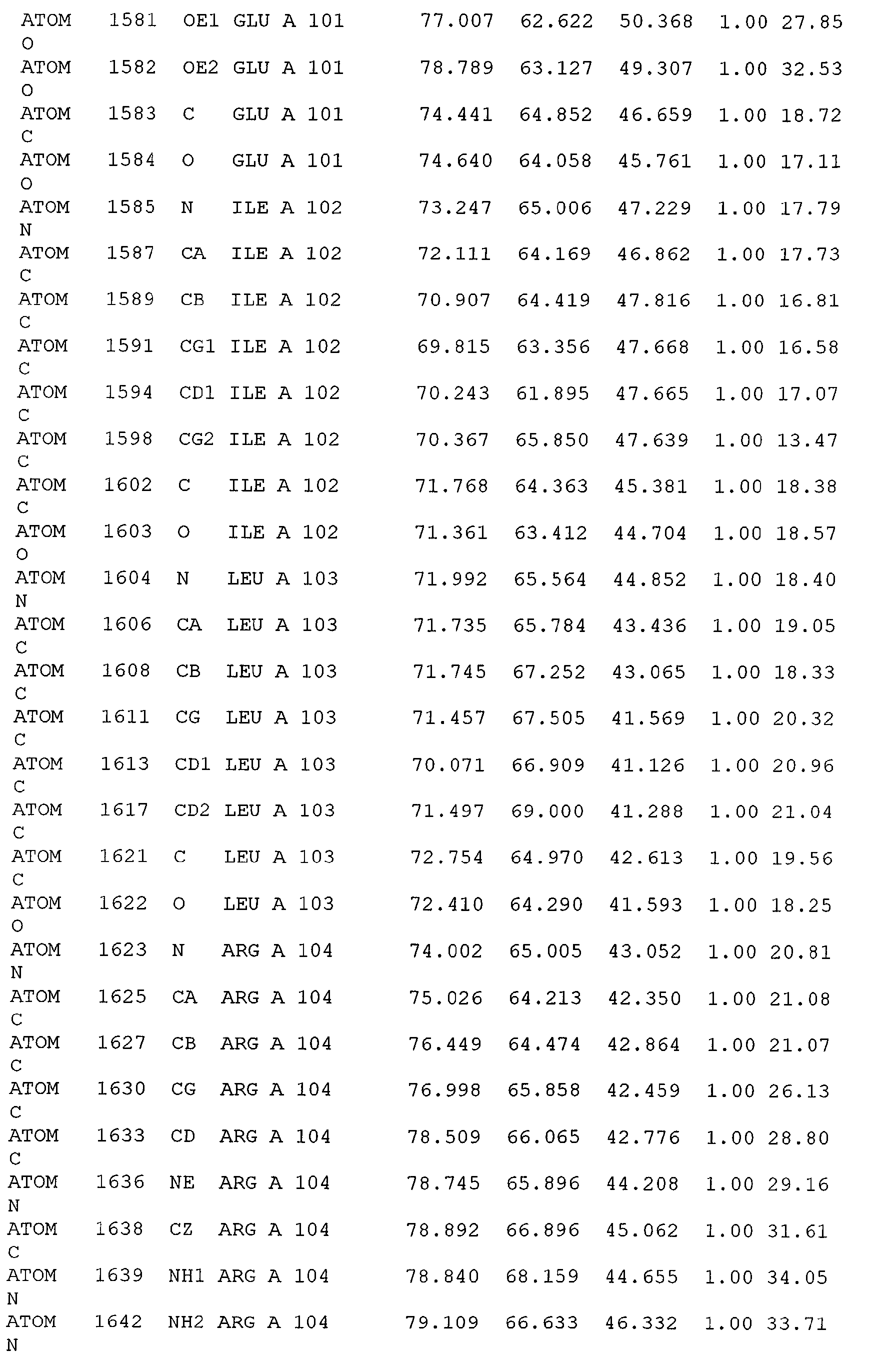

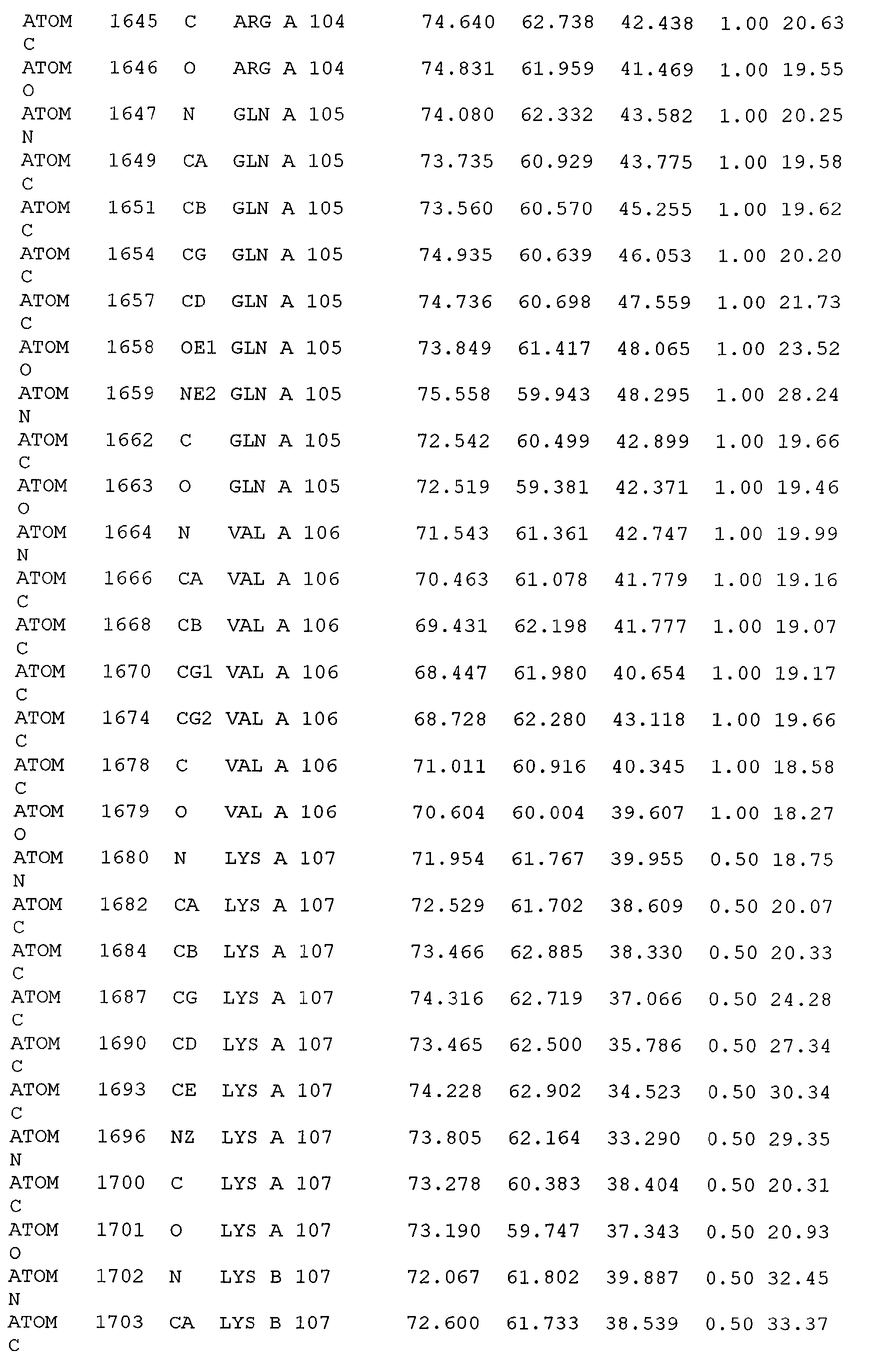

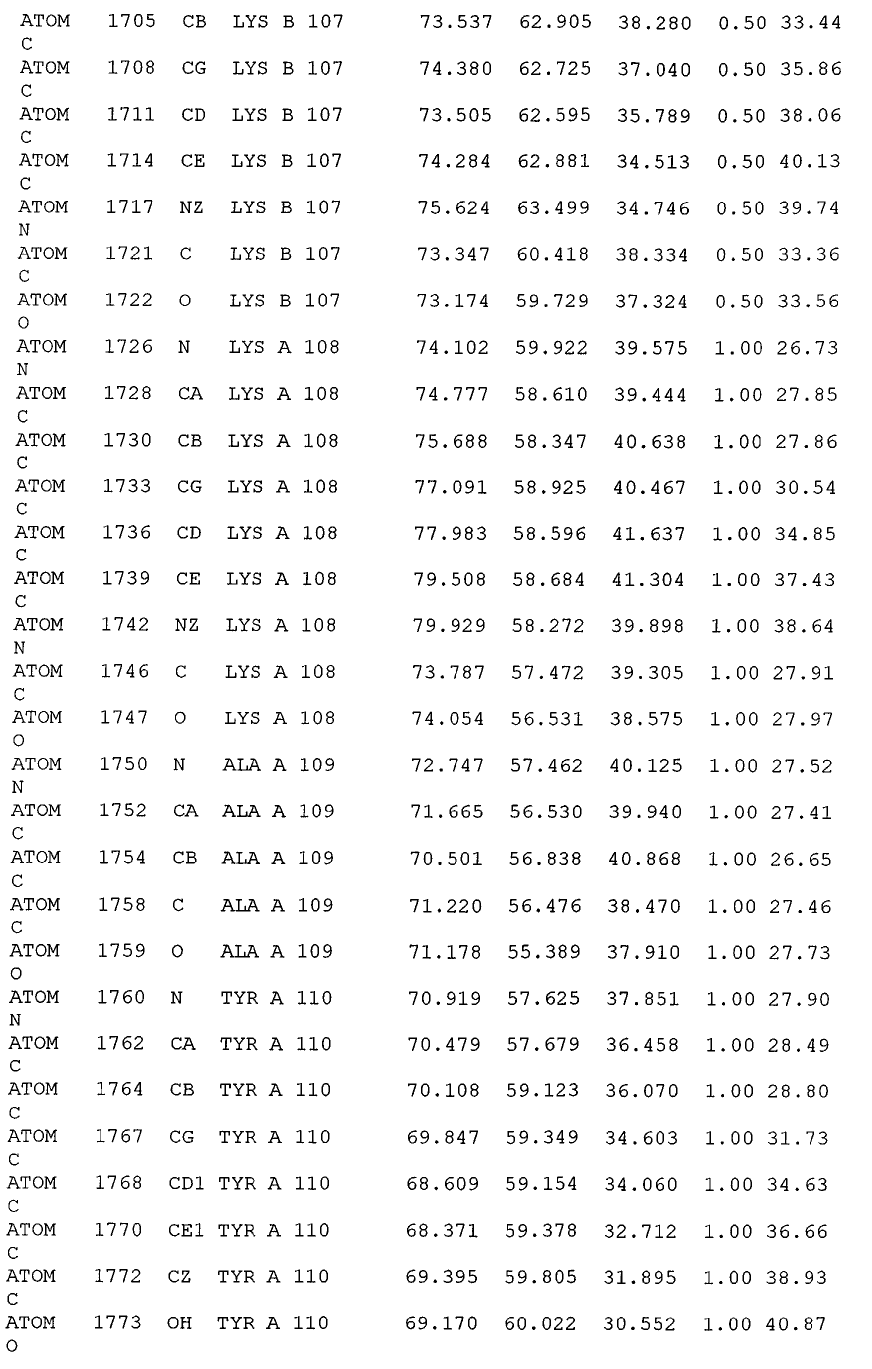

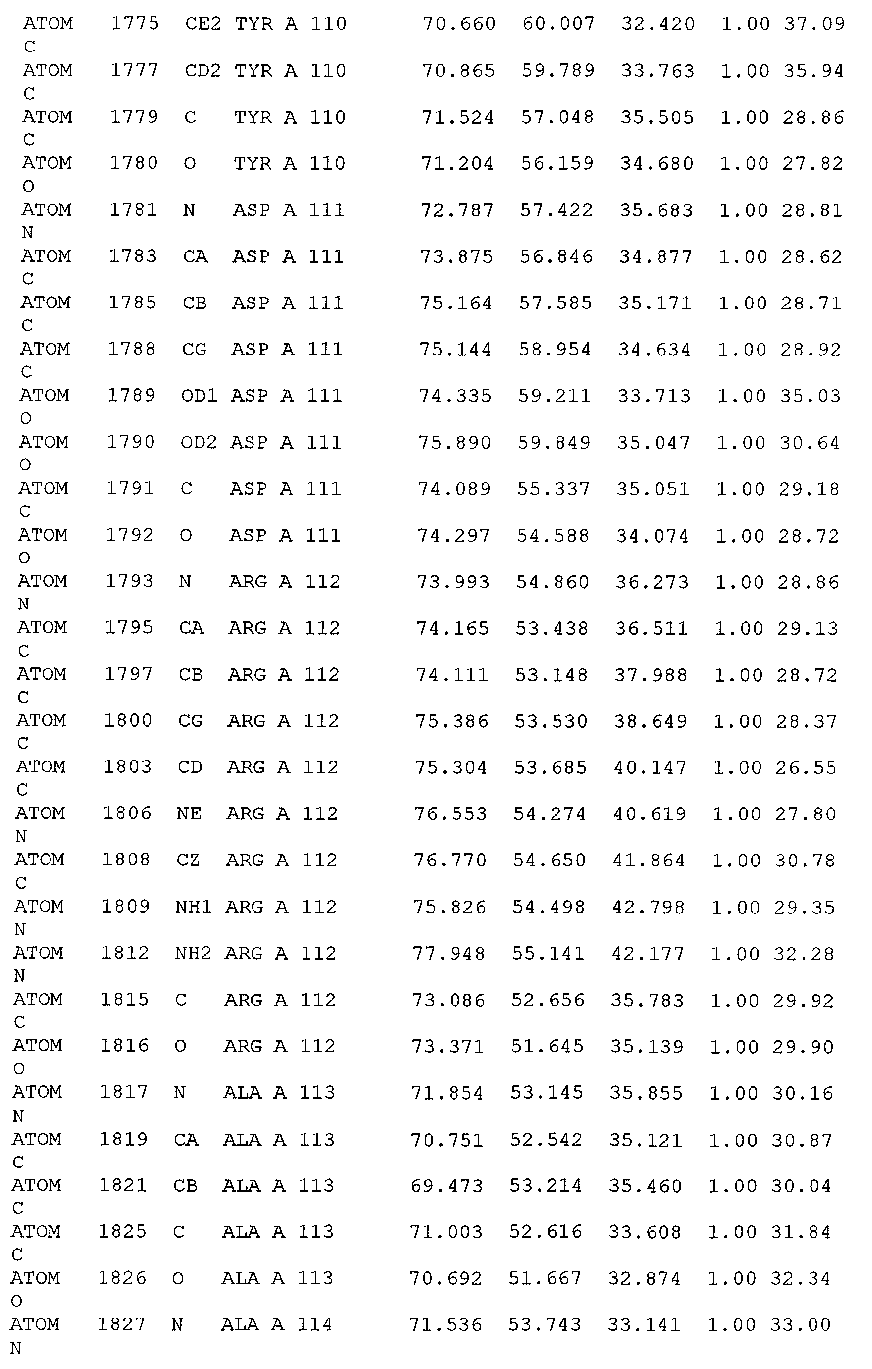

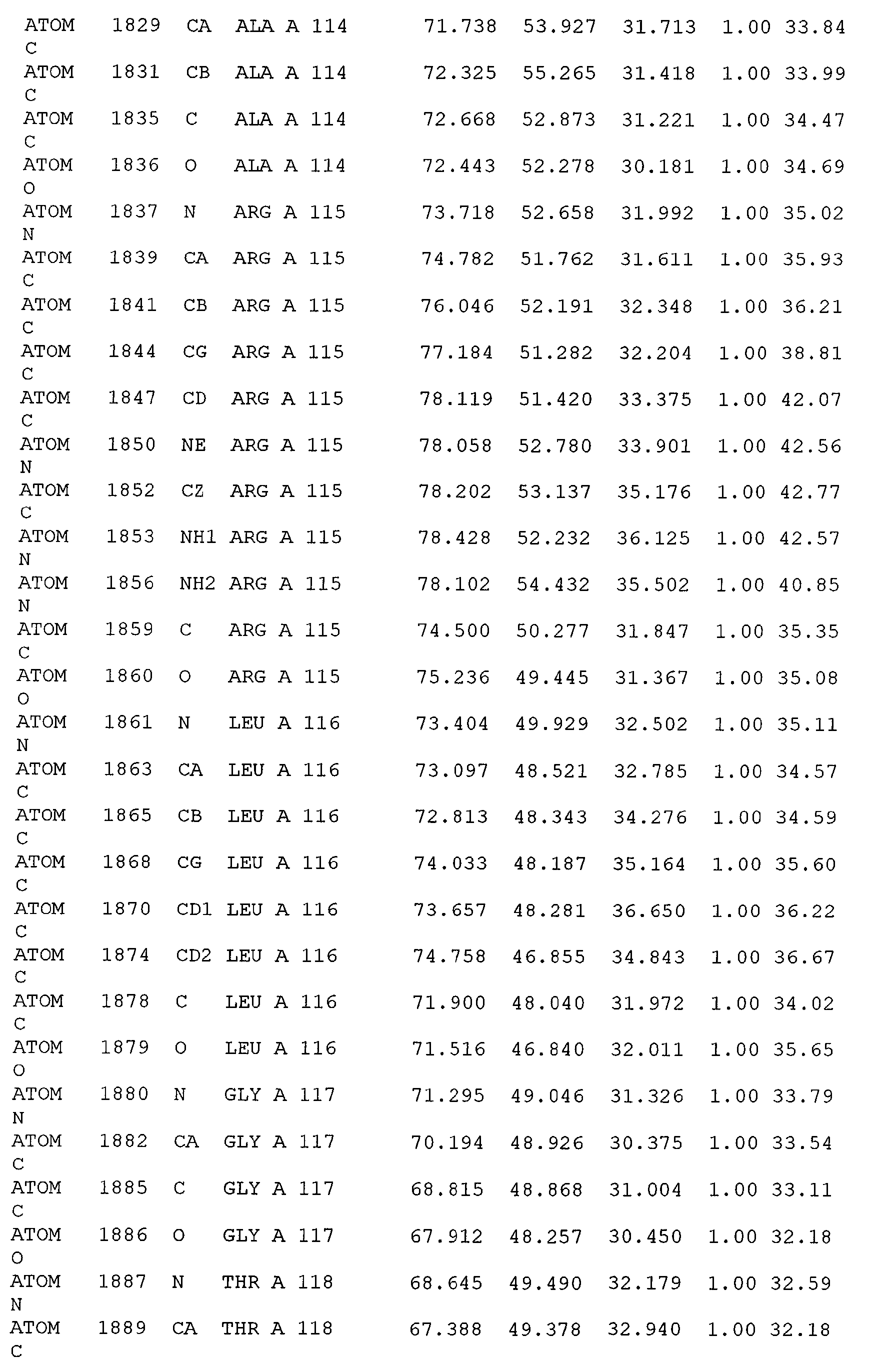

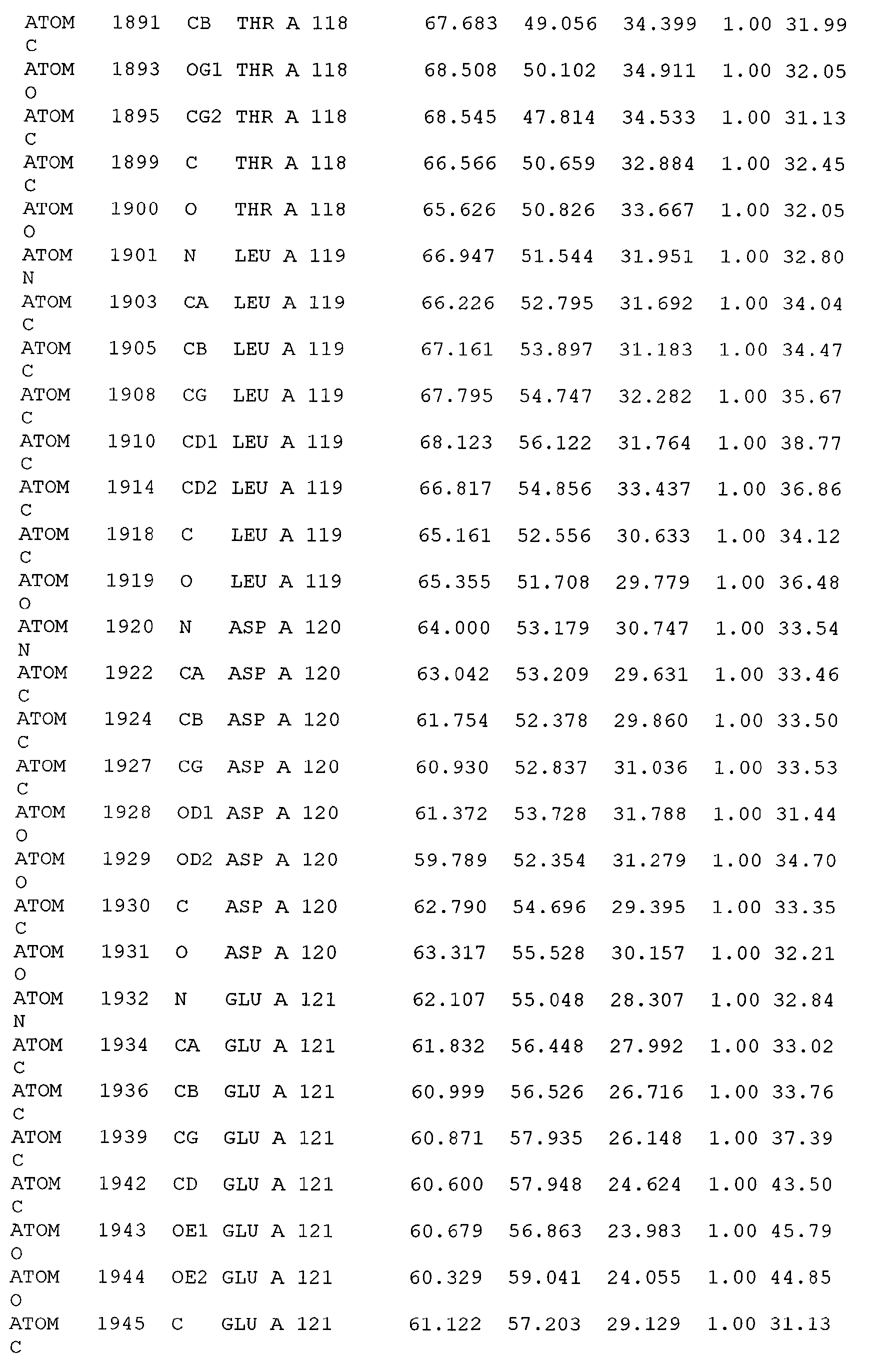

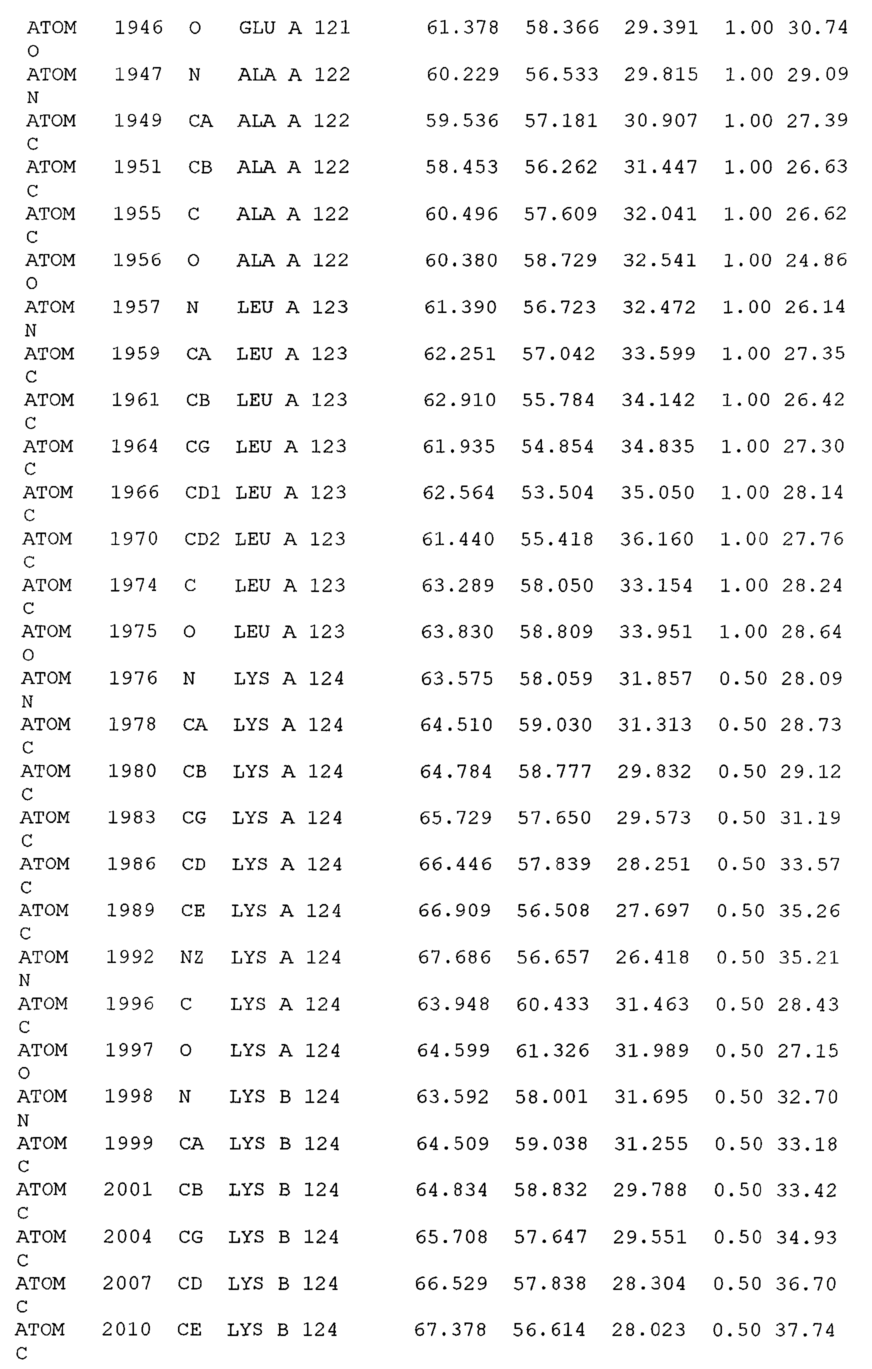

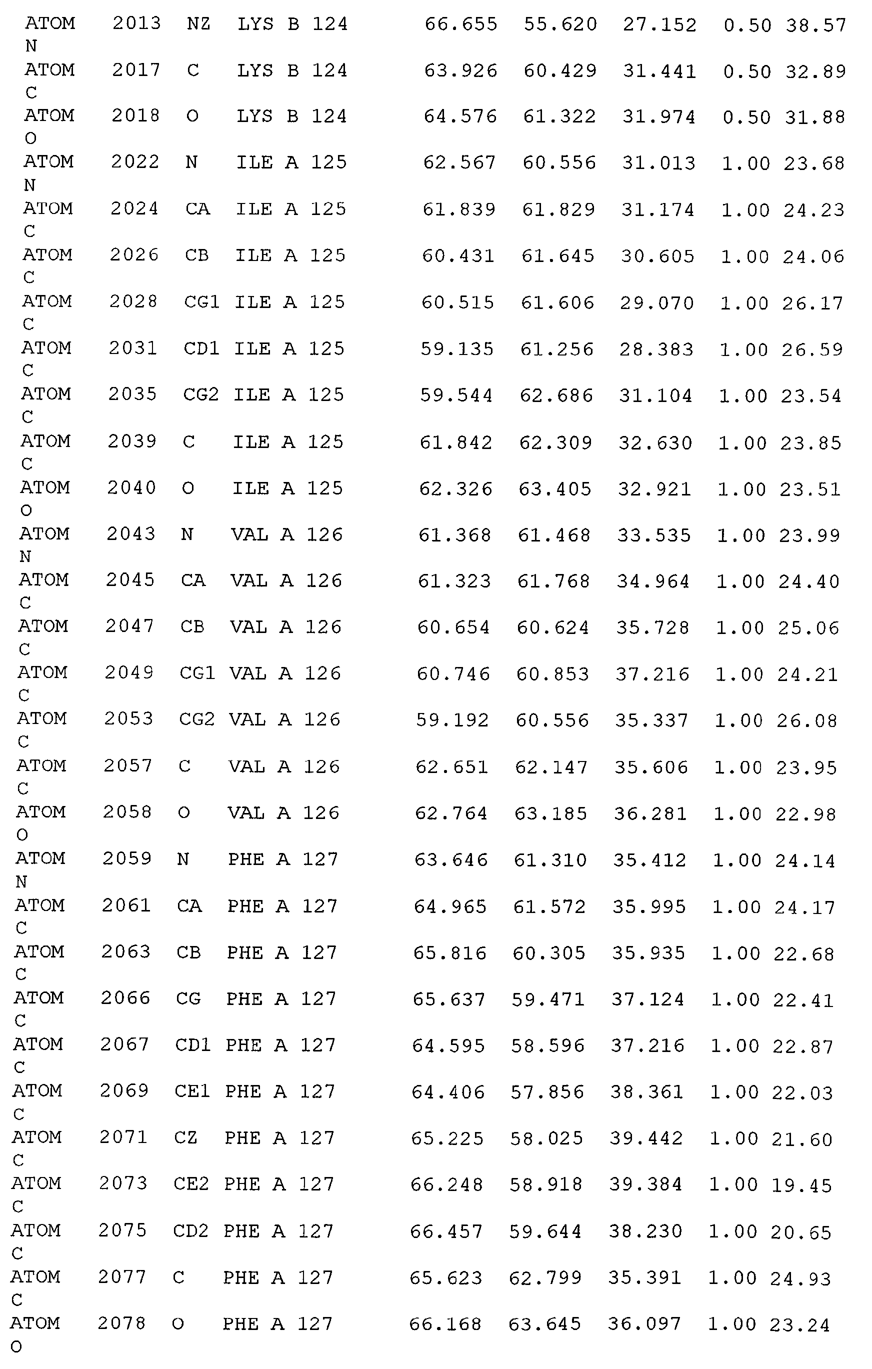

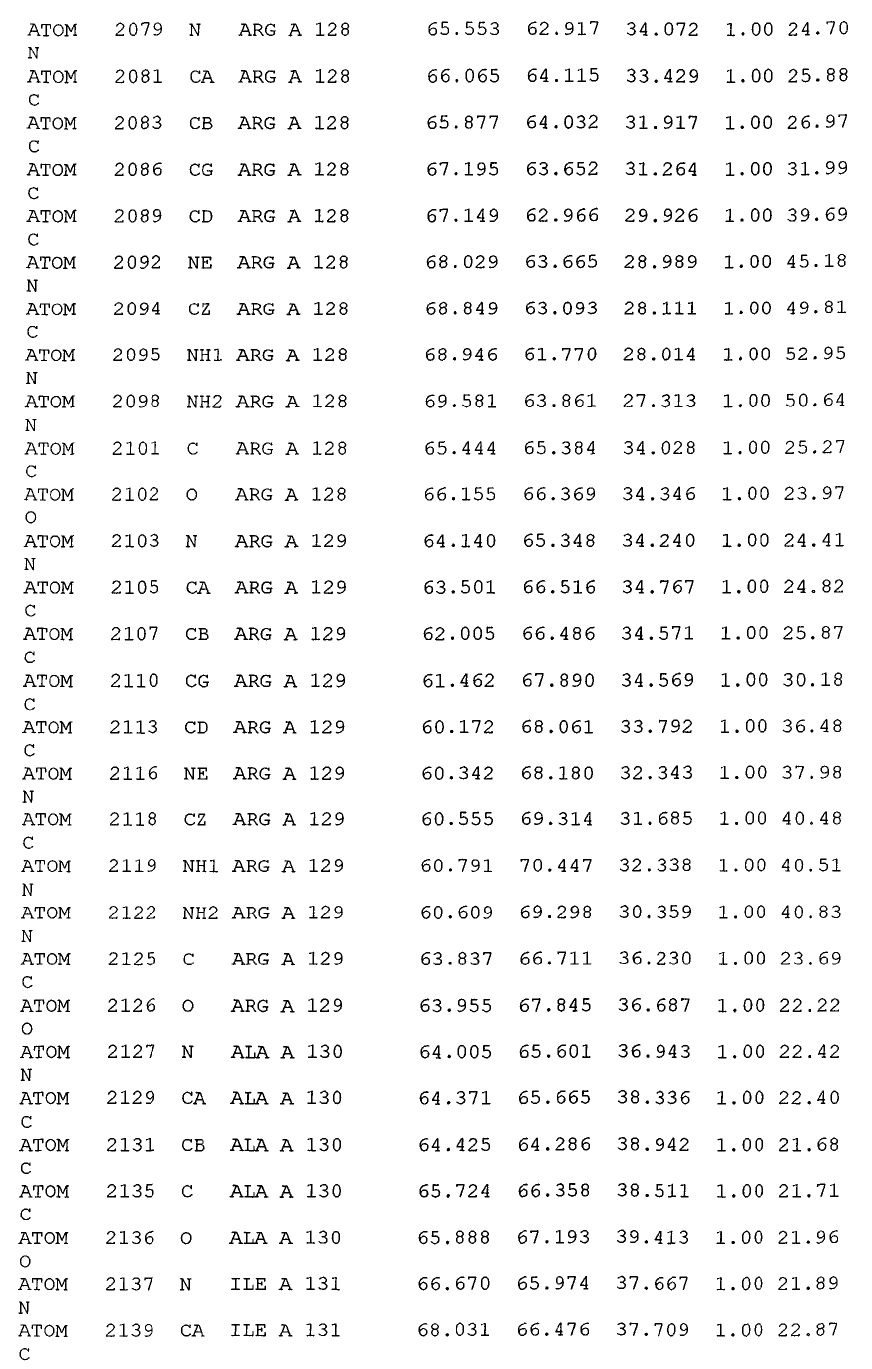

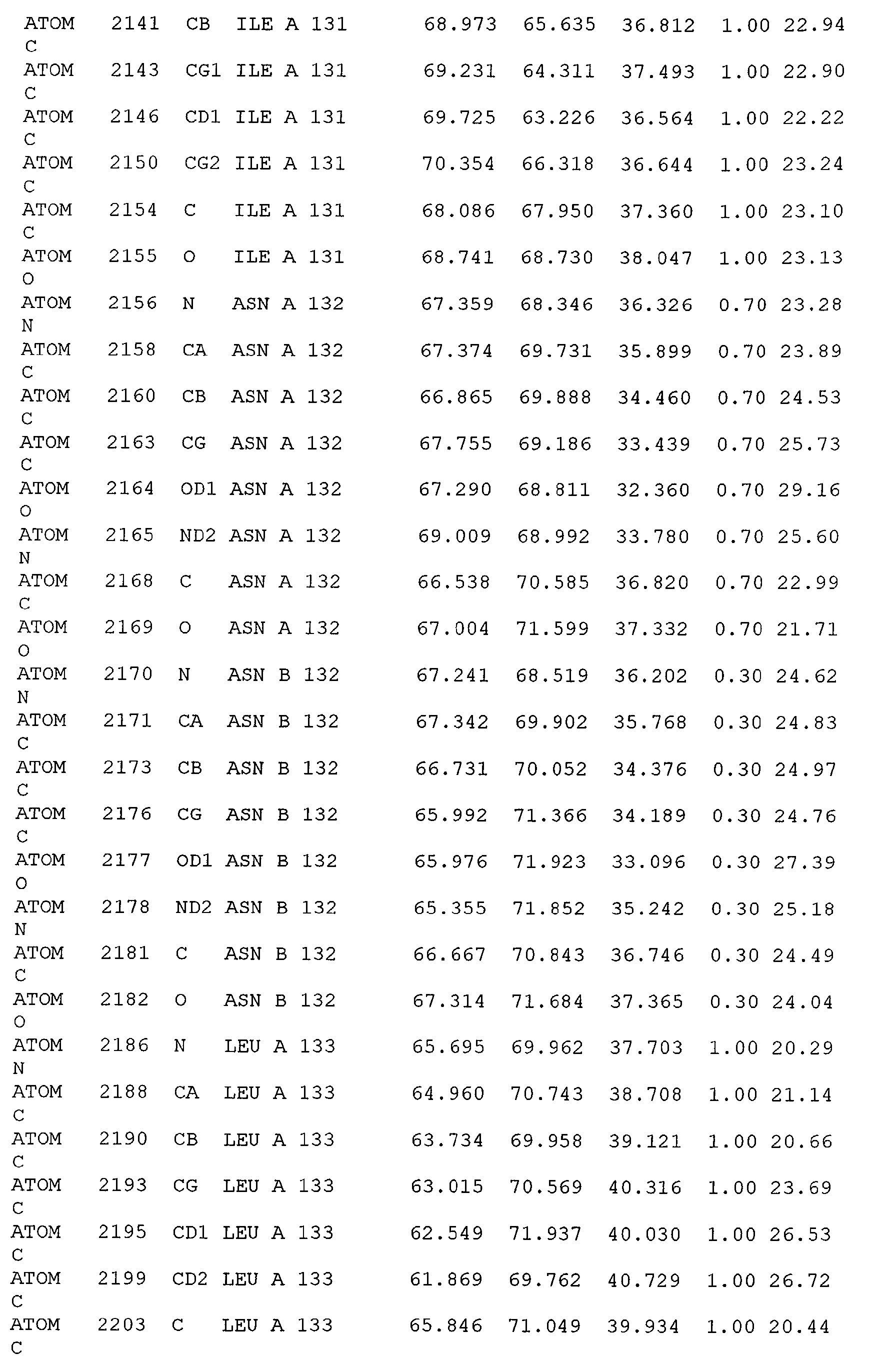

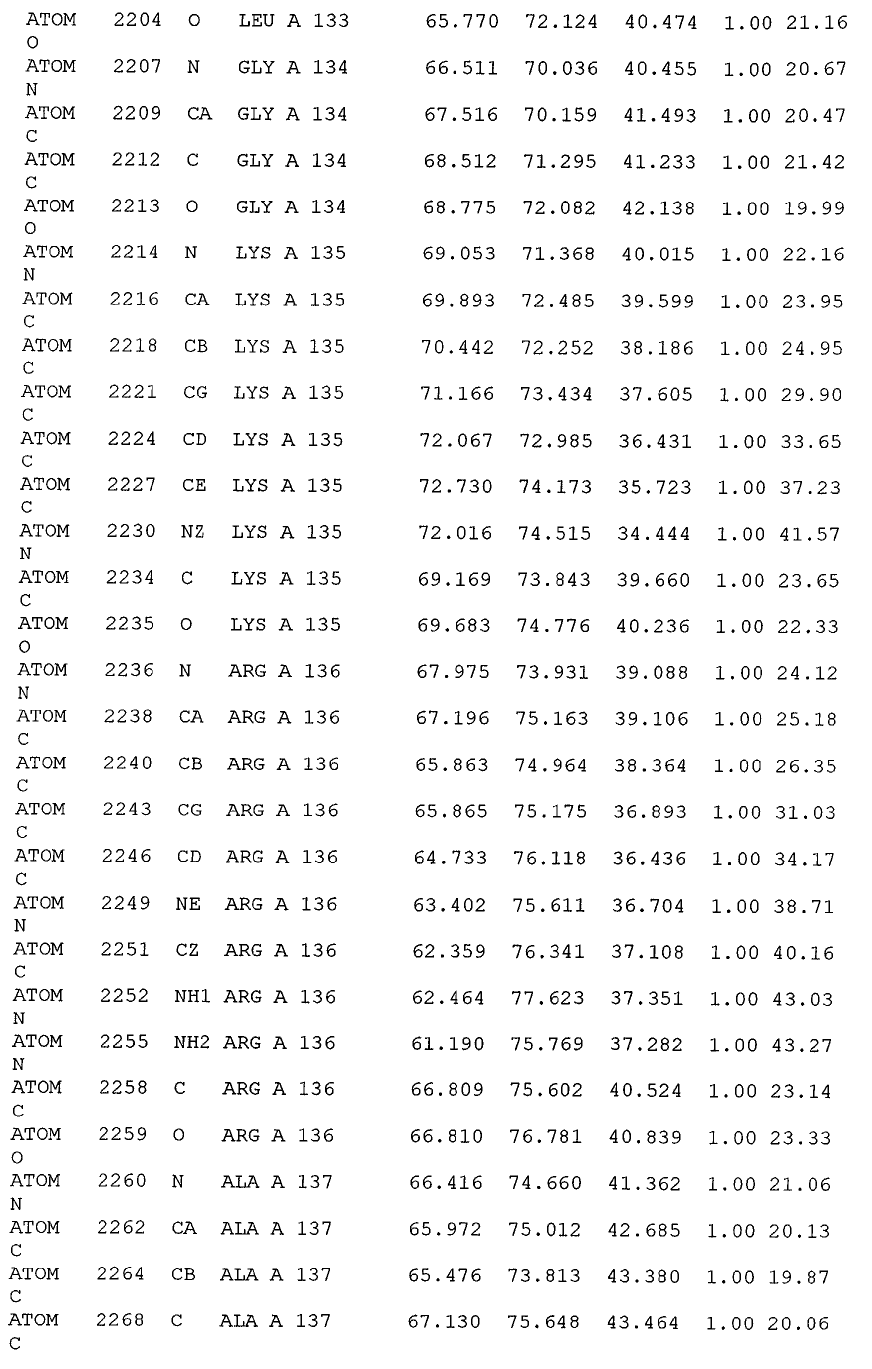

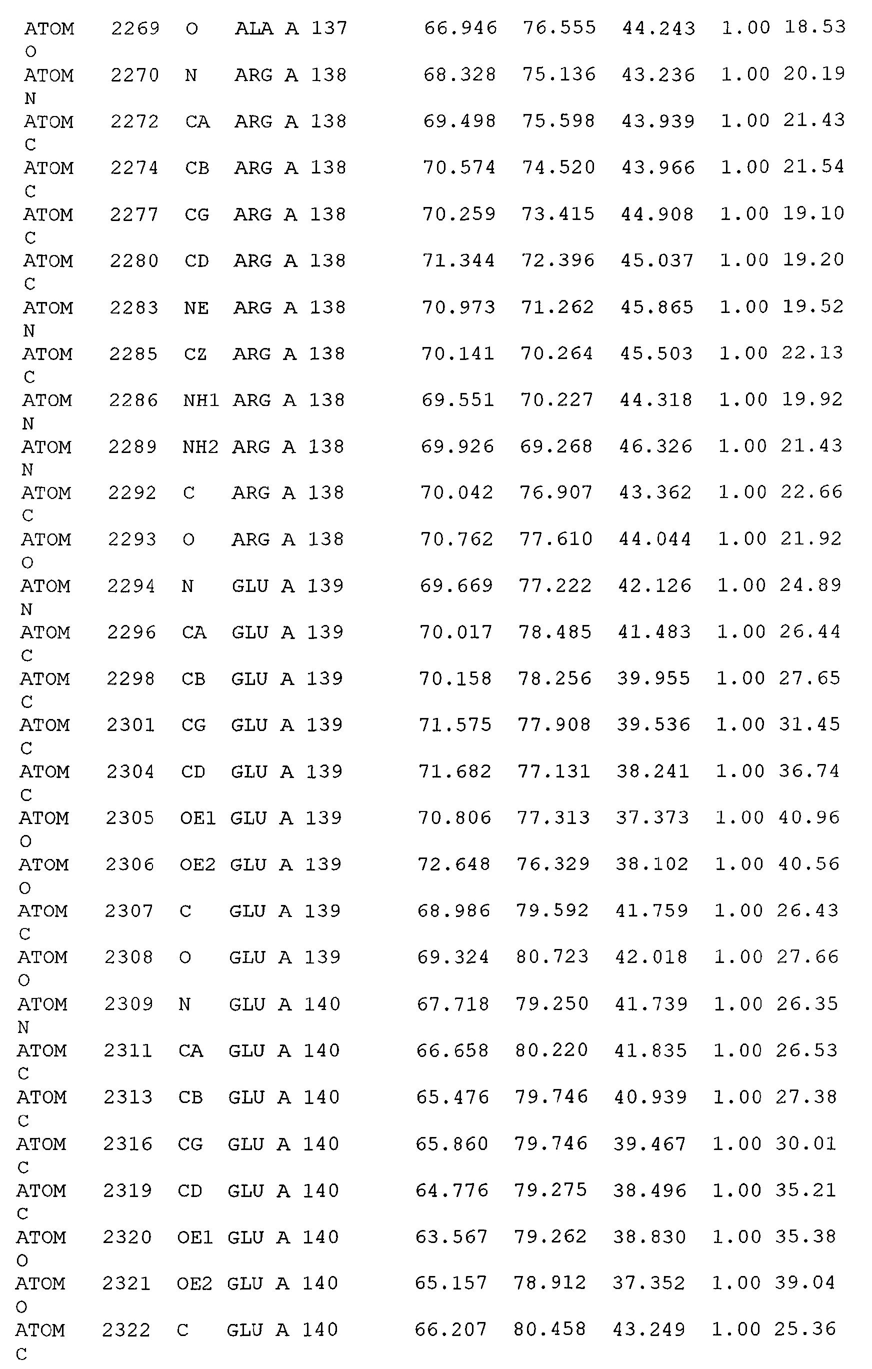

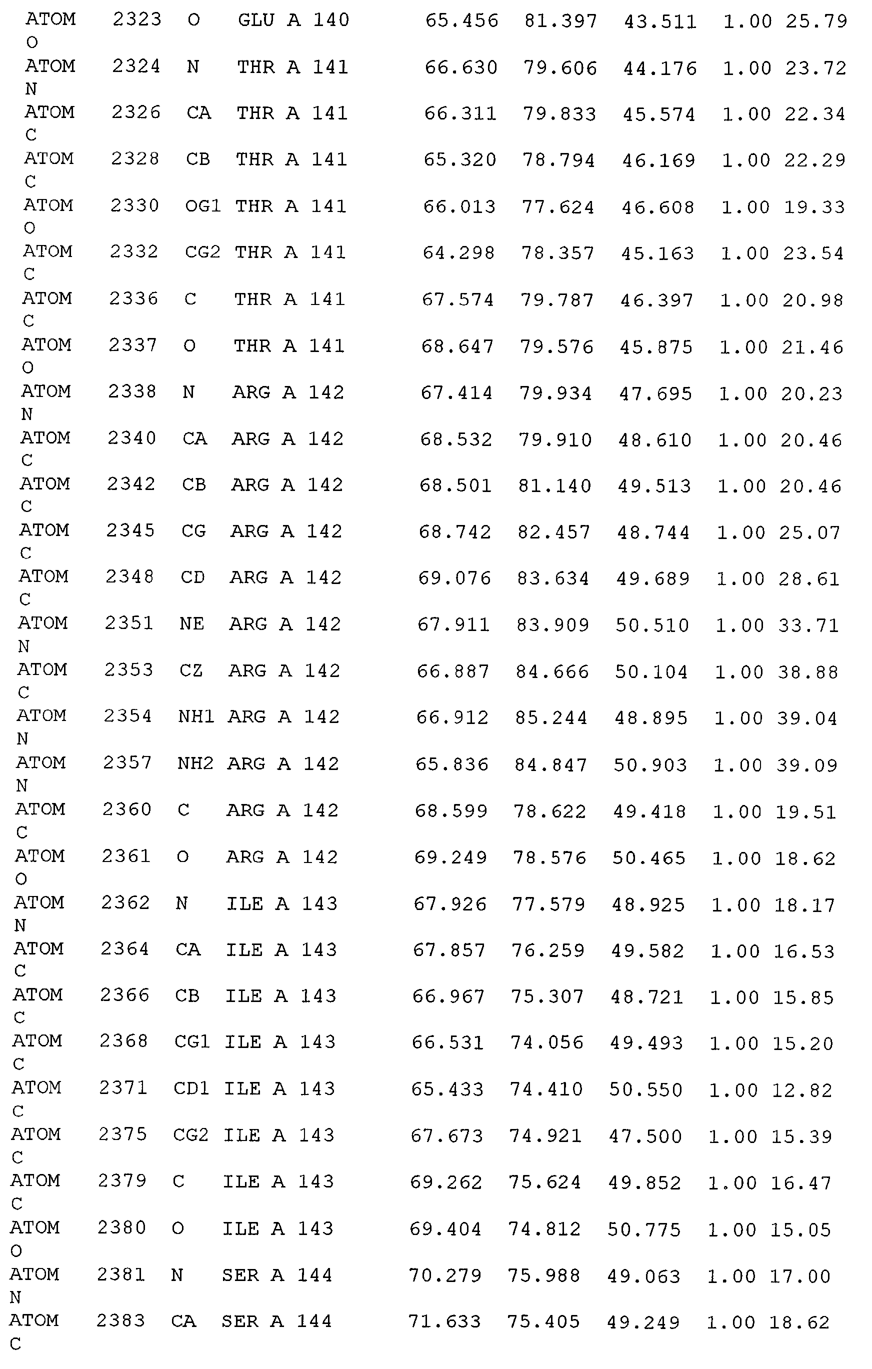

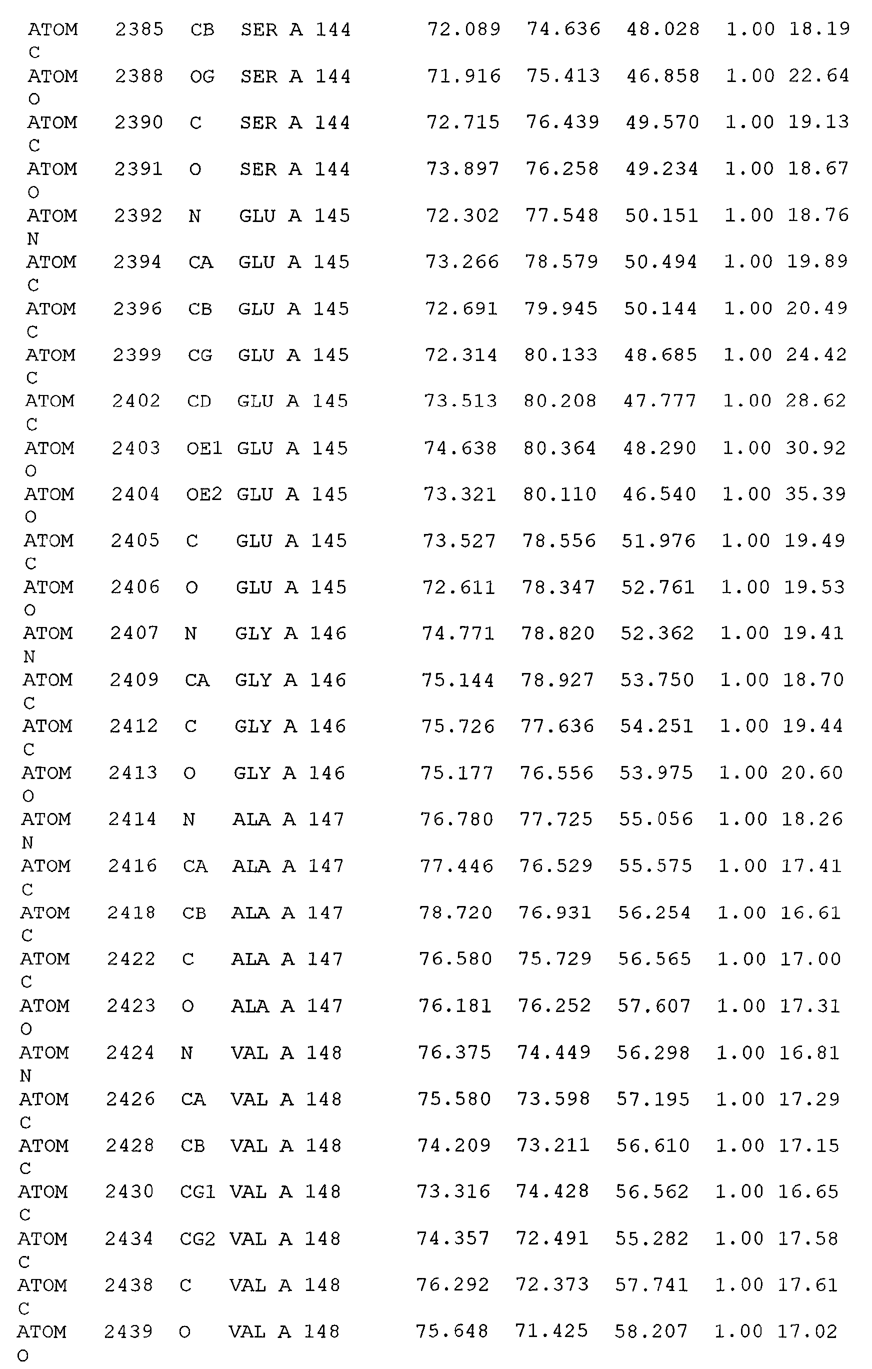

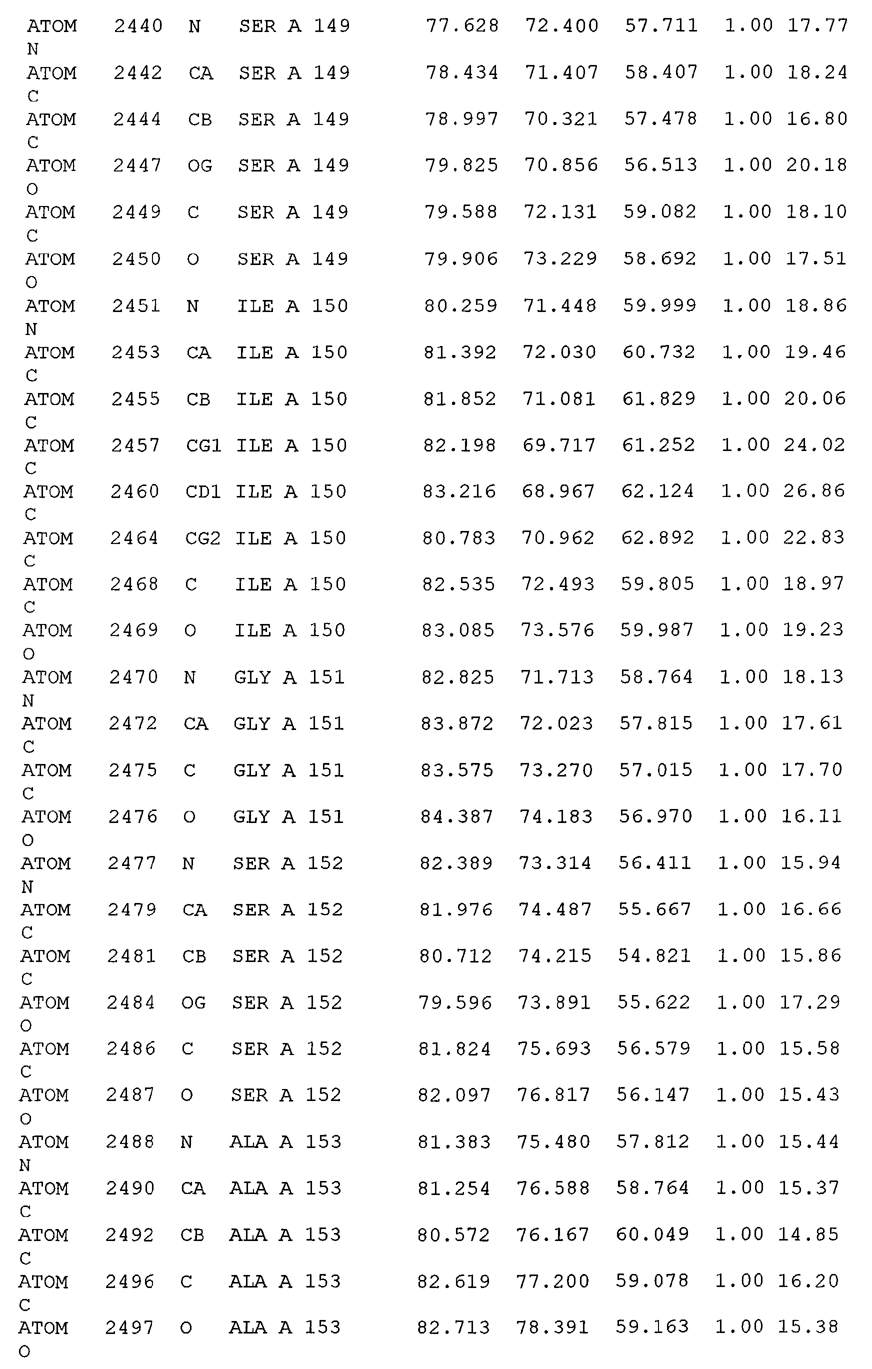

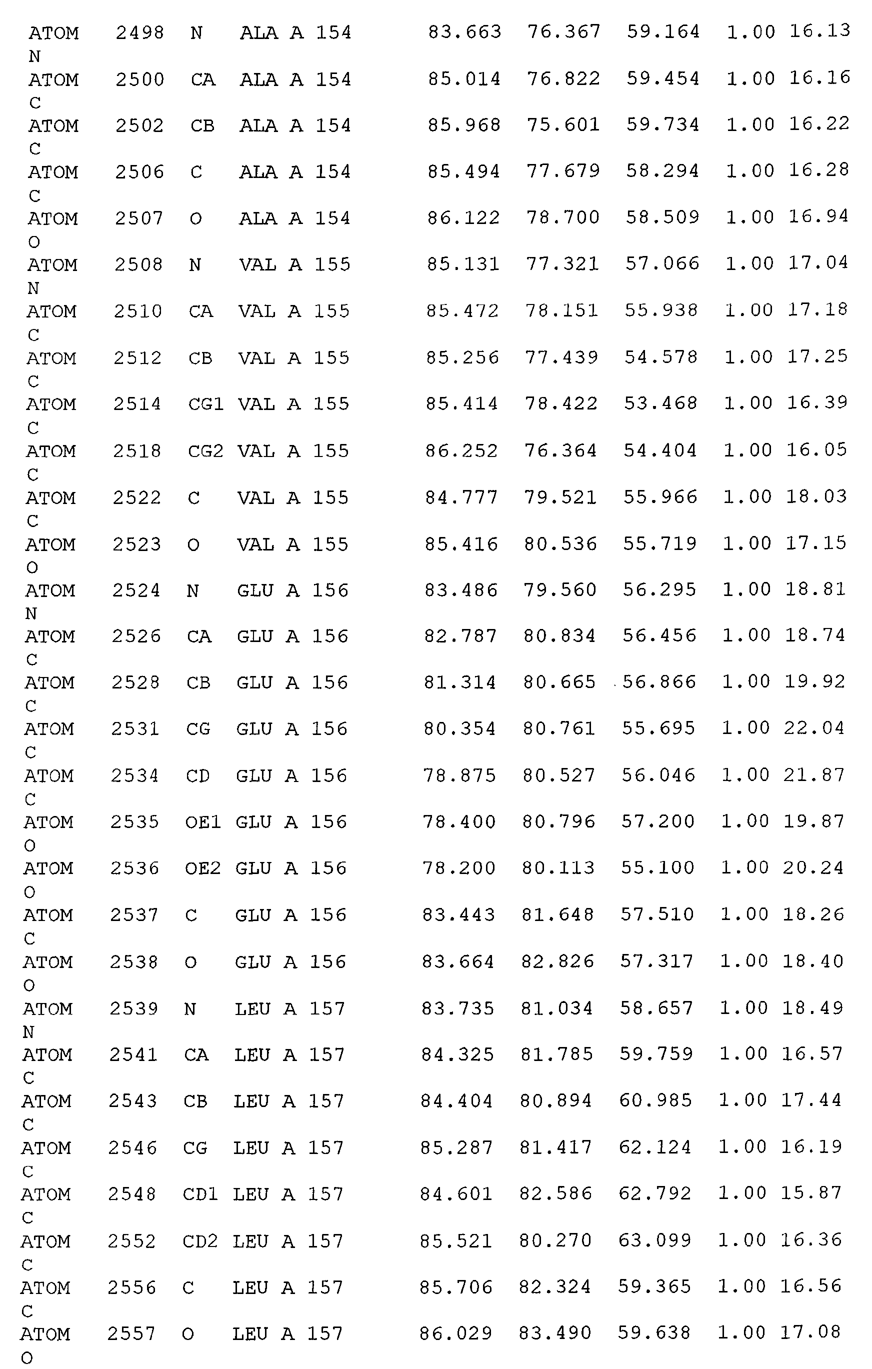

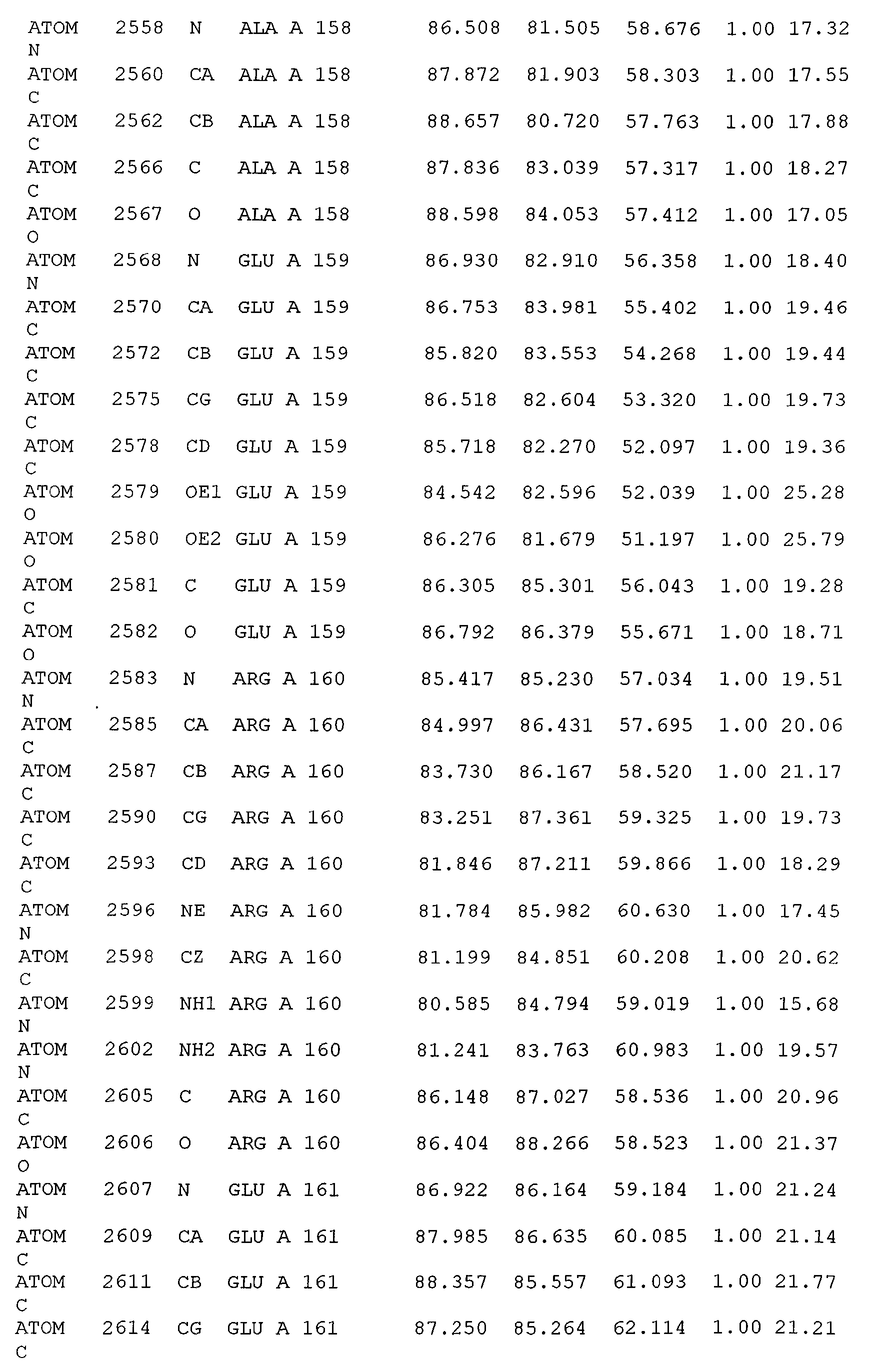

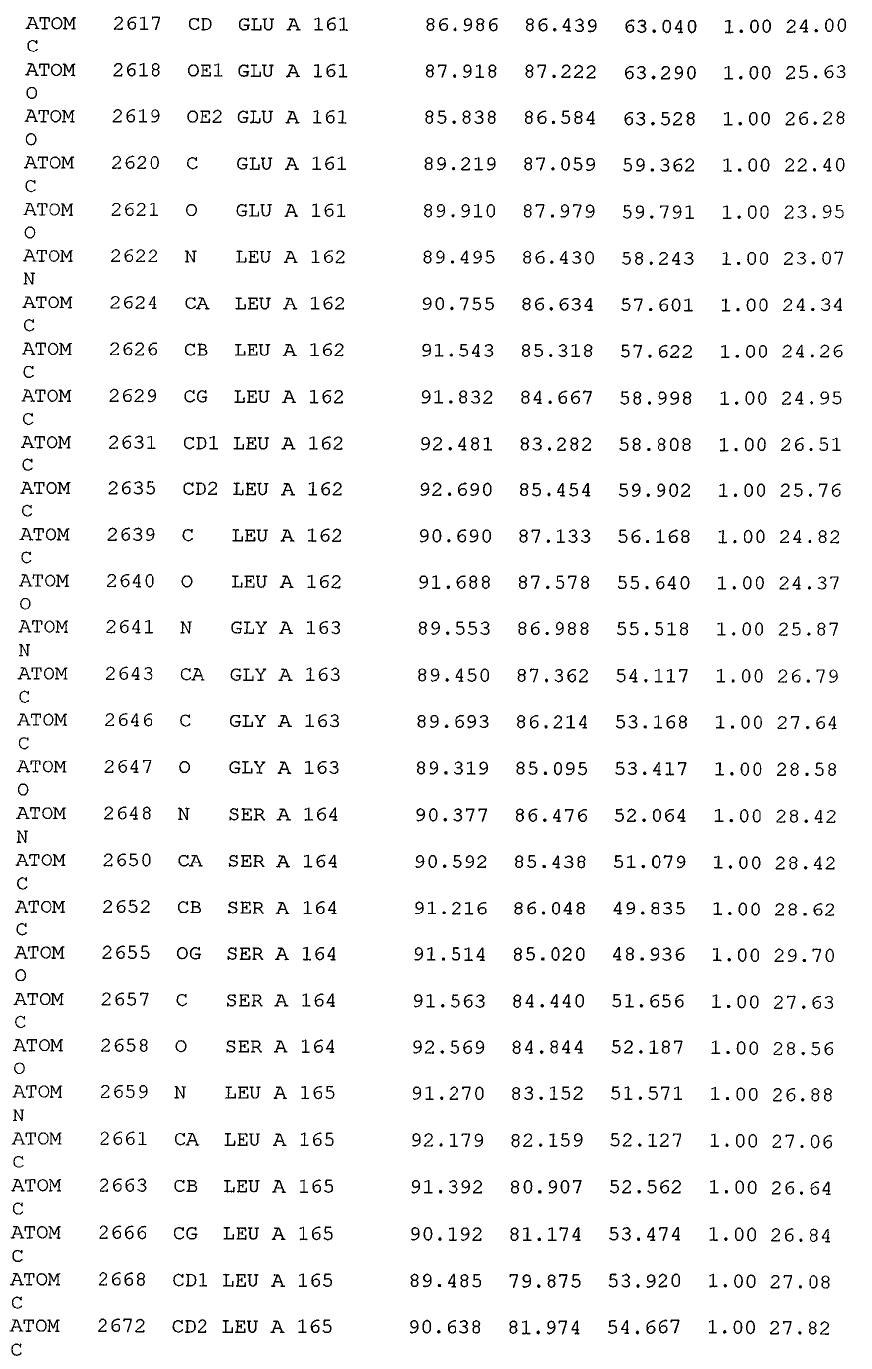

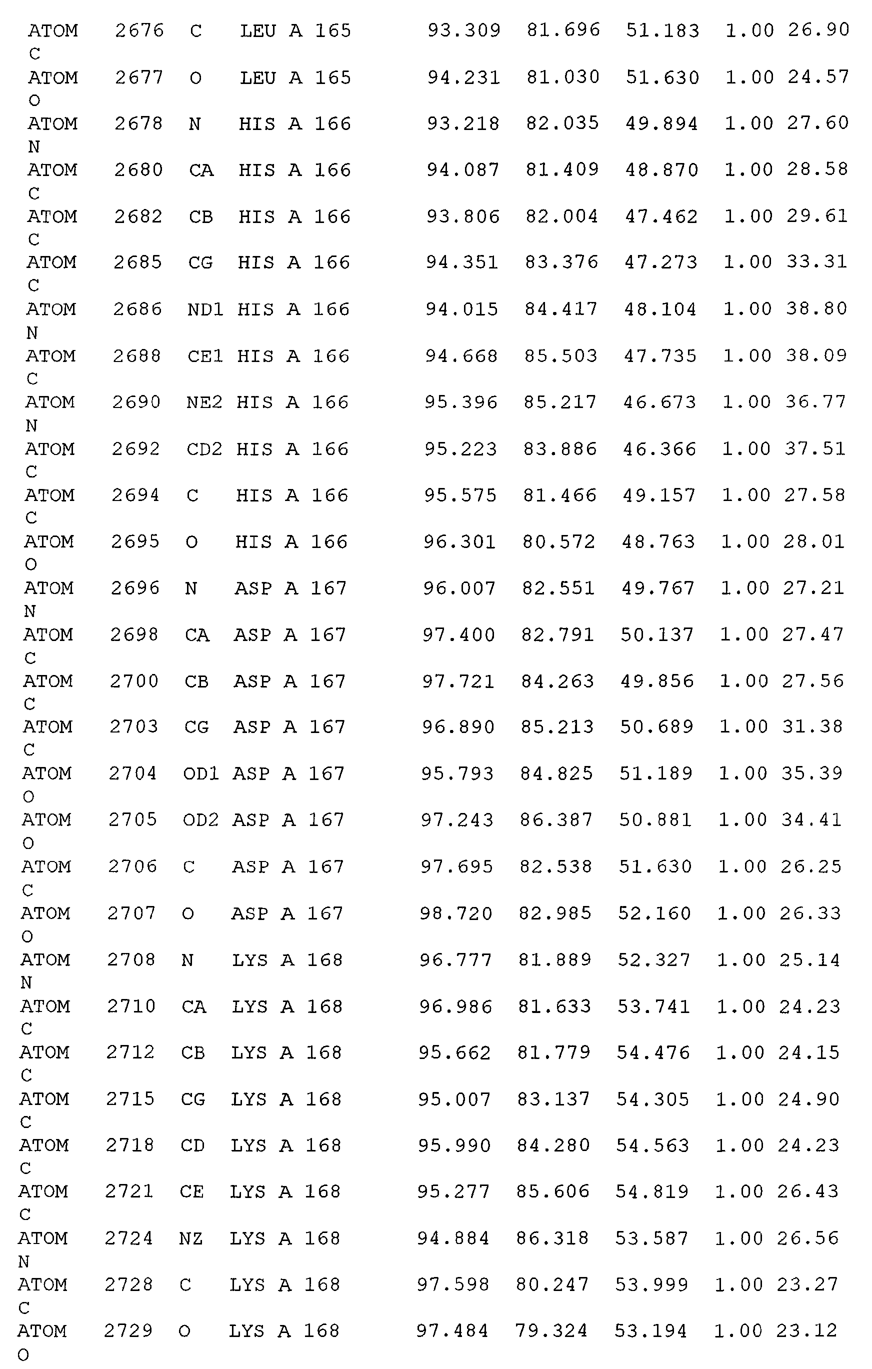

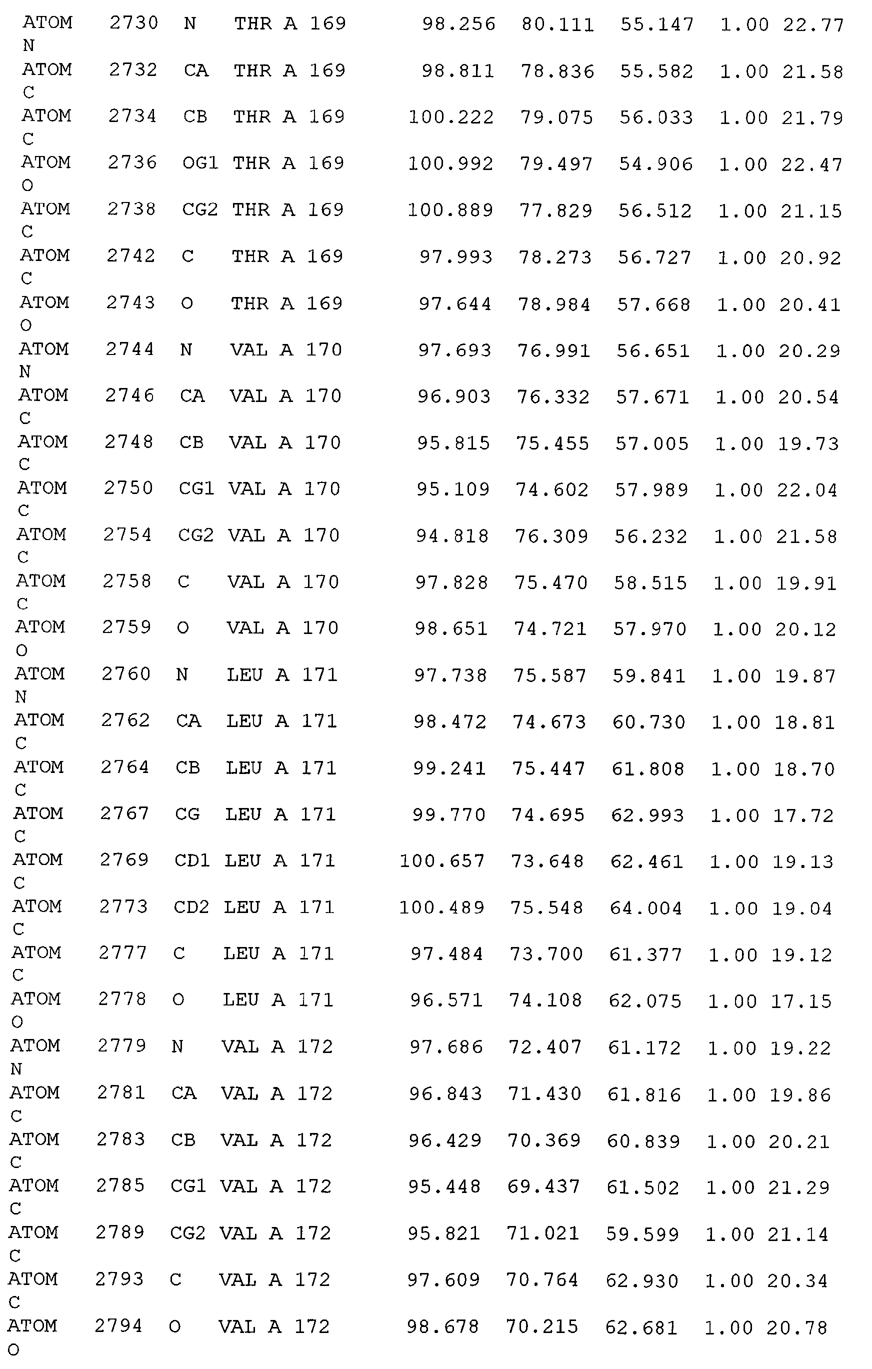

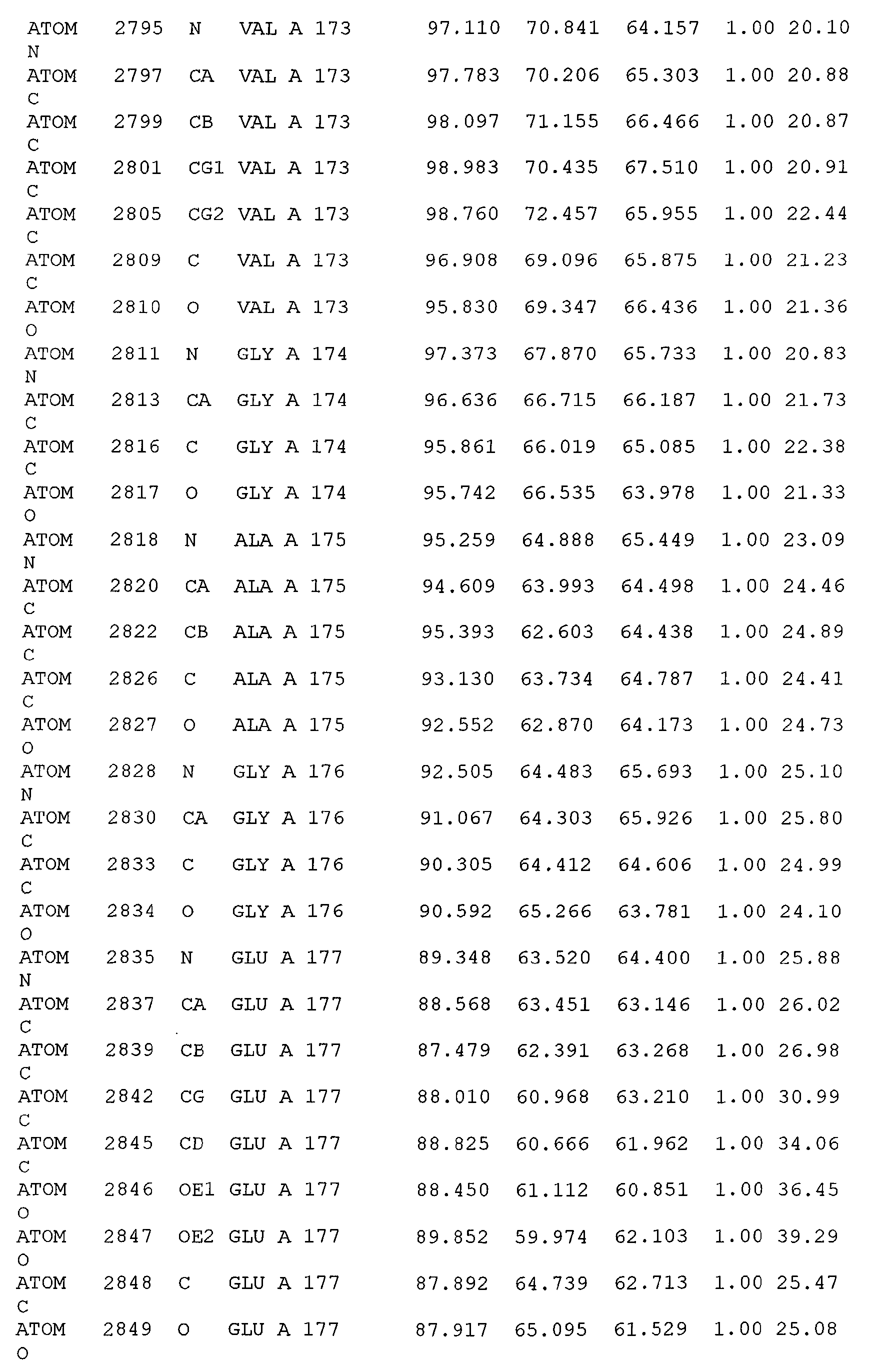

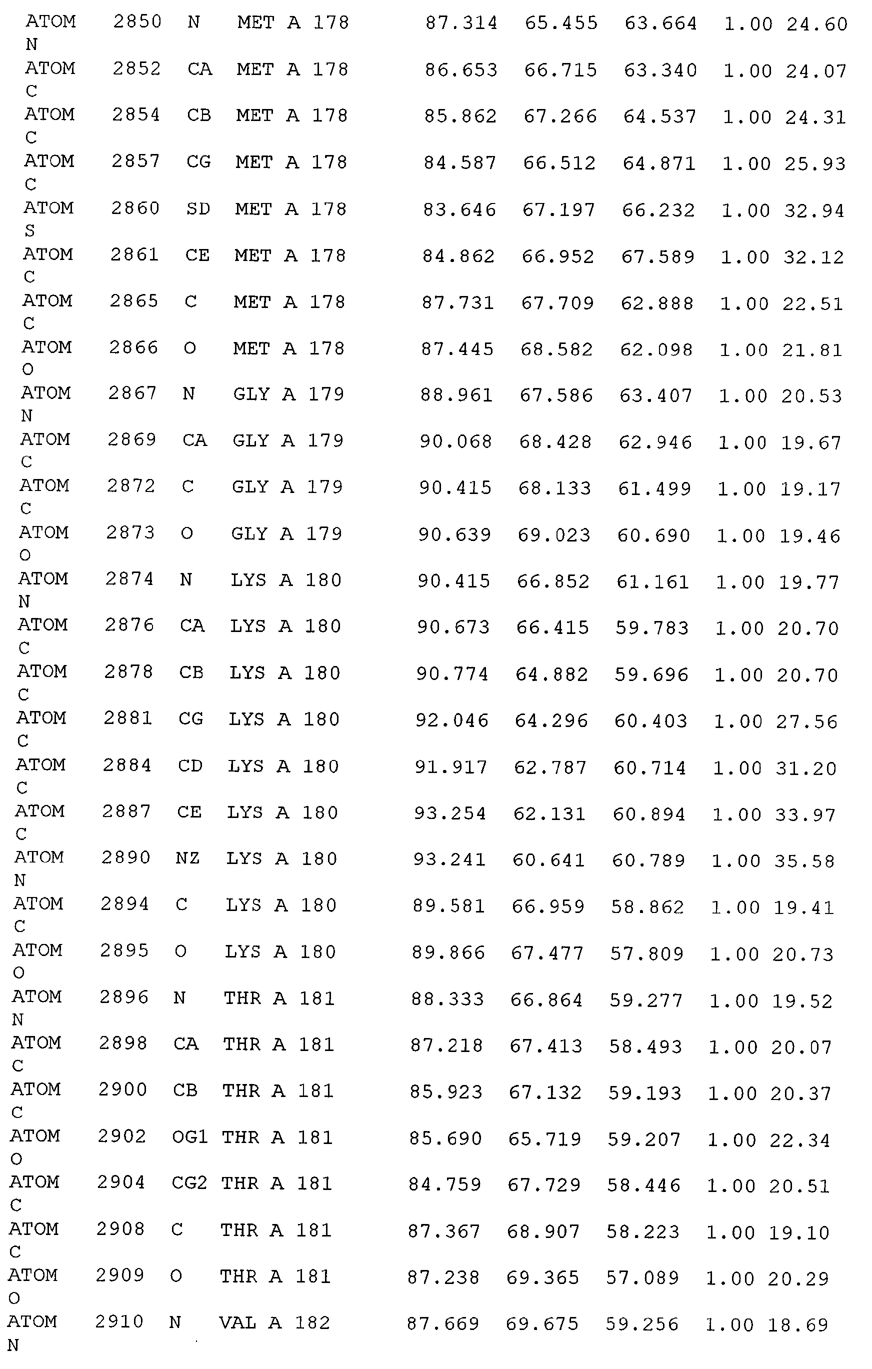

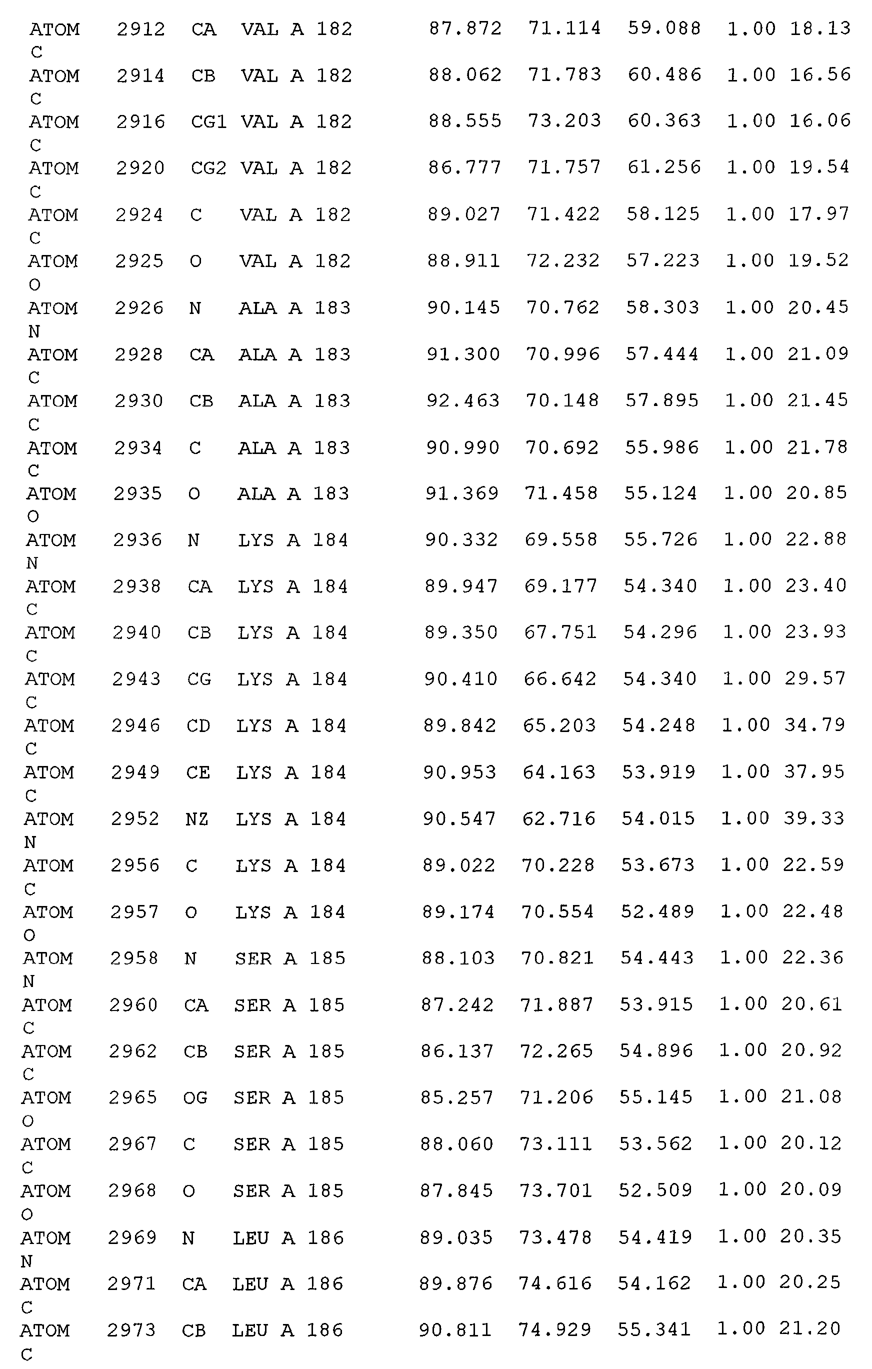

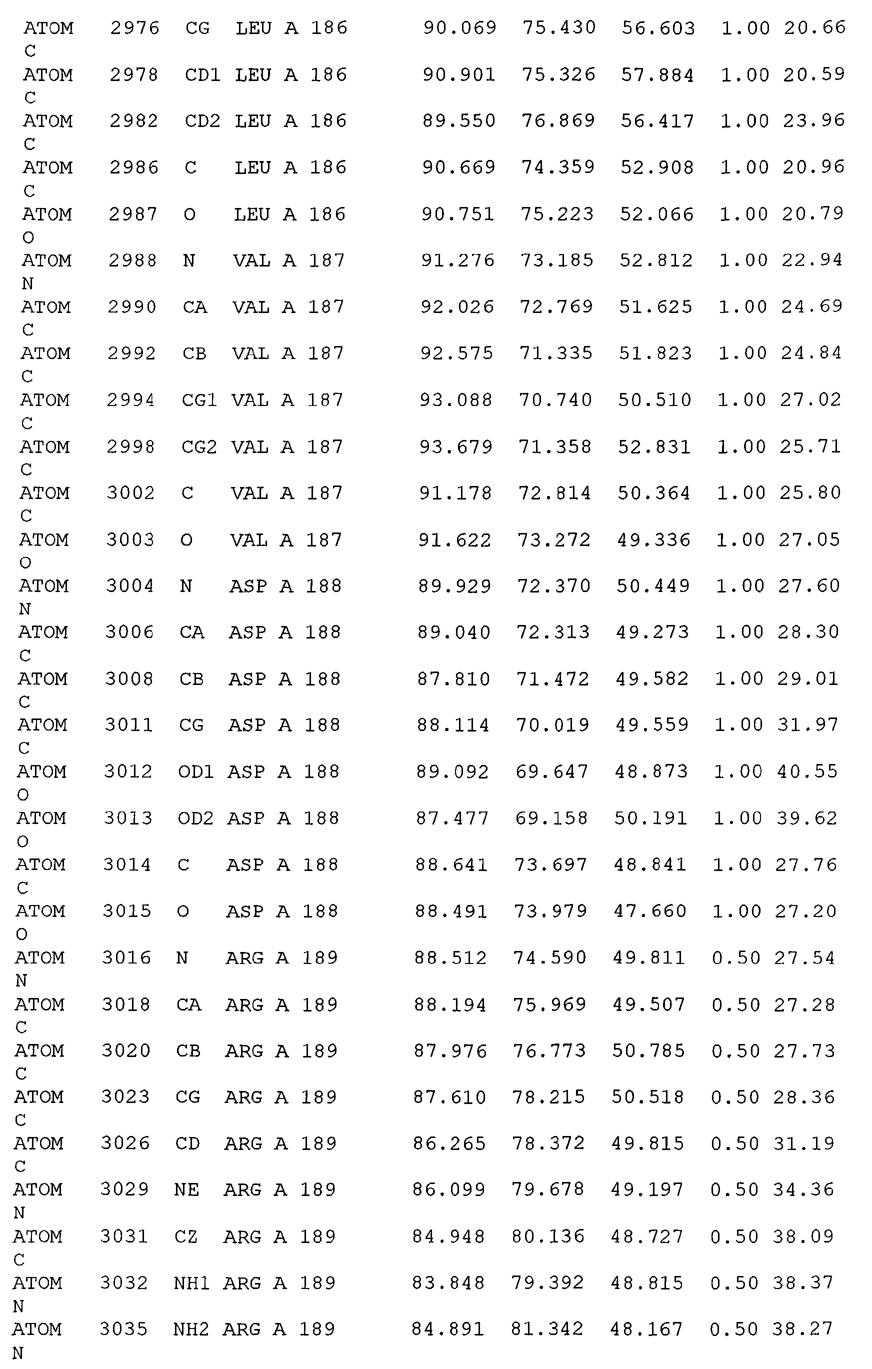

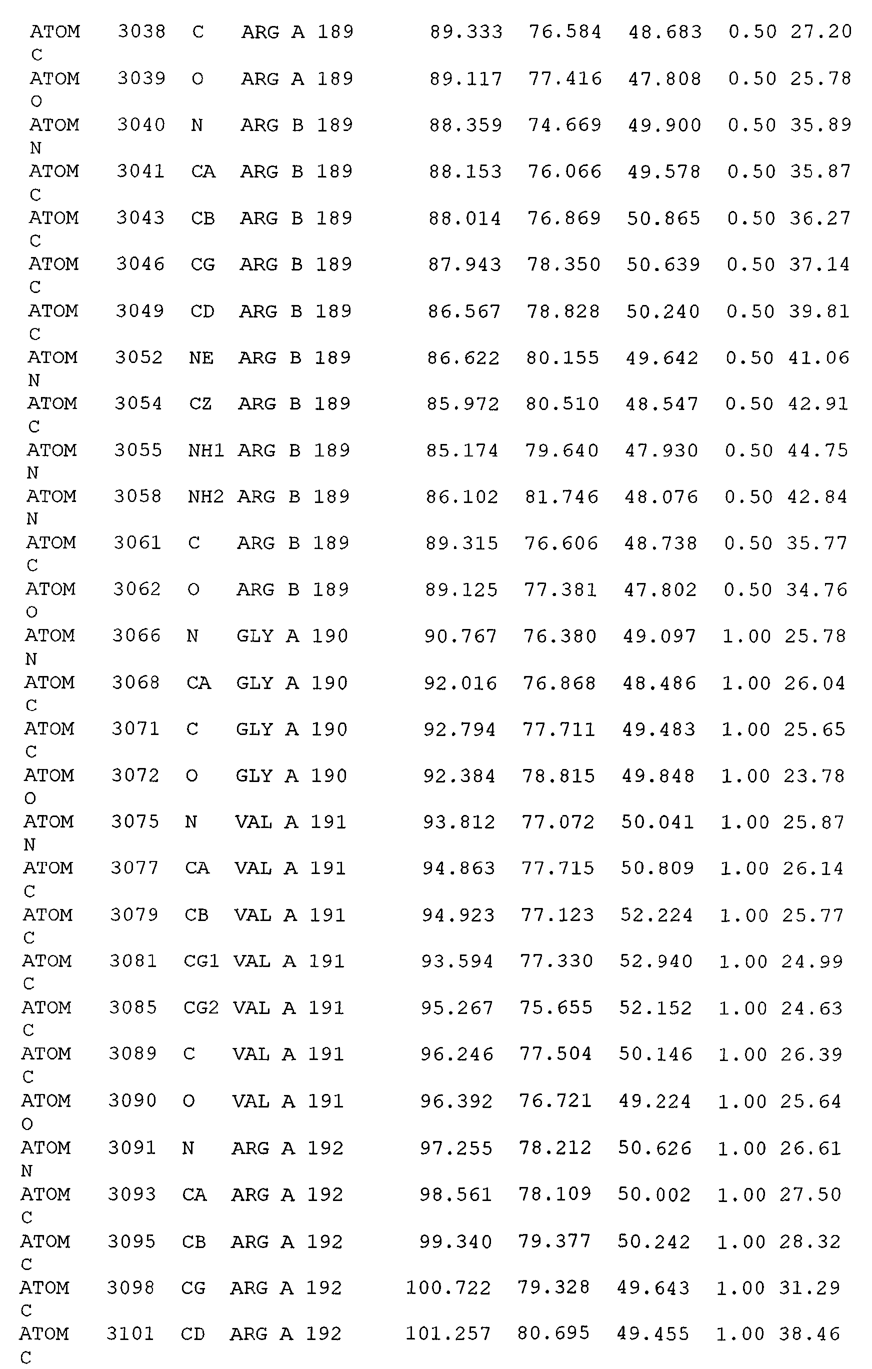

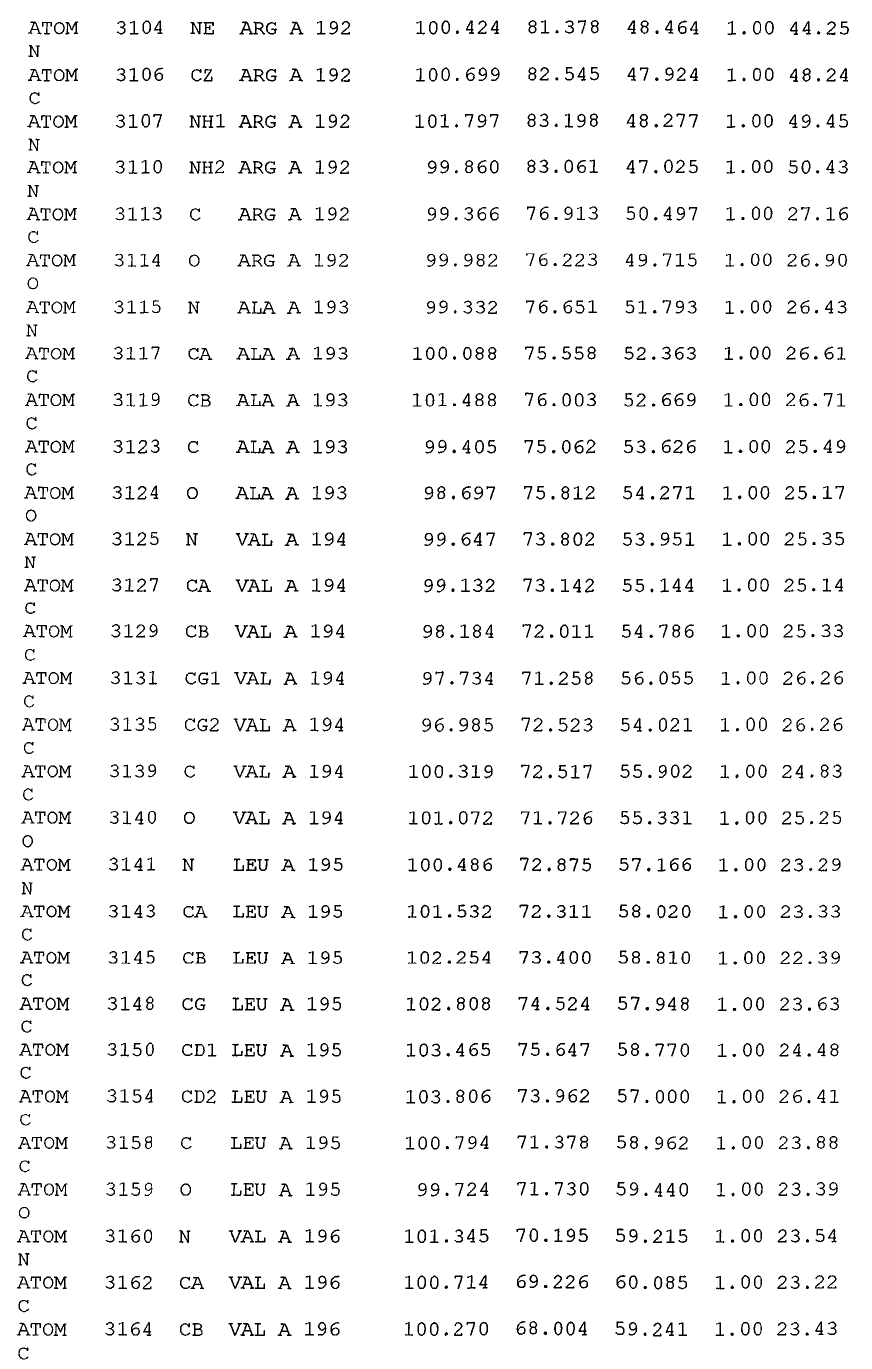

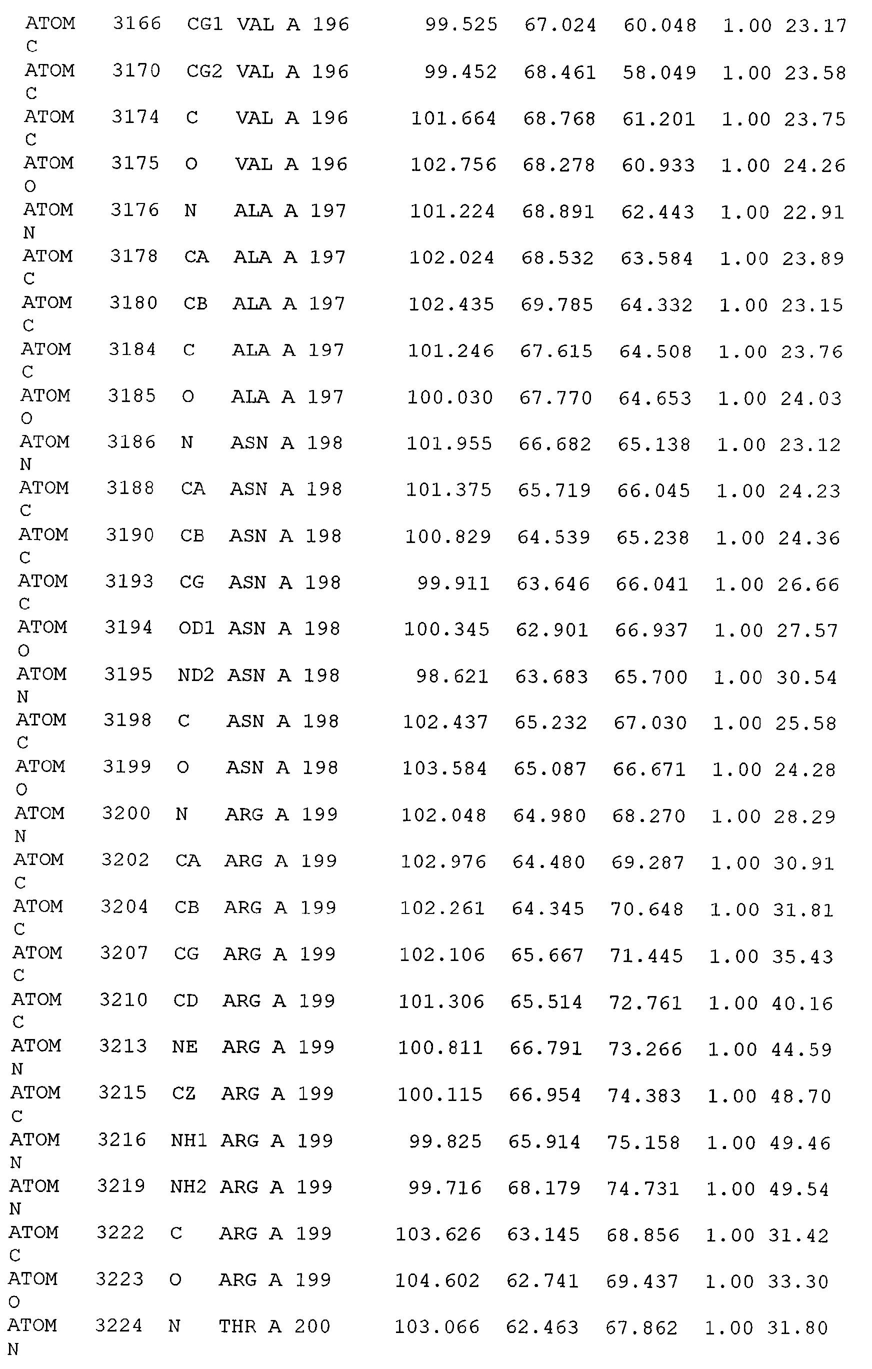

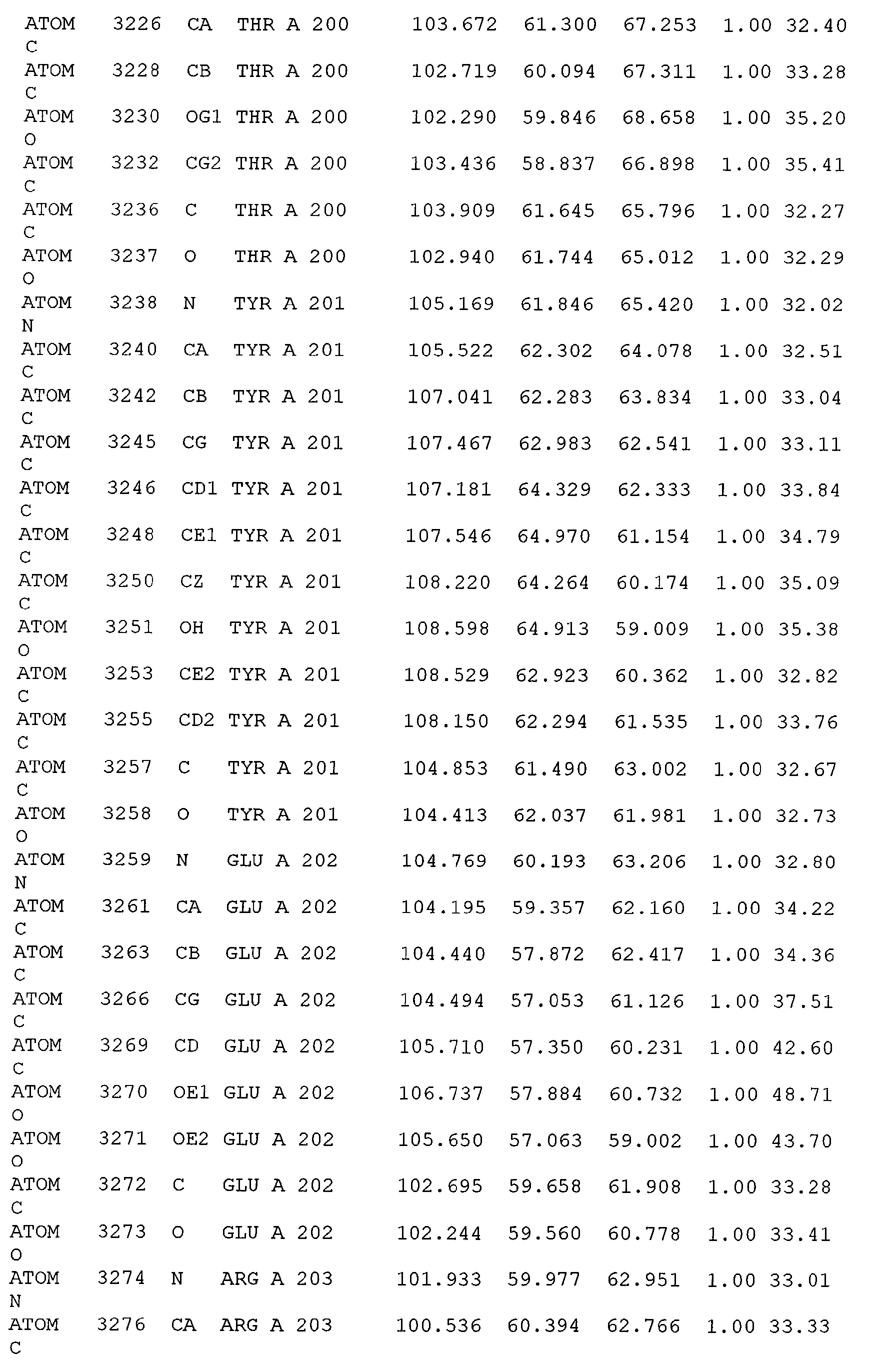

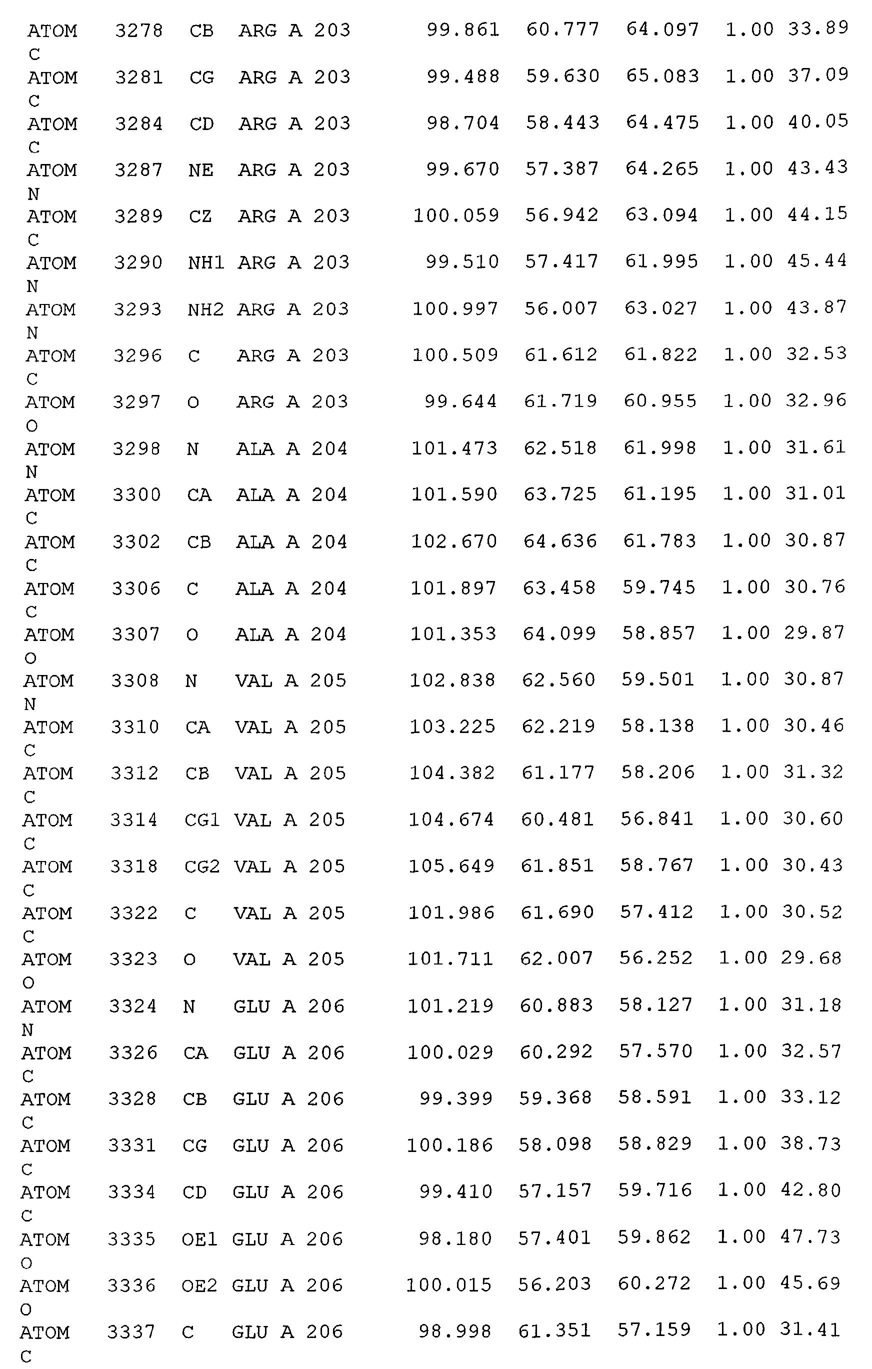

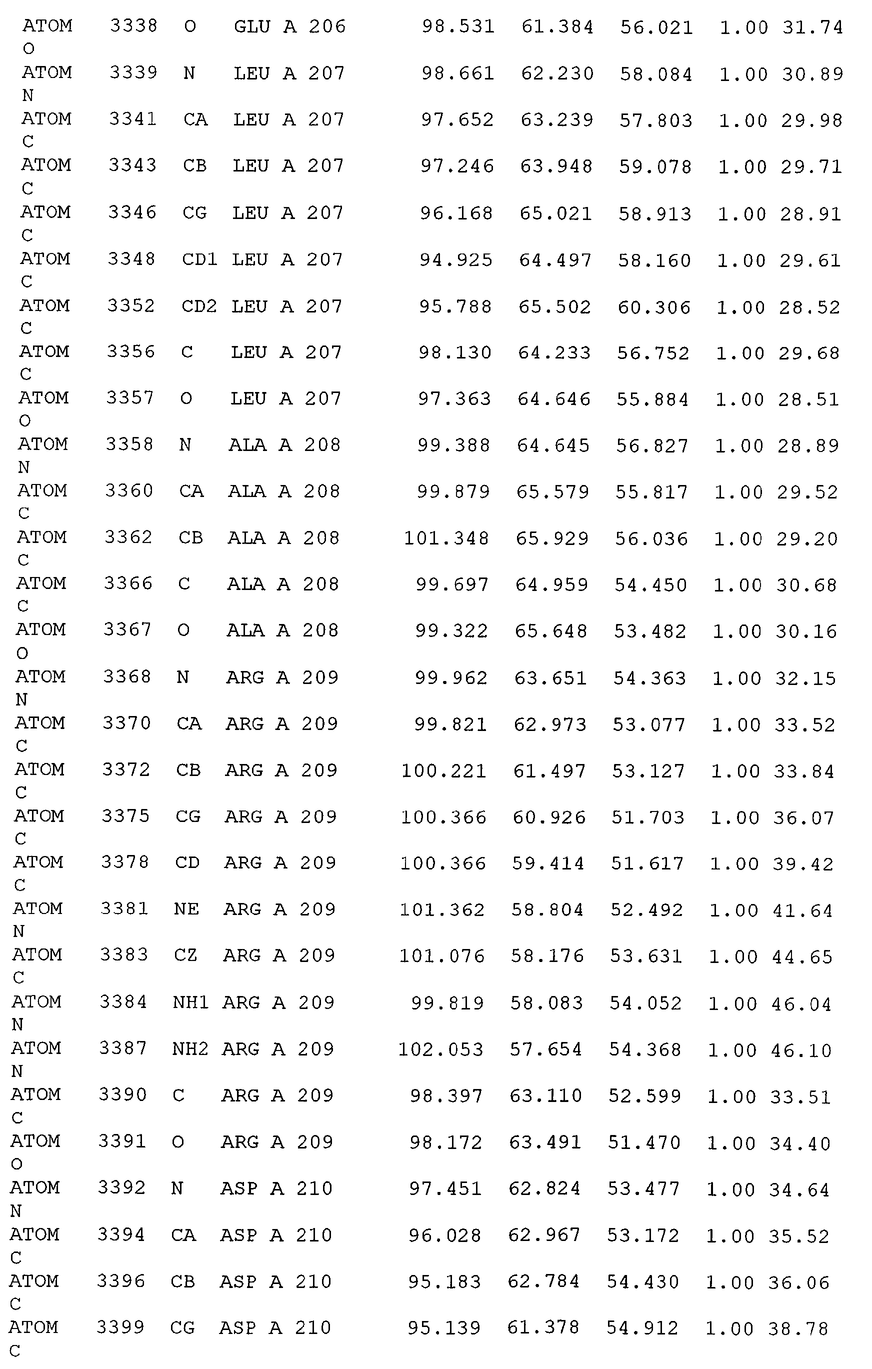

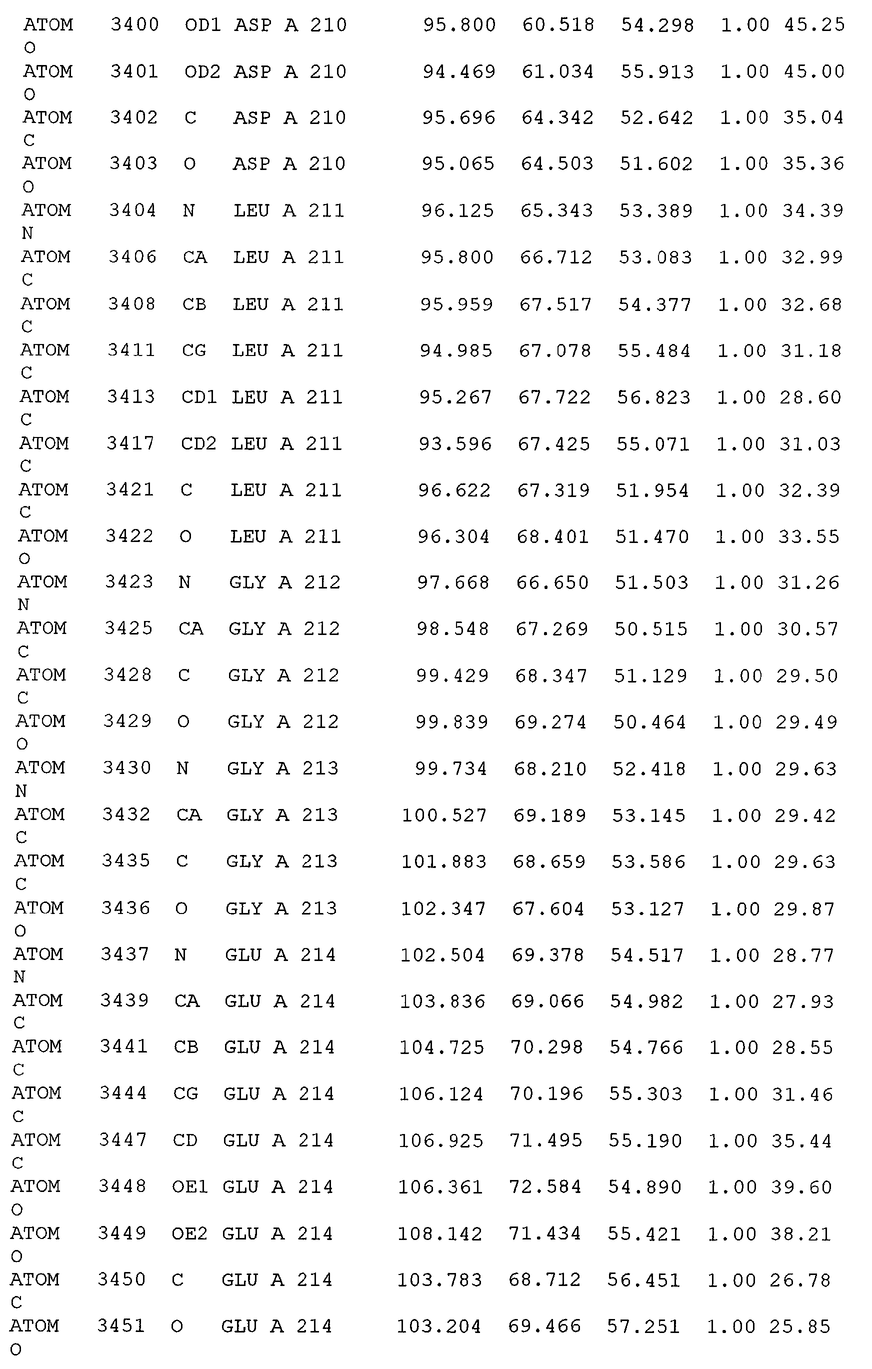

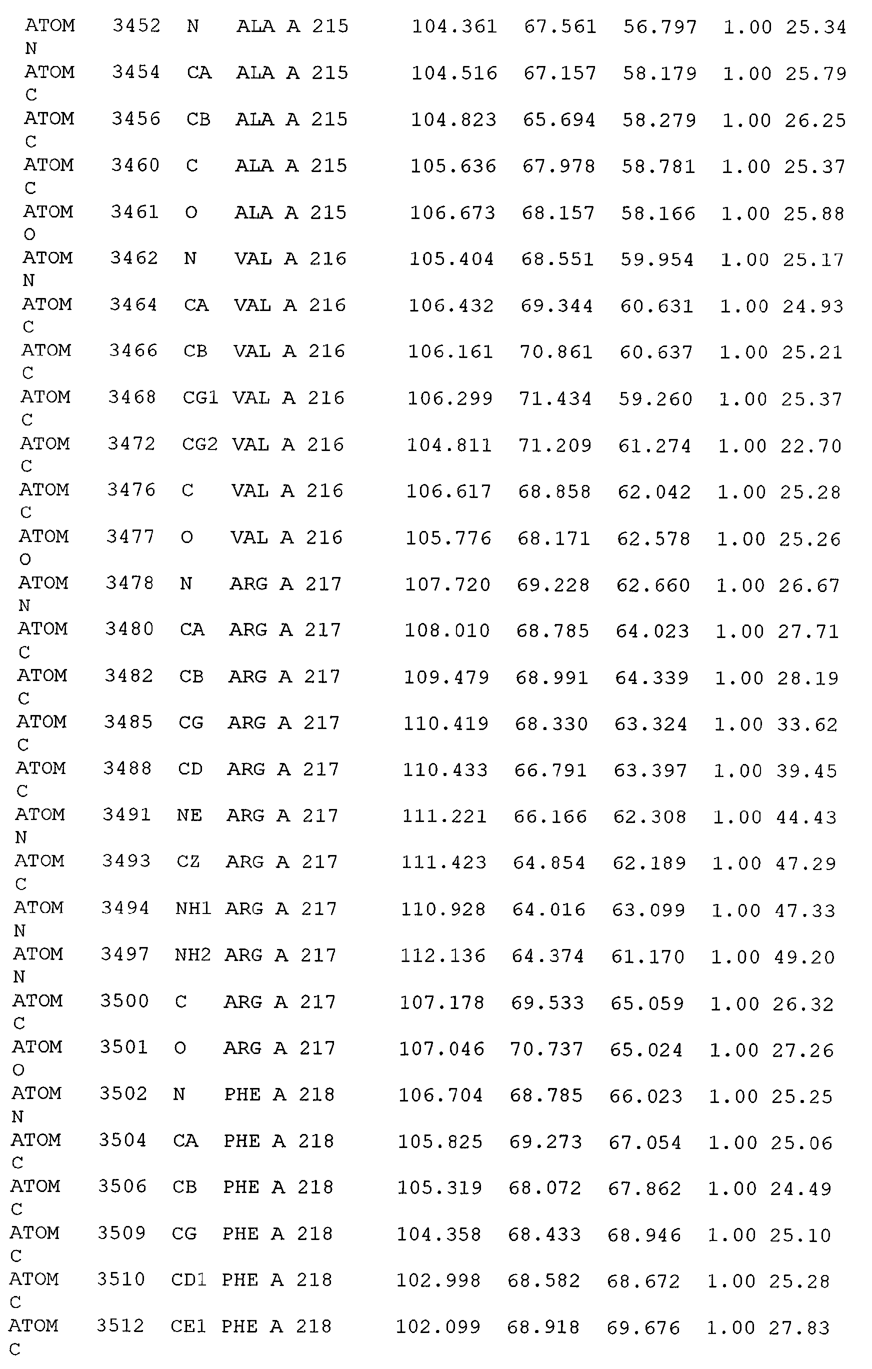

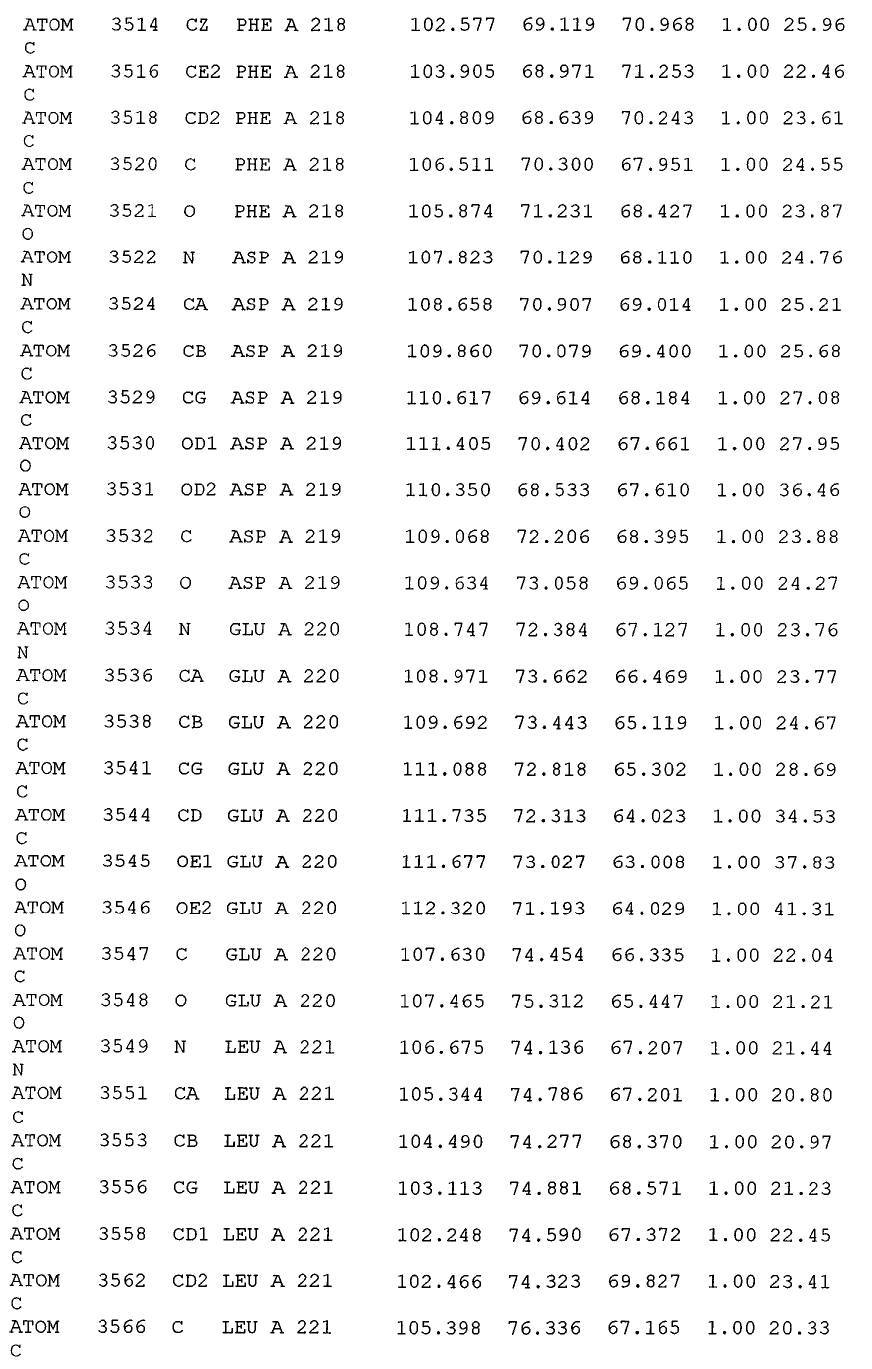

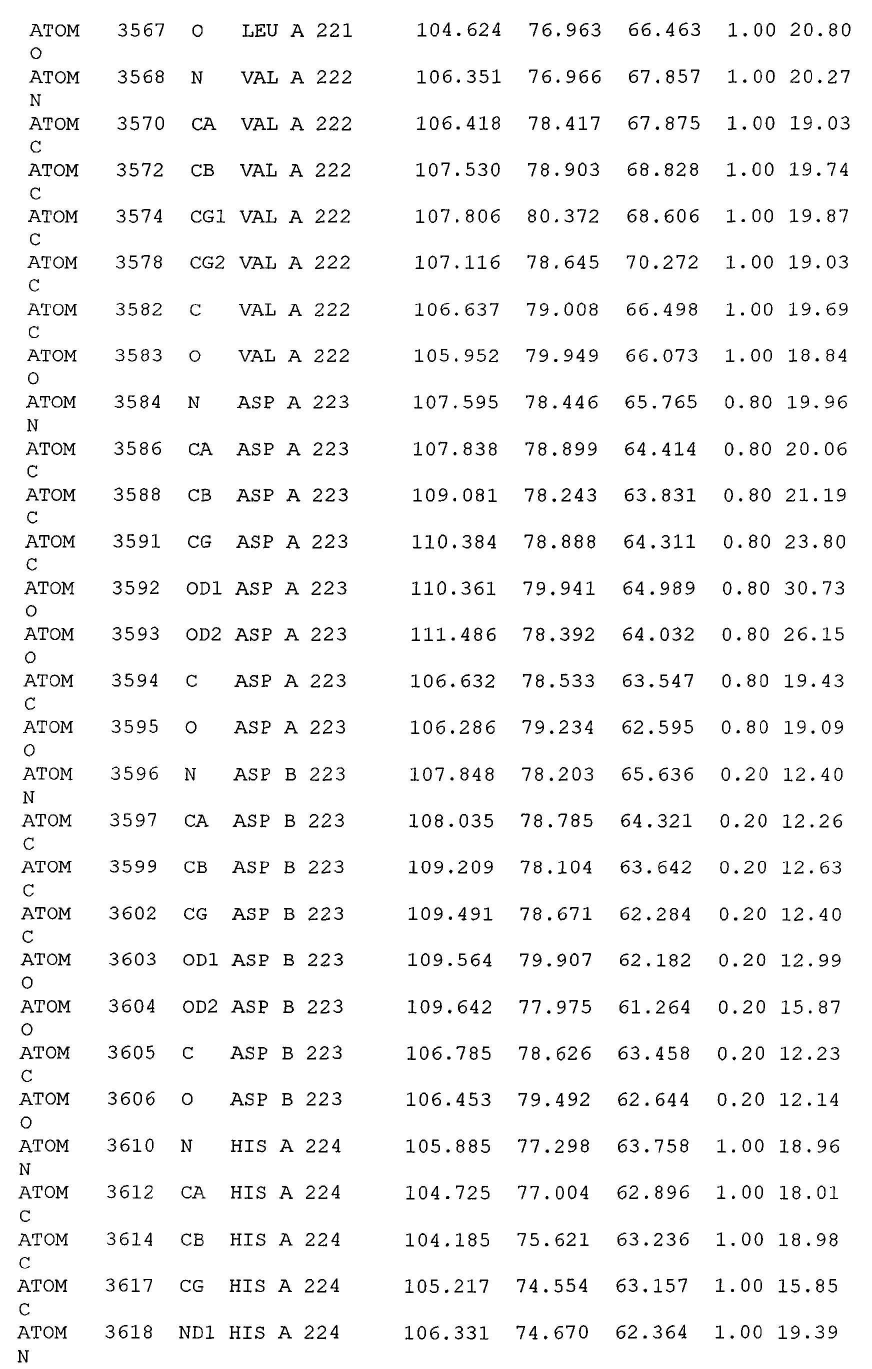

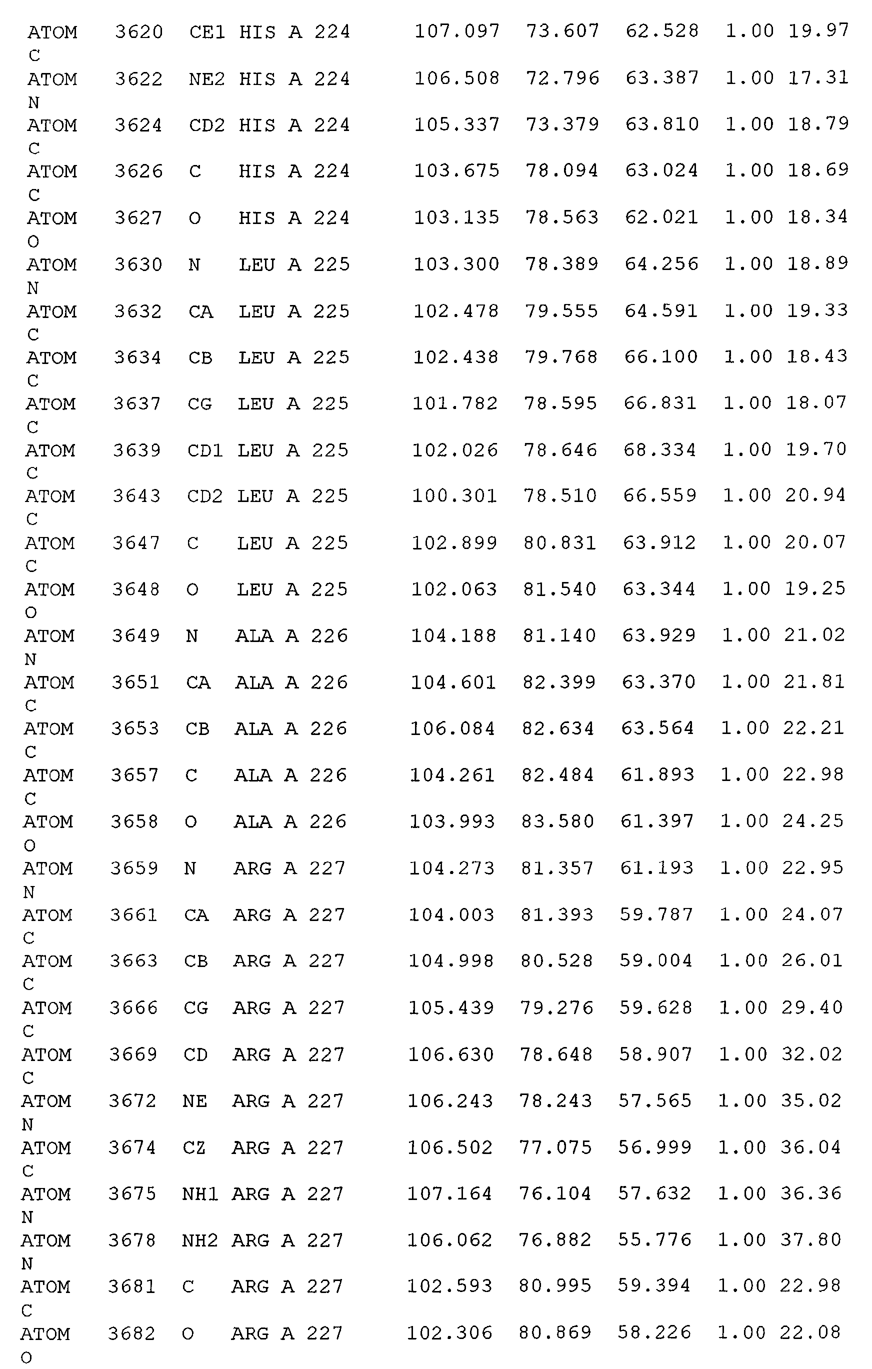

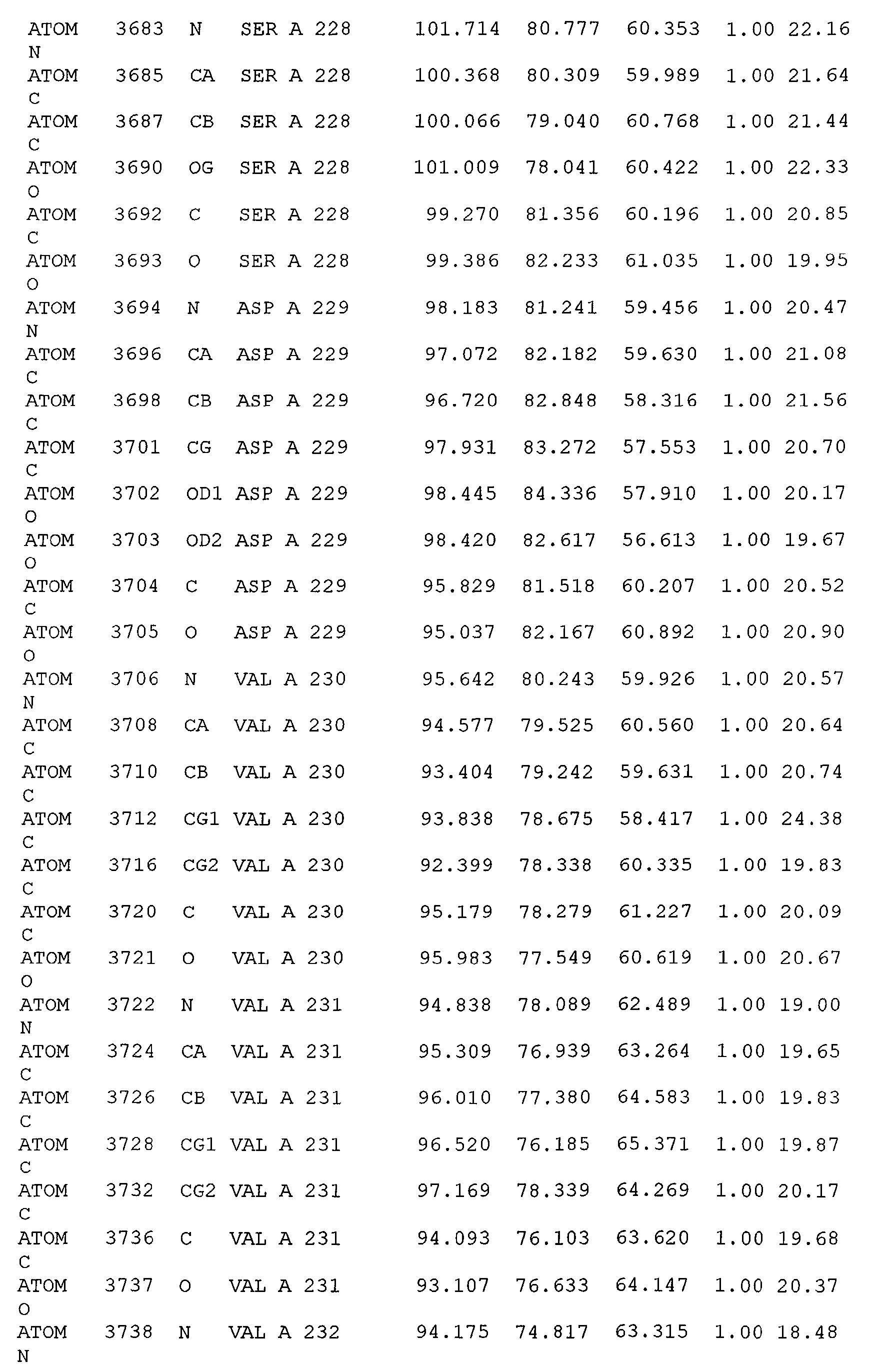

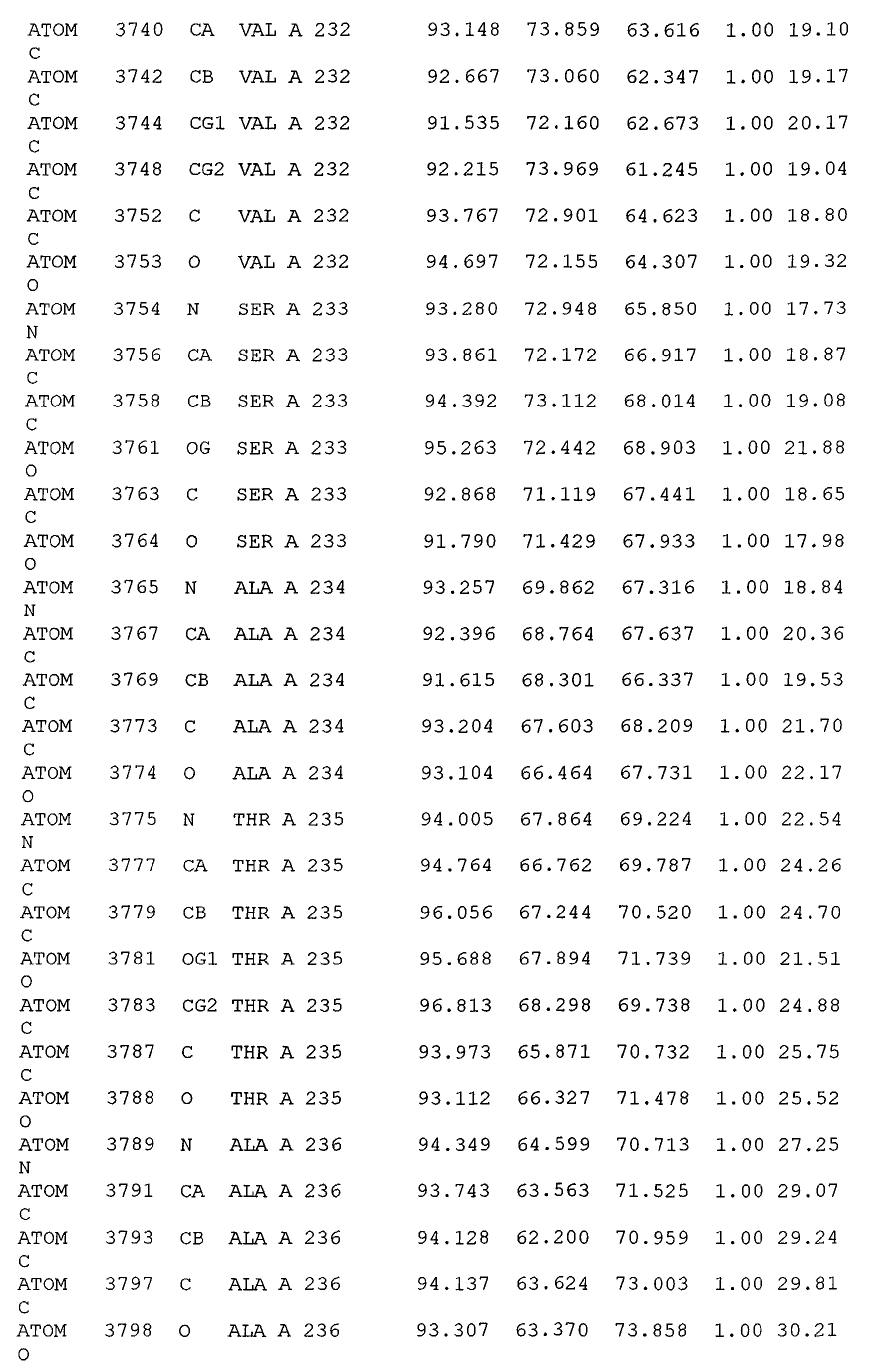

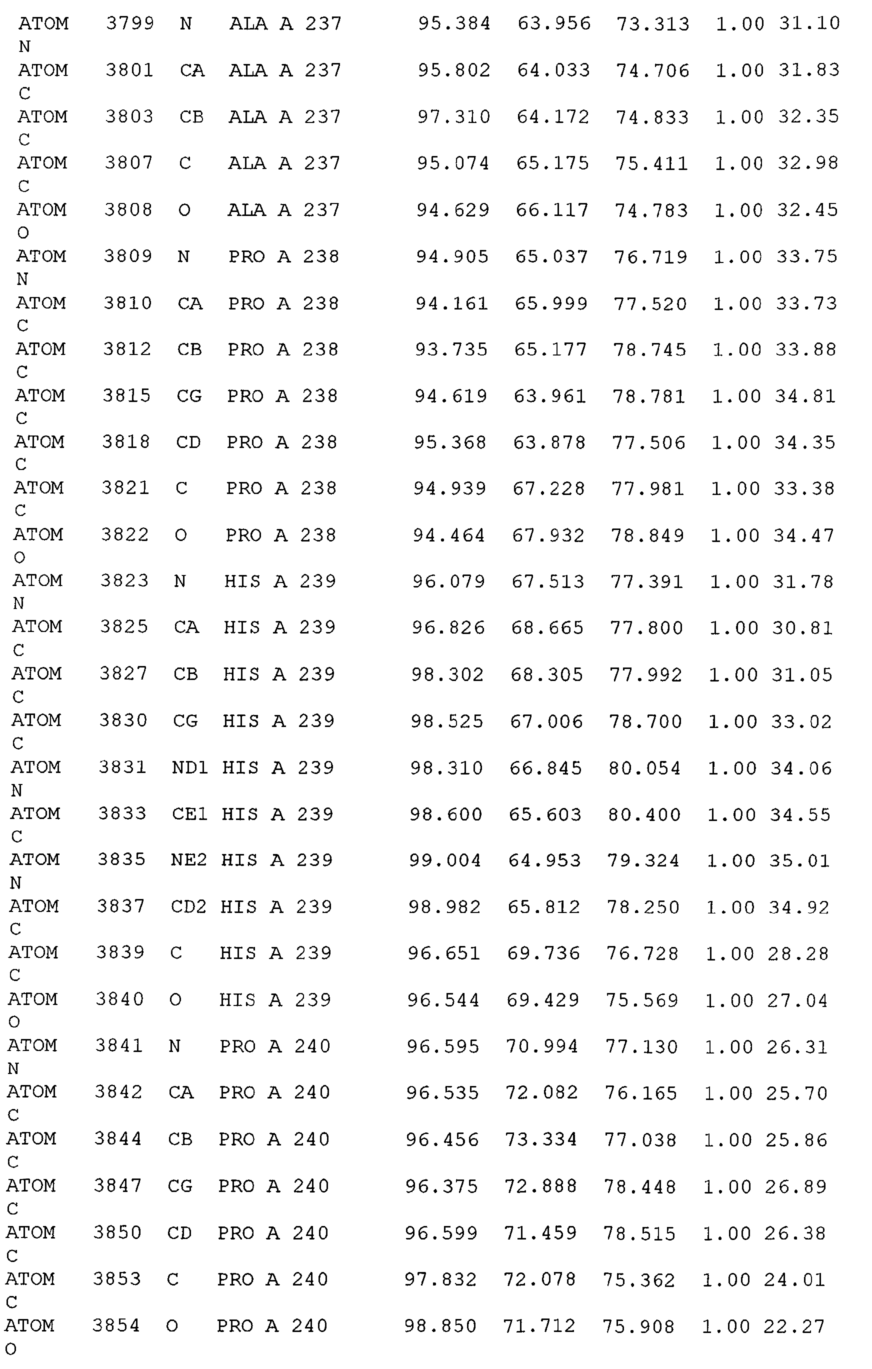

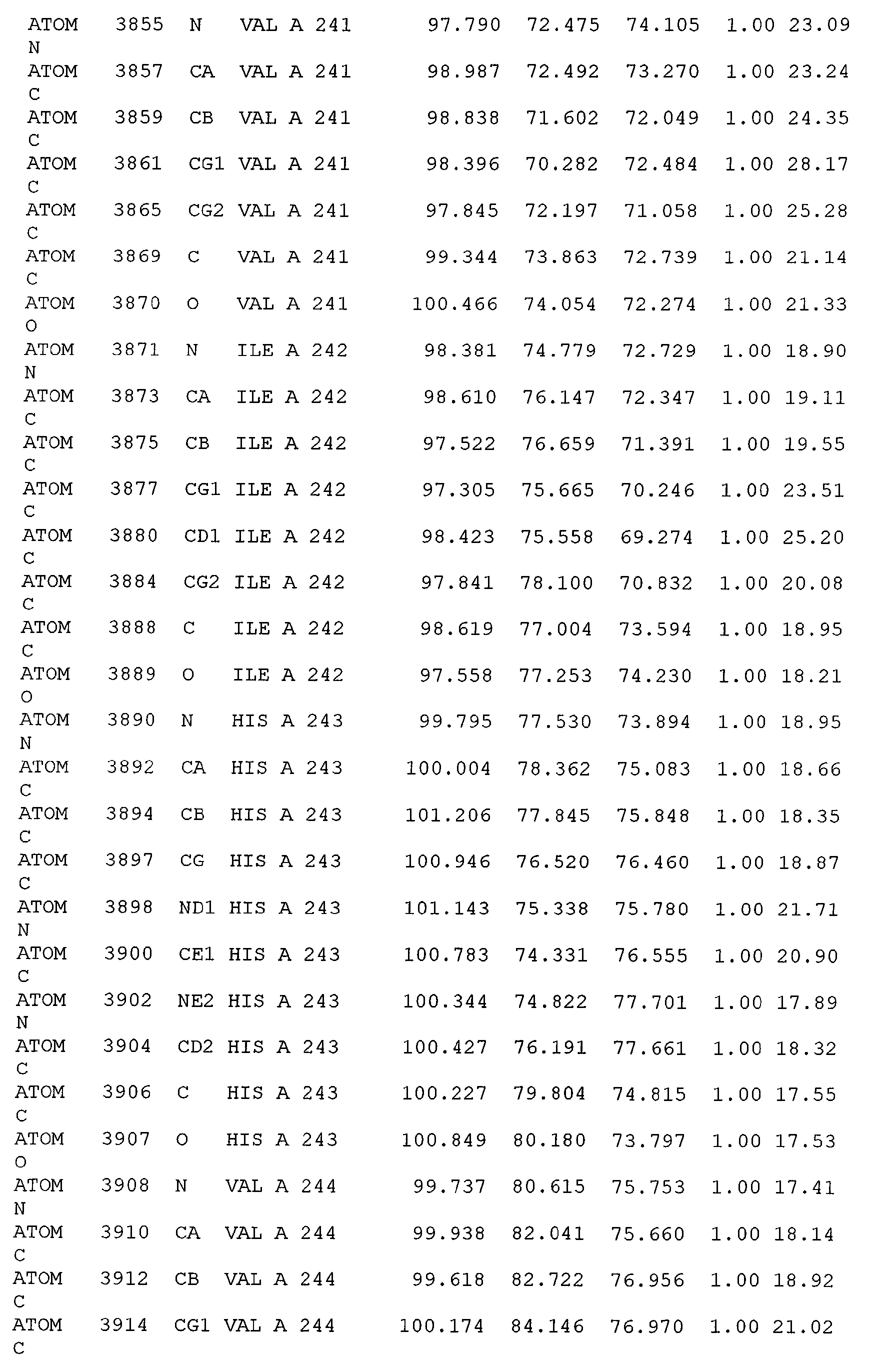

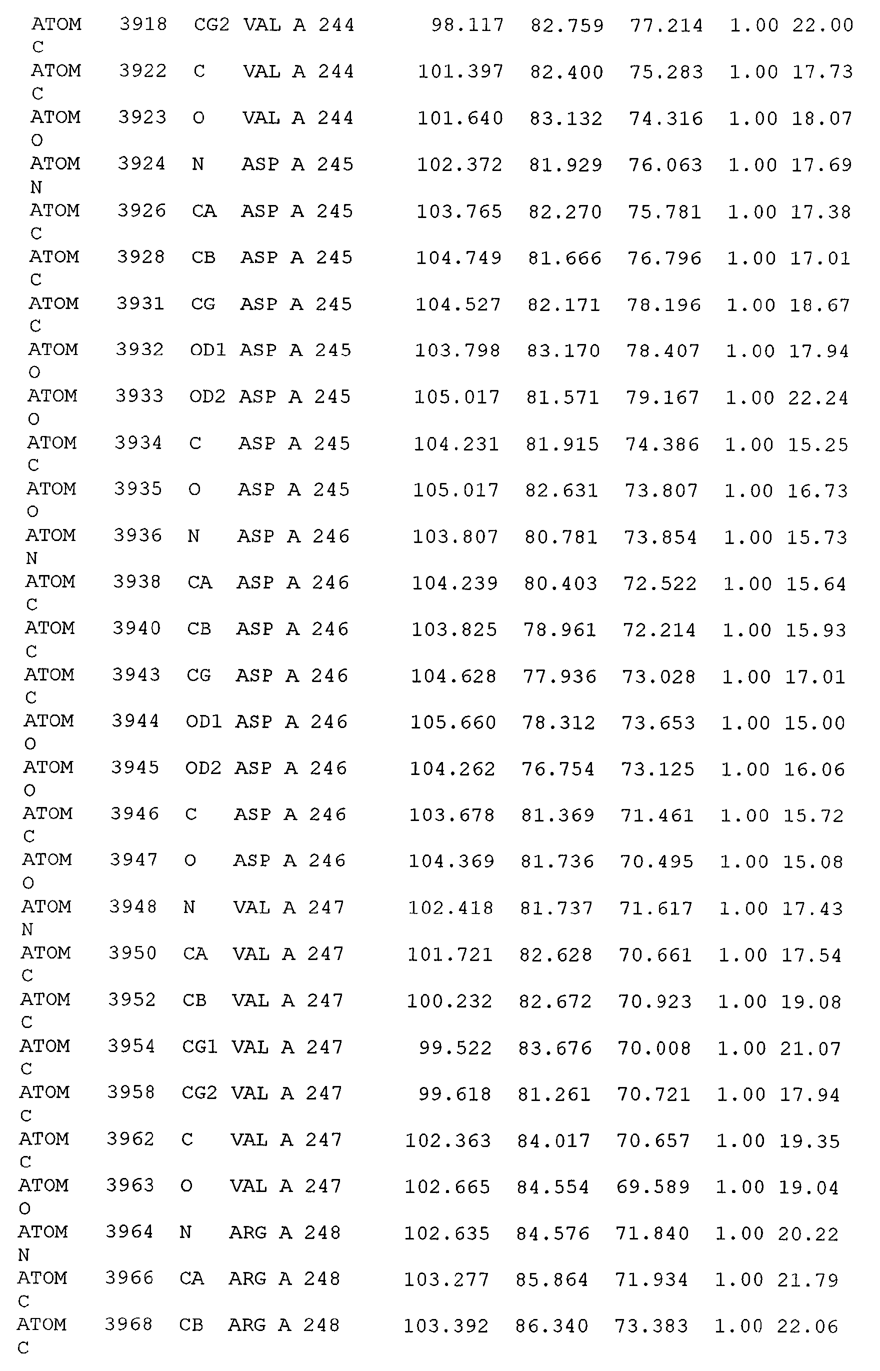

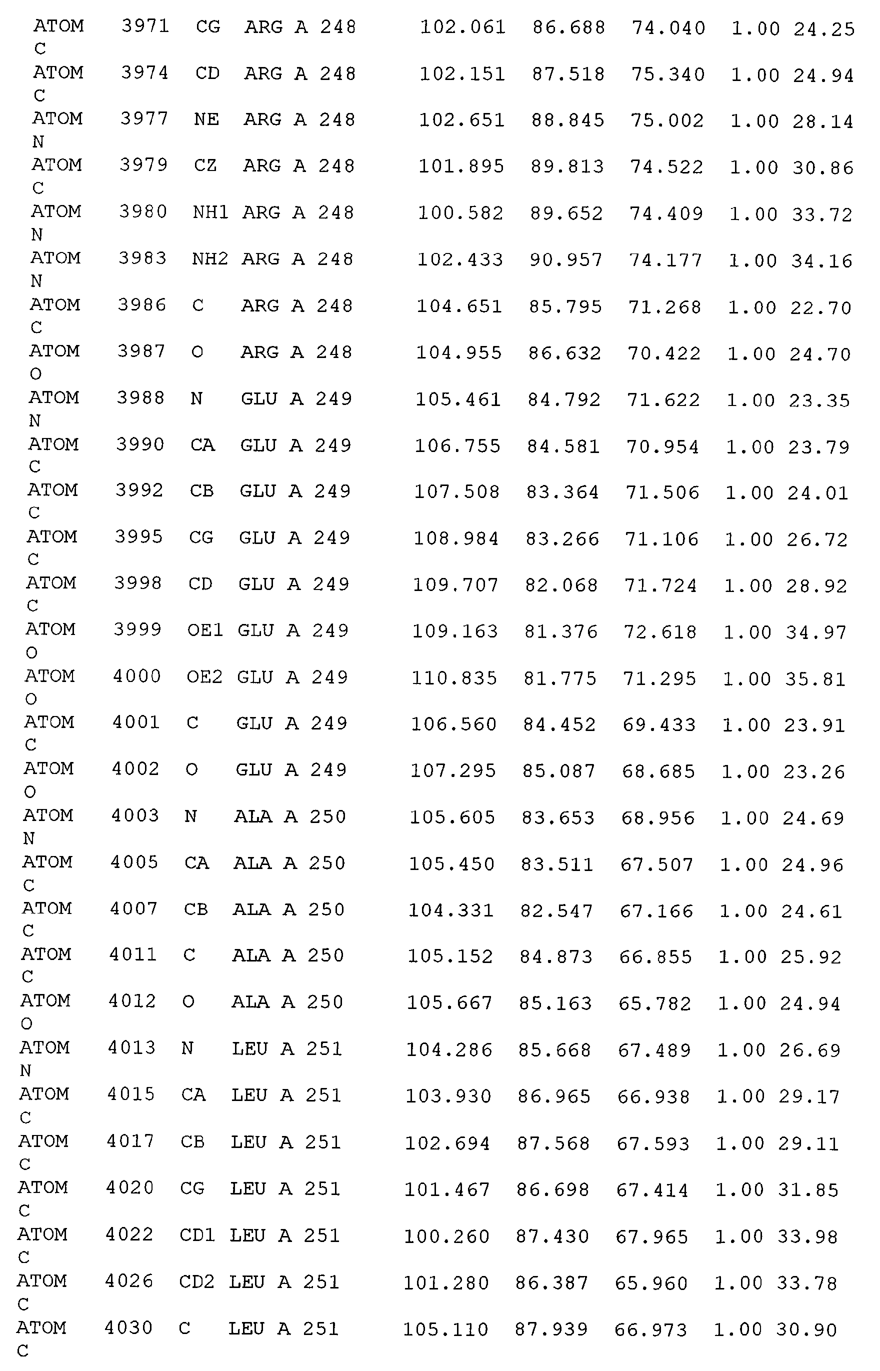

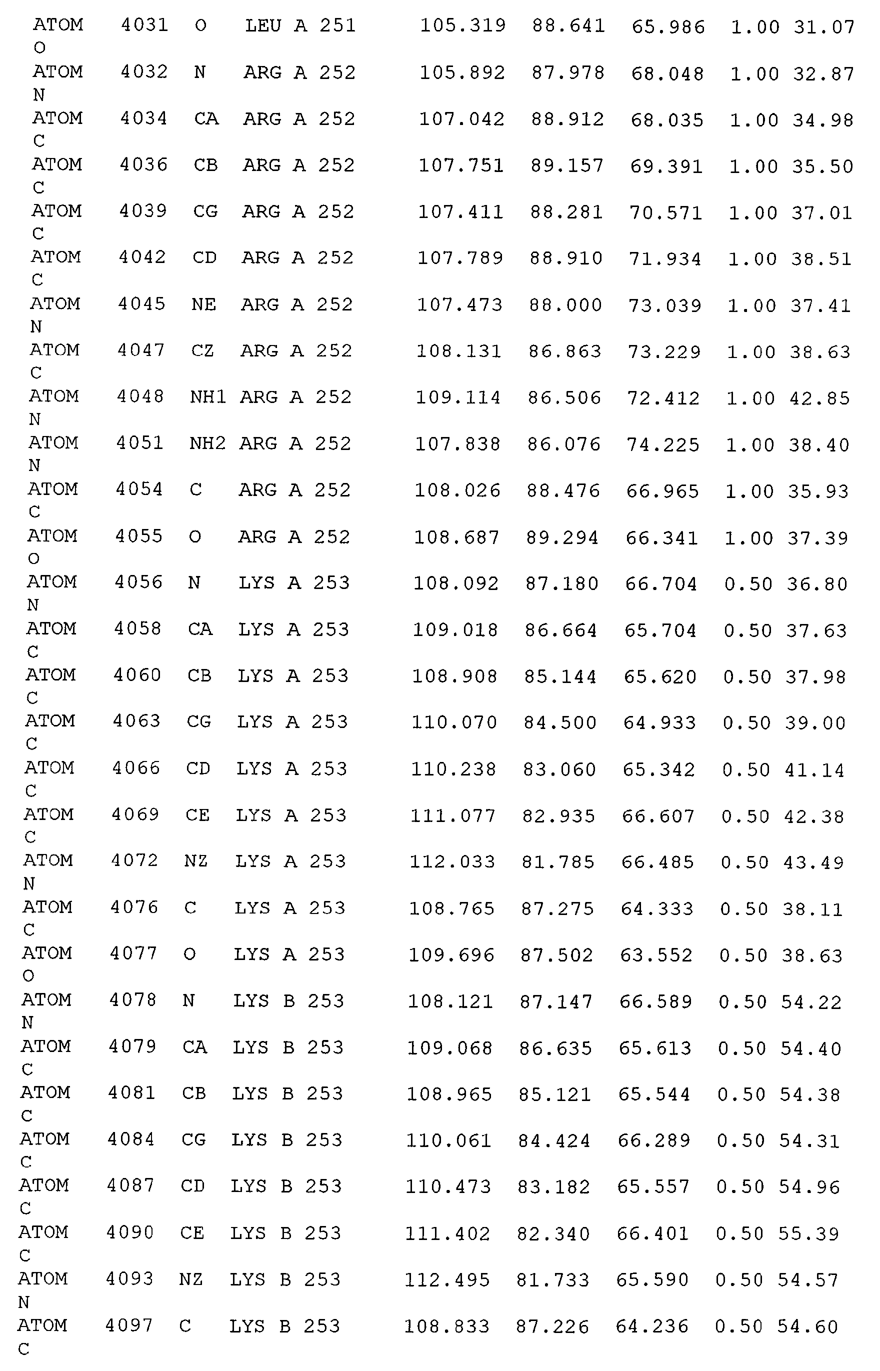

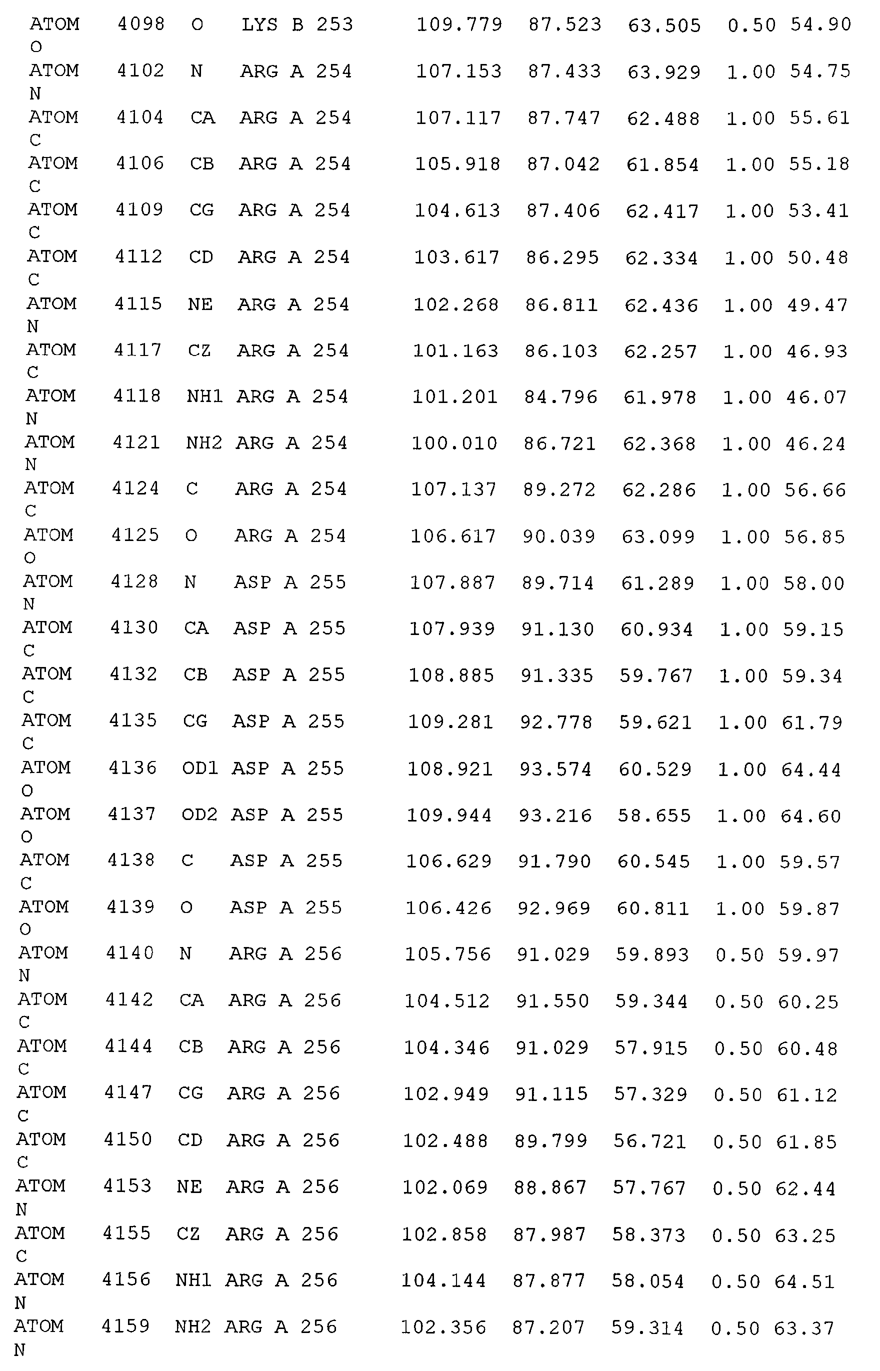

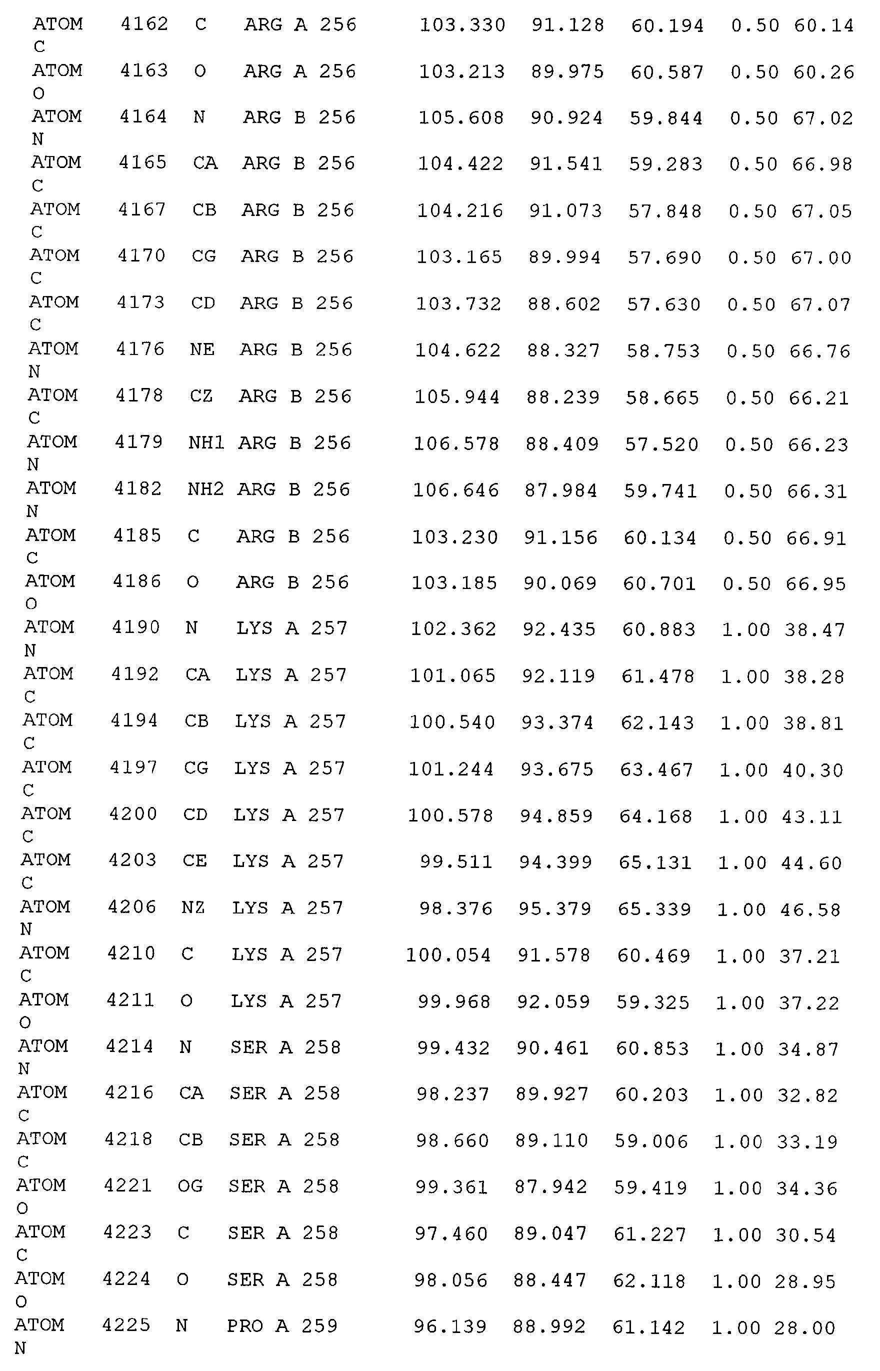

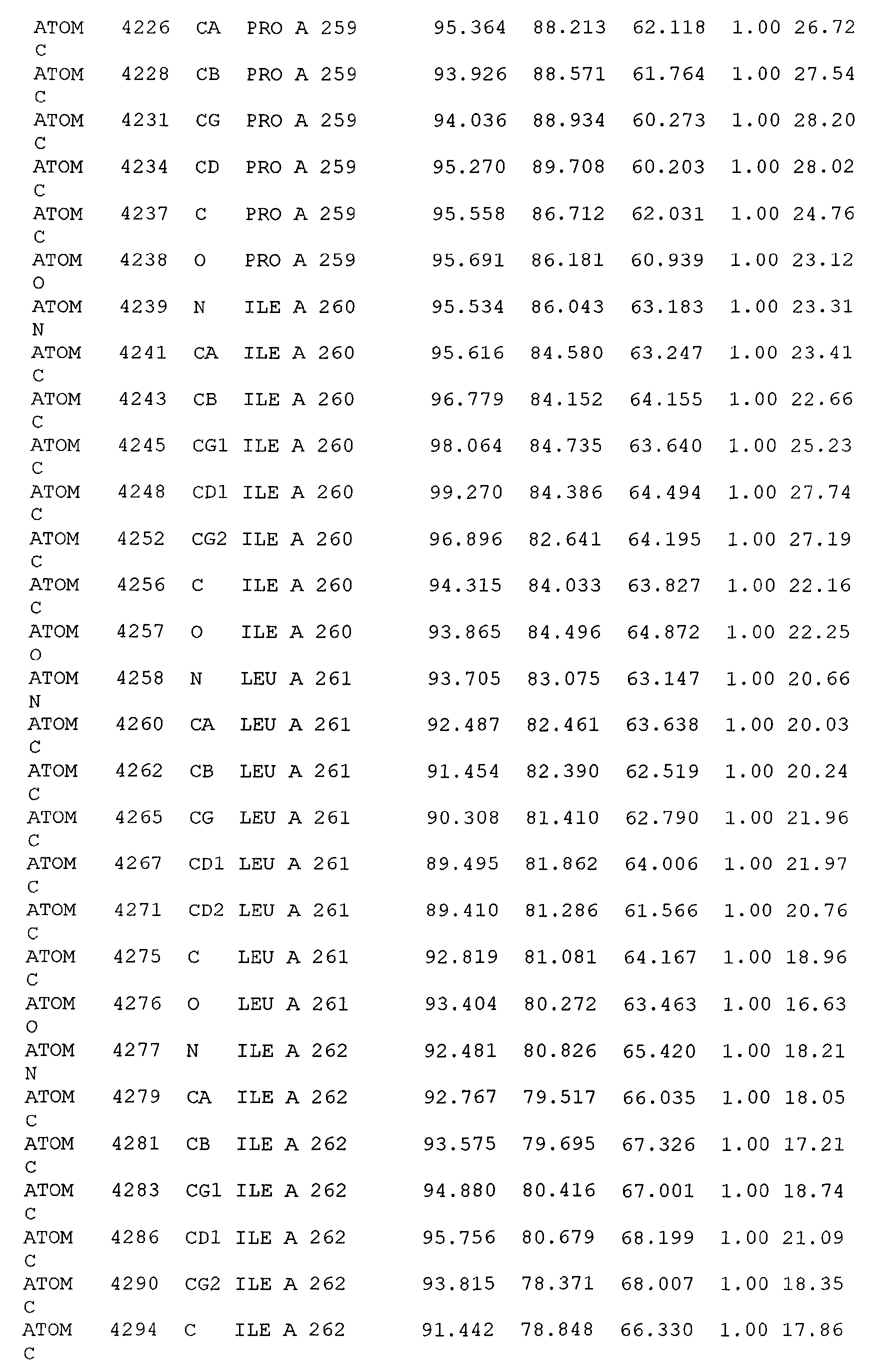

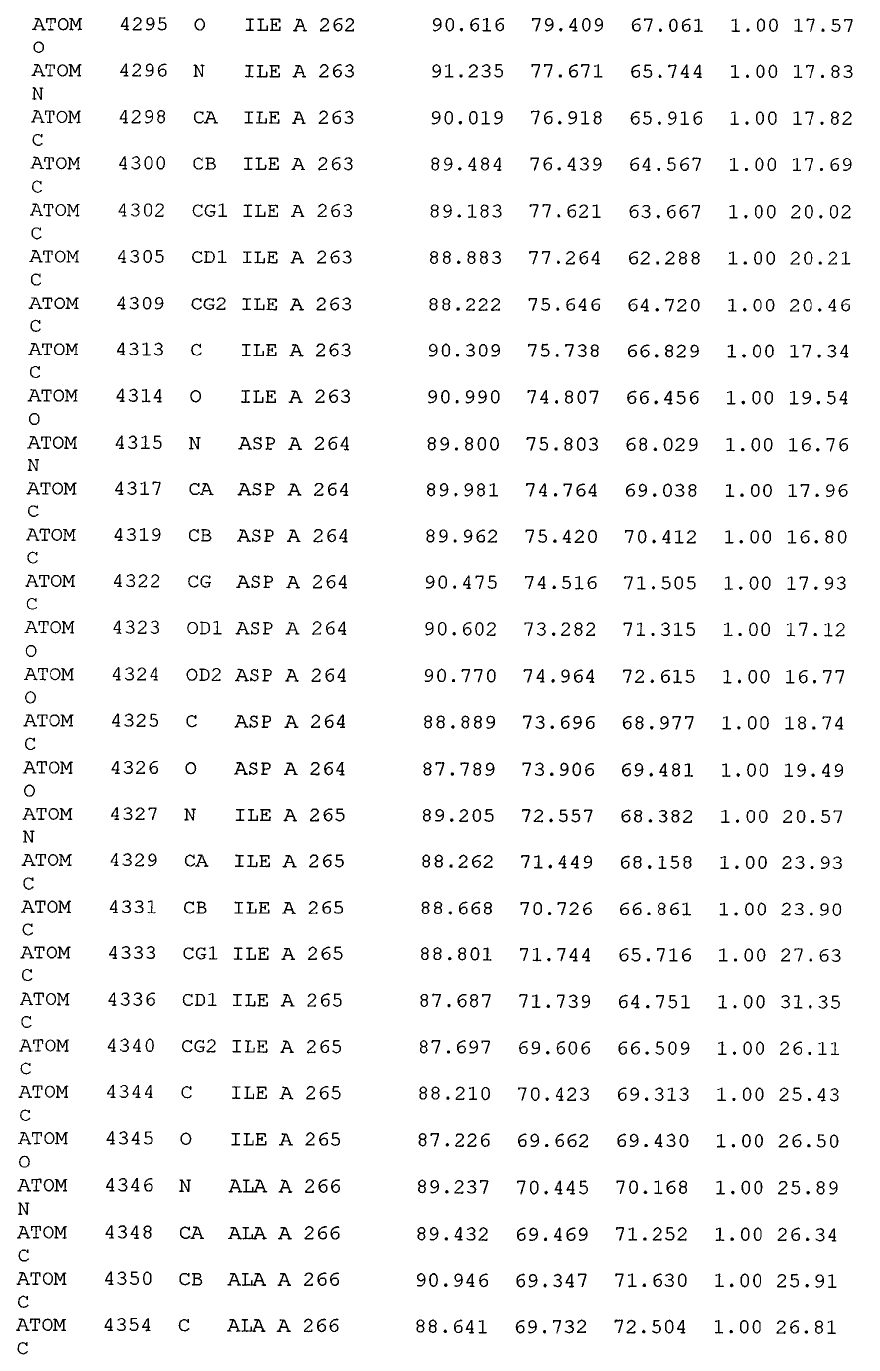

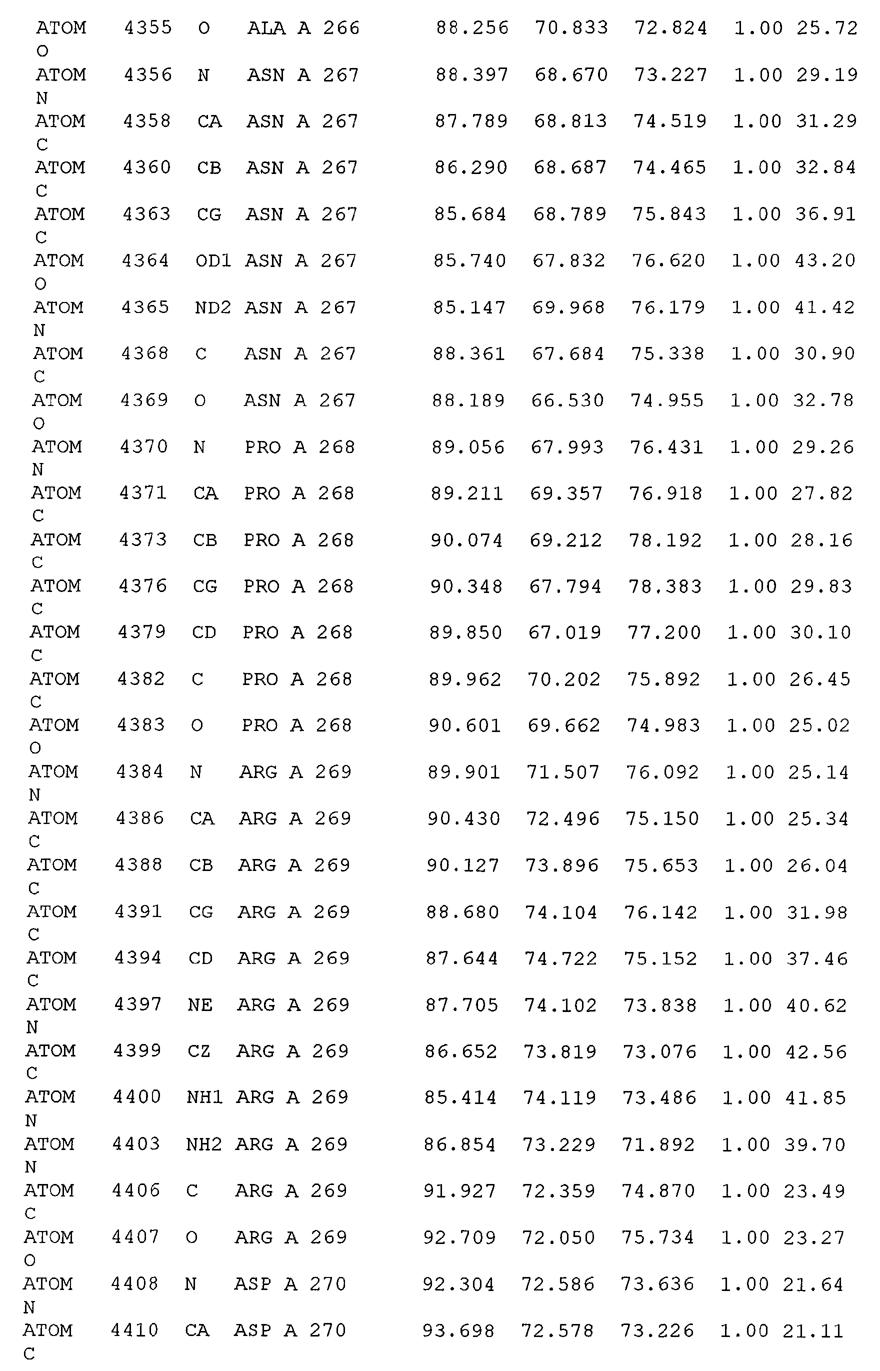

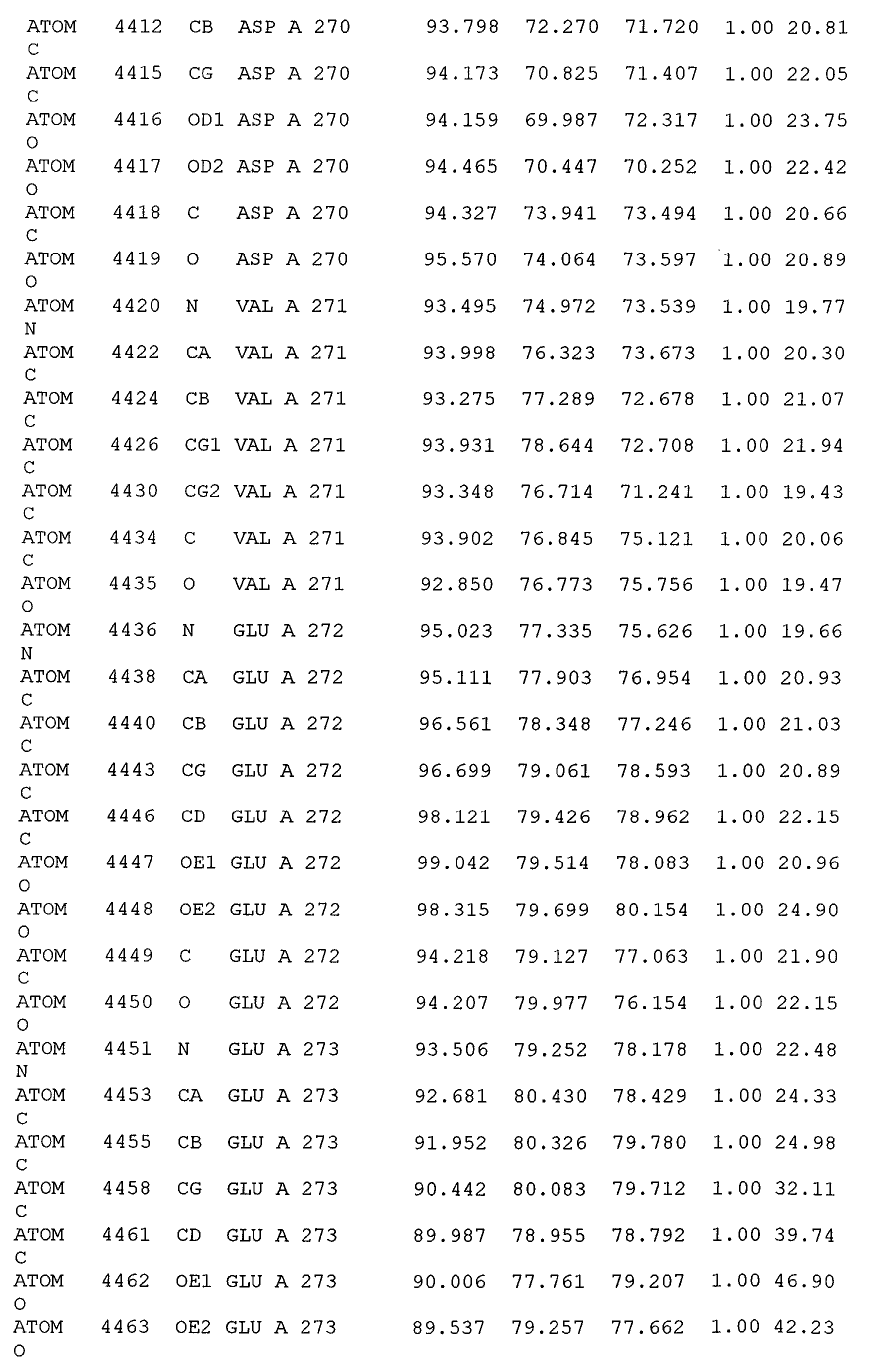

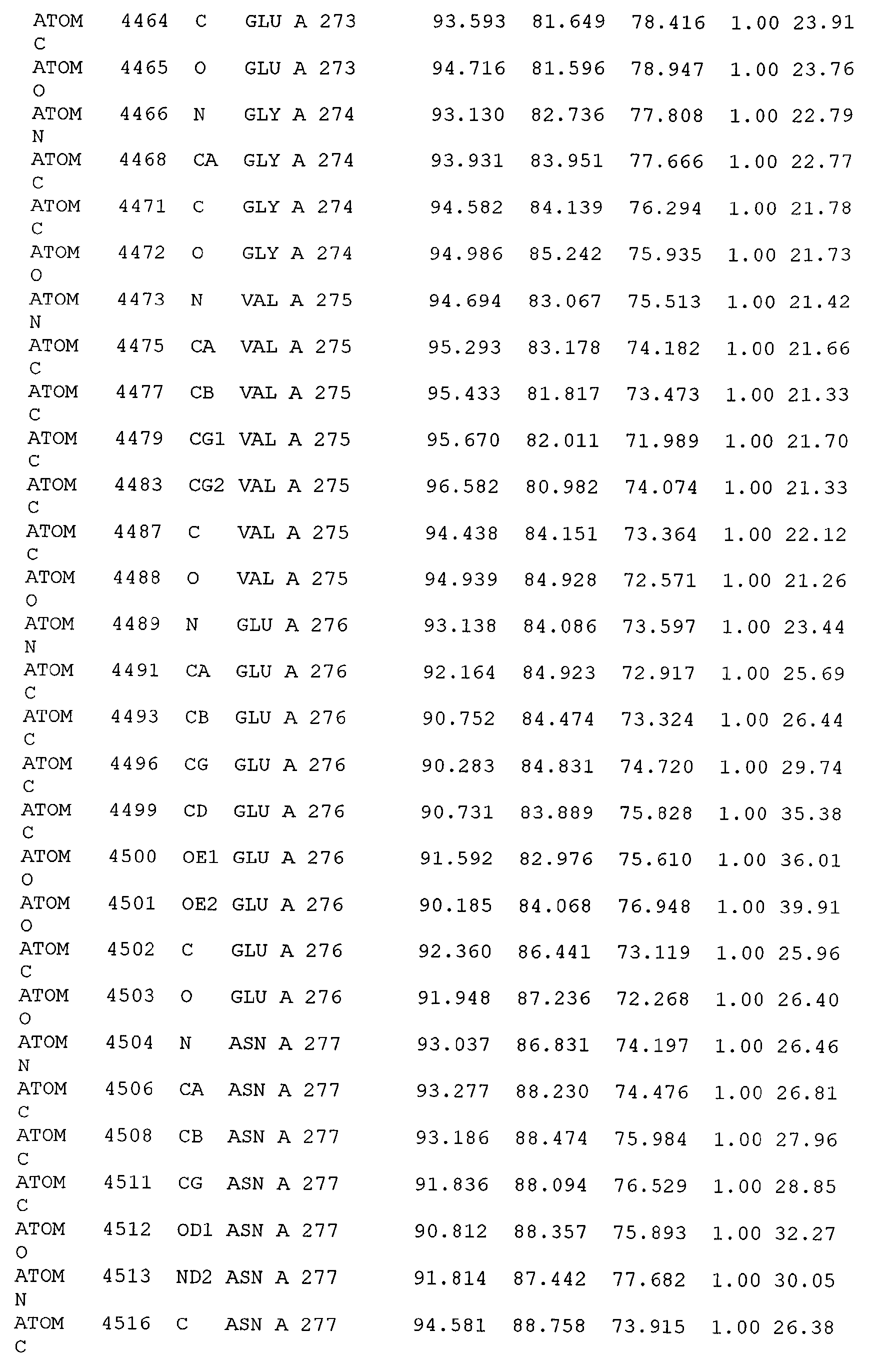

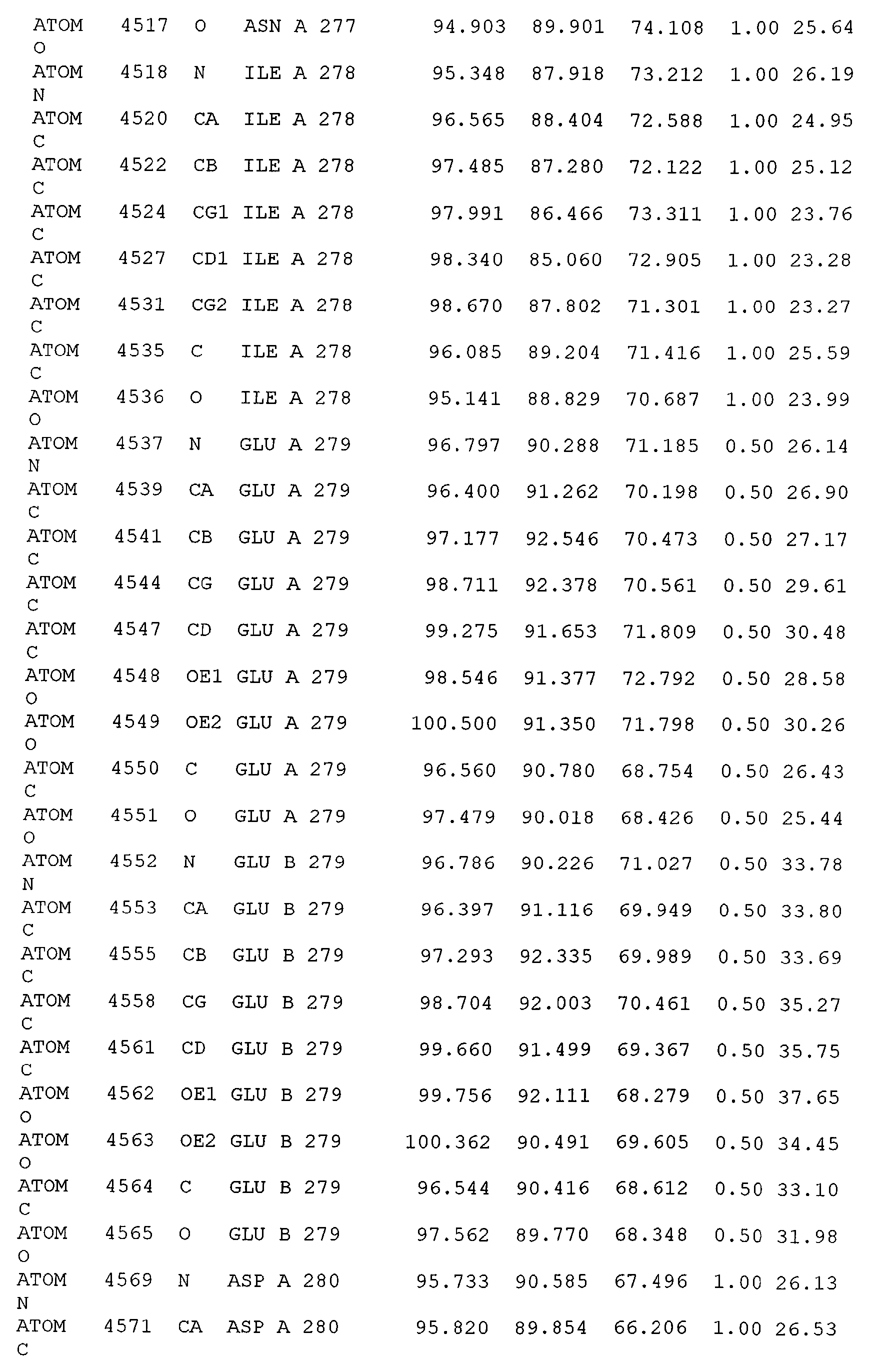

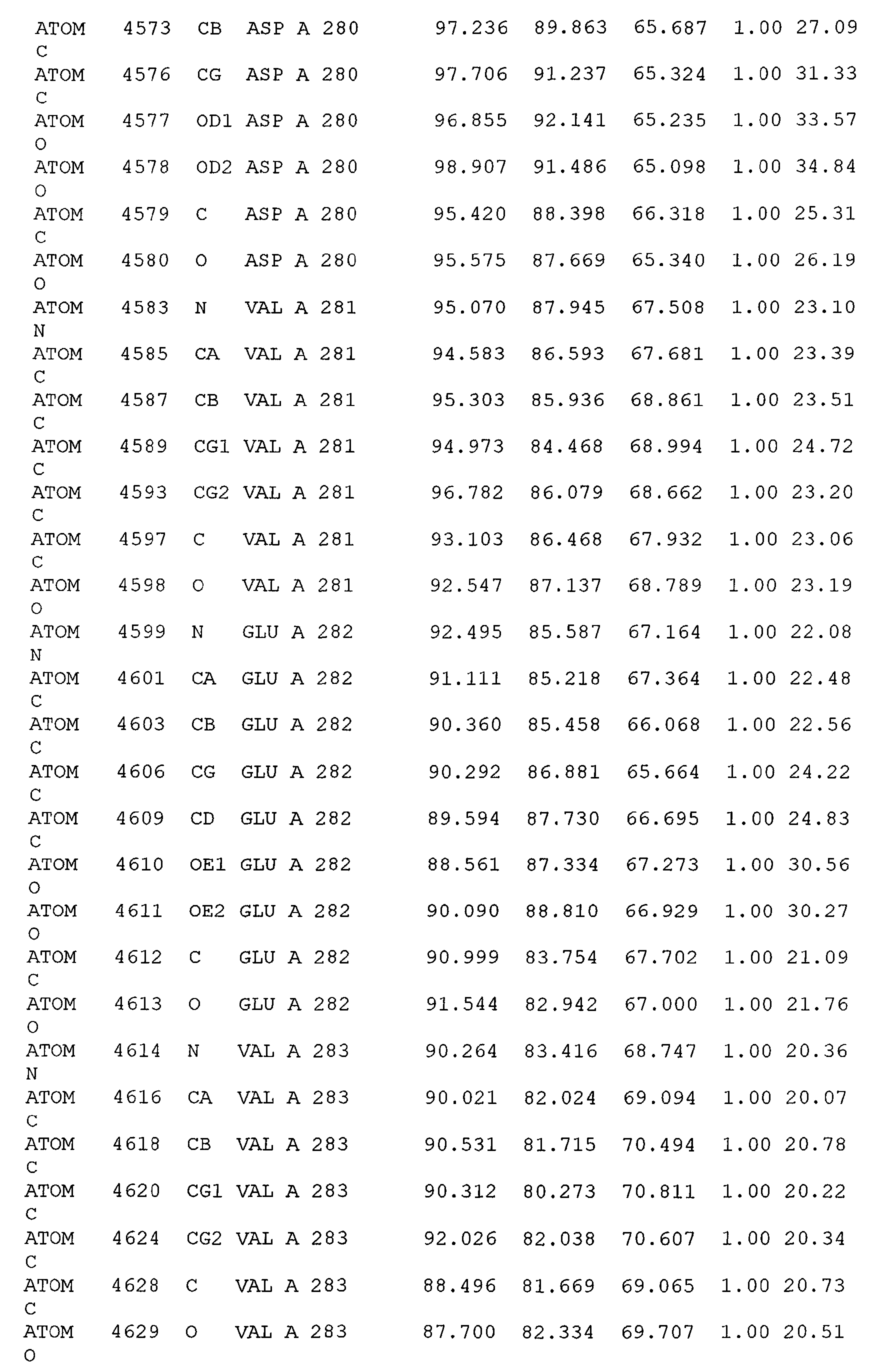

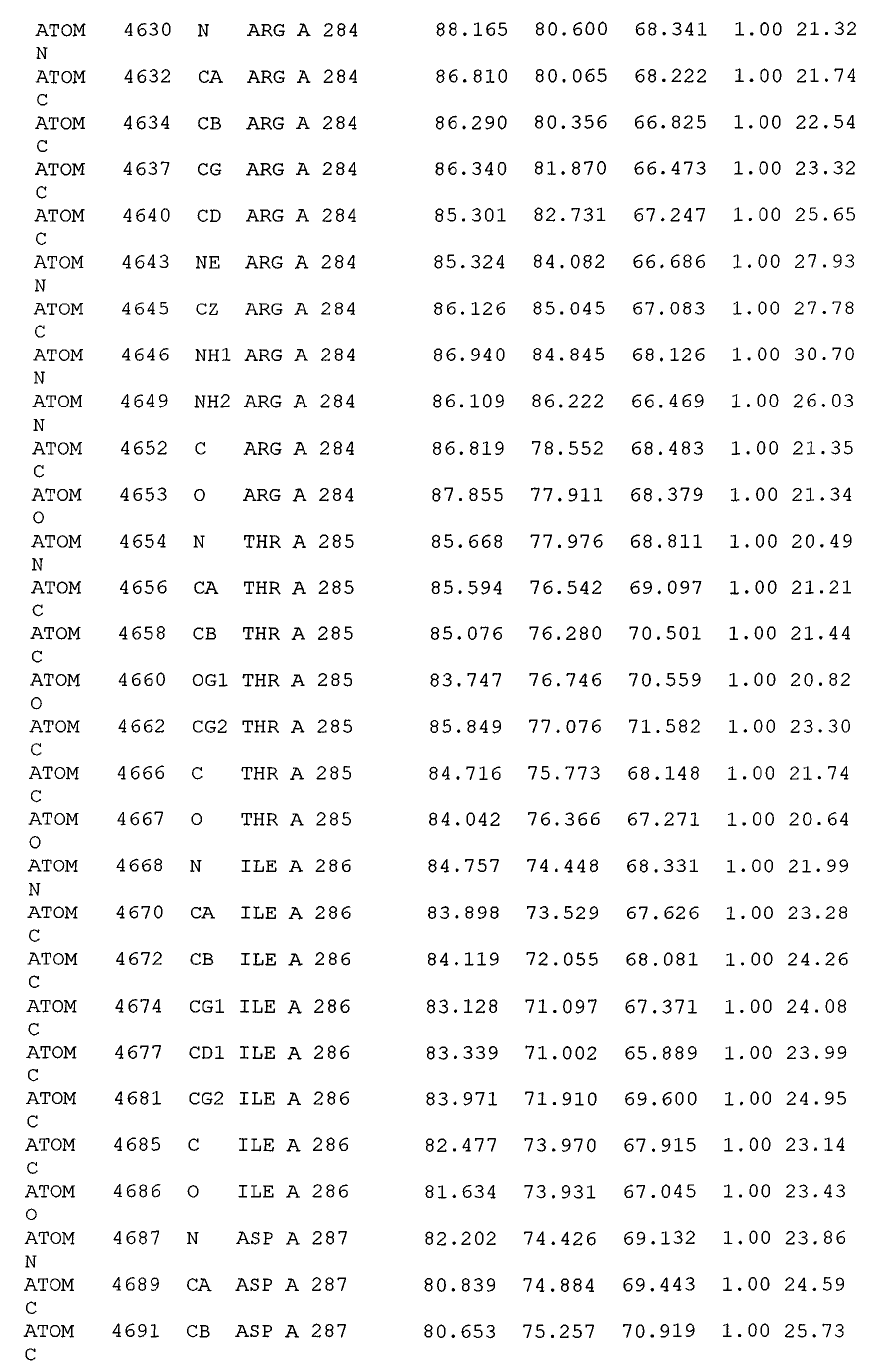

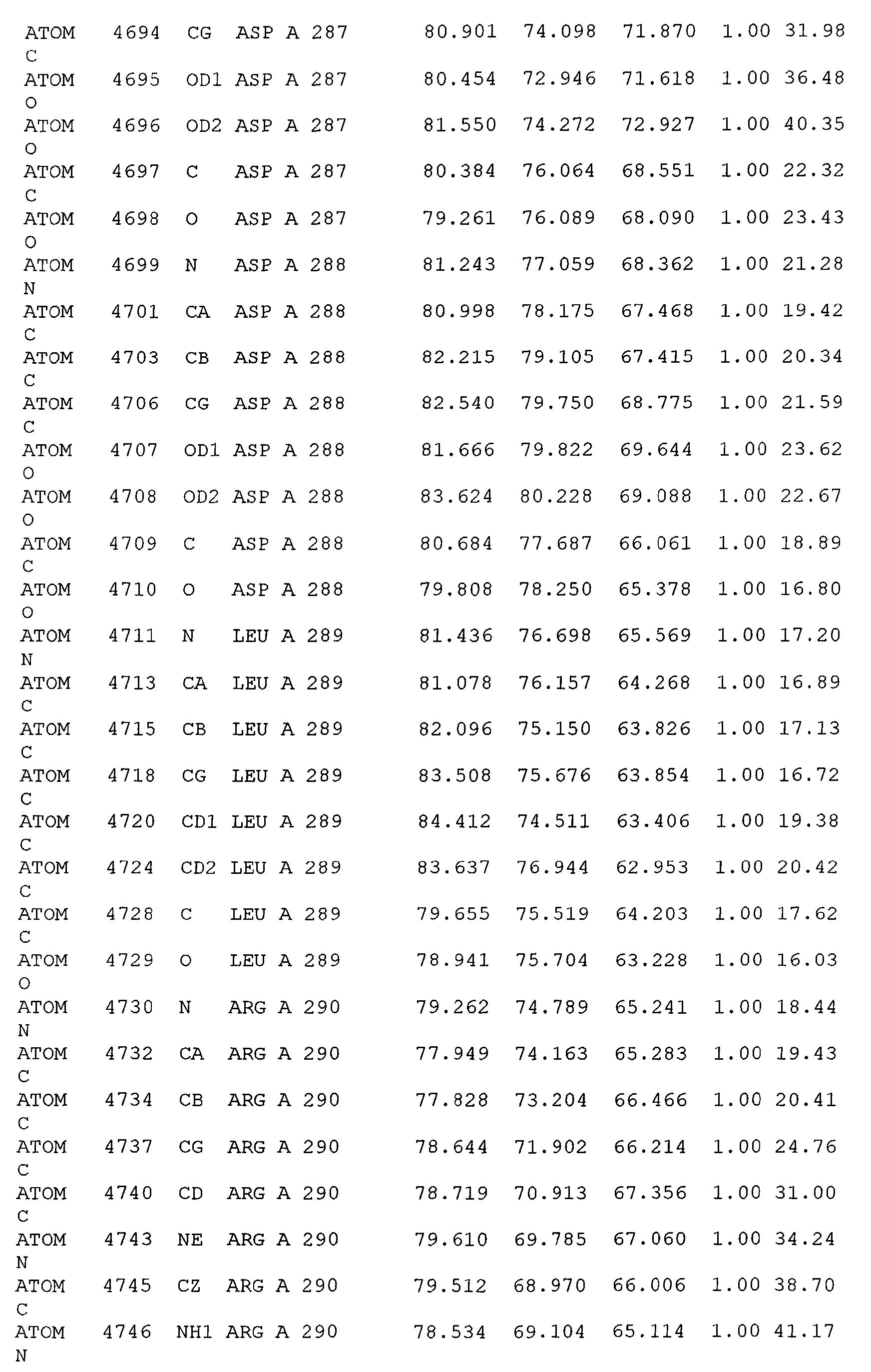

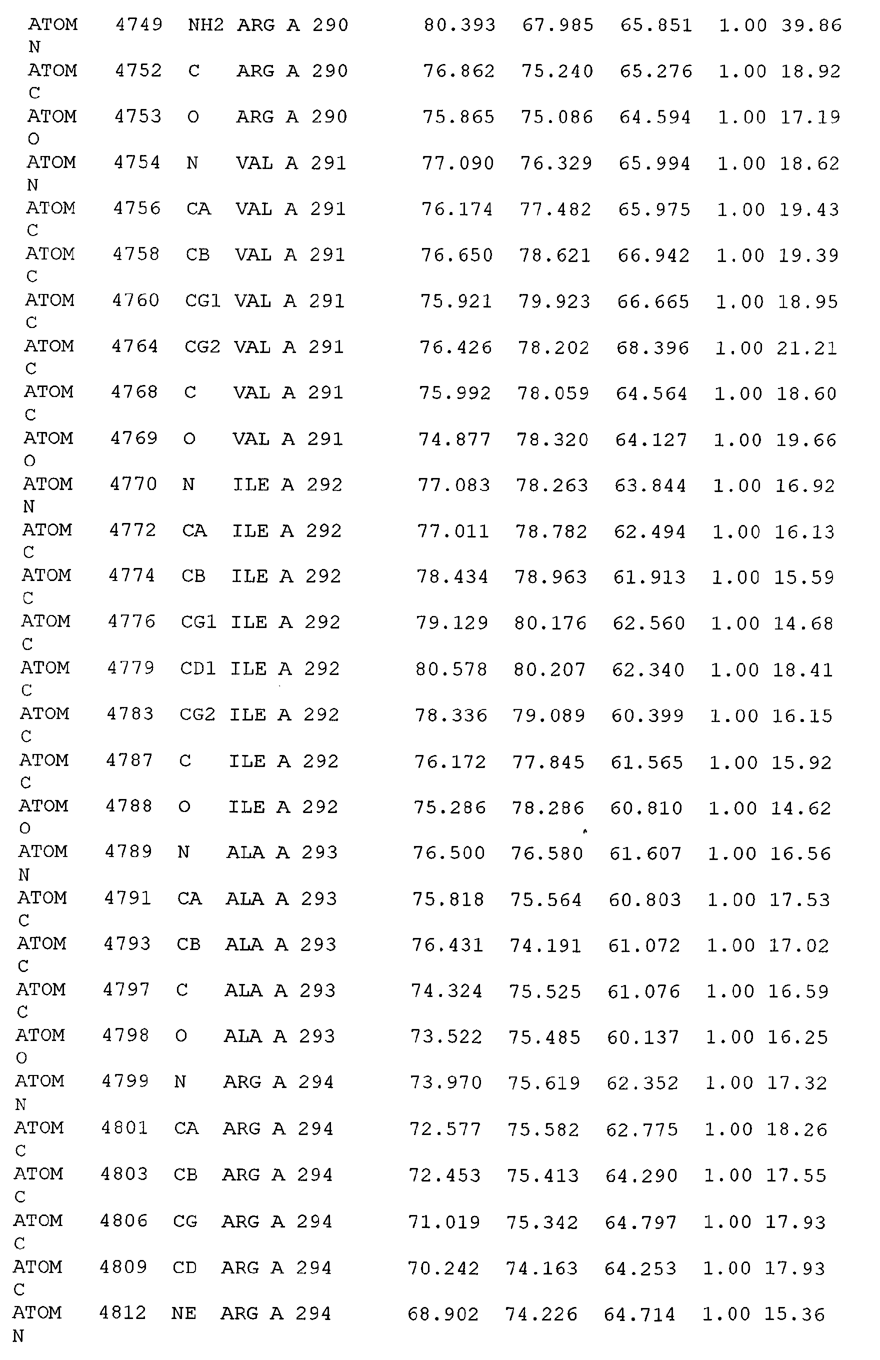

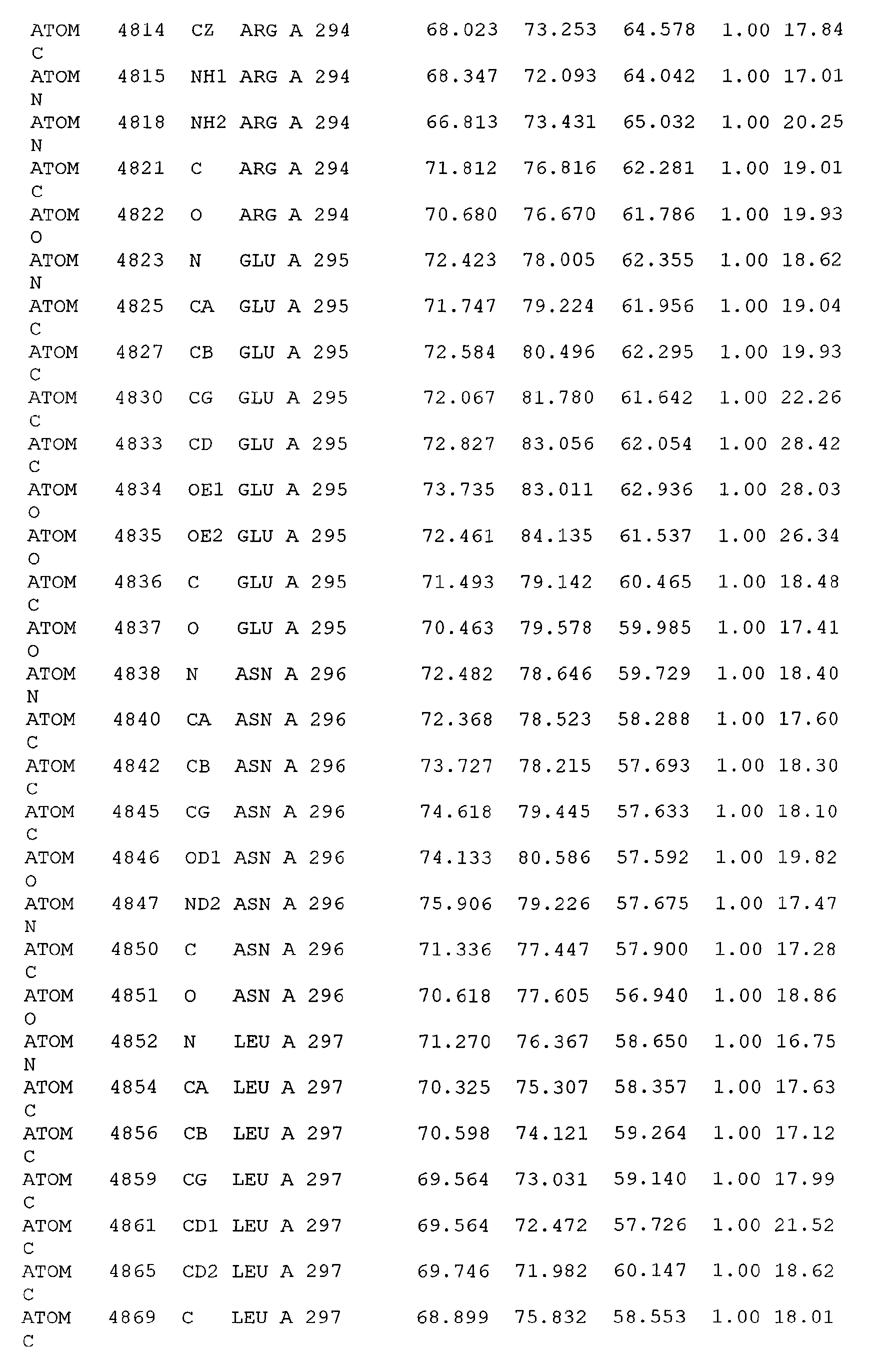

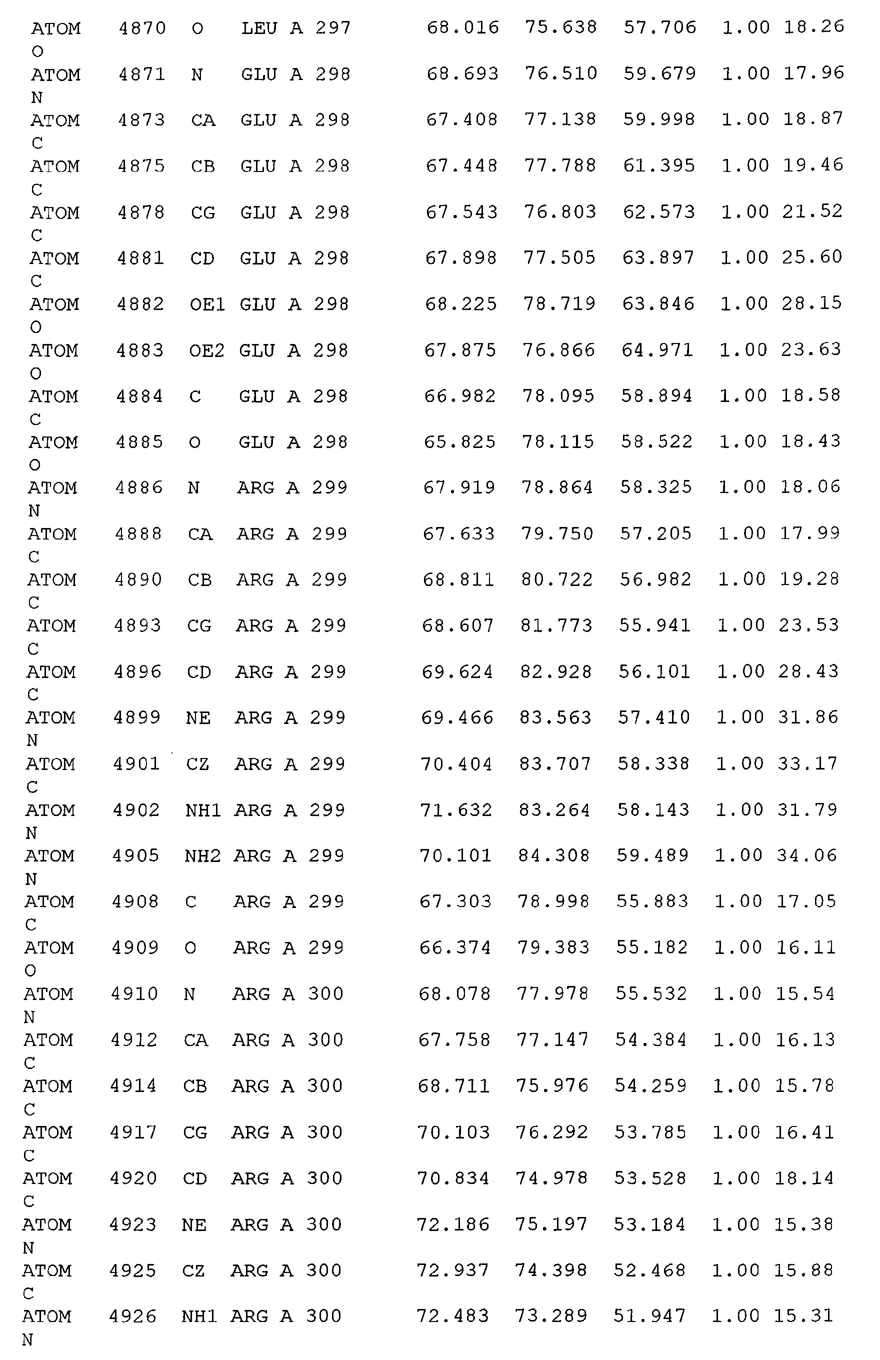

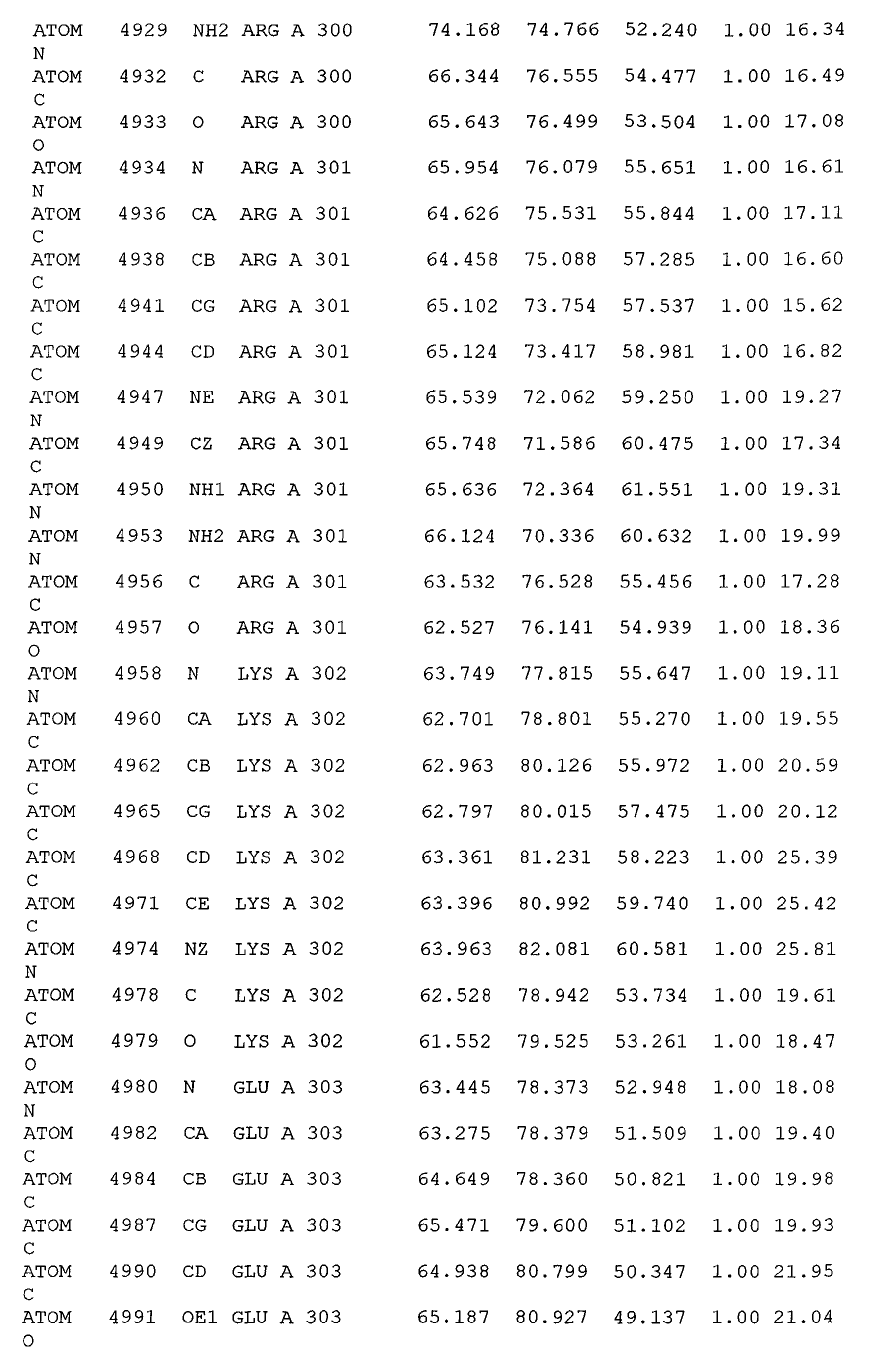

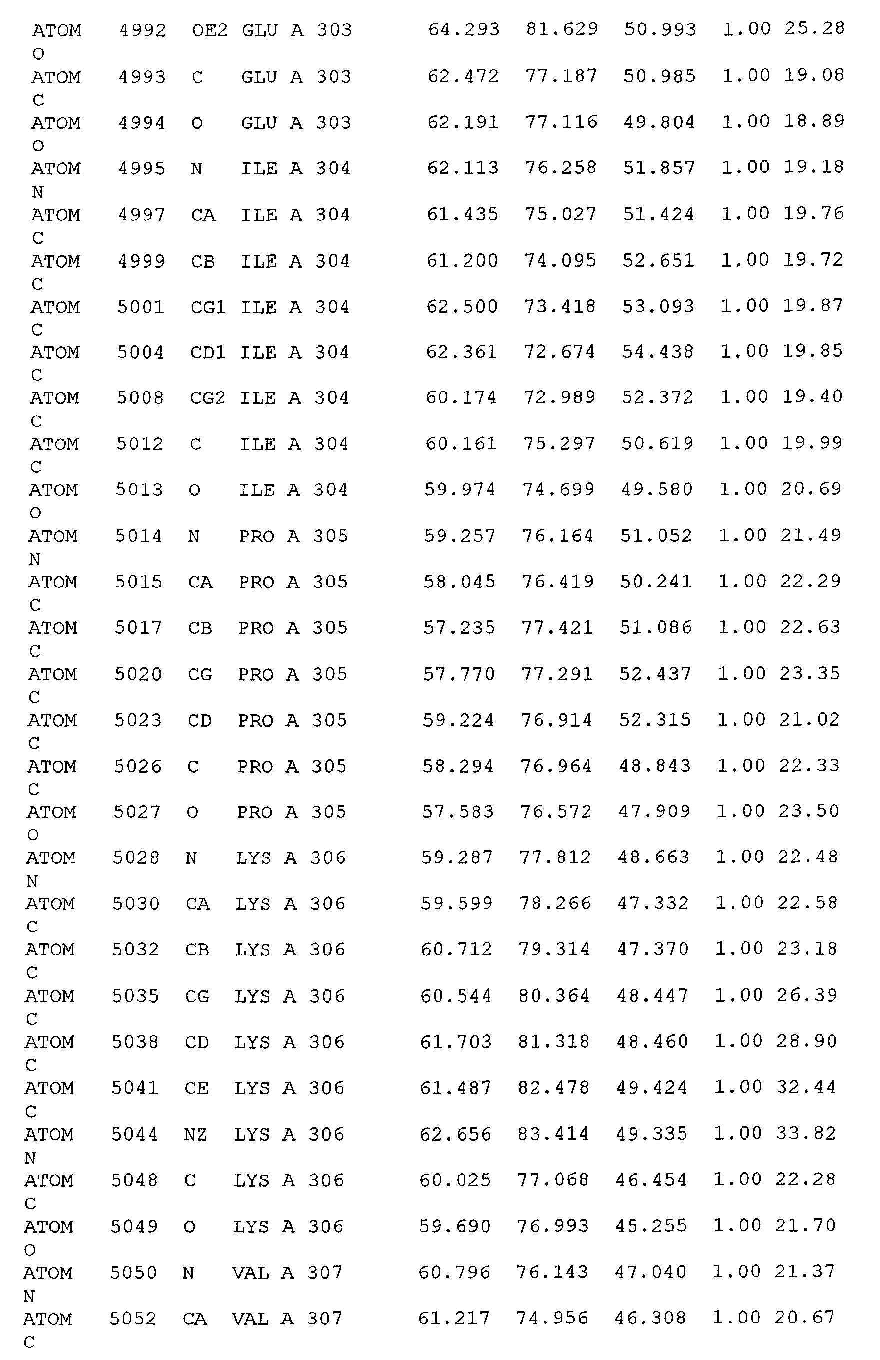

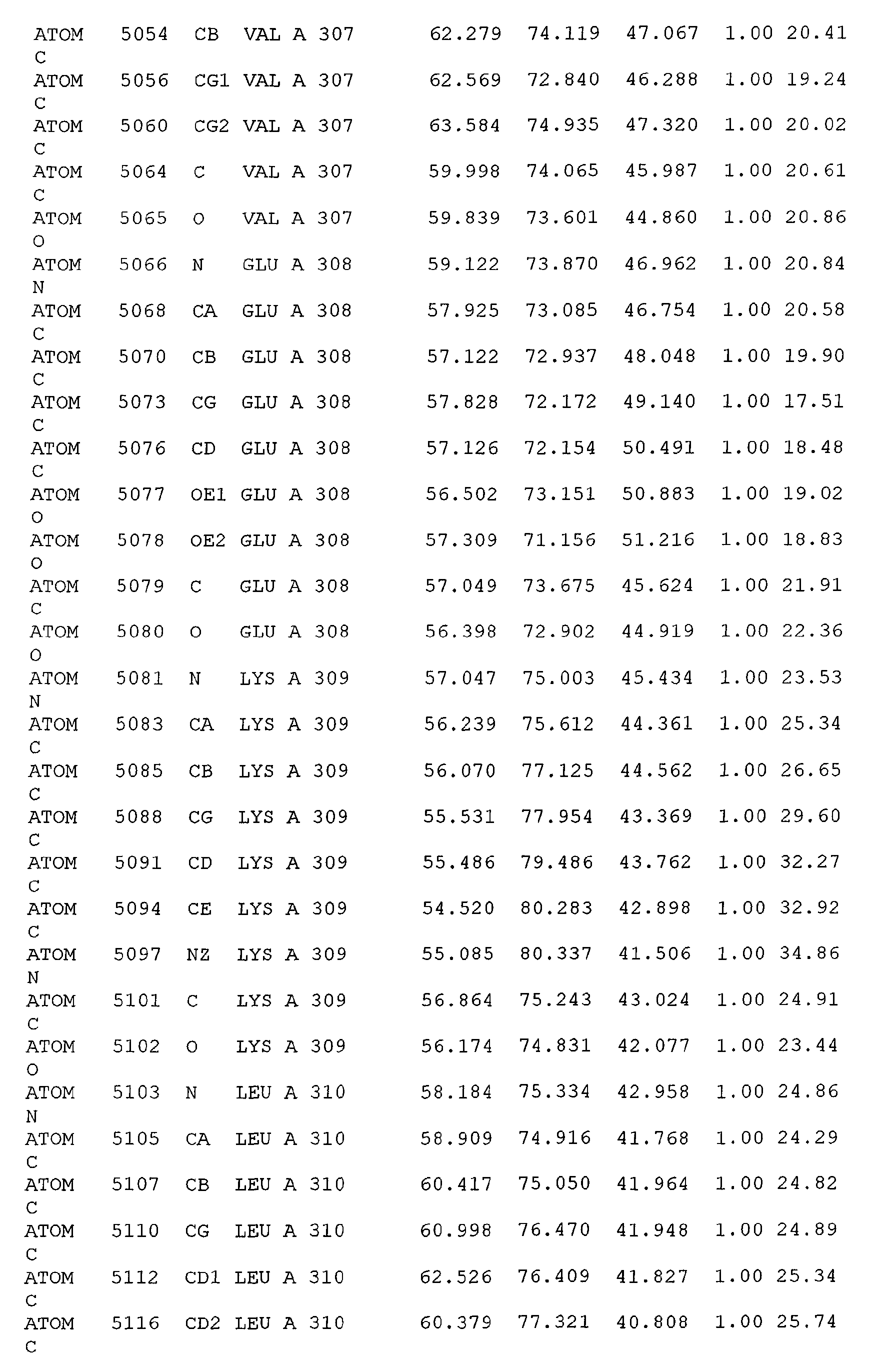

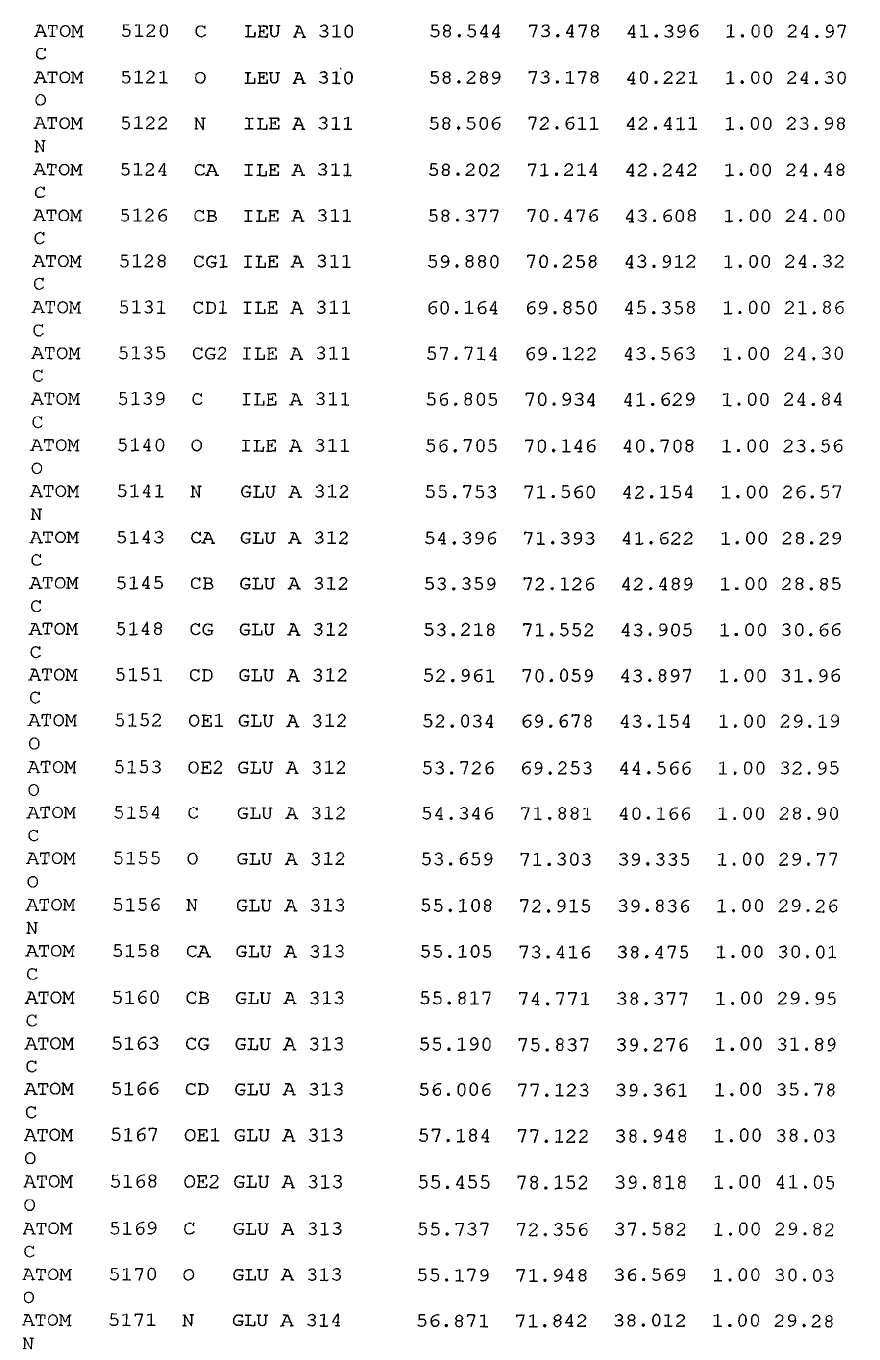

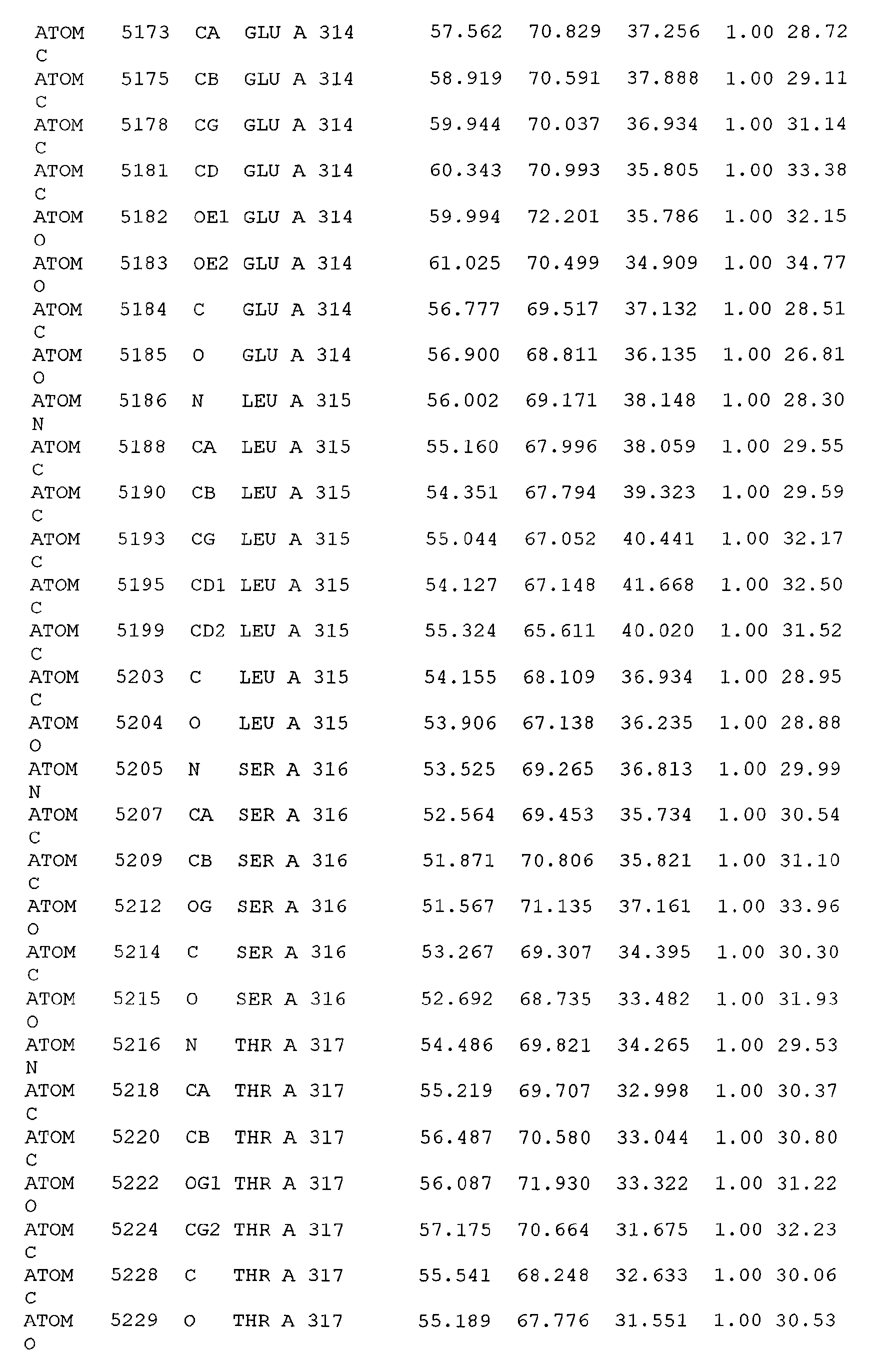

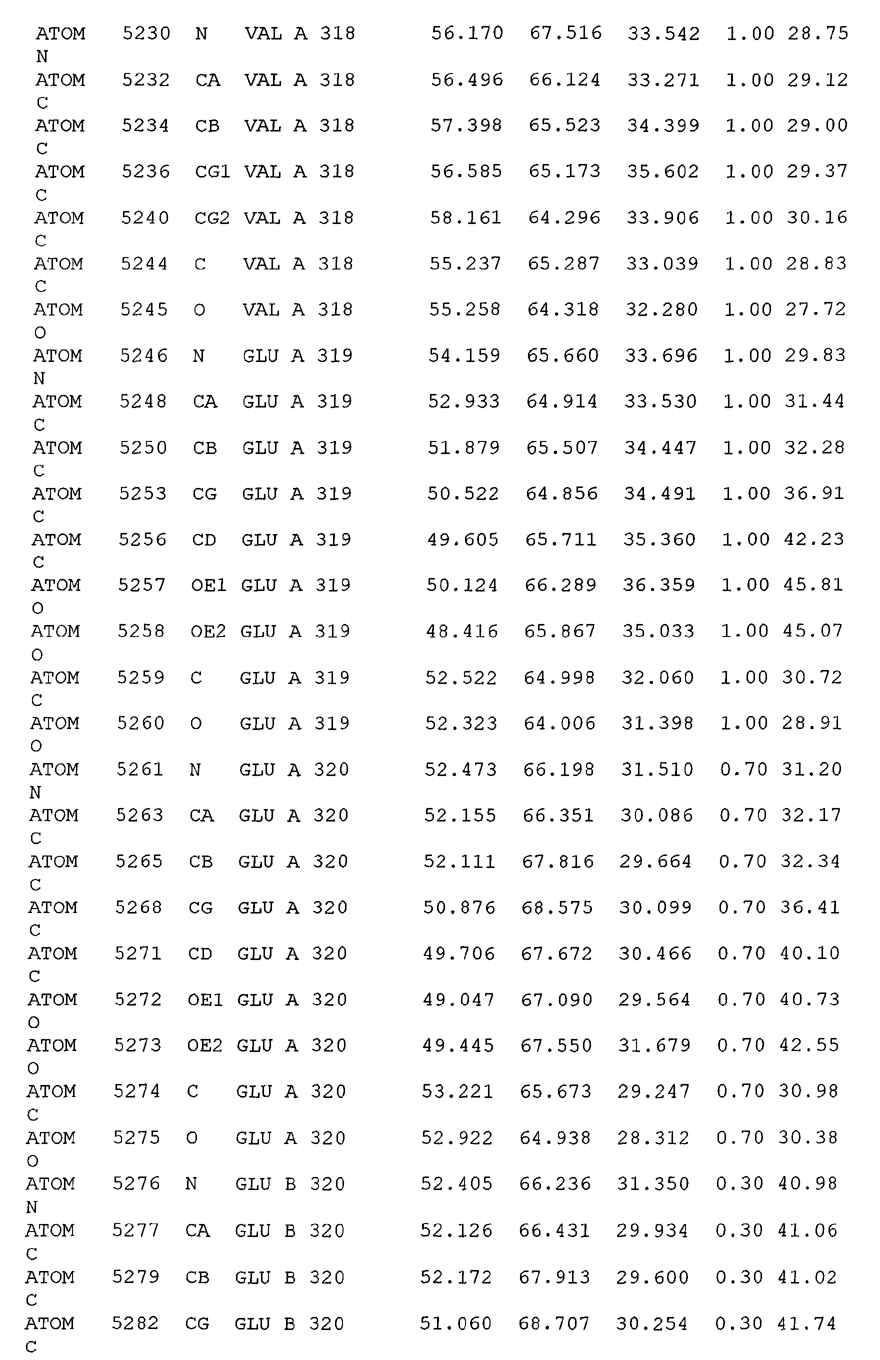

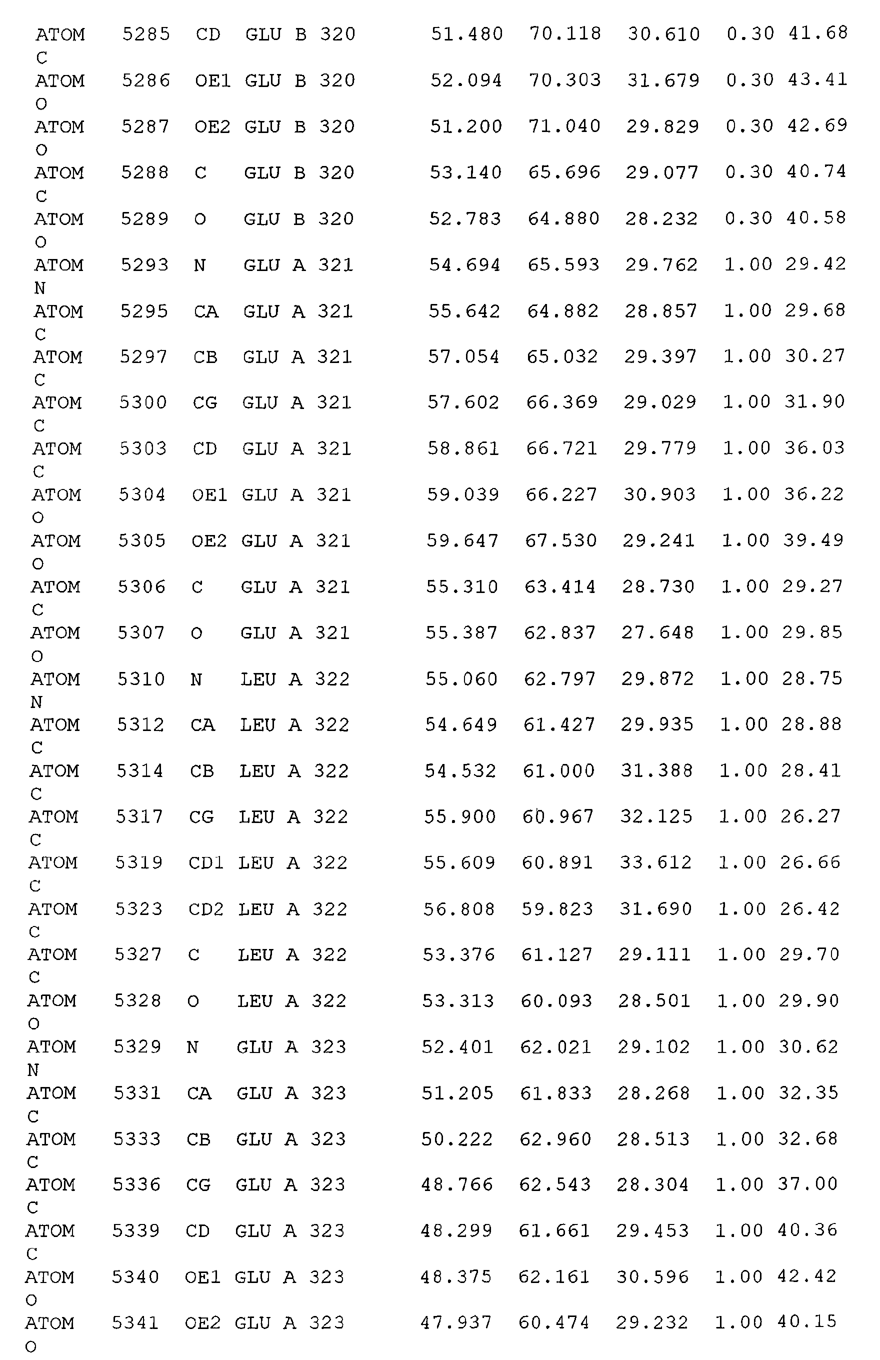

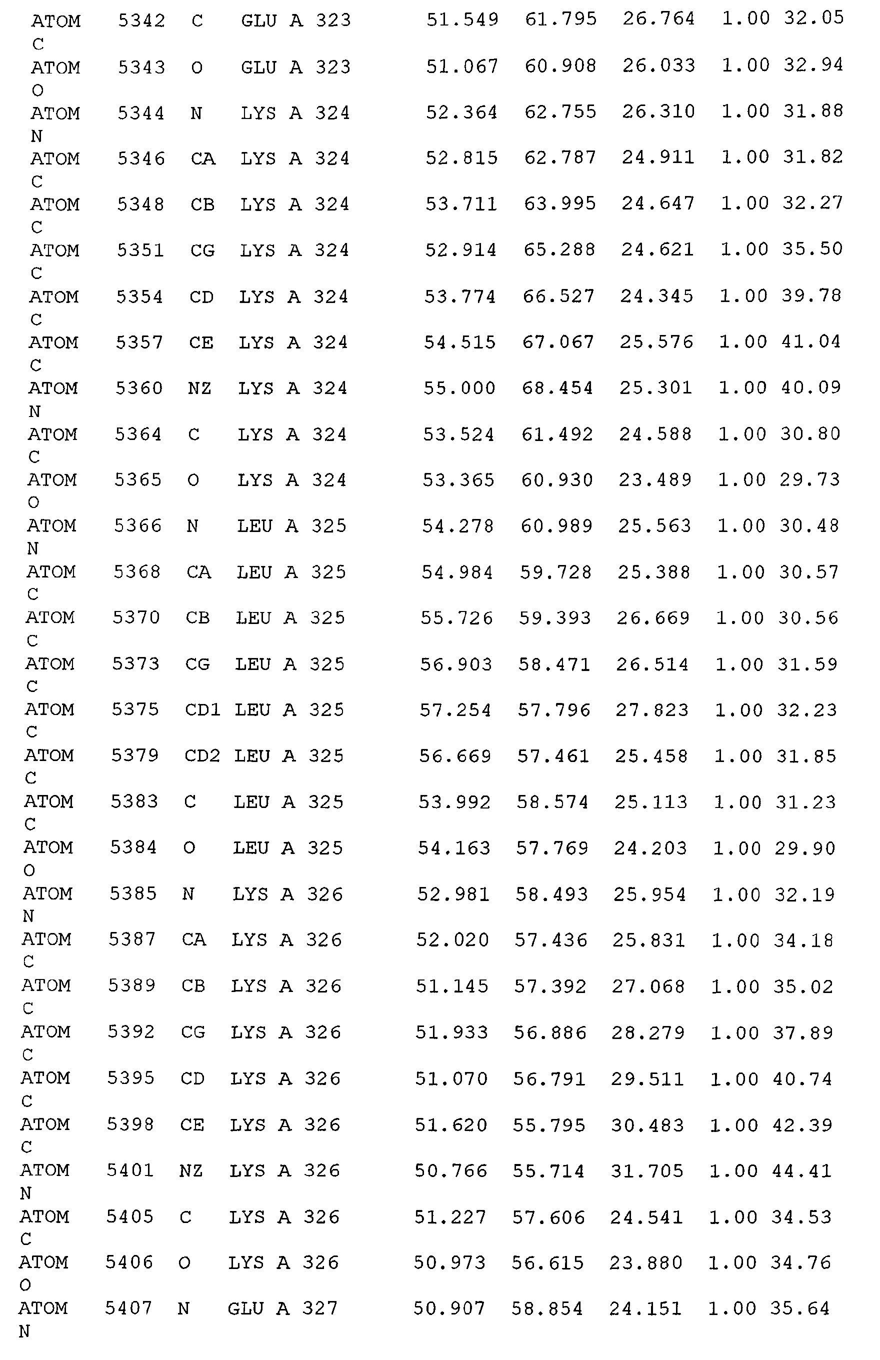

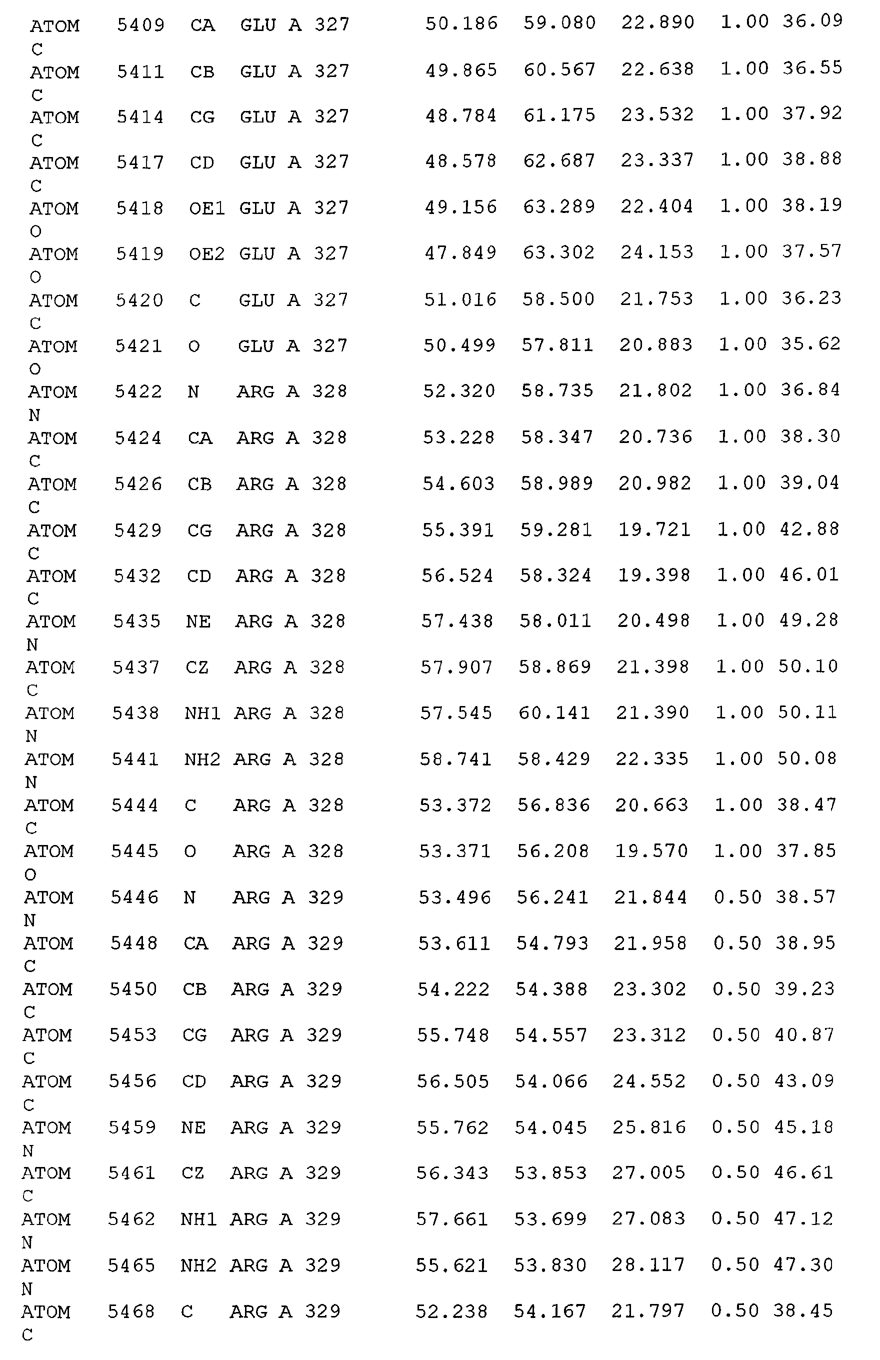

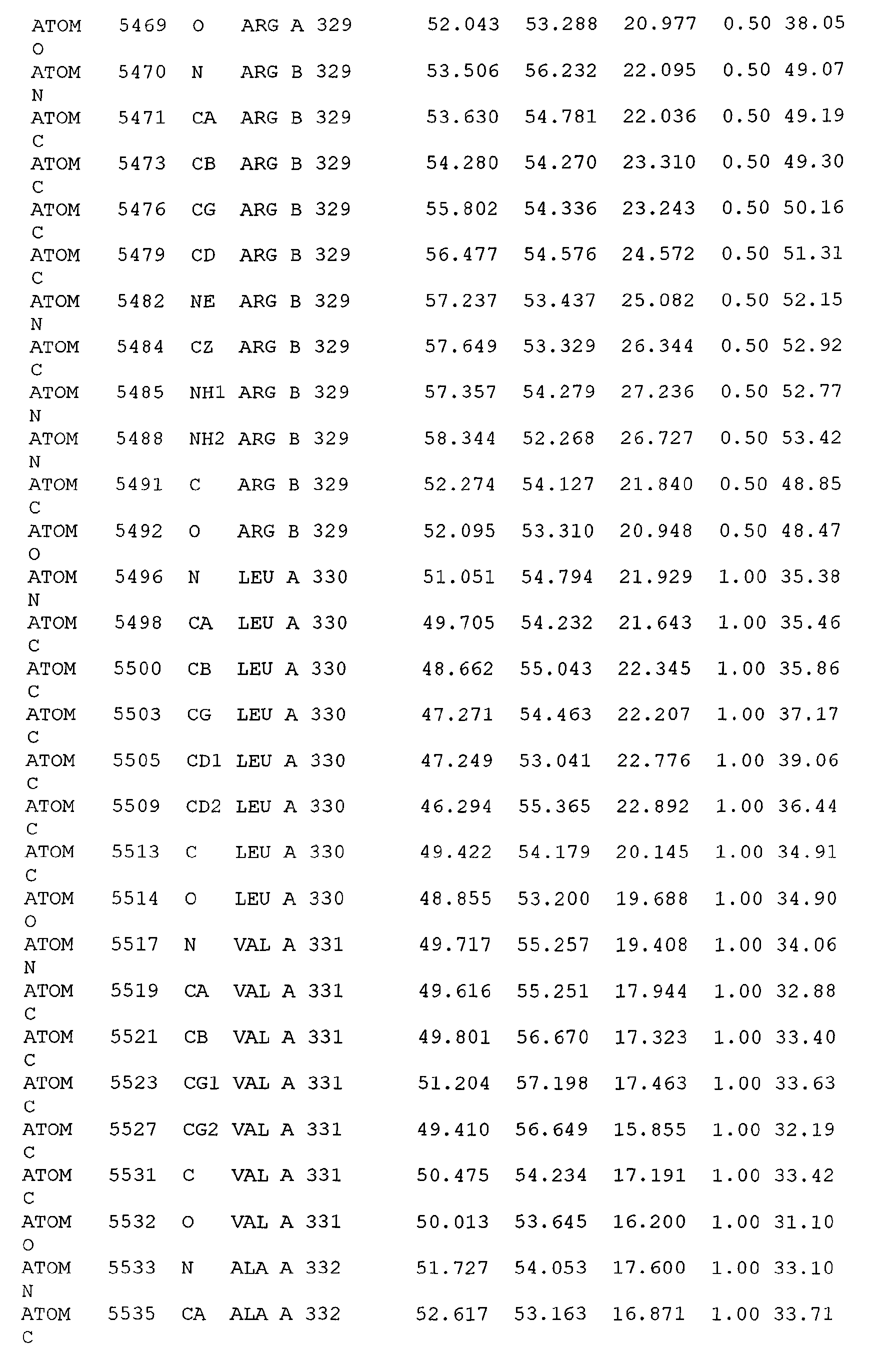

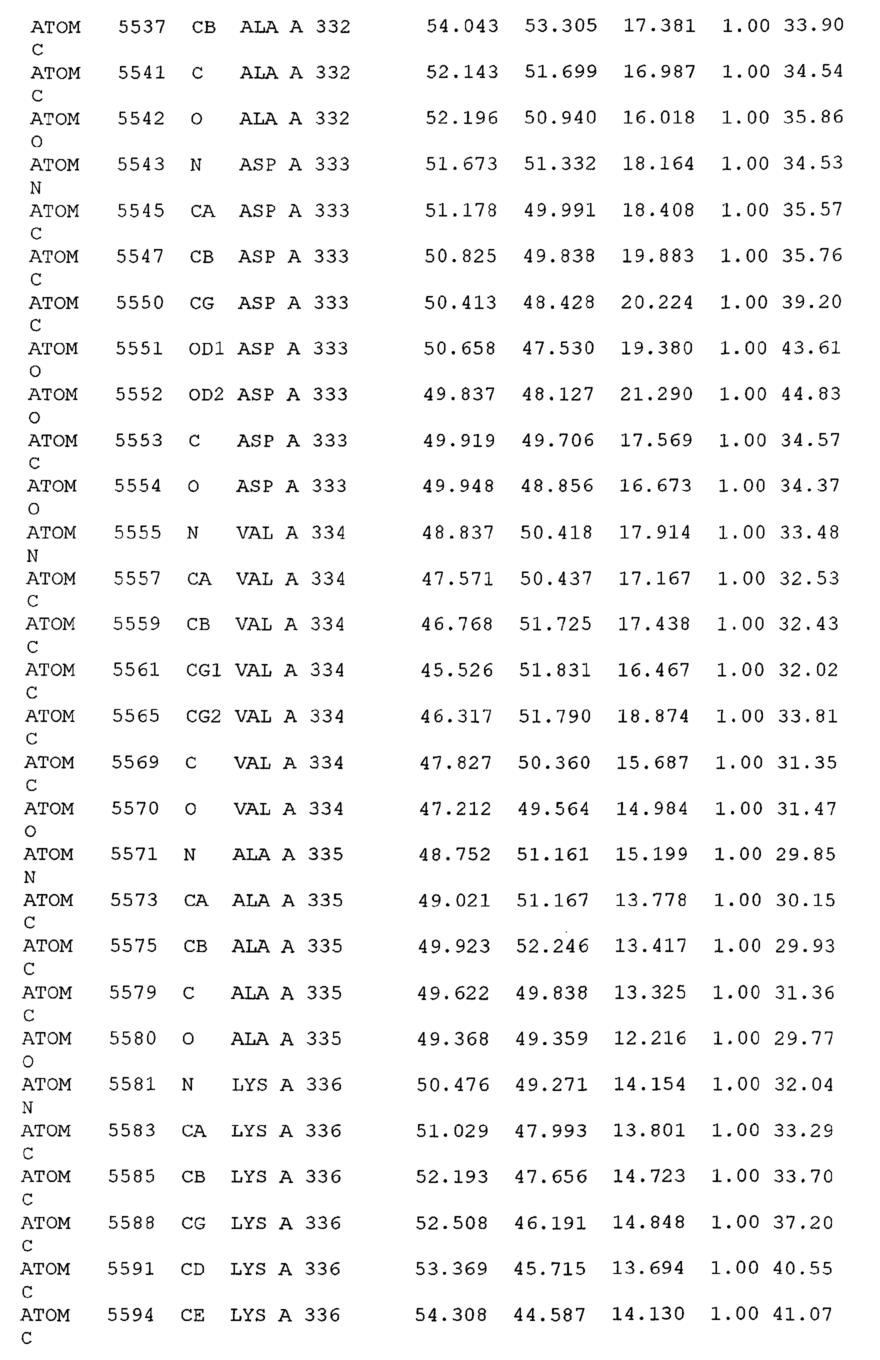

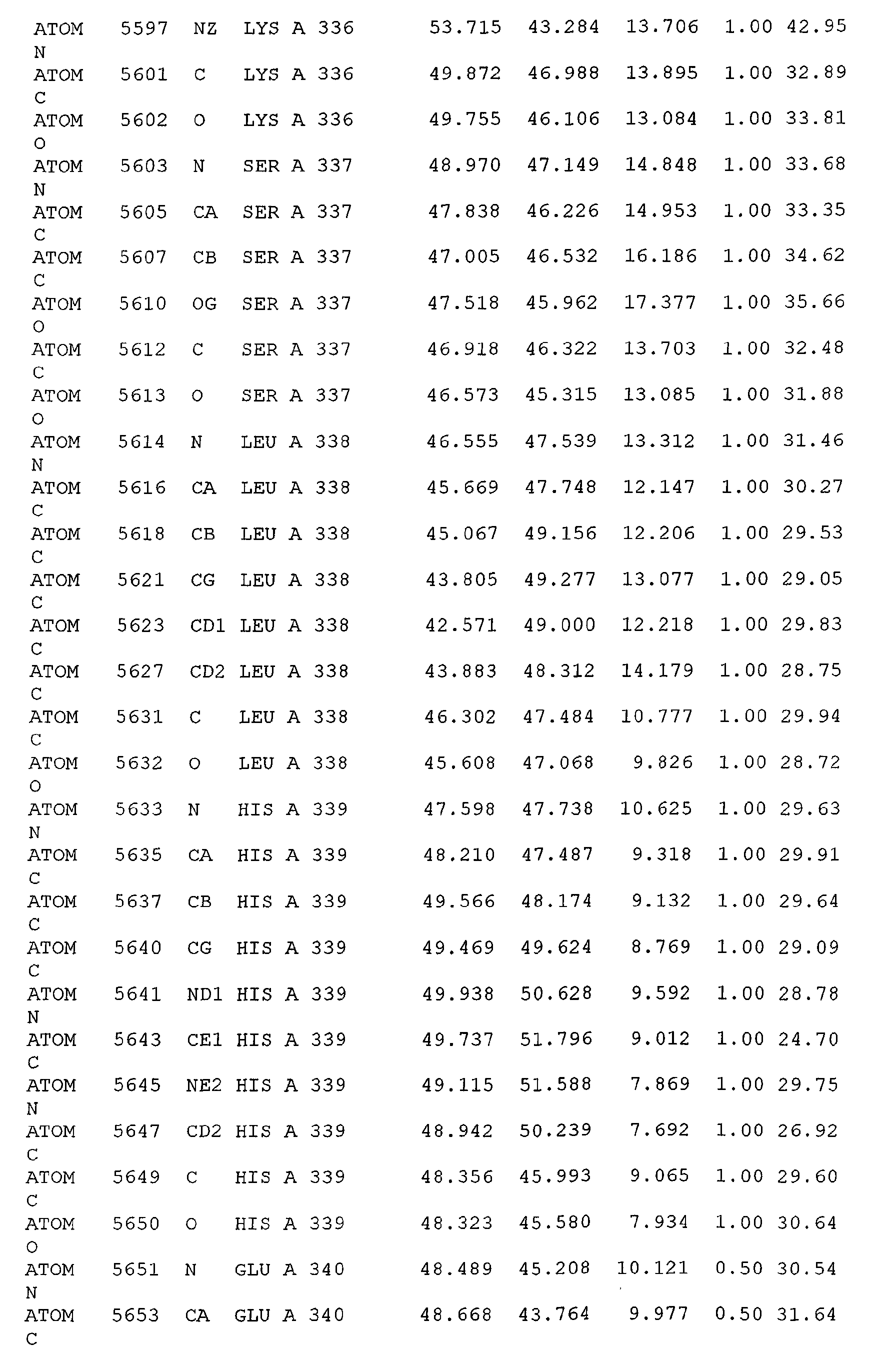

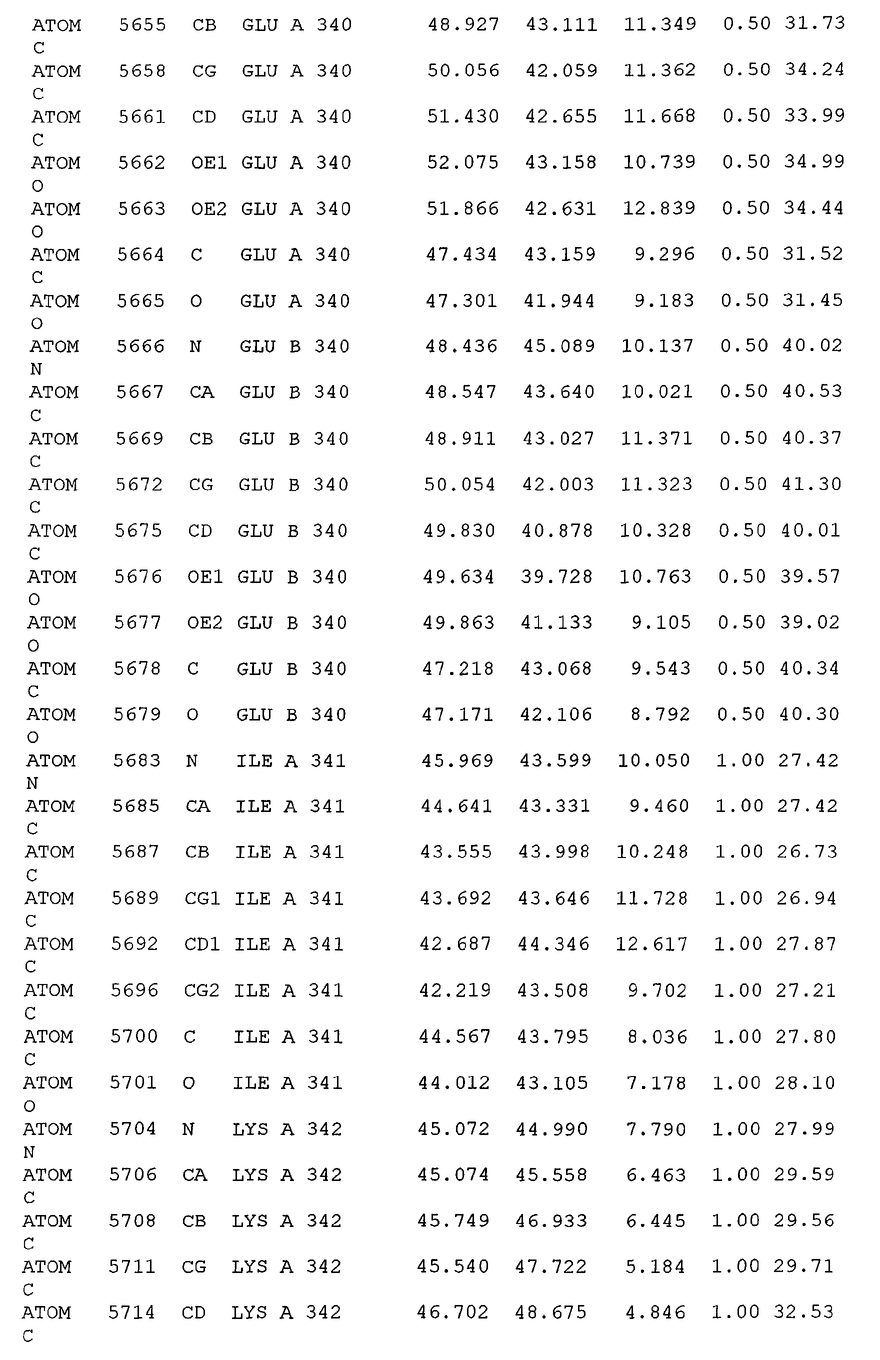

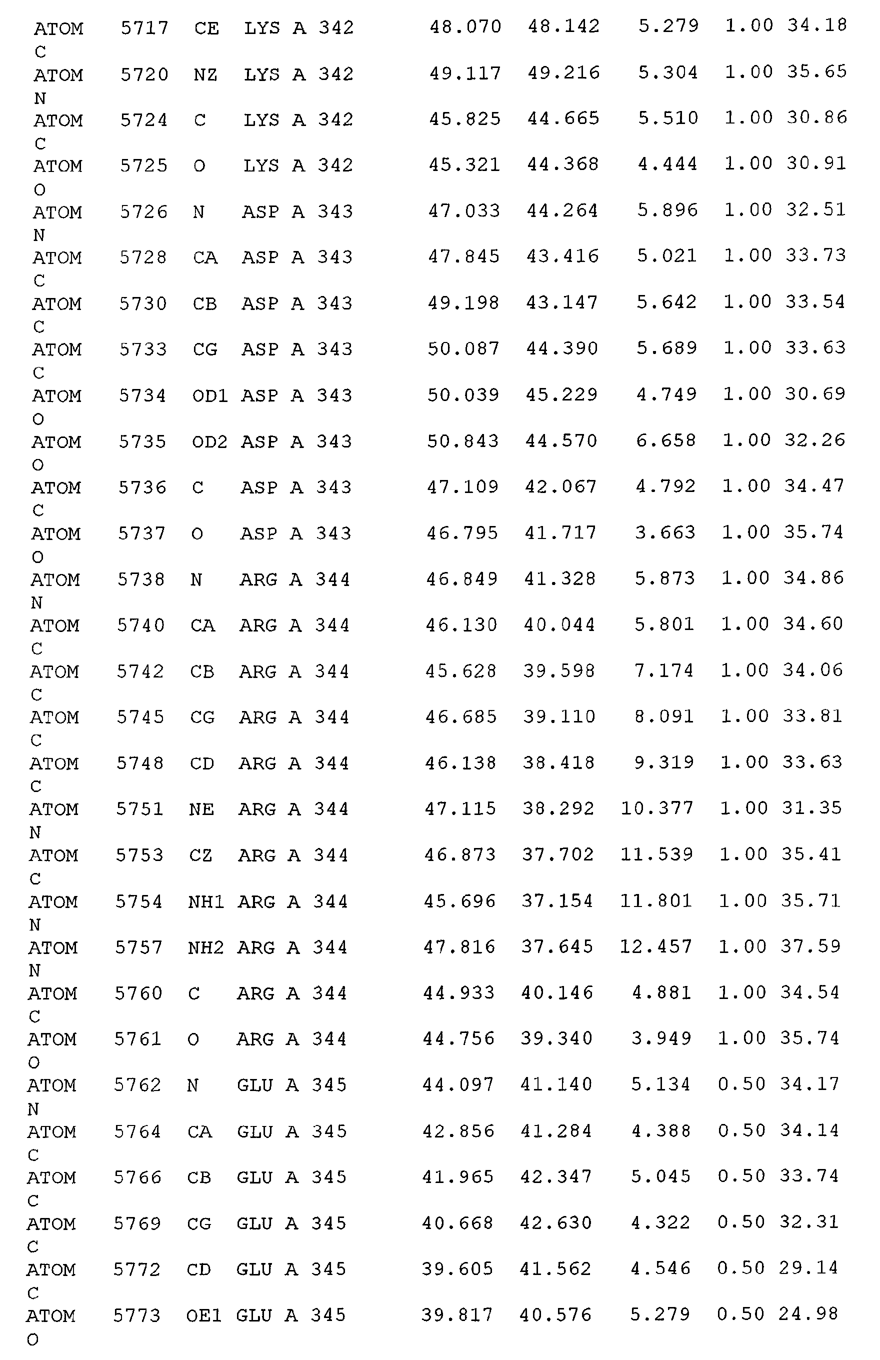

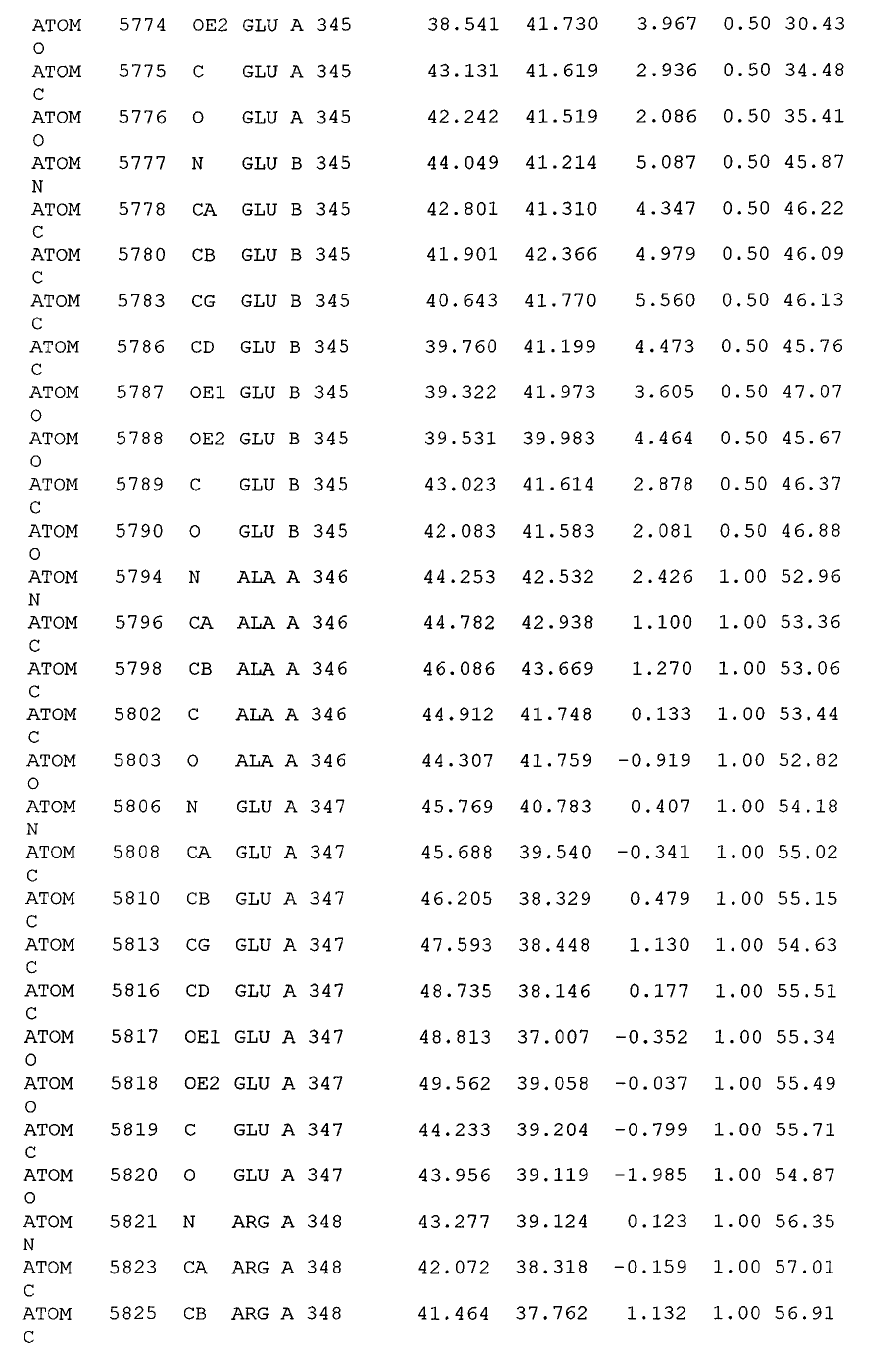

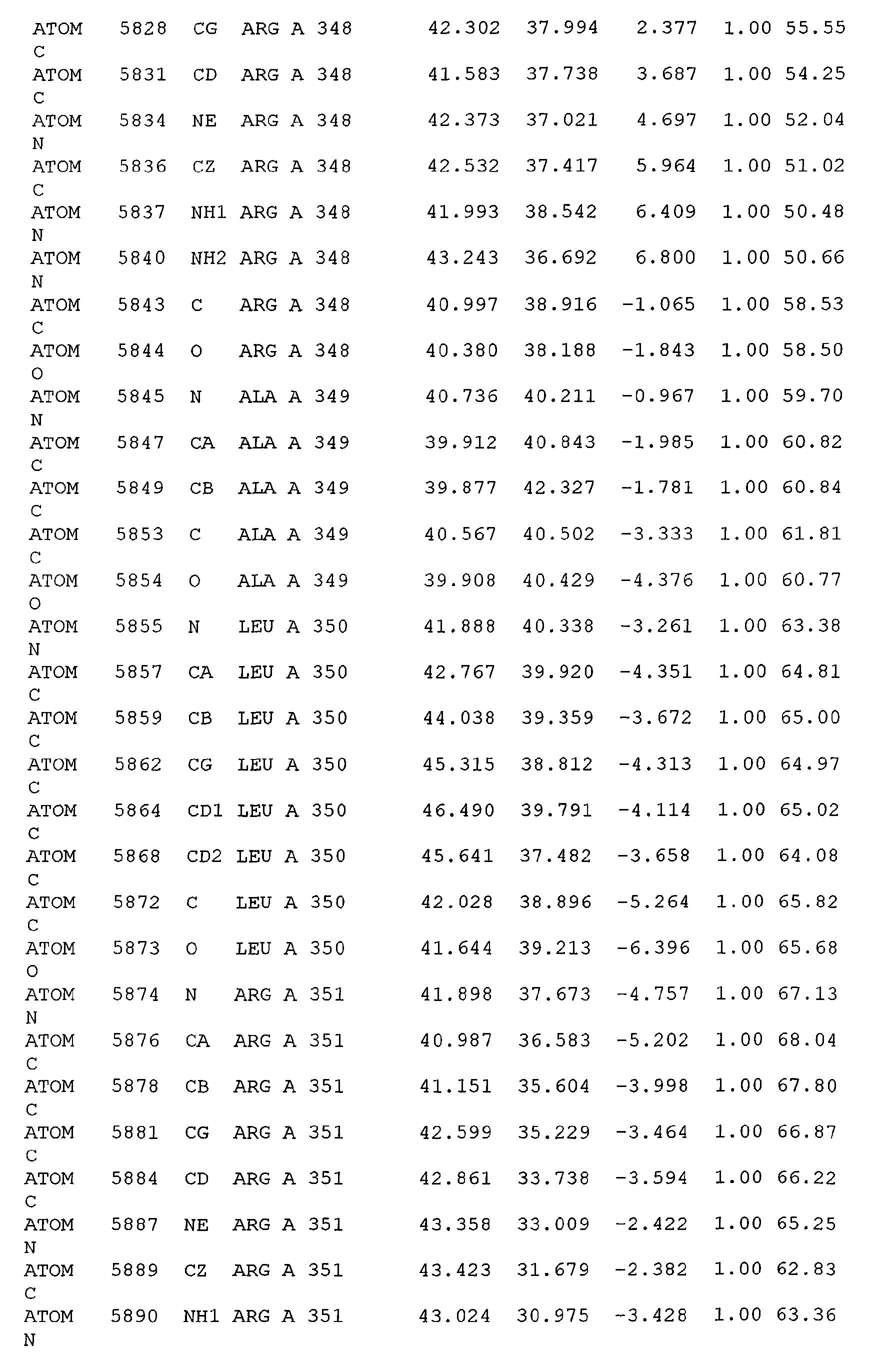

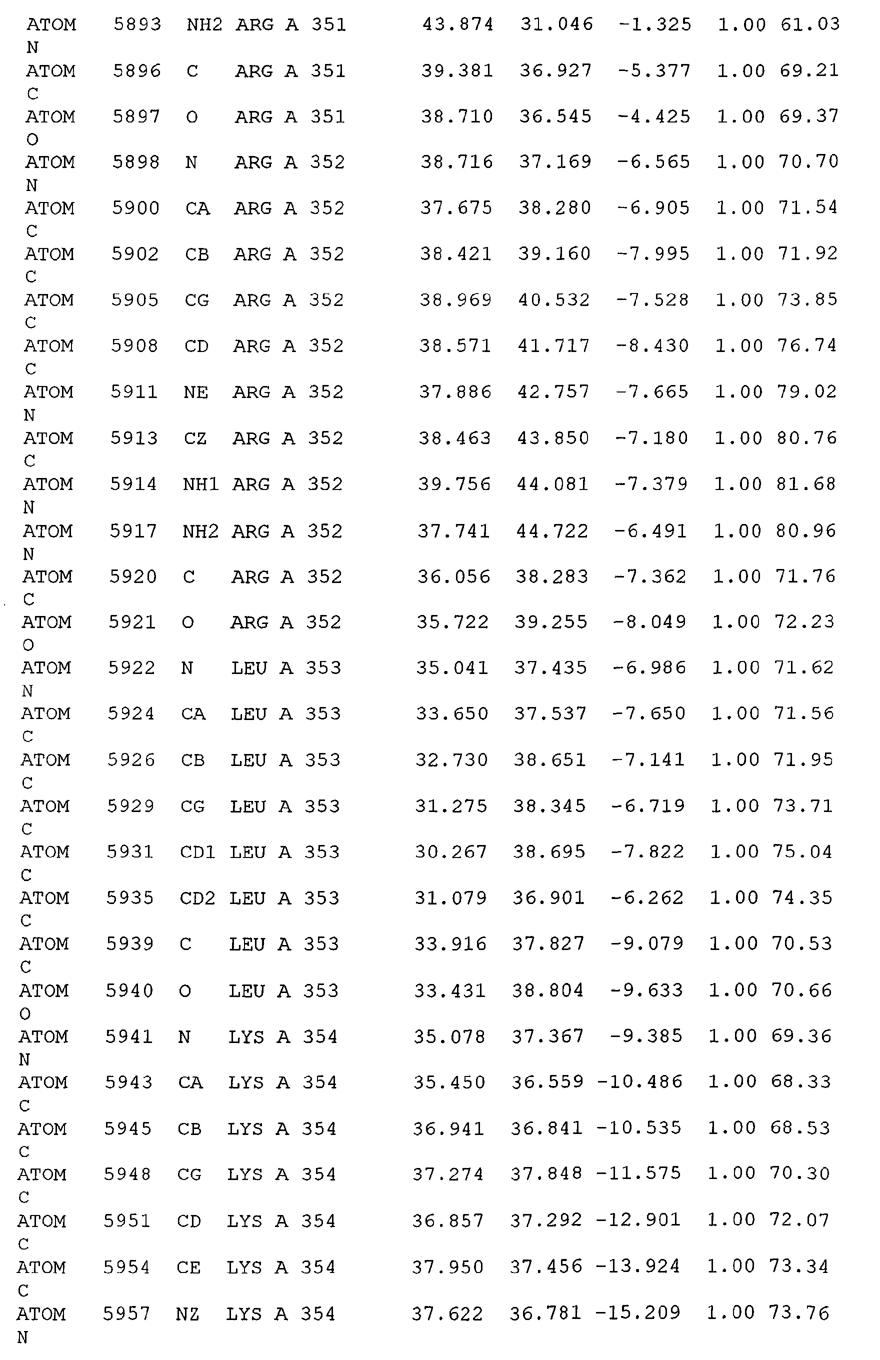

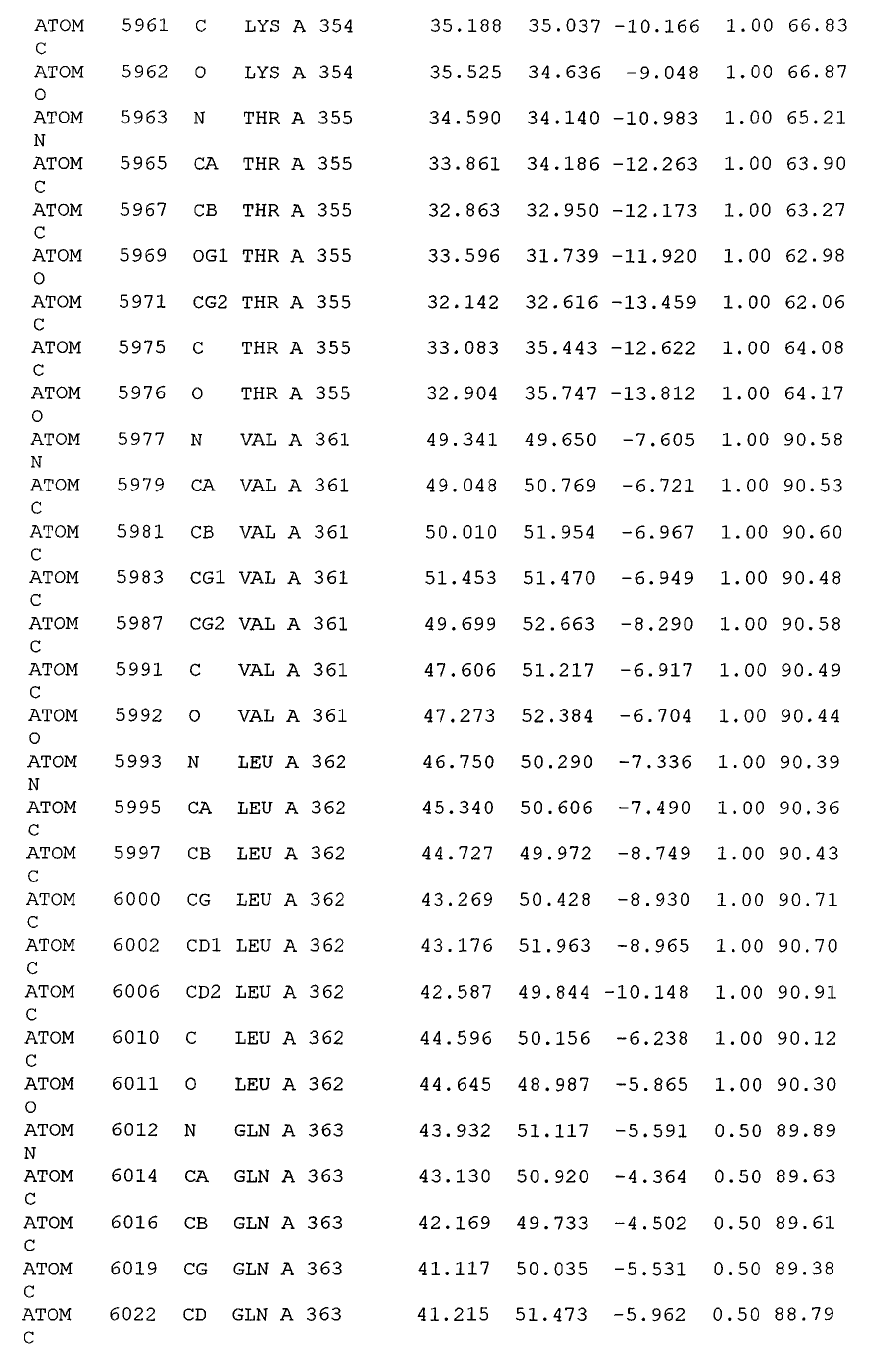

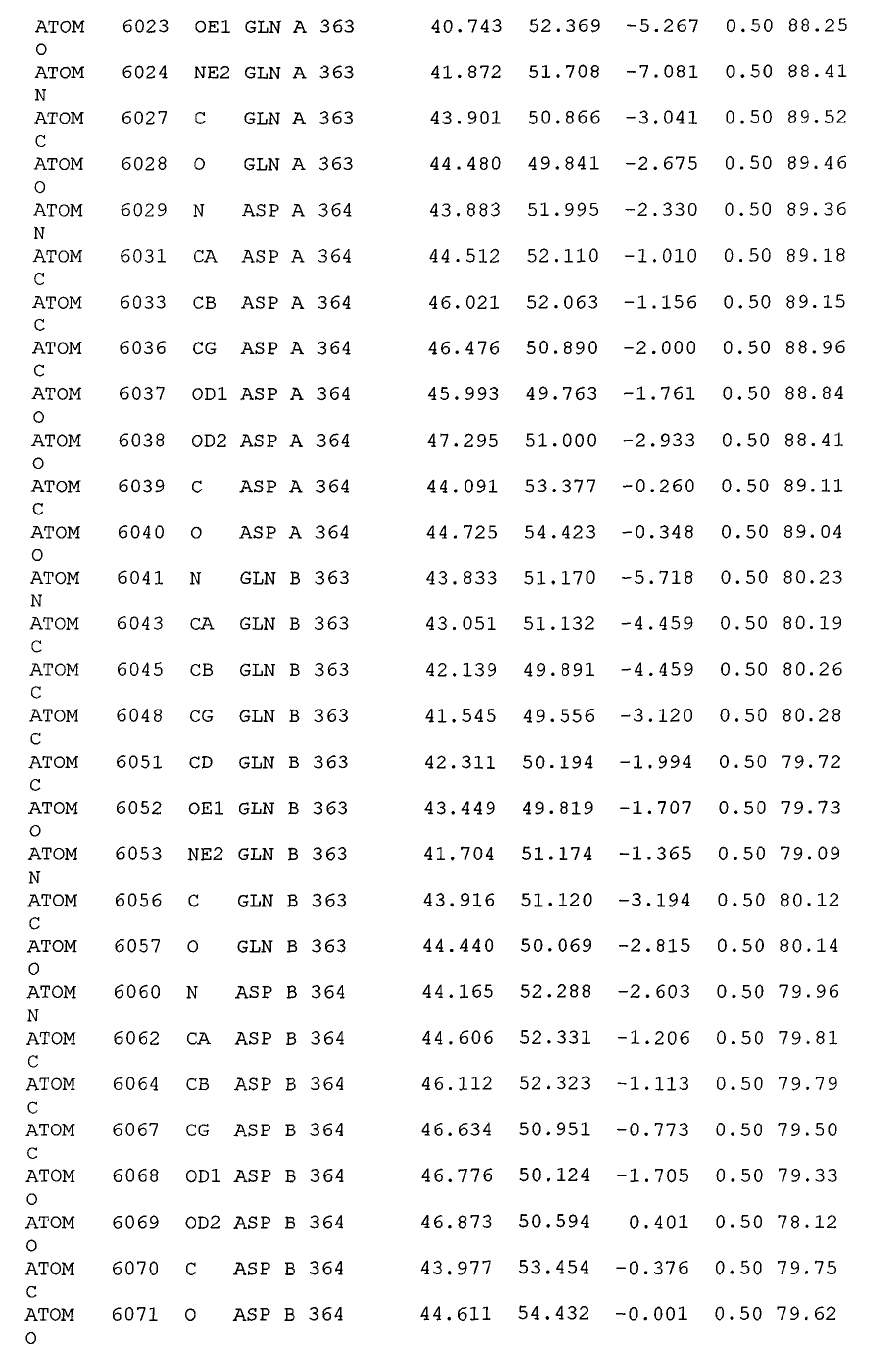

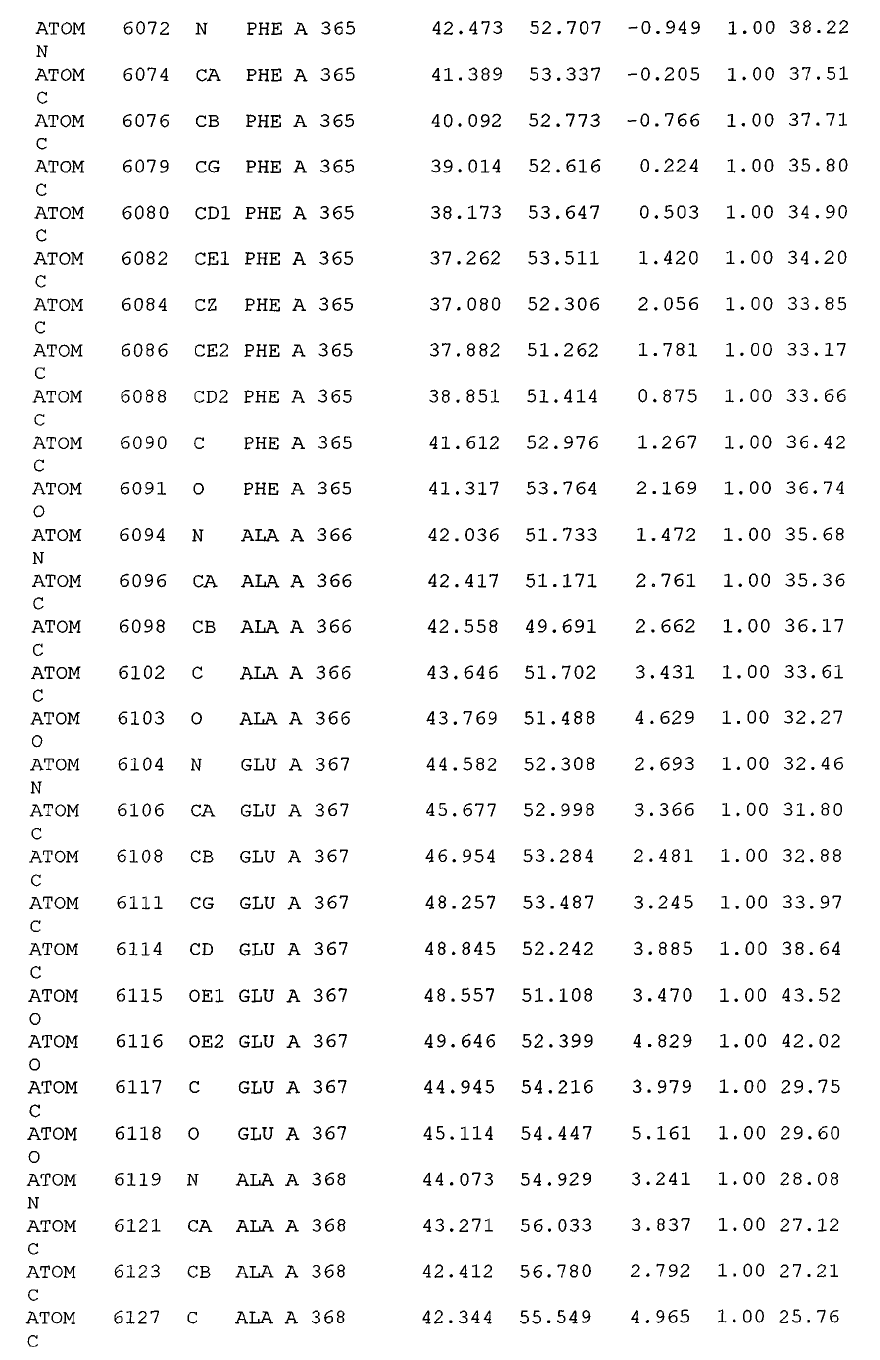

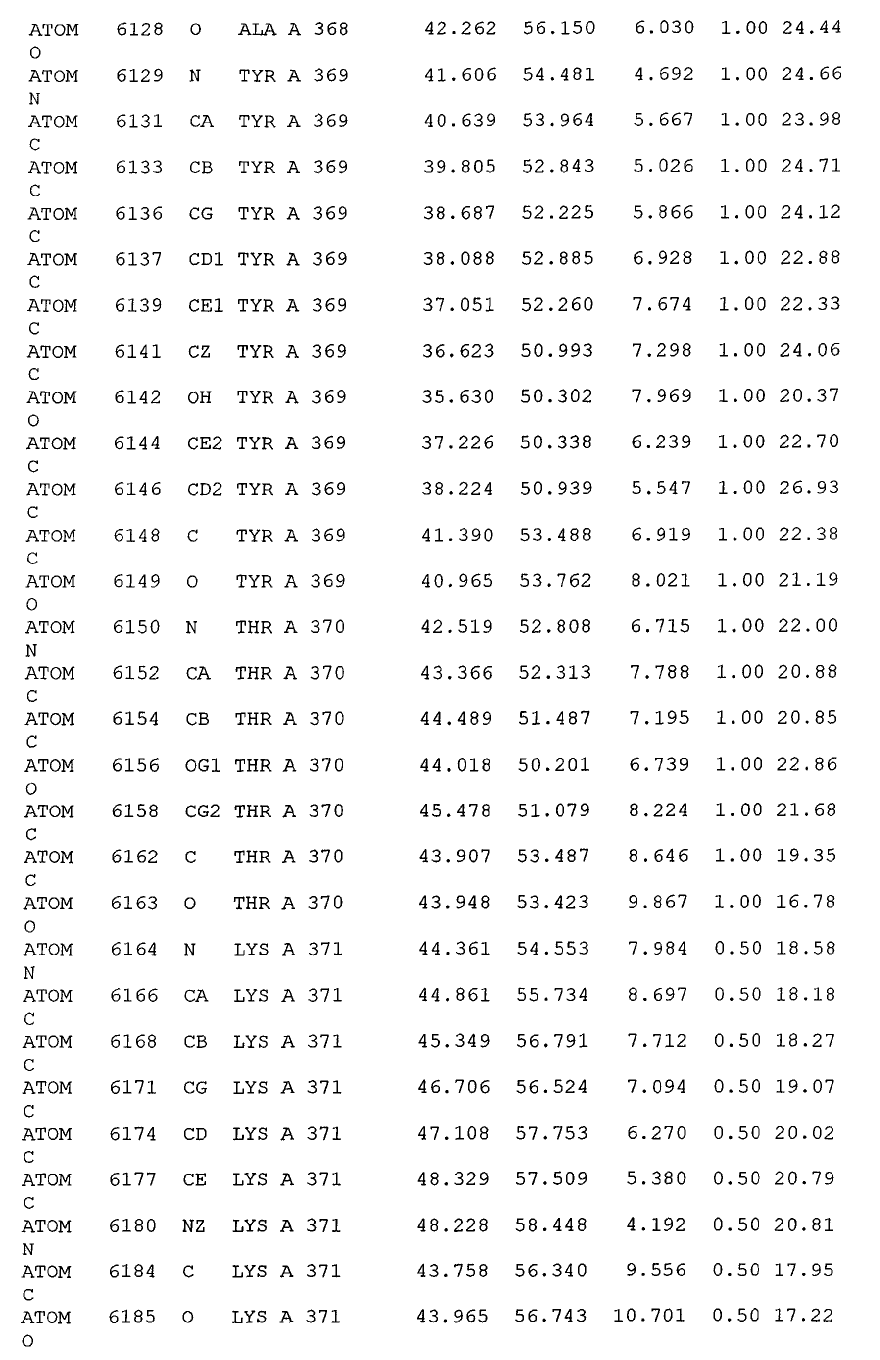

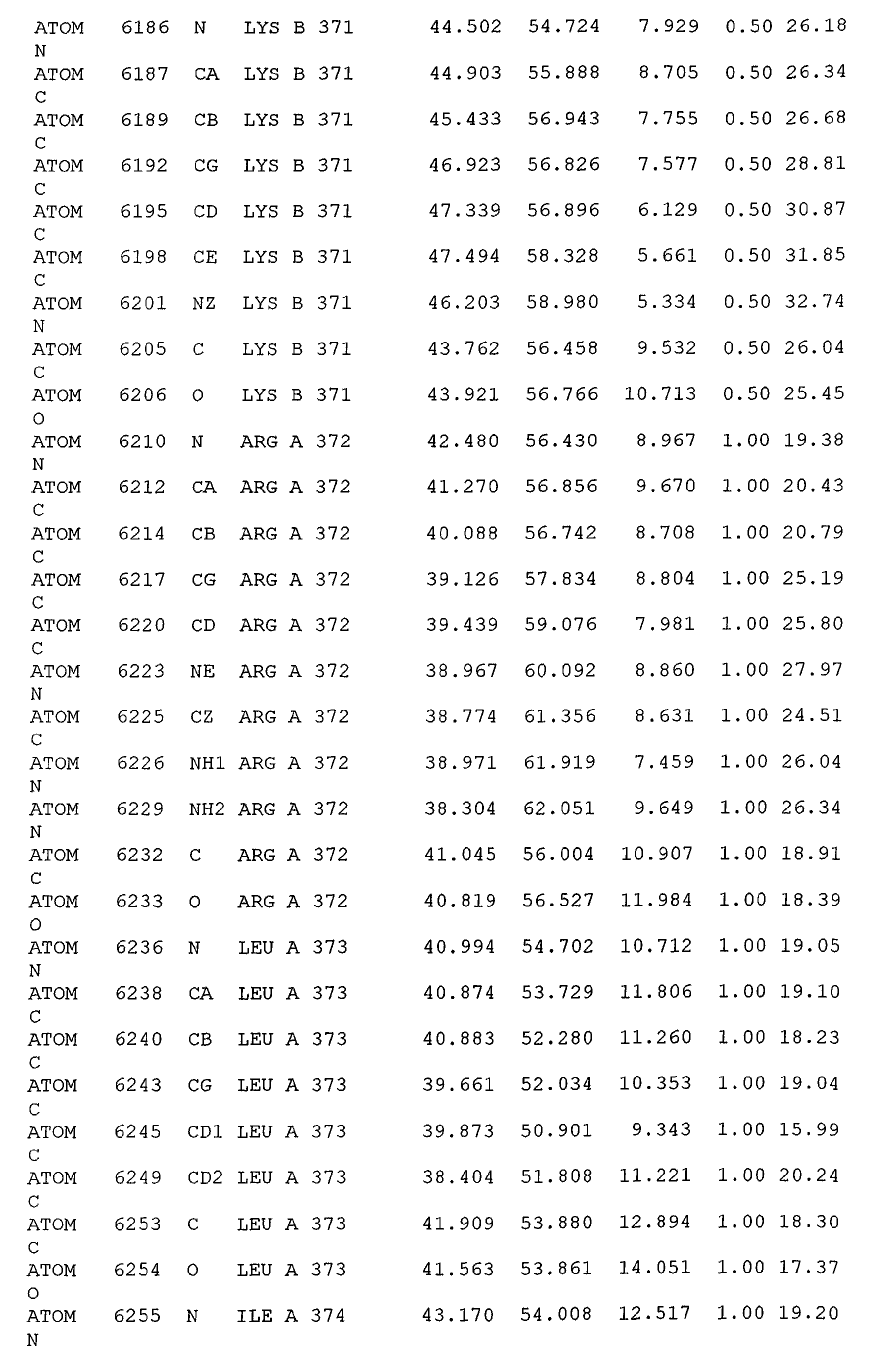

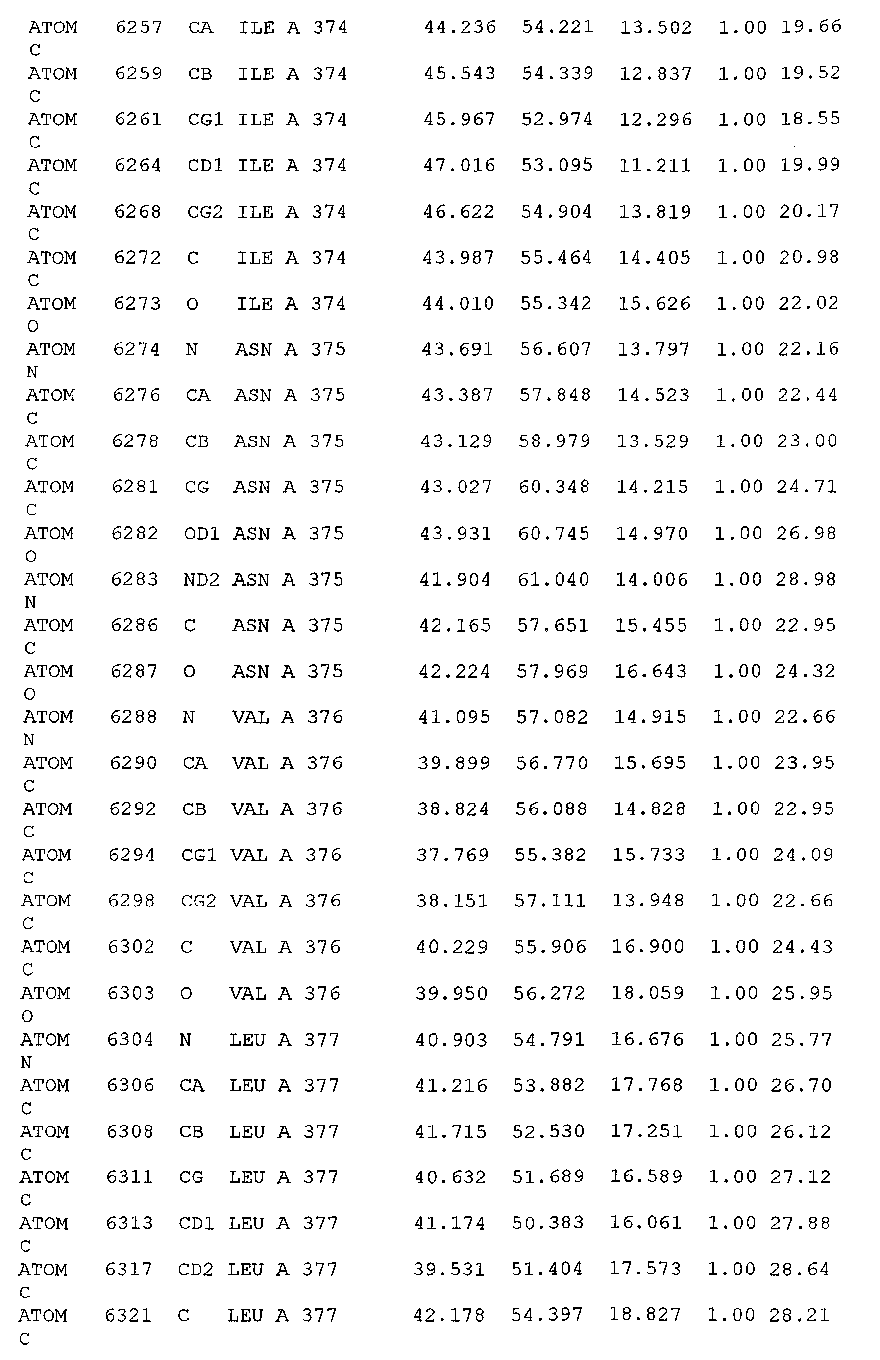

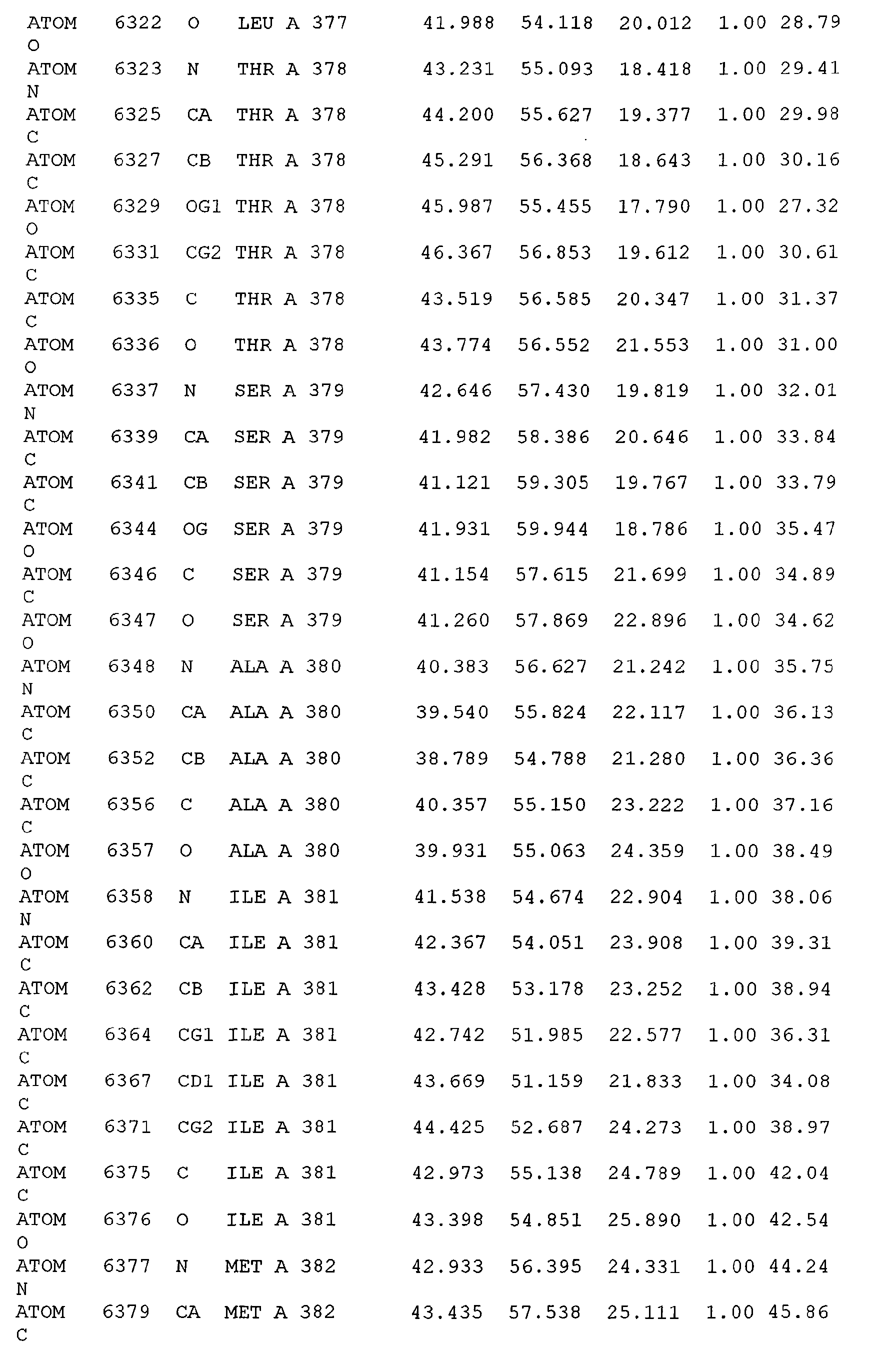

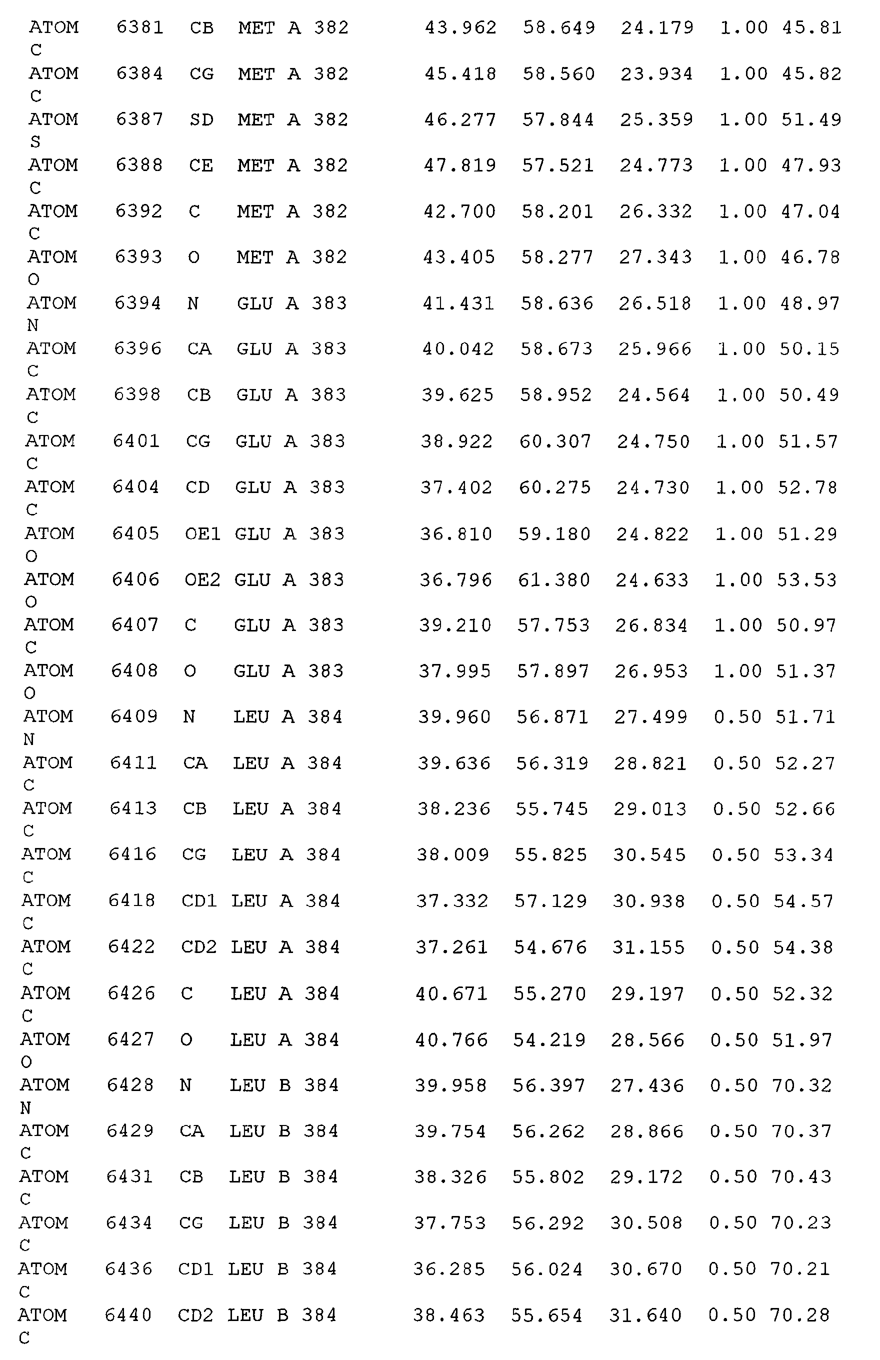

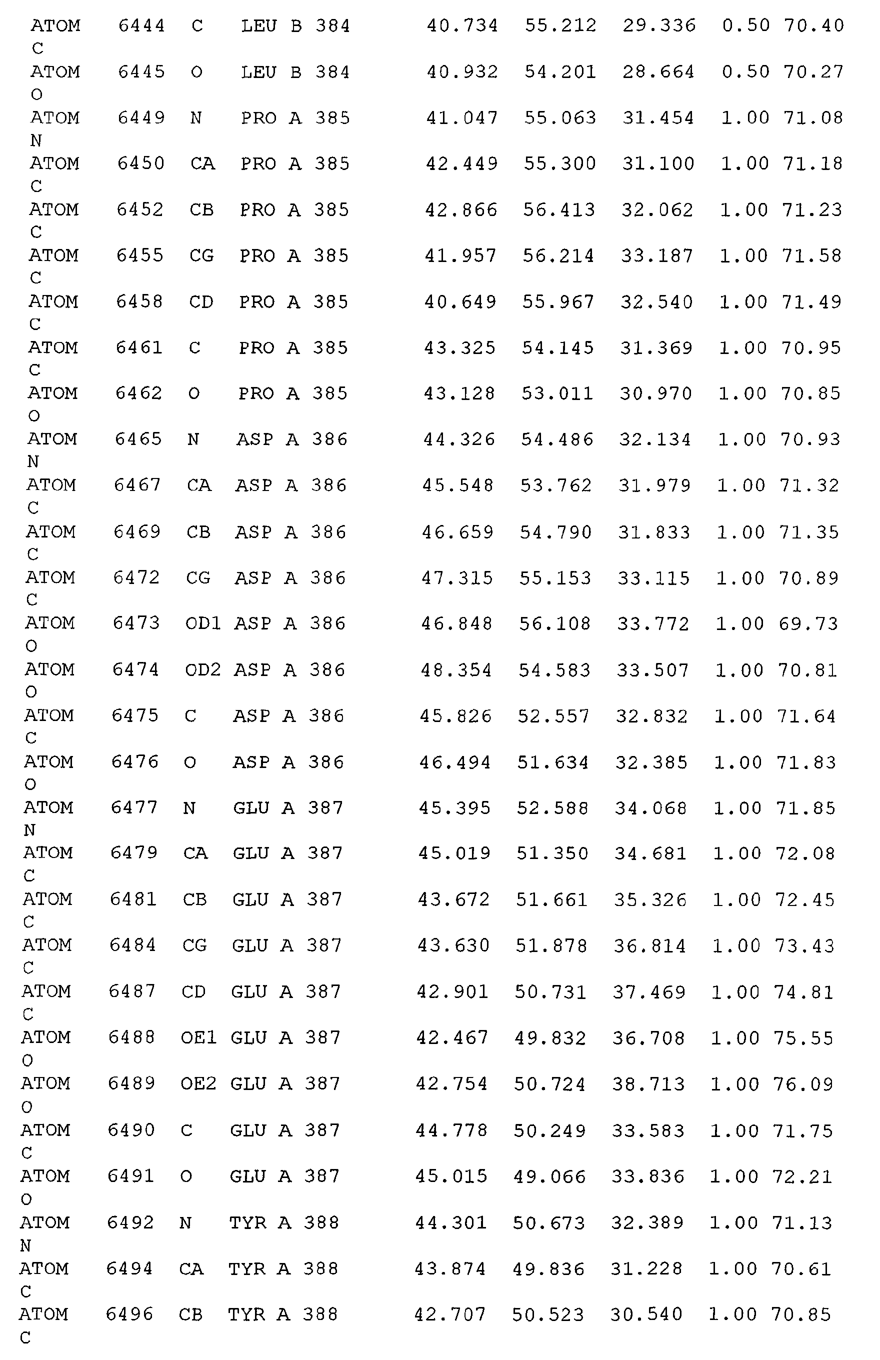

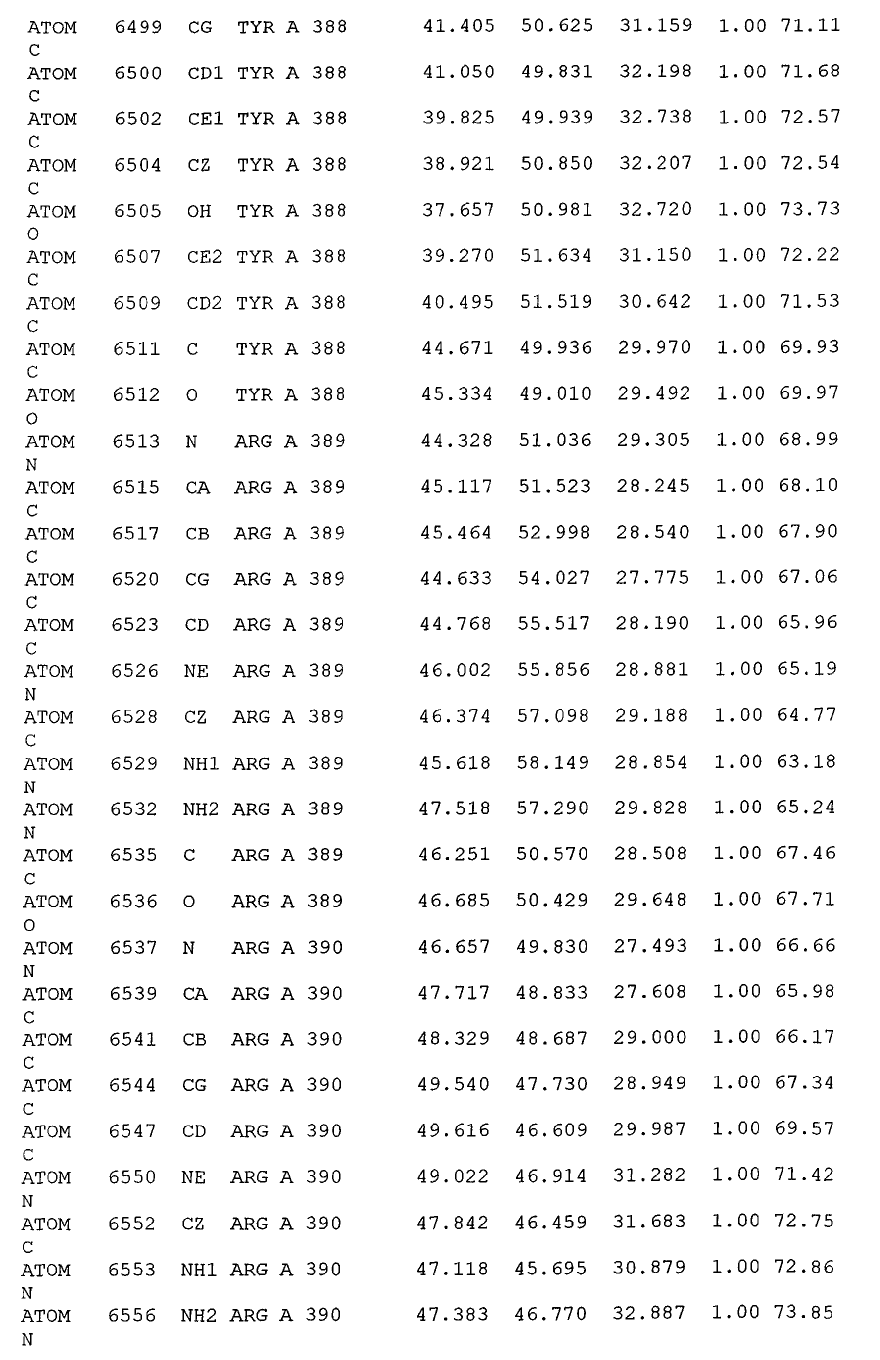

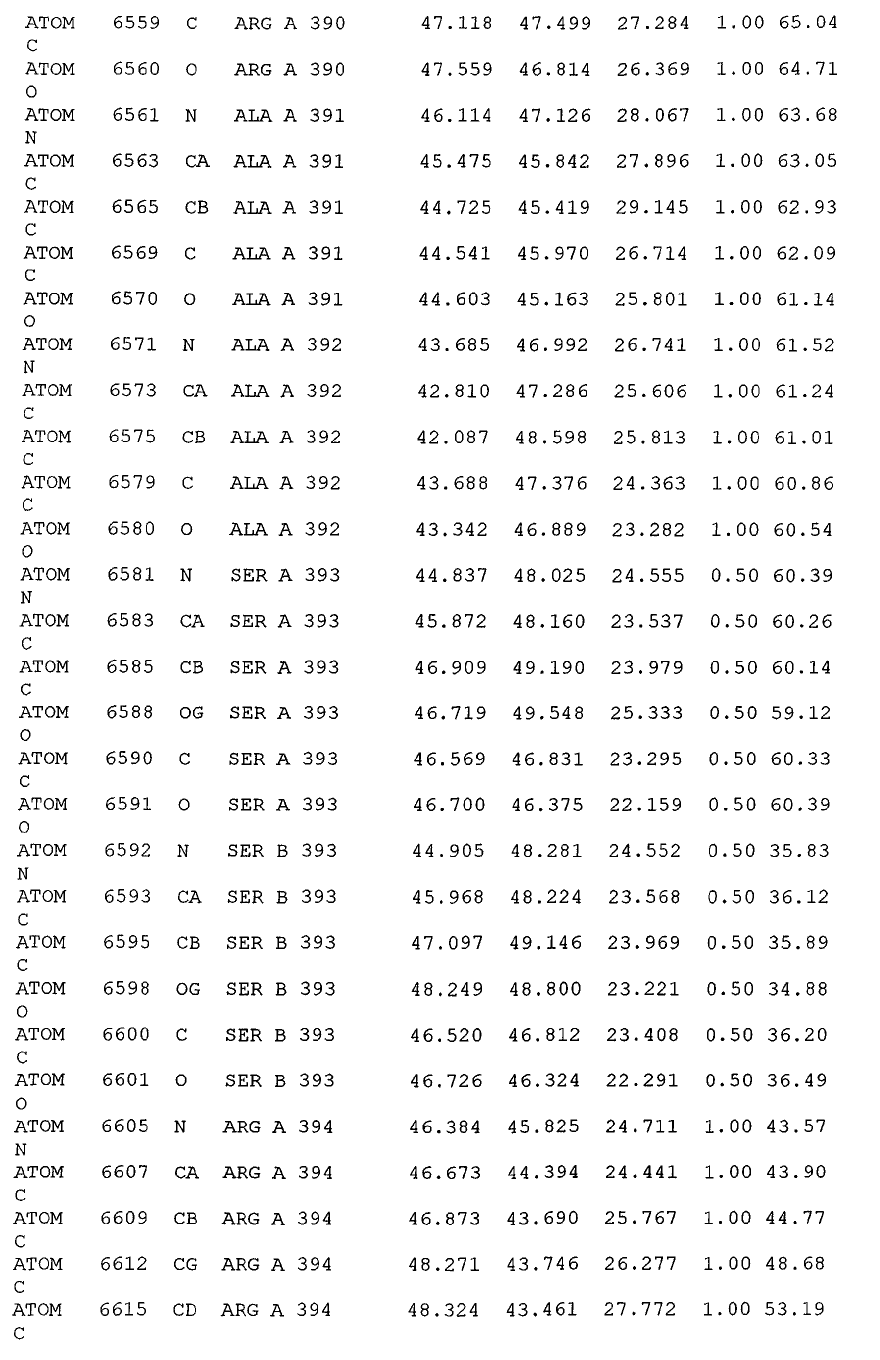

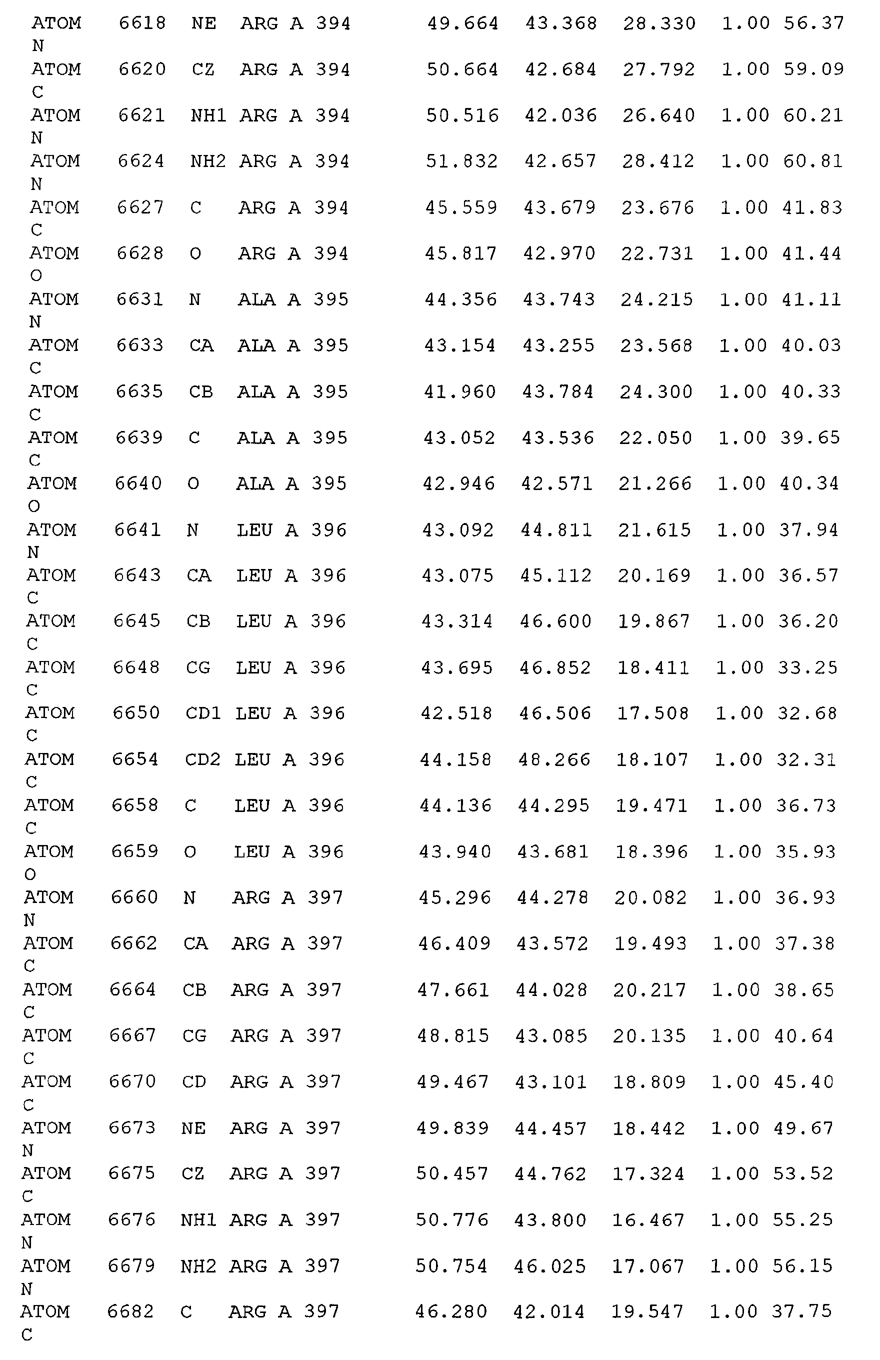

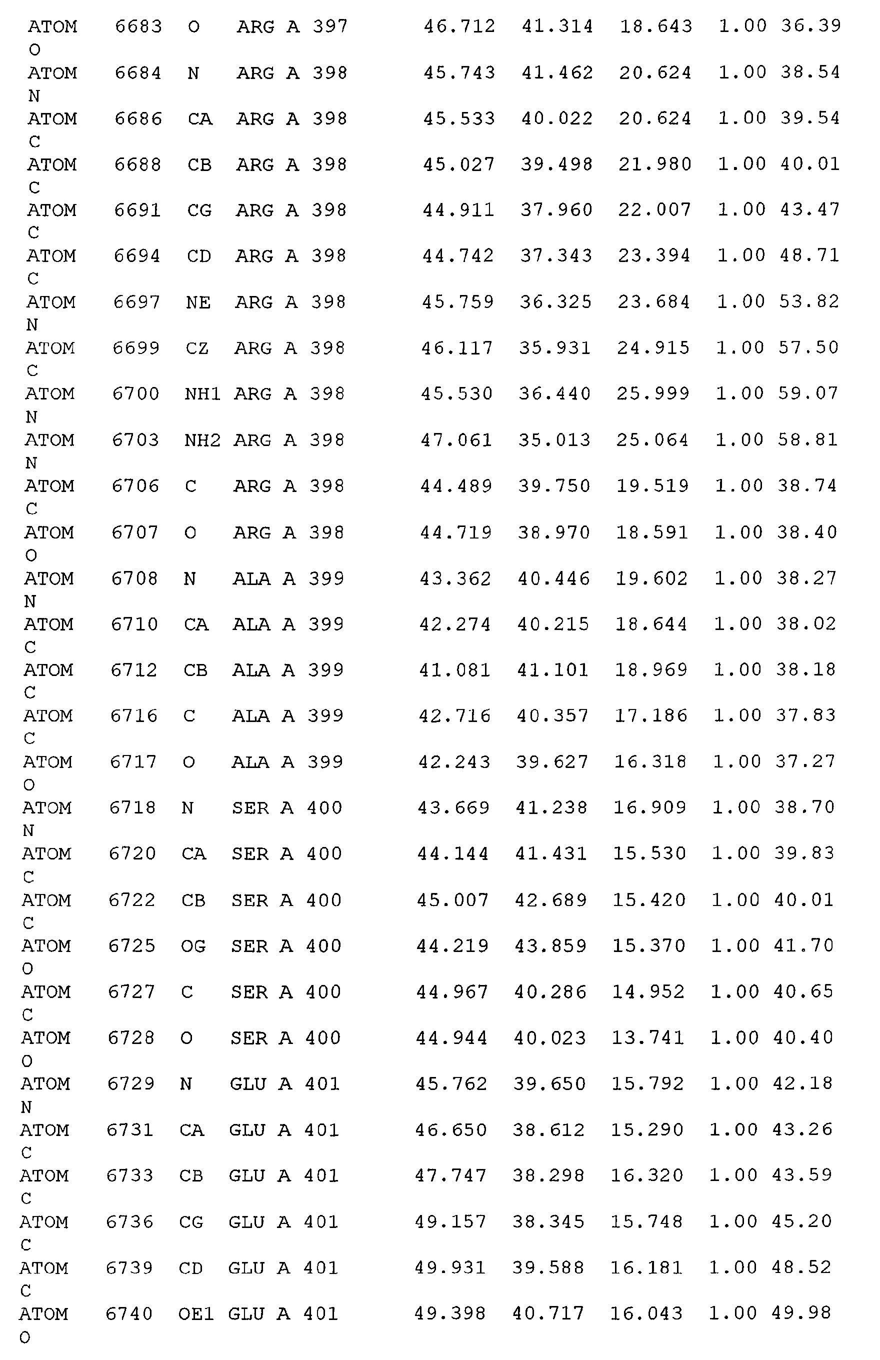

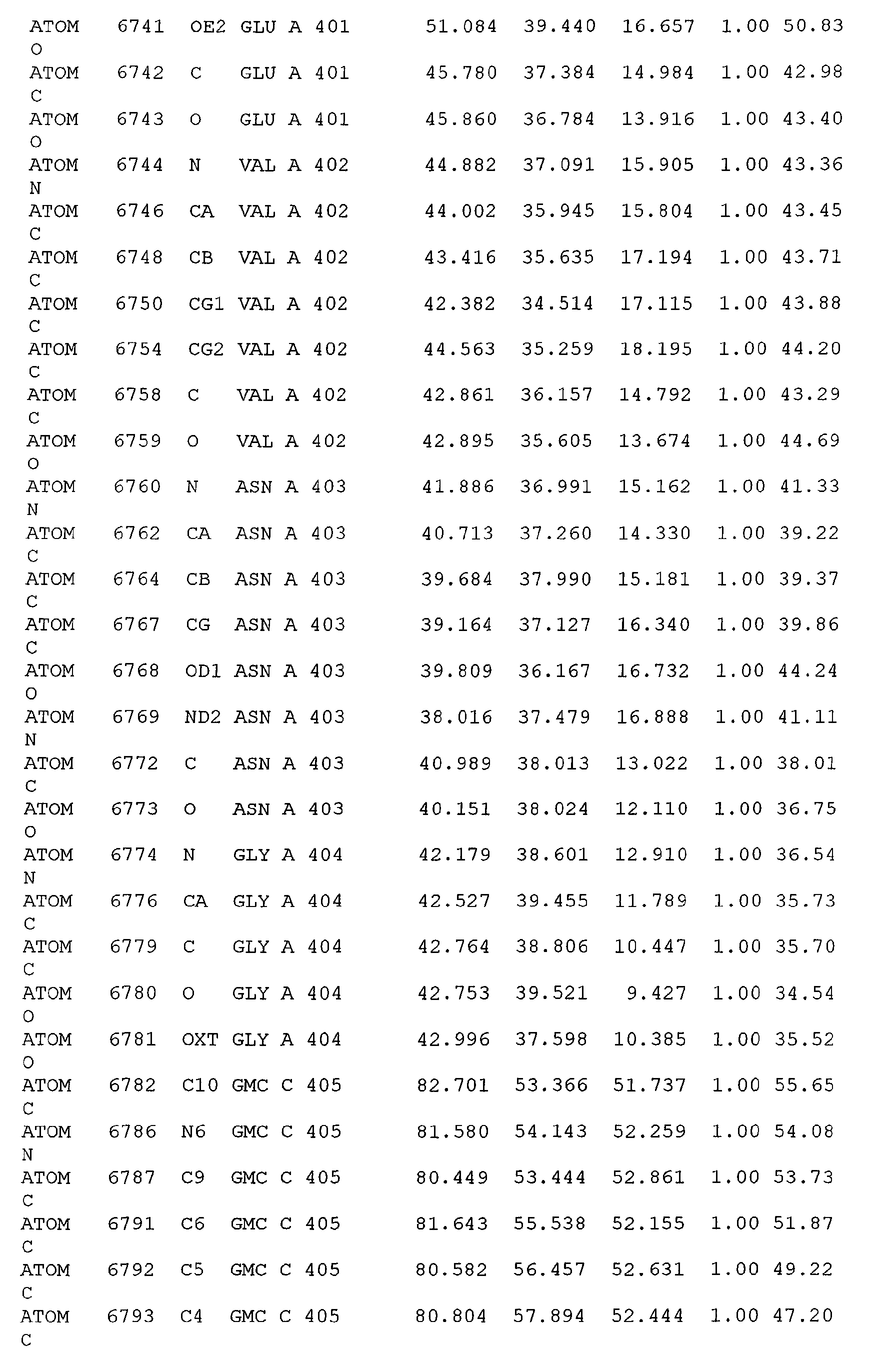

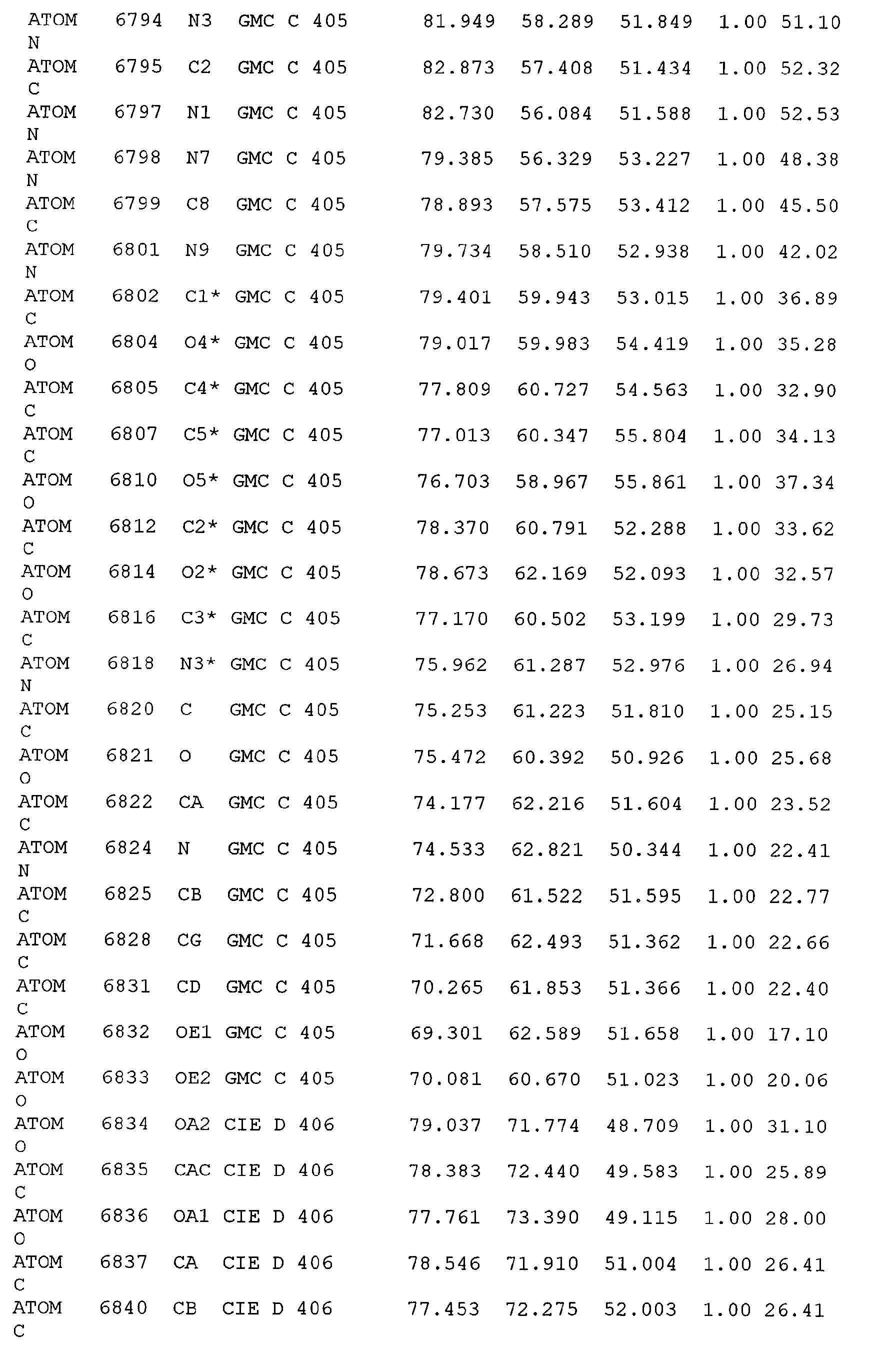

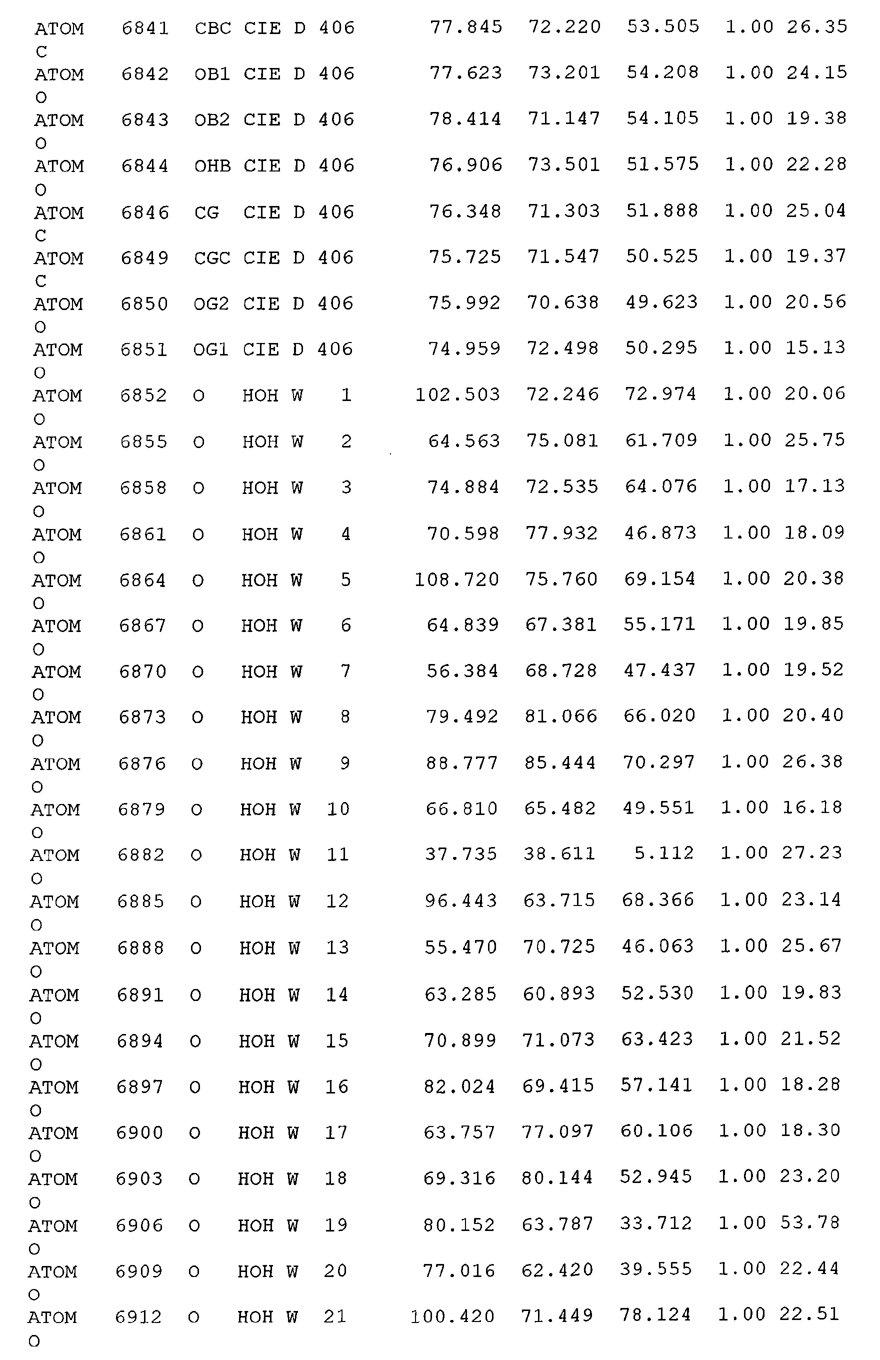

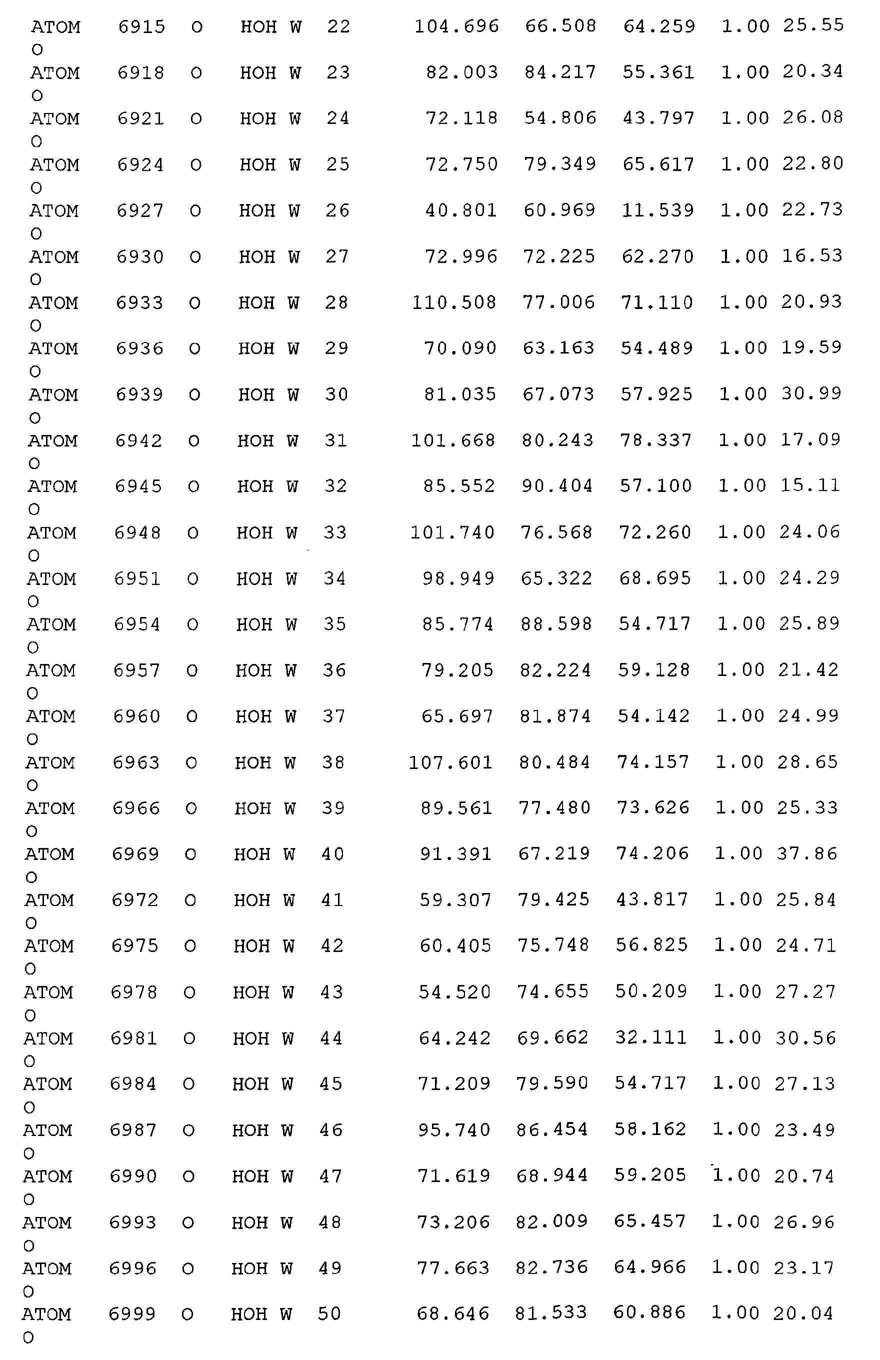

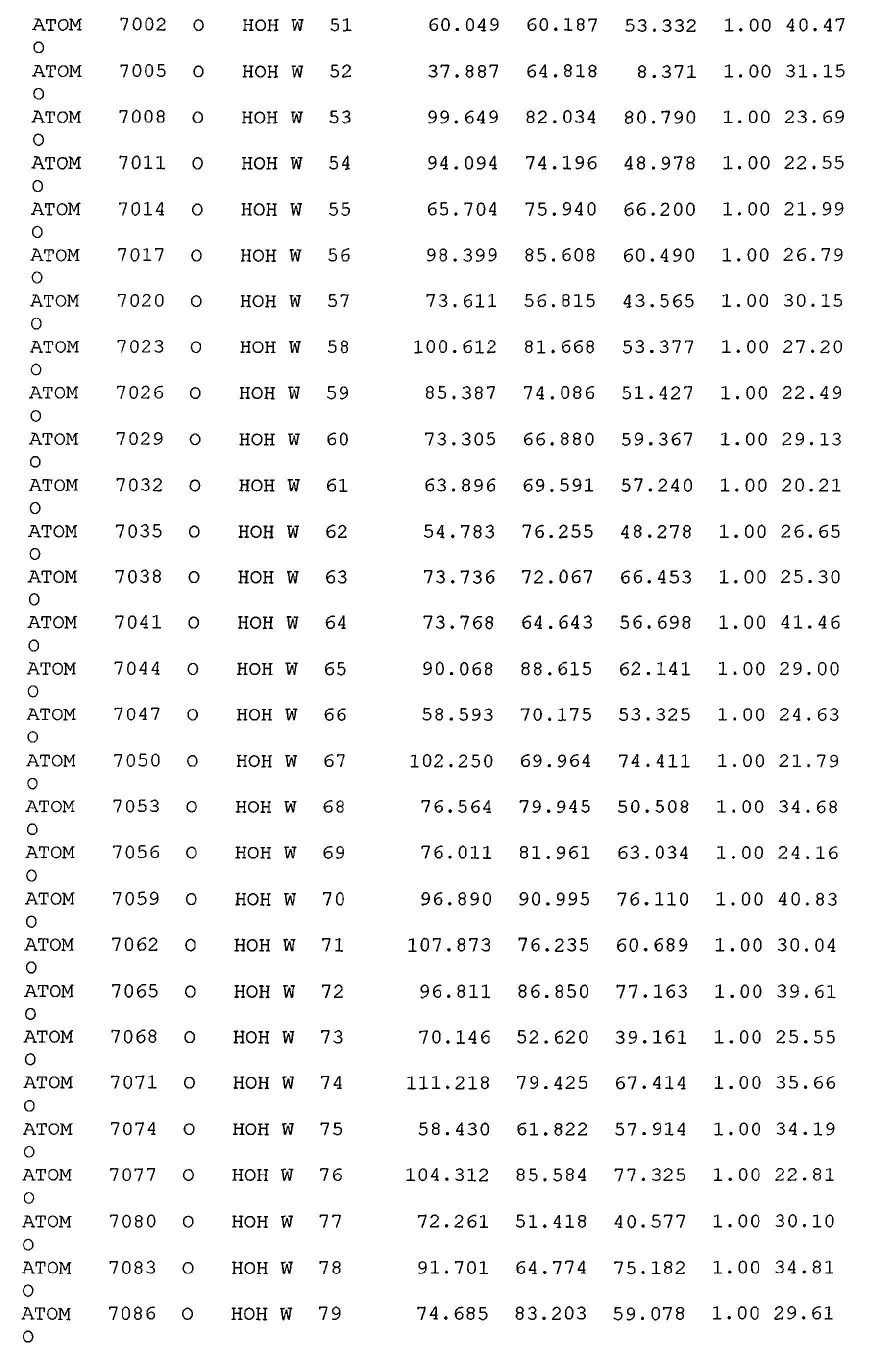

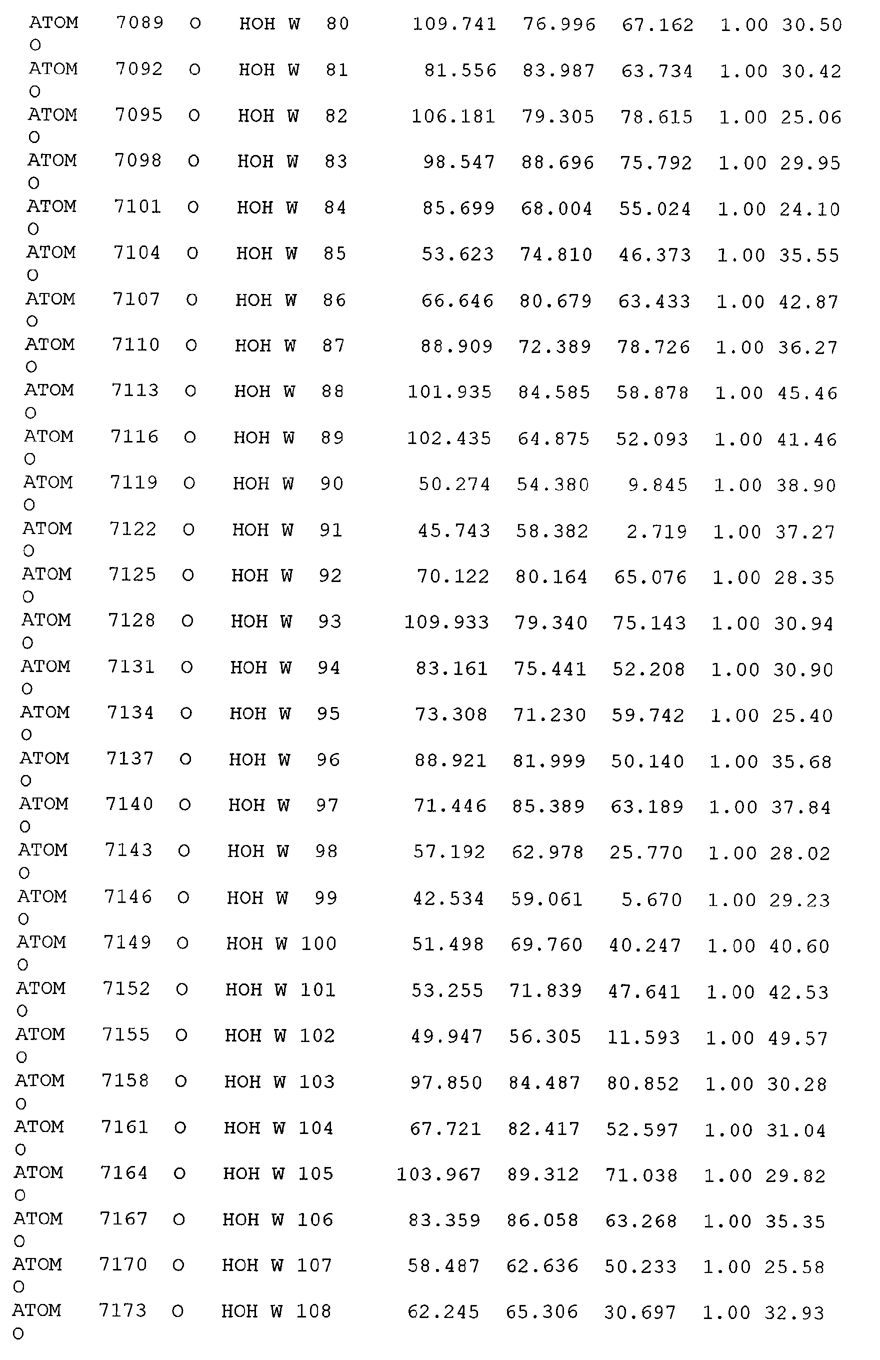

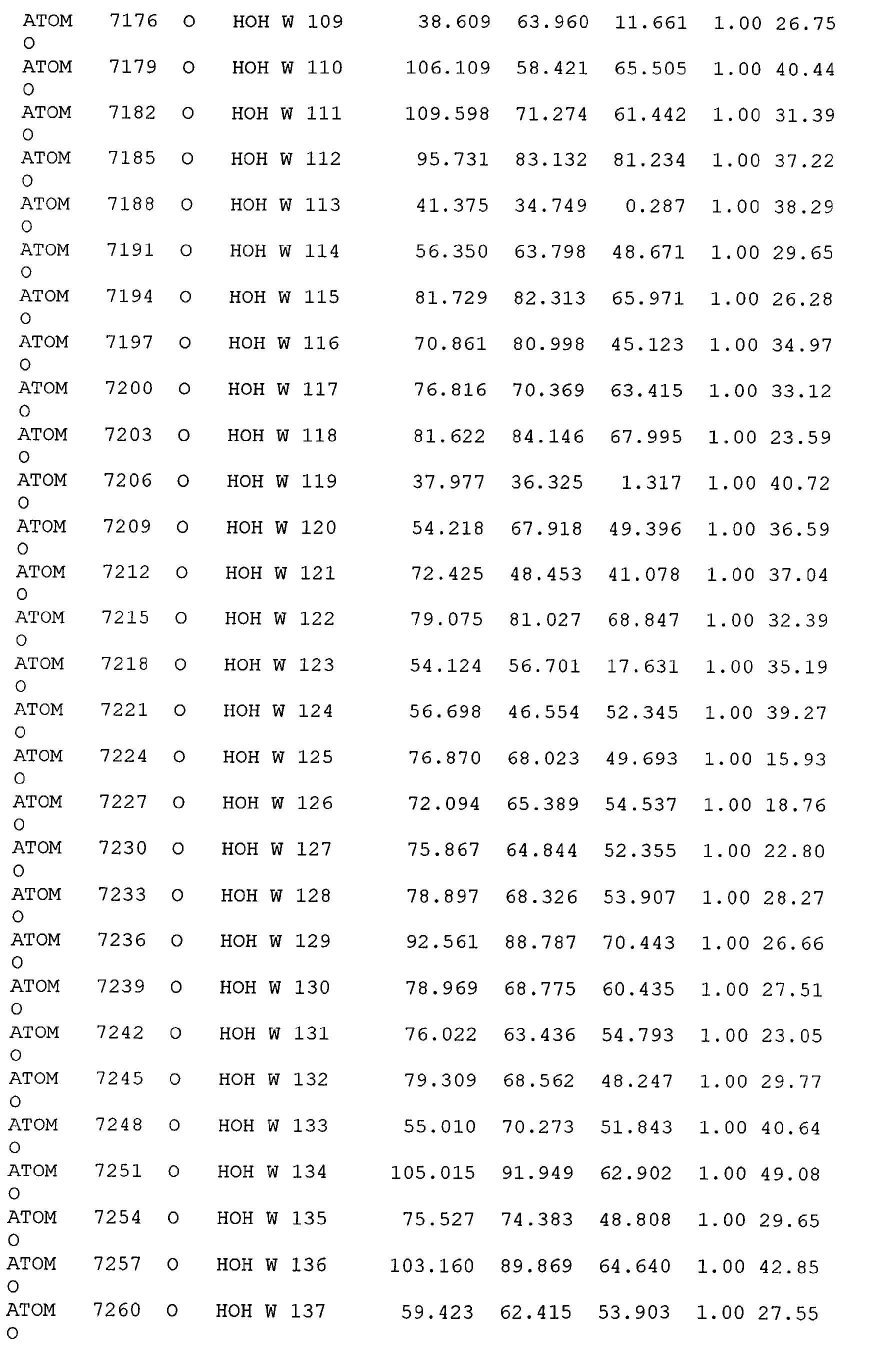

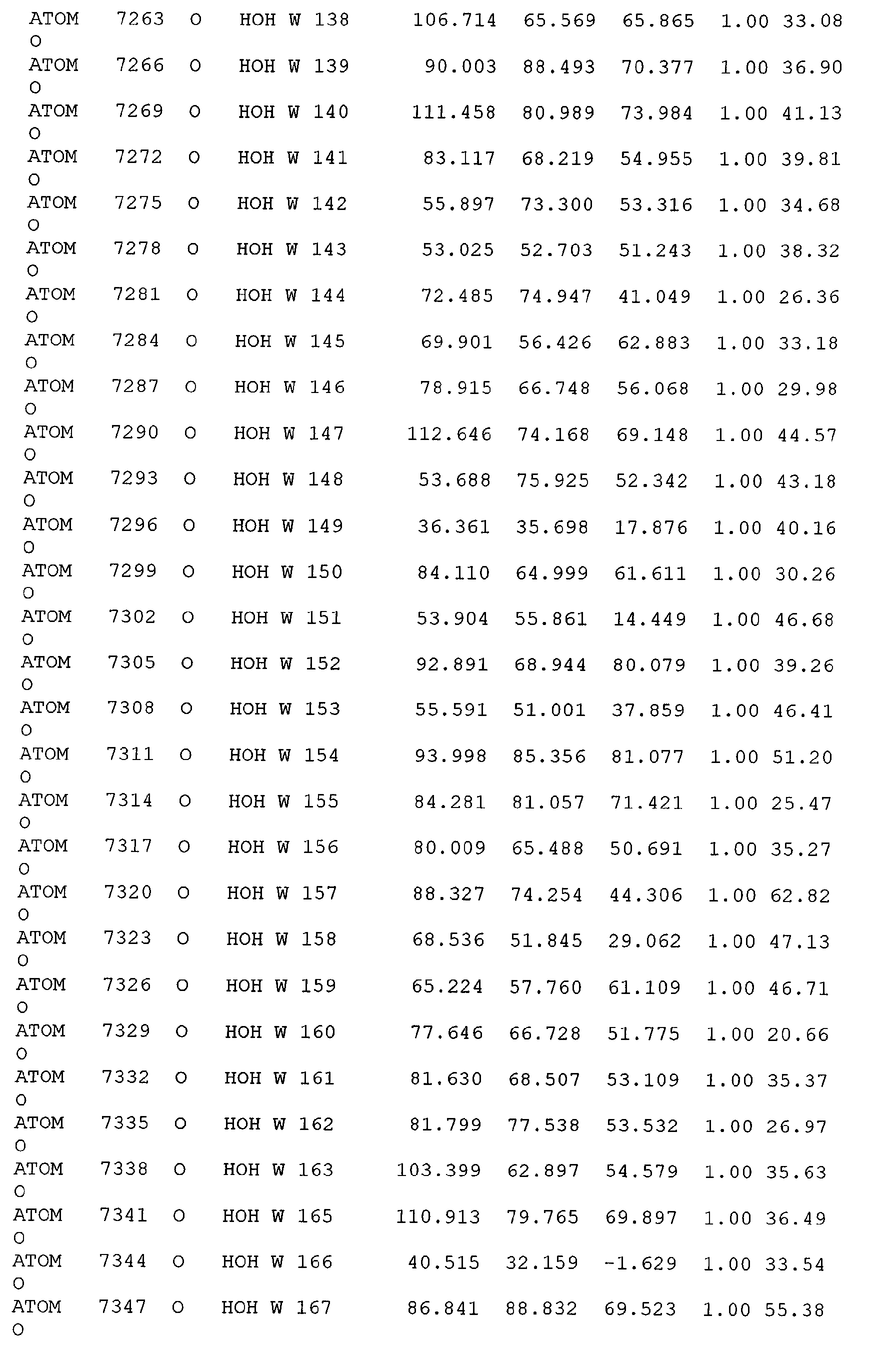

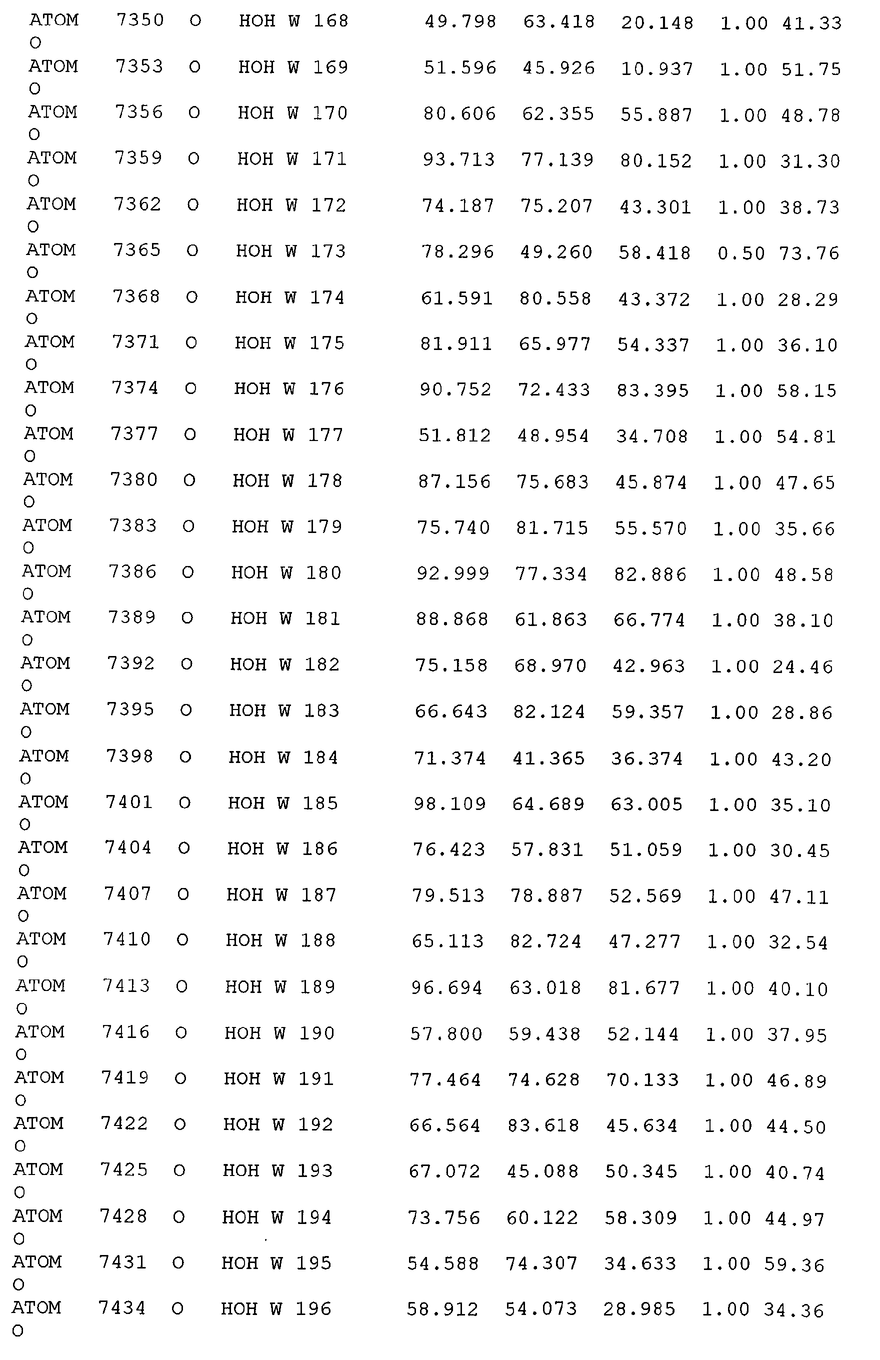

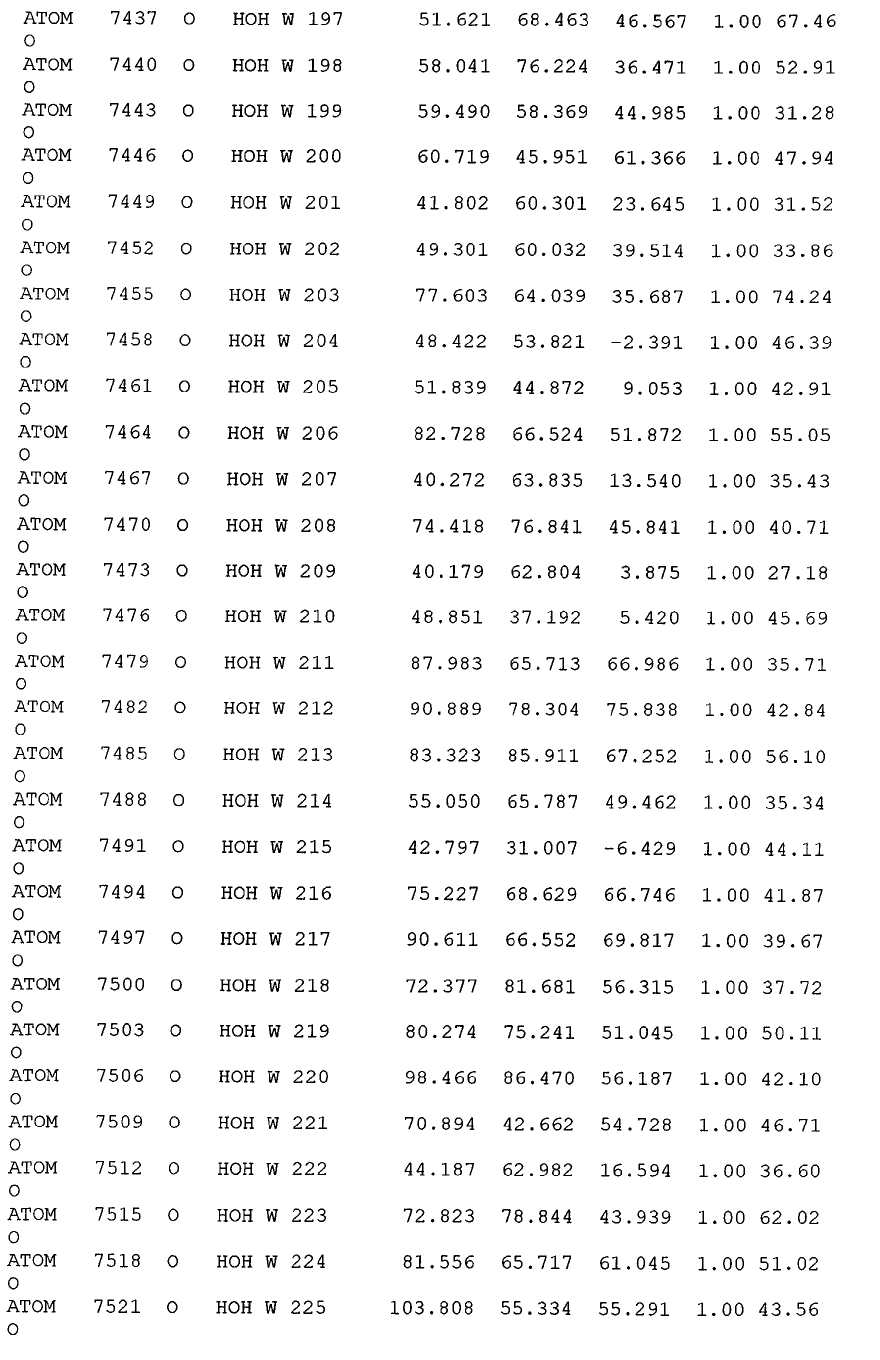

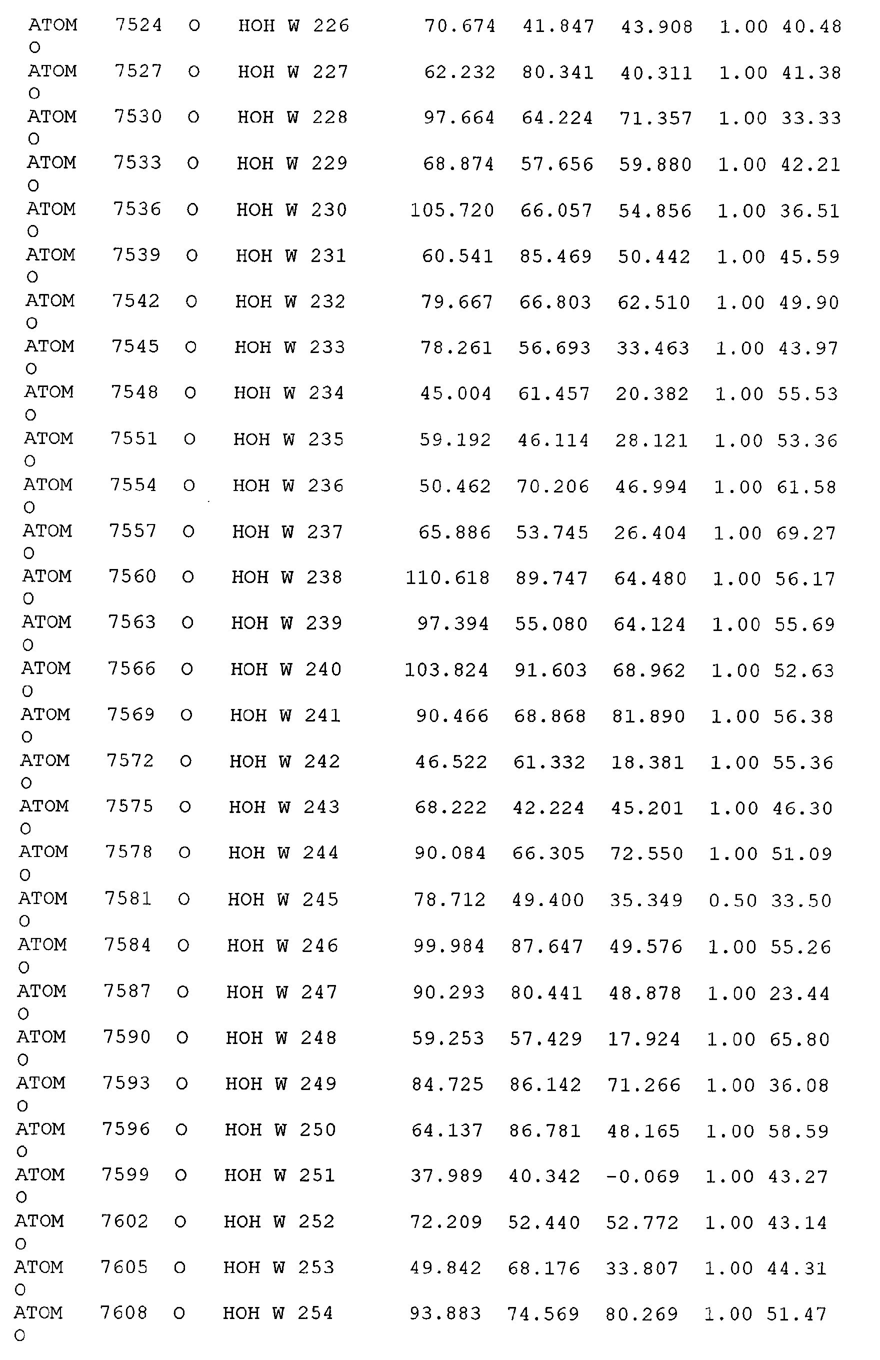

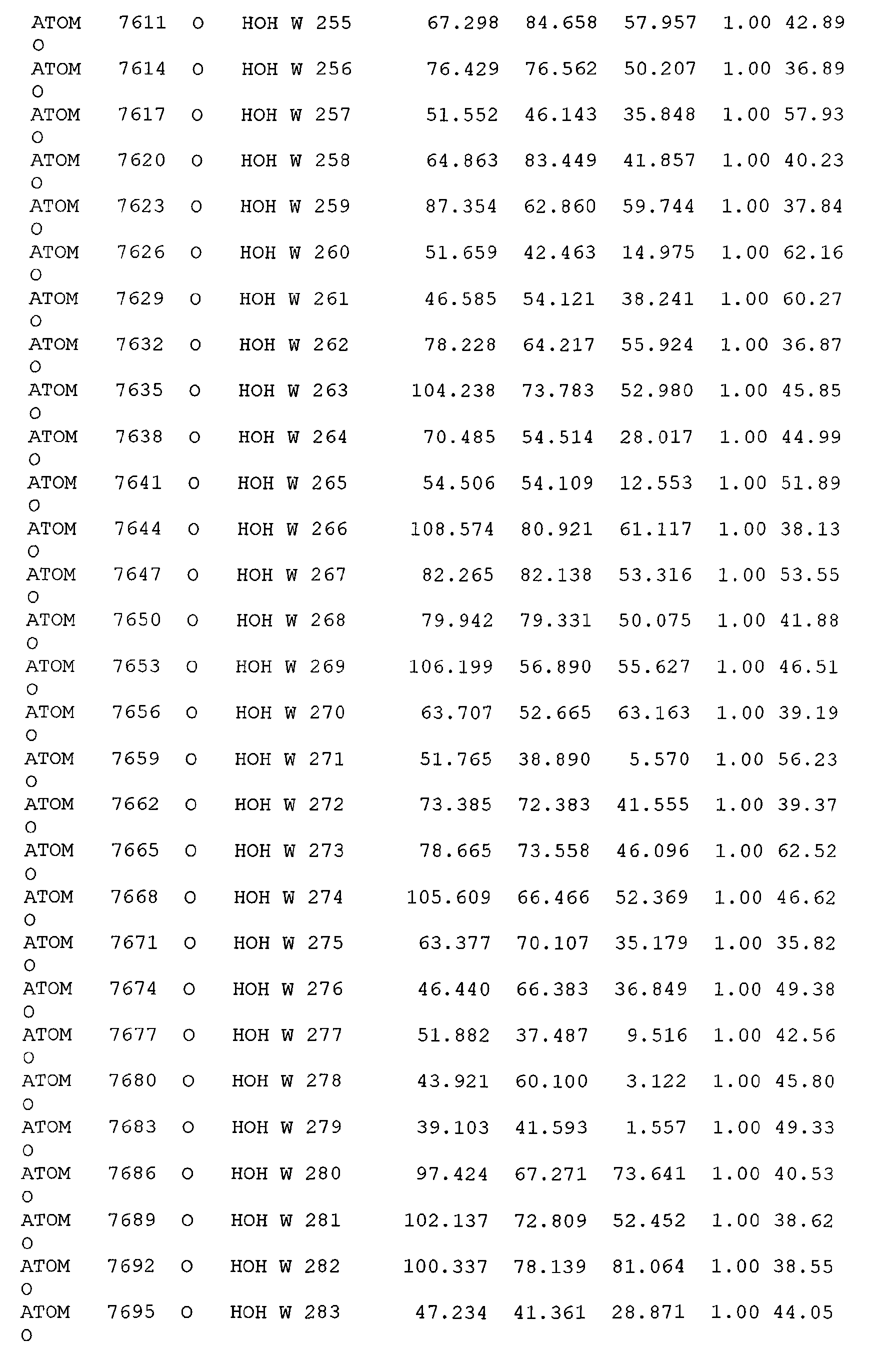

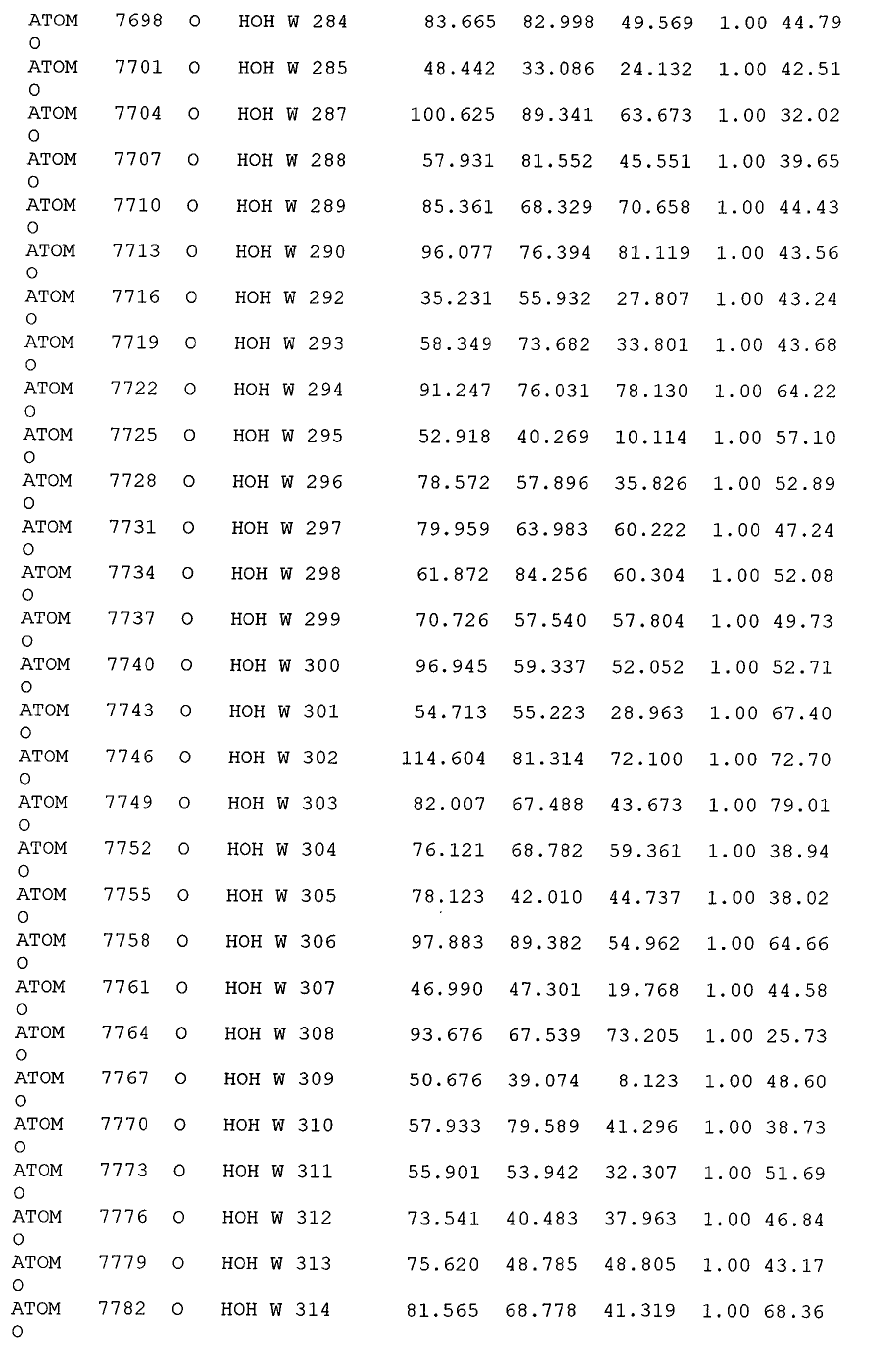

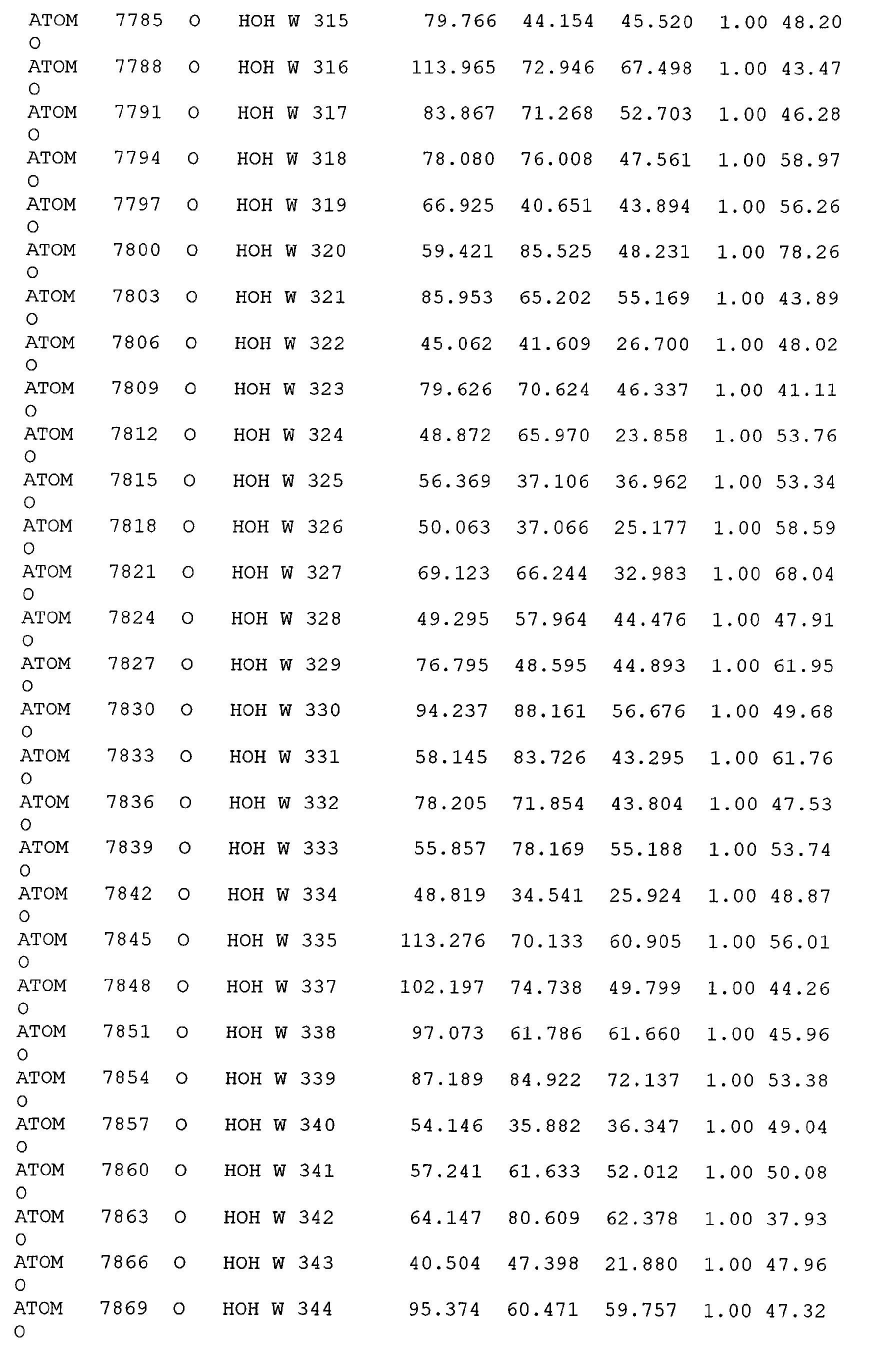

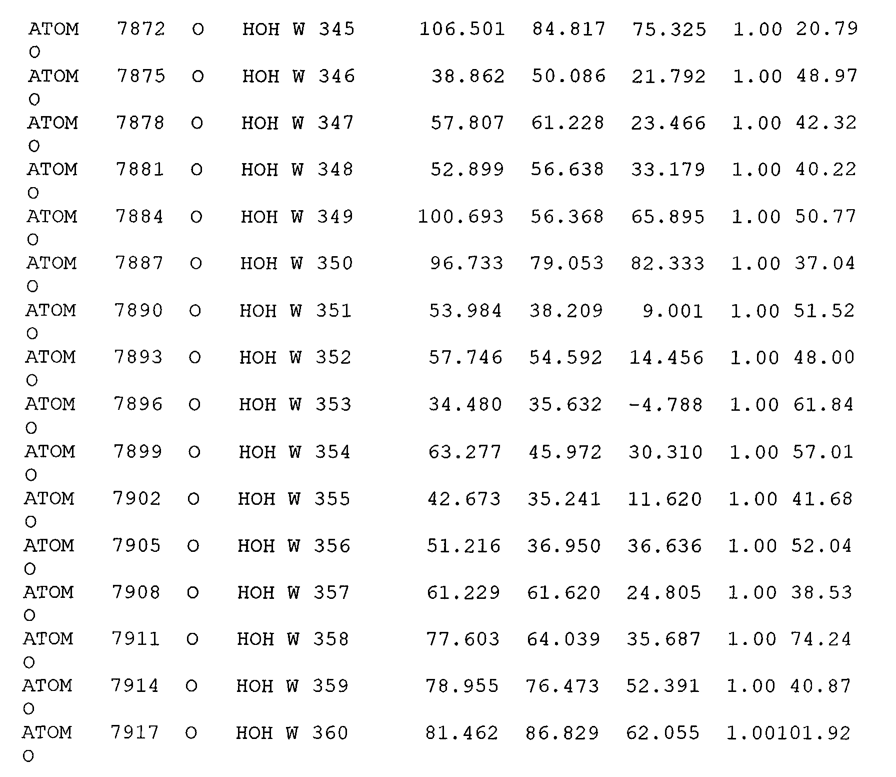

### Summary

Processes vital to life such as respiration and photosynthesis critically depend on the availability of tetrapyrroles including hemes and chlorophylls. tRNA-dependent catalysis is generally associated with protein biosynthesis. An exception is the reduction of glutamyl-tRNA to glutamate-1-semialdehyde by the enzyme glutamyl-tRNA reductase. This reaction is the indispensable initiating step of tetrapyrrole biosynthesis in plants and most prokaryotes.The crystal structure of glutamyl-tRNA reductase from the archaeon *Methanopyrus kandleri* in complex with the substrate-like inhibitor glutamycin at 1.9 Å resolution reveals an extended yet planar V-shaped dimer. The well-defined interactions of the inhibitor with the active site support a thioester-mediated reduction process. Modelling the glutamyl-tRNA onto each monomer reveals an extensive protein-tRNA interface. We furthermore propose a model whereby the large void of glutamyl-tRNA reductase is occupied by glutamate-1-semialdehyde-1,2-mutase, the subsequent enzyme of this pathway, allowing for the efficient synthesis of 5-aminolevulinic acid, the common precursor of all tetrapyrroles.

Glutamyl-tRNA reductase (GluTR) catalyses the initial step of the vital tetrapyrrole biosynthesis in plants, most bacteria and archaea. The enzyme is highly conserved between all investigated organisms. Inhibition of this enzyme totally abolishes plant and bacterial growth. The enzyme is not present in humans and animals. Therefore, inhibitors for glutamyl-tRNA reductase should have no obvious effect on their metabolism and are therefore perfect candidates for the development of novel herbicides and antibiotics. Moreover, glutamyl-tRNA reductase catalysis is one major rate-limiting step, a major target for feedback inhibition and controlled proteolysis during vitamin B₁₂ biosynthesis.

The detailed knowledge of the unique molecular architecture of *Methanopyrus kandleri* GluTR at the atomic level and further derived structures are the ideal basis for the rational design of novel inhibitors and their use as novel herbicides and antibiotics. Structure-based protein engineering allows the generation of inhibitor insensitive GluTRs for herbicide resistant plants. Moreover, structure-based protein engineering allows the construction of end-product, intermediate and proteinase insensitive GluTRs for the improvement of vitamin B₁₂ production. All further GluTR structure based strategies for the development of interacting substances and directed molecular changes of the enzymes and its biochemical and biophysical characteristics are covered by the invention.

### References

Abrahams,J.P. and Leslie,A.G.W. (1996) Methods used in the structure determination of bovine mitochondrial F1 ATPase. *Acta Crystallogr. D,* **52**, 30-42.
Al-Karadaghi,S., Kristensen,O. and Liljas,A. (2000) A decade of progress in understanding the structural basis of protein synthesis. Prog. *Blophys. Mol. Biol*., **73**, 167-193.
Allaire,M., Li,Y., MacKenzie,R.E. and Cygler, M. (1998) The 3-D structure of a folate-dependent dehydrogenase/cyclohydrolase bifunctional enzyme at 1.5 Å resolution. *Structure,* **6,** 173-182.
Brody,S.S., Gough,S.P. and Kannangara,C.G. (1999) Predicted structure and fold recognition for the glutamyl tRNA reductase family of proteins. *Proteins: Struct. Funct. Genet.* **37**, 485-493.
Brünger,A.T. (1992) Free R-value: a novel statistical quantity for assessing the accuracy of crystal structures. *Nature,* **355**, 472-475.
Brünger,A.T. *et al*. (1998) Crystallography and NMR system (CNS): A new software system for macromolecular structure determination. *Acta Crystallogr. D,* **54**, 905-921.
Carugo,O. and Argos,P. (1997) NADP-dependent enzymes. I: Conserved stereochemistry of cofactor binding. *Proteins: Struct. Genet.,* **28**, 10-28.
Collaborative Computational Project No. 4. (1994) The CCP4
Suite: Programs for Protein Crystallography. *Acta Crystallogr. D,* **50,** 760-763.
Contestabile,R., Angelaccio,S., Maytum,R., Bossa,F. and John,R.A. (2000) The contribution of a conformationally mobile, active site loop to the reaction catalyzed by glutamate semialdehyde aminomutase. *J. Biol. Chem.,* **275,** 3879-3886.
de la Fortelle,E. and Bricogne,G. (1997) Maximum-likelihood heavy-atom parameter refinement for the multiple isomorphous replacement and multiwavelenth anomalous diffraction methods. *Methods Enzymol*., **276,** 472-494.
Eiler,S., Dock-Bregeon,A., Moulinier,L., Thierry,J.C. and Moras,D. (1999) Synthesis of aspartyl-tRNA(Asp) in *Escherichia coli -* a snapshot of the second step. *EMBO J*., **18,** 6532-6541.
Hennig,M., Grimm,B., Contestabile,R., John,R.A. and Jansonius,J.N. (1997) Crystal structure of glutamate-1-semialdehyde aminomutase: An α2-dimeric vitamin B6-de-pendent enzyme with asymmetry in structure and active site reactivity. *Proc. Natl. Acad. Sci. USA.,* **94**, 4866-4871.
Holm,L. and Sander,C. (1996) Alignment of three-dimensional protein structures: network server for database searching. *Meth. Enzymol*., **266**, 653-662.
Jahn,D., Verkamp,E. and Söll,D. (1992) Glutamyl-transfer RNA: a precursor of heme and chlorophyll biosynthesis. *Trends Biochem*. *Sci*., **17**, 215-218.
Jones,T.A., Zou,J.Y., Cowan,S.W. and Kjeldgaard,M. (1991) Improved methods for building protein models in electron density maps and the location of errors in these models. *Acta Crystallogr*. *A*, **47**, 110-119.
Kikuchi, G., Kumar,A., Talmage,P. and Shemin, D. (1958) The enzymatic synthesis of δ-aminolevulinic acid. *J. Biol. Chem.*, **233**, 1214-1219.
Kraulis,P.J. (1991) MOLSCRIPT: a program to produce both detailed and schematic plots or protein structures. *J. Appl. Crystallogr*., **24**, 946-950.
Laskowski,R.A., MacArthur,M.W., Moss,D.S. and Thornton,J.M. (1993) PROCHECK: a program to check the stereochemical quality of protein structures. *J. Appl. Crystallogr*., **26**, 283-291.
Lindahl,M. *et al.* (1994) Crystal structure of the ribosomal protein S6 from *Thermus thermophilus. EMBO J.,* **13**, 1249-1254.
May,B.K, Dogra,S.C., Sadlon,T.J., Bhasker,C.R., Cox,T.C. and Bottomley,S.S. (1995) Molecular regulation of heme biosynthesis in higher vertebrates. *Prog. Nucleic Acid Res. Mol. Blol*. 51,1-51.
Merrit, E.A. and Murphy,M.E.P. (1994) Raster3D version 2.0: a program for photorealistic molecular graphics. *Acta Crystallogr. D,* **50**, 869-873.
Moser,J., Lorenz,S., Hubschwerlen,C., Rompf,A. and Jahn,D. (1999) *Methanopyrus kandleri* glutamyl-tRNA-reductase. *J. Biol. Chem.,* **274,** 30679-30685.
Murshudov,G.N., Vagin,A.A. and Dodson,E.H. (1997) Refinement of macromolecular structures by the maximum-likelihood method. *Acta Crystallogr. D,* **53**, 240-255.
Nissen, P., Kjeldgaard,M., Thirup,S., Polekhina,G., Reshetnikkova, L., Clark,B.F. and Nyborg,J. (1995) Crystal structure of the ternary complex of Phe-tRNAPhe, EF-Tu, and a GTP analog. *Science,* **270**, 1464-1472.
Nissen, P., Thirup,S., Kjeldgaard,M. and Nyborg,J. (1999) The crystal structure of Cys-tRNACys-EF-Tu-GDPNP reveals general and specific features in the ternary complex and in tRNA. *Struct. Fold. Des.,* **7,** 143-156.
Otwinowski,Z. and Minor,W. (1997) Processing of X-ray diffraction data collected in oscillation mode. *Meth. Enzymol.*, **276**, 307-326.
Rath,V.L., Silvian,L.F., Beijer,B., Sproat,B.S. and Steitz,T.A. (1998) How glutaminyl-tRNA synthetase selects glutamine. *Structure,* **6,** 439-449 (1998).
Schluenzen,F., Tocil,A., Zarivach,R., Harms, J., Gluehmann,M., Janell,D., Bashan,A., Bartels,H., Agmon,I., Franceschi,F. and Yonath,A. (2000) Structure of functionally activated small ribosomal subunit at 3.3 angstroms resolution. *Cell,* **102,** 615-623.
Schmitt,E., Panvert,M., Blanquet,S. and Mechulam,Y. Crystal structure of methionyl-tRNAfMet transformylase complexed with the initiator formyl-methionyl-tRNAfMet. *EMBO J.,* **17,** 6819-6826.
Schön,A., Kannangara,C.G., Gough,S. and Söll,D. (1988) Protein biosynthesis in organelles requires misaminoacylation of tRNA. *Nature,* **331,** 187-190.
Sekine,S., Nureki,O., Shimada,A., Vassylyev,D.G. and Yokoyama,S. (2001) Structural basis for anticodon recognition by discriminating glutamyl-tRNA synthetase. *Nat. Struct. Biol.*, **8**, 189-191.
Smith,M.A., Kannangara,C.G. & Grimm,B. (1992) Glutamate 1-semialdehyde aminotransferase: anomalous enantiomeric reaction and enzyme mechanism. *Biochemistry* **31,** 11249-11254.
Terwilliger,T.C. and Berendzen,J. (1996) Correlated phasing in multiple isomorphous replacement. *Acta Crystallogr. D,* **52**, 749-757.
Wallace,A.C., Laskowski,R.A. and Thornton,J.M. (1995) LIGPLOT: A program to generate schematic diagrams of protein-ligand interactions. *Prot. Eng.,* **8,** 127-134.
Willows,R.D., Kannangara,C.G. and Pontoppidan,B. (1995) Nucleotides of tRNA (Glu) involved in recognition by barley chloroplast glutamyl-tRNA synthetase and glutamyl-tRNA-reductase. *Biochim. Biophys. Acta,* **1263**, 228-234.
Ferreira, G. C. & Gong, J. (1995). 5-Aminolevulinate synthase and the first step of heme biosynthesis. *J. Bioenerg. Biomembr*. **27**, 151-159.
Guex, N. & Peitsch, M. C. (1997) Swiss-Model and the Swiss-PdbViewer: An environment for comparative protein modeling. *Electrophoresis* **18,** 2714-2723.
Hennig, M., Grimm, B., Contestabile, R., John, R. A. & Jansonius, J.N. (1997). Crystal structure of glutamate-1-semialdehyde aminomutase: An α2-dimeric vitamin B6-dependent enzyme with asymmetry in structure and active site reactivity. *Proc. Natl. Acad. Sci. USA*., **94**, 4866-4871.
Jahn, D., Verkamp, E. & Soll, D. (1992). Glutamyl-transfer RNA: a precursor of heme and chlorophyll biosynthesis. *Trends Blochem. Sci.,* **17**, 215-218.
Kikuchi, G., Kumar, A., Talmage, P. & Shemin, D. (1958). The enzymatic synthesis of δ-aminolevulinic acid. J. *Biol. Chem.* 233, 1214-1219.
Loida, P. J., Thompson, R. L., Walker, D. M. & CaJacob, C. A. (1999). Novel inhibitors of glutamyl-tRNA^{Glu} reductase identified through cell-based screening of the heme/chlorophyll biosynthetic pathway. *Arch. Blochem. Biophys*. **372**, 230-237.
Lorber, D. M. & Shoichet, B. K. (1998). Flexible ligand docking using conformational ensembles. *Prot. Sci.* **7**, 938-950.
Lybrand, T. P. Ligand-protein docking and rational drug design. (1995). *Curr. Opin. Struc. Biol. 5,* 224-228.
Moser, J., Lorenz, S., Hubschwerlen, C., Rompf, A. & Jahn, D. (1999). *Methanopyrus kandleri* glutamyl-tRNA-reductase. *J. Biol. Chem.,* **274**, 30679-30685.
Otyepka, M., Krystof, V., Havlicek, L., Siglerova, V., Strnad, M. & Koca, J. (2000). Docking-based development of purine-like inhibitors of cyclin-dependent kinase-2. J. *Med. Chem.* **43**, 2506-2513.
Sekine, S., Nureki, O., Shimada, A., Vassylyev, D. G. & Yokoyama, S. (2001). Structural basis for anticodon recognition by discriminating glutamyl-tRNA synthetase. *Nature Struct. Biol*., **8,** 189-191.
Rotstein, S. H. & Murcko, M. A. (1993a). GenStar - a method for de novo drug design. *J. Comput. Aided Mol. Des.* **7**, 23-43.
Rotstein, S. H. & Murcko, M. A. (1993b). GroupBuild: a fragment-based method for de novo drug design. *J. Med. Chem.* 36, 1700-1710.
Sayle, R. A. & Milner-White, E. J. (1995). RasMol: Biomolecular graphics for all. *Trends Biochem*. *Sci.* **20**, 374-376.
Stigler, R.-D., Hoffmann, B., Abagyan, R. & Schneider-Mergener, J. (1999). Soft docking an L and a D peptide to an anticholera toxin antibody using internal coordinate mechanics. *Structure* **7,** 663-670.
Taylor, J. S. & Burnett, R. M. (2000). DARWIN: A program for docking flexible molecules. *Proteins* **41,** 173-191.
Verlinde, C. L. M. J. & Hol, W. G. (1994). Structure-based drug design: progress, results and challenges. *Structure* **2,** 577-587.
Vothknecht, U. C., Kannangara, C. G. & von Wettstein, D. (1998). Barley glutamyl-tRNAGlu reductase: mutations affecting haem inhibition and enzyme activity. *Pytochemistry* **47**, 513-519.
Wang, L., Elliott, M. & Elliott, T. (1999). Conditional stability of the HemA protein (glutamyl-tRNA reductase) regulates heme biosynthesis in *Salmonella typhimurium*. *J.* Bacteriol. 181, 1211-1219.
Whittle, P. J. & Blundell, T. L. (1994). Protein structure-based drug design. Annu. Rev. Biophys. Biomol. Struct. 23, 349-375.

## Claims

1. Method for the preparation of antibiotics and herbicides comprising the steps of
(a) generating a space-filling model of glutamyl-tRNA reductase, or of functional domains thereof, and of candidate compounds,
(b) docking said candidate compounds to the surface of said space-filling model of said glutamyl-tRNA reductase, or of functional domains thereof, and estimating the fitting accuracy on the basis of the three-dimensional structure and functional surface amino acid residues information mediated by said space-filling model of said glutamyl-tRNA reductase, and
(c) selecting and optionally, chemically synthesizing candidate compounds which fit to said surface of said space-filling model of said glutamyl-tRNA reductase, and
(d) optionally, qualitatively and quantitatively detecting the potential inhibiting activity of said candidate compounds for glutamyl-tRNA reductase obtained in step (c) in a biological assay.

2. Method according to claim 1 wherein in step (b) as said functional domain of said glutamyl-tRNA reductase one ore more domain selected from the group consisting of the catalytic or active site domain, the glutamyl-tRNA substrate binding domain, the NADPH binding domain, the glutamate-1-semialdehyde-2,1-aminomutase docking domain and the glutamyl-tRNA reductase dimerization domain is used.

3. Compounds having glutamyl-tRNA reductase inhibiting activity obtainable by conducting a method according to anyone of claims 1 or 2, or a salt, solvate, hydrate or formulation thereof, wherein the salt, solvate, hydrate or formulation thereof is acceptable for antibiotics or herbicides.

4. Pro-drugs, which are composed of a compound according to claim 3 and at least one protective group which is acceptable for antibiotics or herbicides and will be cleaved off under physiological conditions.

5. Compositions containing a least one compound according to claim 3 as the active agent or at least one pro-drug according to claim 4 and optionally carriers and/or diluents and/or adjuvants.

6. Use of a compound, a pro-drug or a composition according to anyone of claims 3 to 5 for the manufacture of antibiotics or herbicides.
